# EUROPEAN PATENT SPECIFICATION

(11) **EP 3 981 438 B1**
(45) Date of publication and mention of the grant of the patent: **28.10.2026**
(21) Application number: 21197262.5
(22) Date of filing: 04.05.2016
(51) Int. Cl.: A61K 48/00, C12N 15/00, C12Q 1/70, C07H 21/04

(54) **ONCOLYTIC HSV1 VECTOR AND METHODS OF USE**
ONKOLYTISCHER HSV1-VEKTOR UND VERFAHREN ZUR VERWENDUNG
VECTEUR HSV1 ONCOLYTIQUE ET PROCÉDÉS D'UTILISATION

(30) Priority: 04.05.2015 US 201562156447 P
(43) Date of publication of application: 13.04.2022
(62) Document number(s) of the earlier application(s) in accordance with Art. 76 EPC: 16789977.2 / 3 291 841
(73) Proprietor: The Brigham and Women's Hospital, Inc., Boston, MA 02115 (US); Nakashima, Hiroshi, Brookline, Massachusetts 02446 (US)
(72) Inventor: NAKASHIMA, Hiroshi, Sharon, 0267 (US); CHIOCCA, Ennio Antonio, Weston, 02493 (US)
(74) Representative: Barker Brettell LLP

(56) References cited:
- WO-A1-2014/078529
- US-A1- 2010 272 686
- US-A1- 2011 177 032
- HIROKAZU KAMBARA ET AL: "An Oncolytic HSV-1 Mutant Expressing ICP34.5 under Control of a Nestin Promoter Increases Survival of Animals even when Symptomatic from a Brain Tumor", CANCER RESEARCH, AMERICAN ASSOCIATION FOR CANCER RESEARCH, US, vol. 65, no. 7, 1 April 2005 (2005-04-01), pages 2832 - 2839, XP008162812, ISSN: 0008-5472, DOI: 10.1158/0008-5472.CAN-04-3227
- KAZUE KASAI ET AL: "Toxicology and Biodistribution Studies for MGH2.1, an Oncolytic Virus that Expresses Two Prodrug-activating Genes, in Combination with Prodrugs", MOLECULAR THERAPY-NUCLEIC ACIDS, vol. 2, 1 January 2013 (2013-01-01), pages e113, XP055518225, ISSN: 2162-2531, DOI: 10.1038/mtna.2013.38
- HIROKAZU KAMBARA ET AL: "Cyclophosphamide Allows for In vivo Dose Reduction of a Potent Oncolytic Virus", CANCER RESEARCH, vol. 65, no. 24, 15 December 2005 (2005-12-15), US, pages 11255 - 11258, XP055517801, ISSN: 0008-5472, DOI: 10.1158/0008-5472.CAN-05-2278

## Description

### FIELD OF INVENTION

The present invention is directed to compositions comprising a tumor-selective oncolytic herpes simplex viral vector, and to said compositions for use in selectively killing intracranial tumor cells in a subject.

### BACKGROUND

Many malignant tumors are intrinsically resistant to conventional therapies and represent significant therapeutic challenges, e.g. malignant gliomas and recurrent systemic solid tumors such as lung cancer. Malignant gliomas are the most abundant primary brain tumors, having an annual incidence of 6.4 cases per 100,000. These neurologically devastating tumors are the most common subtype of primary brain tumors and are one of the deadliest human cancers. In the most aggressive cancer manifestation, glioblastoma multiforme (GBM), median survival duration for patients is 14 months, despite maximum treatment efforts. Treatments for the most malignant types of brain tumors (malignant glioma /glioblastoma multiforme, GBM) fail to provide long-lasting control with 50% of afflicted patients dying within 15 months from diagnosis, in spite of a variety of treatments such as surgery, radiation and chemotherapy. A variety of experimental treatments have been tried and tested. Because few good treatment options are available for many of these refractory tumors, the exploration of novel and innovative therapeutic approaches is important.

One area of experimental therapy has involved the use of oncolytic (tumor-killing) viruses, whose infection and replication into tumor cells has been engineered to be tumor-selective. Replication selective oncolytic viruses have shown great promise as anti-tumor agents for solid tumors. The viruses have been constructed genetically so that they are able to preferentially replicate within tumor cells, while being at least somewhat restricted in their ability to replicate in normal cells. The principal anti-tumor mechanism of oncolytic viruses is through a direct cytopathic effect as they propagate and spread from initially infected tumor cells to surrounding tumor cells, achieving a larger volume of distribution and anticancer effects.

Oncolytic herpes simplex viruses (HSVs) were initially designed and constructed for the treatment of brain tumors. Subsequently, they have been found to be effective in a variety of other human solid tumors, including breast, prostate, lung, ovarian, colon and liver cancers. The safety of oncolytic HSVs has also been extensively tested in mice and primates, which are extremely sensitive to HSV. One oncolytic HSV-1 mutant that has been studied as a glioma-specific therapeutic is rQNestin34.5 (Kambara et al. An oncolytic HSV-1 mutant expression ICP34.5 under control of a Nestin promoter increases survival of animals even when symptomatic from a brain tumor, (2005) Cancer Res. 65(7): 2832-2839).

Despite encouraging preclinical studies, results from early clinical trials have suggested that most of the current versions of oncolytic viruses, although acceptably safe, may only have limited anti-tumor activity on their own.

Considering the limited effective treatment options available for certain types of cancer, including certain types of brain cancer, there remains a need in the art for improved oncolytic viruses.

International patent application WO2014/078529A1 discloses oncolytic viruses capable of killing target cells, such as glioblastoma cells, and related compositions and methods. US patent application US2010/272686A1 discloses oncolytic viruses that can specifically replicate in cancer cells leading to their destruction and at the same time secrete robust amounts of a therapeutic polypeptide, and related compositions and methods.

The article by Kambara et al., "An Oncolytic HSV-1 Mutant Expressing ICP34.5 under Control of a Nestin Promoter Increases Survival of Animals even when Symptomatic from a Brain Tumor", Cancer Res (2005) 65 (7): 2832-2839, discloses glioma-selective HSV-1 mutant "rQNestin34.5". The article by Kambara et al., "Cyclophosphamide Allows for In vivo Dose Reduction of a Potent Oncolytic Virus", Cancer Res (2005) 65 (24): 11255-11258, discloses that cyclophosphamide enhances animal survival and oncolytic virus tumor distribution, and reduces tumor volumes, with rQNestin34.5 doses of 3 × 10³ and 3 × 10² pfu.

### SUMMARY OF THE INVENTION

The invention is set out in the appended set of claims.
We have generated an improved oncolytic rQNestin34.5 HSV virus, a second generation virus referred to as rQNestin34.5.v2. The genetically modified HSV1 rQNestin34.5.v2 has been made glioma-selective by the following maneuvers: 1) one of the viral genes that encodes for a viral protein (ICP6) has been removed. Without this gene, HSV1 has to utilize factors within the infected cells to efficiently grow and replicate and we have shown that such factors are existent in cells that are mitotically active or that have a defect in a cellular gene (p16) that is missing in most gliomas; and 2) the two copies of the viral gene that encode for a protein (ICP34.5) needed for robust viral growth in an infected cell have also been removed and one copy was reinserted under control of a Nestin promoter that is also present selectively in gliomas in the adult human brain. These two maneuvers combined with the reduced toxicity due to lack of ICP6-EGFP fusion protein present in the first generation rQNestin34.5 (Kambara et al. An oncolytic HSV-1 mutant expression ICP34.5 under control of a Nestin promoter increases survival of animals even when symptomatic from a brain tumor, (2005) Cancer Res. 65(7): 2832-2839), allow this new HSV1 (designated as rQNestin34.5v.2) to be relatively selective in destroying gliomas and not normal brain cells. We have confirmed this in cultured cells and in animal models.

Accordingly, provided herein are tumor-selective oncolytic herpes simplex viral vectors comprising: a) a deletion or inactivating mutation in both copies of the gene encoding y34.5; b) an insertion of at least one copy of the HSV y34.5 gene under the transcriptional control of a nestin promoter; and c) a deletion or inactivating mutation in the gene that encodes for the HSV viral protein ICP6, wherein the tumor-selective oncolytic herpes simplex viral vector does not express green fluorescent protein. The vector does not express ICP6-EGFP fusion protein present in the first generation HSV-1 vector rQNestin34.5. The tumor-selective oncolytic herpes viral vector does not comprise SEQ ID NO: 7. In one embodiment, the vector does not contain UL39 nucleic acid regulatory sequences (promoter and enhancer elements). In one embodiment, the vector does not contain a fusion protein of ICP6. The at least one copy of the y34.5 gene under the transcriptional control of a nestin promoter may be inserted into UL39 gene that encodes for the large subunit of ribonucleotide reductase ICP6 (infected cell protein 6). In one embodiment, the nestin promoter comprises SEQ ID NO: 2 or a degenerate variant thereof. In one embodiment the tumor-selective oncolytic herpes simplex viral vector comprises SEQ ID NO: 8.

Also provided is a tumor-selective oncolytic herpes simplex viral vector that comprises the sequence of SEQ ID NO: 1, or a degenerate variant thereof.

Also disclosed, but not part of the claimed invention, is a method of selectively killing intracranial tumor cells in a subject, comprising introducing into the vicinity of the tumor-selective oncolytic herpes simplex viral vectors of the invention. We have determined that one additional maneuver allows rQNestin34.5v.2 to be highly effective in injected gliomas. Mammals are able to rapidly counteract HSV1 in the brain through their initial immune responses. Brain cells such as microglia and systemic cells such as NK cells and macrophages can blunt and effectively ensure that viral replication and its untoward consequences on normal brain does not occur. We have discovered that such innate initial host defenses also exist in the context of gliomas injected with rQNestin34.5v.2 that curtail the biodistribution of the virus within the tumor and limit its therapeutic effectiveness. In animal models, we have discovered that a single dose of cyclophosphamide, a commonly utilized agent that modulates the immune response, administered two days before rQNestin34.5v.2 significantly enhances viral biodistribution, replication and efficacy, effectively reducing the dose of virus required to produce a survival effect by two orders of magnitude.

Accordingly, the method may further comprise administration of cyclophoshamide (CPA). The CPA may be administered simultaneously with the HSV oncolytic vector. Alternatively, the CPA is administered hours, days, or weeks before the administration of the HSV-1 oncolytic vector. For example, the CPA may be administered two days before the oncolytic herpes simplex viral vector.

The tumor cells may comprise a glioblastoma cell, or a cancer stem cell. The subject to be treated may be a mammal. The mammal may be a human.

Also disclosed are pharmaceutical compositions of the tumor-selective oncolytic herpes simplex viral vectors described herein for use in selectively killing intracranial tumor cells in a subject. The tumor-selective oncolytic herpes simplex viral vectors comprise: a) a deletion or inactivating mutation in both copies of the gene encoding y34.5; b) an insertion of at least one copy of the HSV y34.5 gene under the transcriptional control of a Nestin promoter; and c) a deletion or inactivating mutation in the gene that encodes for the HSV viral protein ICP6, wherein the tumor-selective oncolytic herpes simplex viral vector does not express green fluorescent protein. The vector does not express ICP6-EGFP fusion protein present in the first generation HSV-1 vector rQNestin34.5. The tumor-selective oncolytic herpes viral vector does not comprise SEQ ID NO: 7. In one embodiment, the vector does not contain UL39 nucleic acid regulatory sequences (promoter and enhancer elements). In one embodiment, the vector does not contain a fusion protein of ICP6. The at least one copy of the y34.5 gene under the transcriptional control of a nestin promoter may be inserted into UL39 gene that encodes for the large subunit of ribonucleotide reductase ICP6 (infected cell protein 6). In one embodiment, the nestin promoter comprises SEQ ID NO: 2 or a degenerate variant thereof. In one embodiment the tumor-selective oncolytic herpes simplex viral vector comprises SEQ ID NO: 8.

### BRIEF DESCRIPTION OF THE DRAWINGS

Exemplary embodiments are illustrated in the referenced figures. It is intended that the embodiments and figures disclosed herein are to be considered illustrative rather than restrictive.

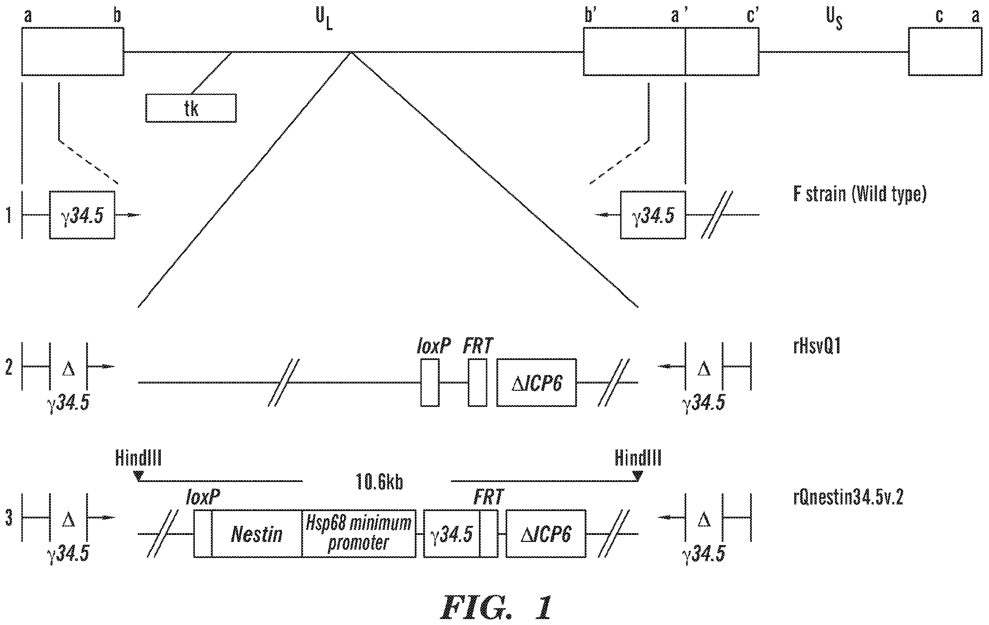

Figure 1 is a schematic of rQNestin34v.2. Schematic maps of HSV strain F (wild-type), rHsvQ1 (double UL39-y34.5 mutant), and rQNestin34.5. All strains contain the typical HSV-1 genome with its two unique segments (UL and US, respectively), each flanked by inverted repeat elements (ab and ca, respectively). The locations of diploid y34.5 genes and of the thymidine kinase gene (tk ) are shown in the top construct, representing wild-type F strain HSV. In the middle construct, UL39 (ICP6) has been deleted of its coding sequences and the deletions within γ1 34.5 are shown for rHsvQ1. These consist of a deletion of about 1,000 bp in the coding region. The bottom construct shows the site of recombination of the hybrid promoter (nestin enhancer and hsp68 minimum promoter)-γ1 34.5 expression cassette into the ICP6 loss, giving rise to the novel mutant oncolytic virus rQNestin34.5v.2. The approximate sizes of the HindIII fragment from rQNestin34.5 are provided (from Kambara et al., Cancer Res., 20051). rQNestin34.5v.2 was sequenced in its entirety to confirm its identity (SEQ ID NO: 1).

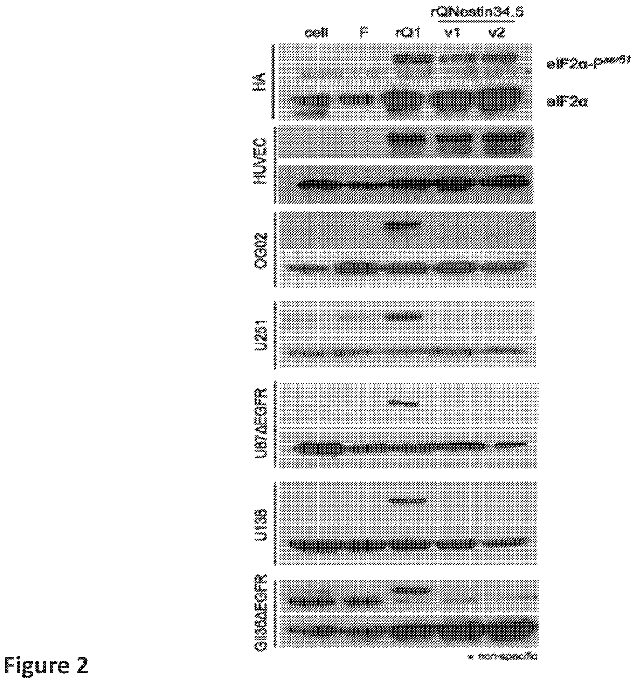

Figure 2 are western blots of several glioma cells (U87dEGFR, U251, U138 and Gli36dEGFR) and freshly explanted glioma "stem-like" cells (GBM 02) and normal human astrocytes (HA) andendothelial cells (HUVEC) were infected with rQNestin 34.5 v.2 or parental rQNestin34.5 (v.1), control rQ1 virus, wild-type F strain or uninfected (cell). Lysates were then harvested and Western blots carried out to visualize total eiF2a and phospho-eiF2a (eiF2a-Pser51). X12 (another GSC) is not included but showed same results as OG02.

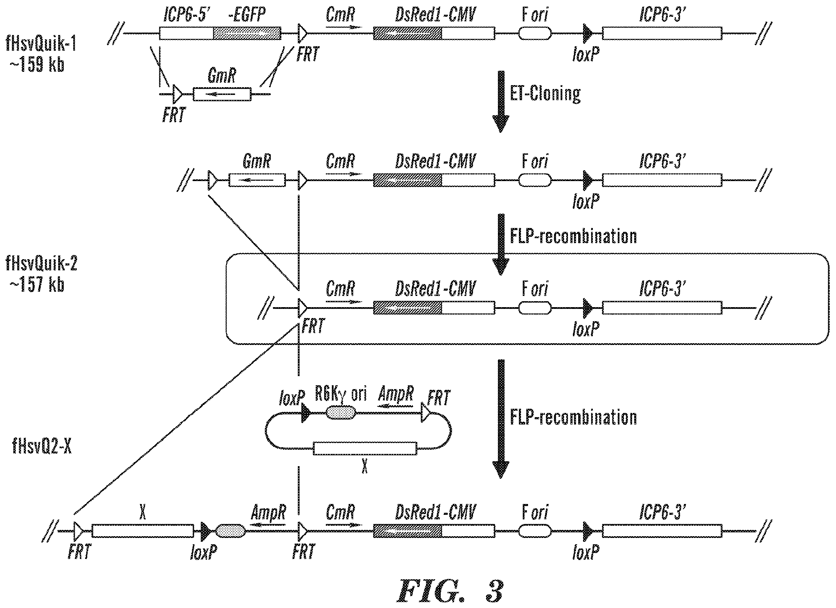

Figure 3 is a schematic of the construction of the fHSVQ2-X series of bacterial artificial chromosomes (BAC) that contain the entire HSV1 genome with endogenous HSV1 ICP34.5 and ICP6 deletions and insertion of a large sequence utilized for transfer of exogenous sequences, such as the Nestin/hsp68 promoter/enhancer sequence (marked by X).

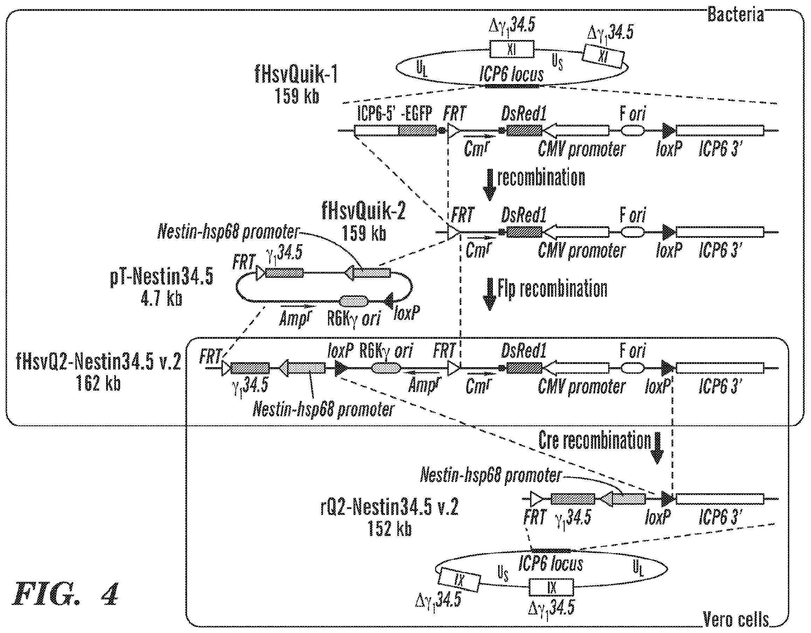

Figure 4 is a schematic of the engineering of rQNestin34.5 v.2 (rQNestin34.5 lacking ICP6GFP). A bacterial artificial chromosome (BAC) containing the HSV1 genome lacking both copies of the HSV1 gene γ134.5, encoding for the viral protein ICP34.5 and with a large deletion of the viral gene for ICP6 (only some 3' sequence remains) was obtained (designated as fHSVQuick-2). A plasmid with the Nestin-promoter enhancer element upstream of a γ1 34.5 transgene (pT-Nestin34.5) is then recombined by site-specific recombination into the FRT locus by employing FLP. The BAC concatenate (fHSVQ2-Nestin34.5 v.2) is then purified from bacteria and electroporated in mammalian Vero cells in the presence of Cre recombinase to remove internal antibiotic resistance sites, fluorescent proteins sites, such as DSRed1, FRT site and F plasmid origin of replication. The viral genome will replicate in Vero cells, generating rQNestin34.5 v.2 virions. (see for example Terada et al. Development of a rapid method to generate multiple oncolytic HSV vectors and their in vivo evaluation using synergenic mouse tumor models. Gene Ther. 13: 705-714, 2006).

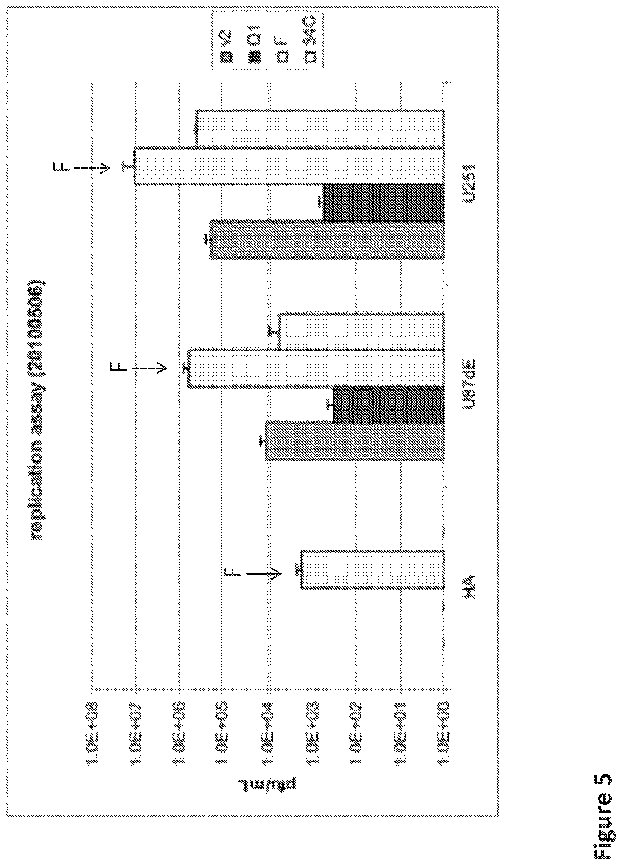

Figure 5 is a graph depicting viral yields in human glioma cells and normal human astrocytes. Normal human astrocytes (HA) and human glioma cells, U87dEGFR (U87dE) and U251, were cultured in the presence of rQNestin34.5v.2 (v2), parental rHSVQ1 (Q1), wild-type F strain (F), and also another oncolytic viral construct unrelated to this project (34C). Viral yields were then assayed 3 days later.

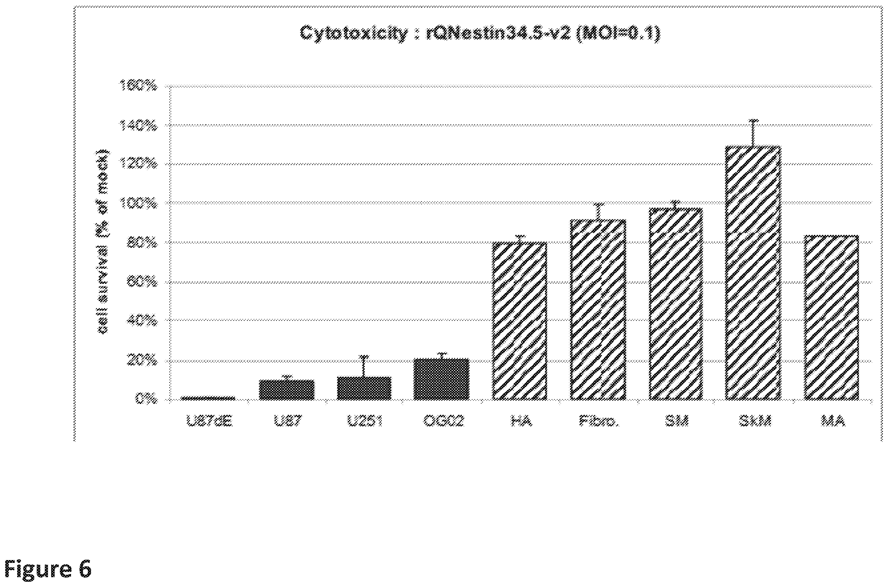

Figure 6 is a graph depicting cell survival in various cells. rQNestin34.5v.2 was added to a panel of glioma cells, U87dEGFR (U87dEGFR), U87, U251 and OG02 and to a panel of normal cells, human astrocytes (HA), human fibroblasts (Fibro.), human smooth muscle (SM), human skeletal muscle cells (SkM) and mouse astrocytes (MA). Surviving cells were then counted 5 days later by Coulter counter enumeration

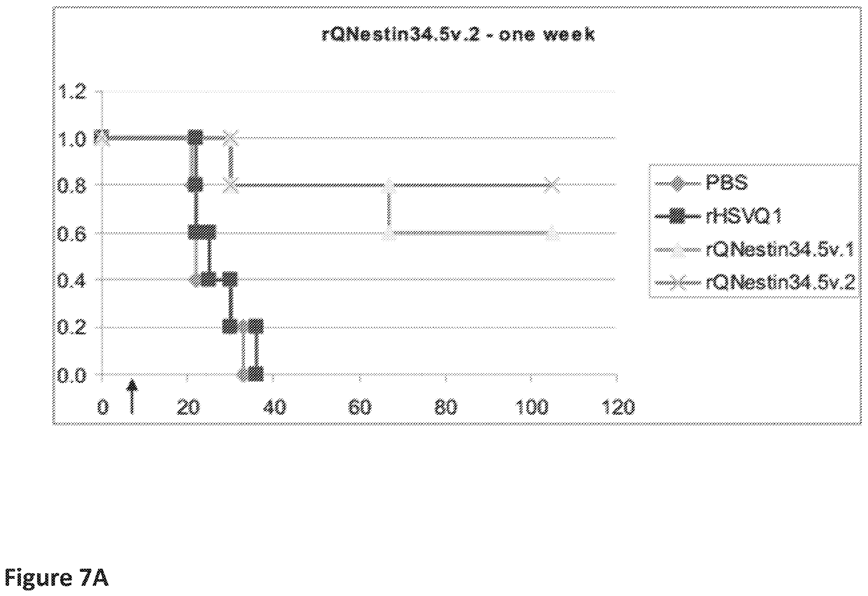

Figures 7A is a graph showing mouse survival (y) over time (x). U87dEGFR glioma cells (2 x 10⁵) were implanted in the brain of 8 week old athymic mice. One week later, PBS, rHSVQ1, rQNestin34.5v.1 and rQNestin34.5v.2 (3 x 10⁵ pfu in 5 ul) were injected into the same location. Survival was then monitored.

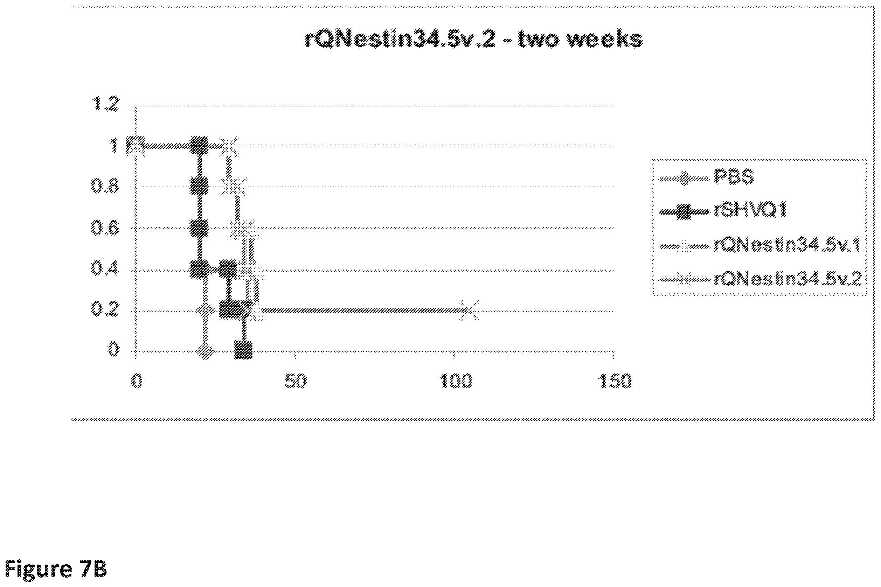

Figure 7B is a graph showing mouse survival rate (y) over time (x). U87dEGFR glioma cells (2 x 10⁵) were implanted in the brain of 8 week old athymic mice. Two weeks later, PBS, rHSVQ1, rQNestin34.5v.1 and rQNestin34.5v.2 (3 x 10⁵ pfu in 5 ul) were injected into the same location. Survival was then monitored.

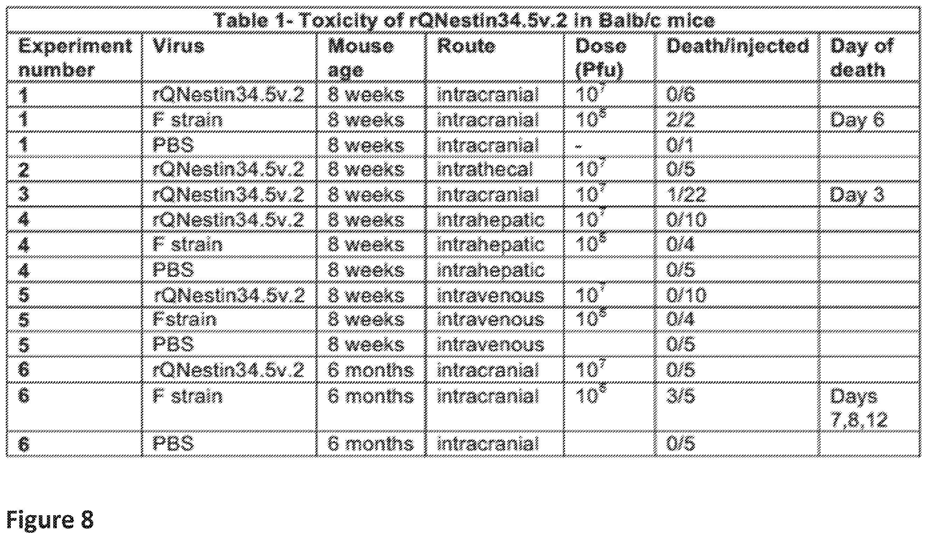

Figure 8 is a table that summarizes the results of all experiments to date with Balb/c mice. Intracerebral injection of 107 pfus in either 8 week old or 6 month old mice was well tolerated (32/33, with one death at day 3 post-injection for unknown reasons)

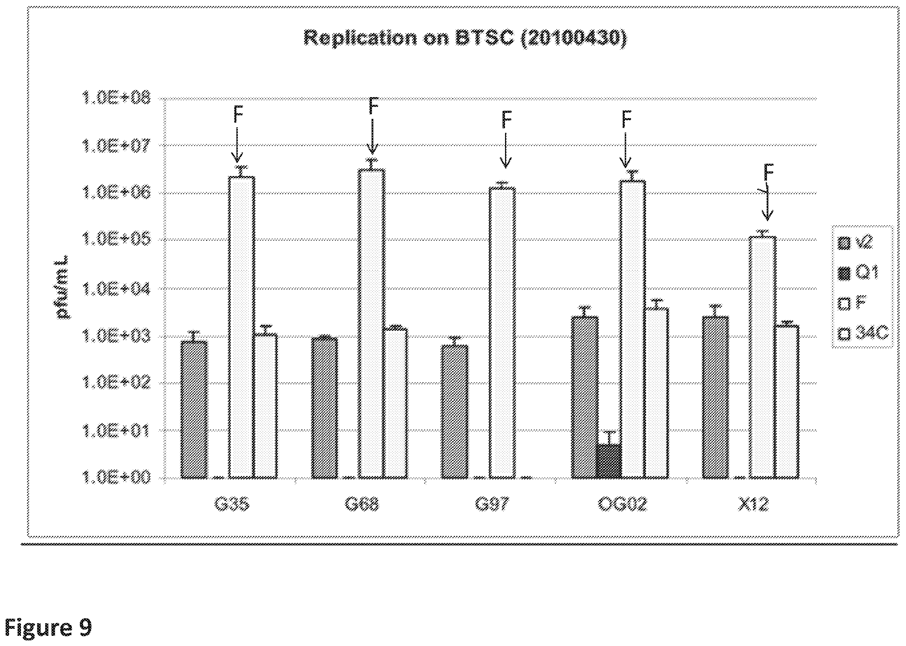

Figure 9 is a graph showing replication of rQNestin34.5v.2 (v2) which was analyzed in 5 glioma stem cells (brain tumor initiating cells - BTIC) freshly generated from humans (G35, G68, G97, OG02, X12). HSVQ1 (Q1) an ICP34.5 deleted virus showed no or minimal replication. F (wild-type HSV) showed the most replication. 34C is an unrelated HSV recombinant HSV.

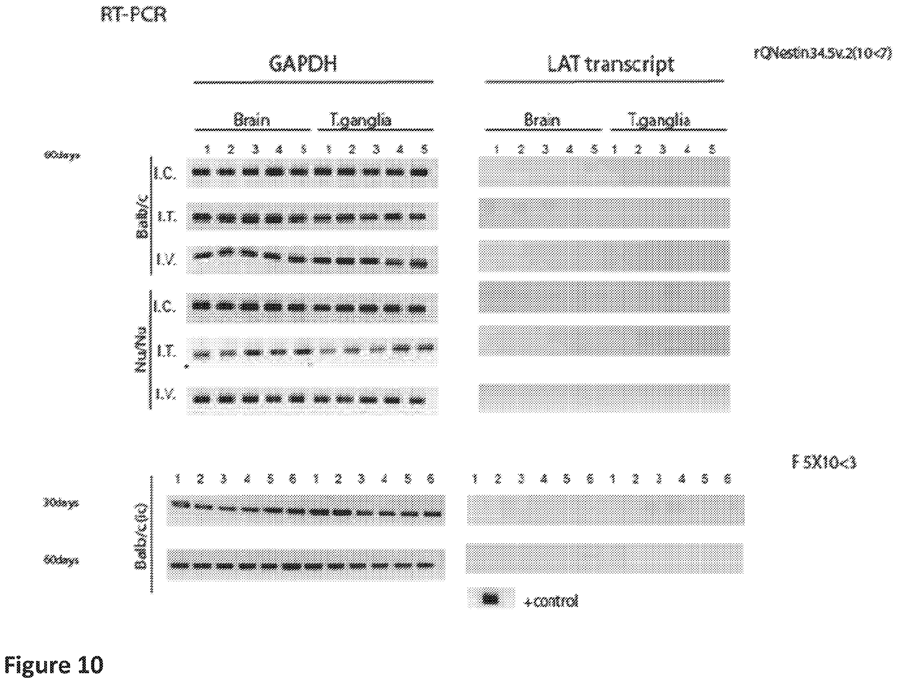

Figure 10 shows RT-PCR for LAT transcript in mice treated with rQNestin34.5v.2 or F. The former was injected ic., i.t. or i.v,. at a dose of 10⁷ pfus. 60 days later, RT-PCR in 6 different brains and 6 different trigeminal ganglia was performed for the LAT transcript (35 cycles). The positive control for the reaction consisted of glioma cells infected with rQNestin34.5v.2. F strain virus was injected i.c. and mice were sacrificed 30 or 60 days later to detect LAT.

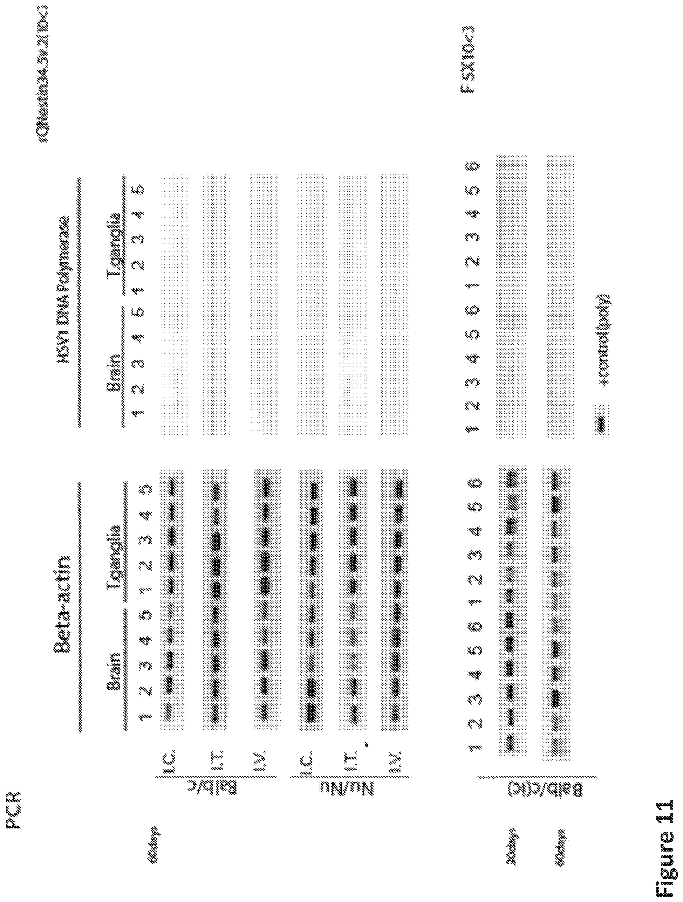

Figure 11 shows PCR for DNA pol transcript in mice treated with rQNestin34.5v.2 or F. The former was injected ic., i.t. or i.v,. at a dose of 10⁷ pfus. 60 days later, PCR in different brains and 5 different trigeminal ganglia was performed for the LAT transcript (35 cycles). The positive control for the reaction consisted of glioma cells infected with rQNestin34.5v.2. F strain virus was injected i.c. and mice were sacrificed 30 or 60 days later to detect DNA pol.

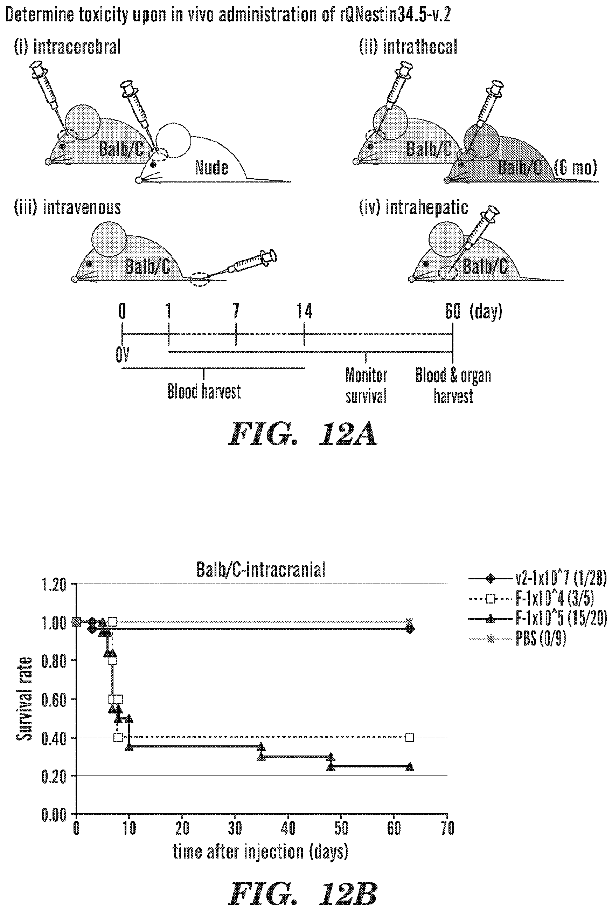

Figures 12A to 12B show a schematic and graph. Figure 12A schematic of the toxicity studies in mice. Figure 12B a graph showing survival rate vs time after injection of rQNestin34.5v.2 (v2-1x10⁷) and wild type HSV1 F strain.

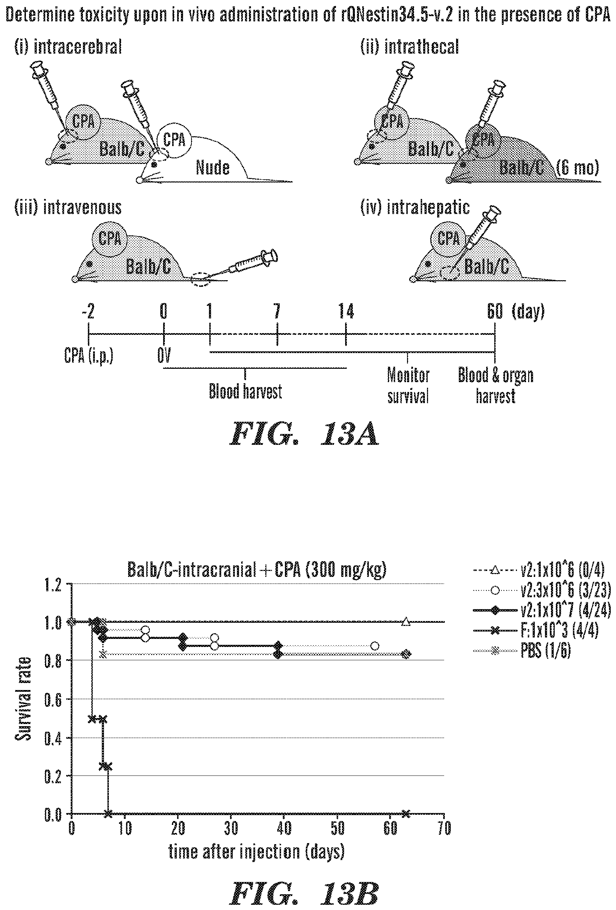

Figures 13A and 13B show a schematic and graph. Figure 13A schematic of the preclinical studies in mice using CPA. Figure 13B a graph showing survival rate vs time after injection of rQNestin34.5v.2 (v2-1x107) and wild type HSV1 F strain. CPA (300 mg/kg) was administered i.p., two days before intracranial inoculation of rQNestin34.5v2 (v2) at 10⁶ pfus (blue triangle with broken line), 3 x 10⁶ pfus (light blue circle/line), or 10⁷ pfus (blue diamond/line). The numerator represents animal euthanized (due to veterinary criteria of toxicity) or found dead, while the denominator represents total number injected. F represents wild-type HSV1 F strain, injected at 10³ pfus (red cross/line). PBS represents intracranial vehicle injection. The experiment was terminated at day 62-63 with planned euthanasia of all surviving animals to harvest organs for toxicity/biodistribution analyses.

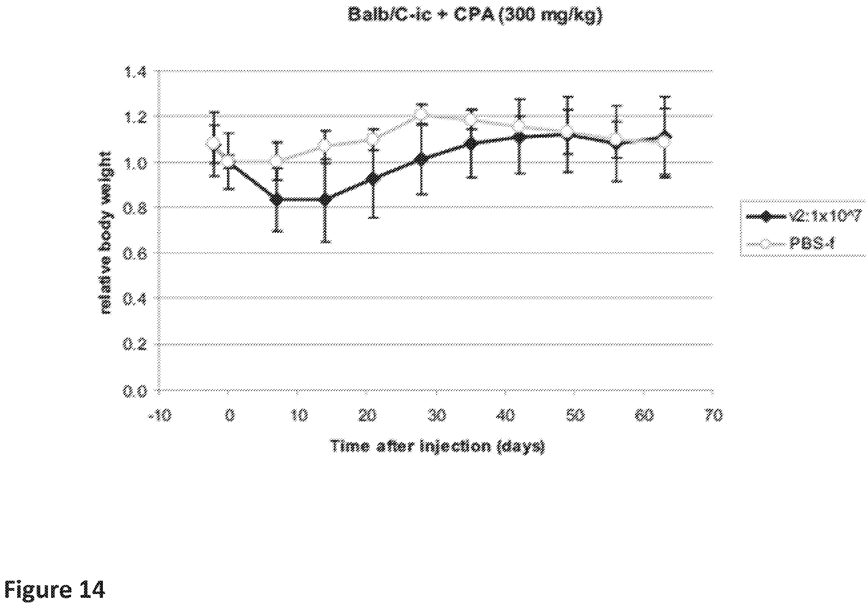

Figure 14 shows a graph of the relative body weight over time after injection of the mice used in Figure 13.

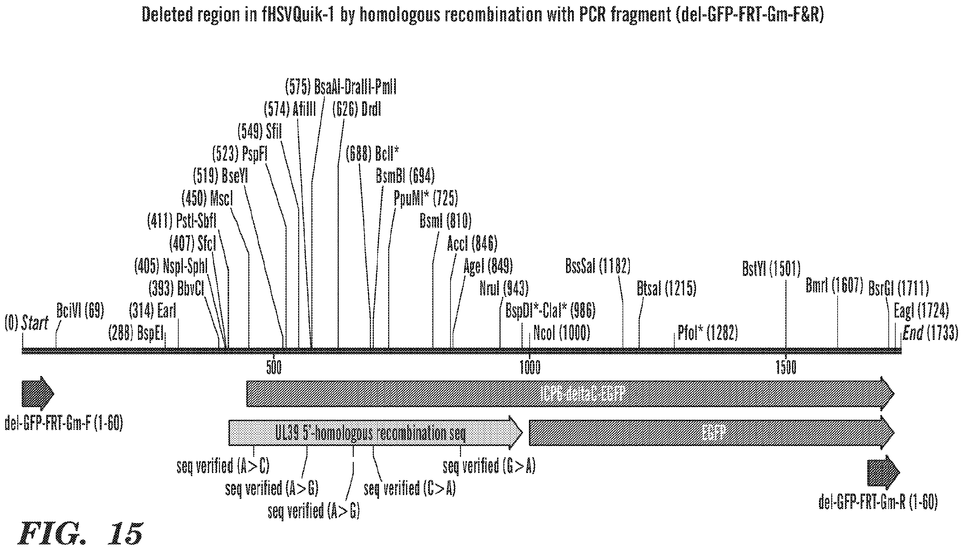

Figure 15 shows a schematic of the deleted region in fHSVQuick-1 by homologous recombination with PCR fragment (del-GFP-FRT-Gm-F&R).

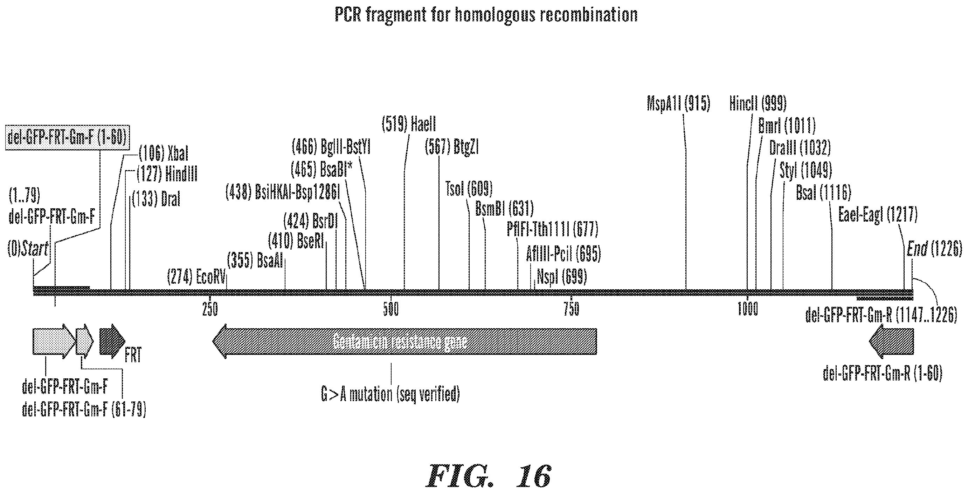

Figure 16 shows a schematic of the PCR Fragment del-GFP-FRT-Gm-F&R used for homologous recombination.

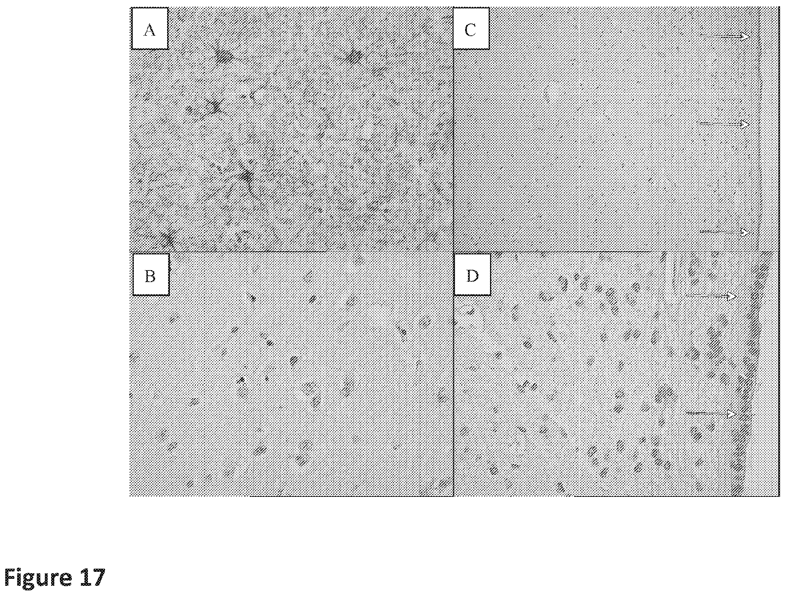

Figures 17A to 17D show Nestin expression in human brain adjacent to gliomas. The subject is a 50+ year old male with a MG. As part of the resection, brain adjacent to tumor and devoid of gross tumor was also resected up to the ventricle. Figure 17A shows GFAP immunohistochemistry with several positive astrocytes in the high power microphotograph. Figure 17B shows that Nestin immunohistochemistry of a similar area is relatively negative. Figure 17C shows Nestin immunohistochemistry (IHC) of the subventricular zone (SVZ) (Arrows) was also negative (low power). Figure 17D shows high power microphotograph of Nestin IHC of SVZ (arrows).

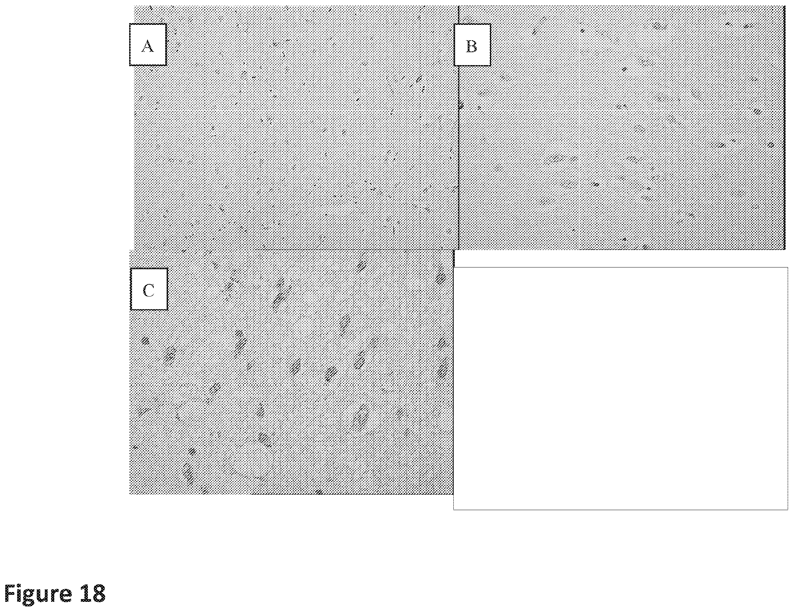

Figures 18A to 18C show Nestin expression in human brain after treatment. The subject was a 50+ year old male with a history of MG, who underwent surgery, radiation and chemotherapy. The patient passed away of causes other than tumor. At postmortem, there was no tumor in brain. Nestin IHC again showed little if any evidence of positivity within the white matter where the subject was treated for his tumor. Figure 18A- Low power; Figure 18B- Mid-power; Figure 18C- High power microphotograph.

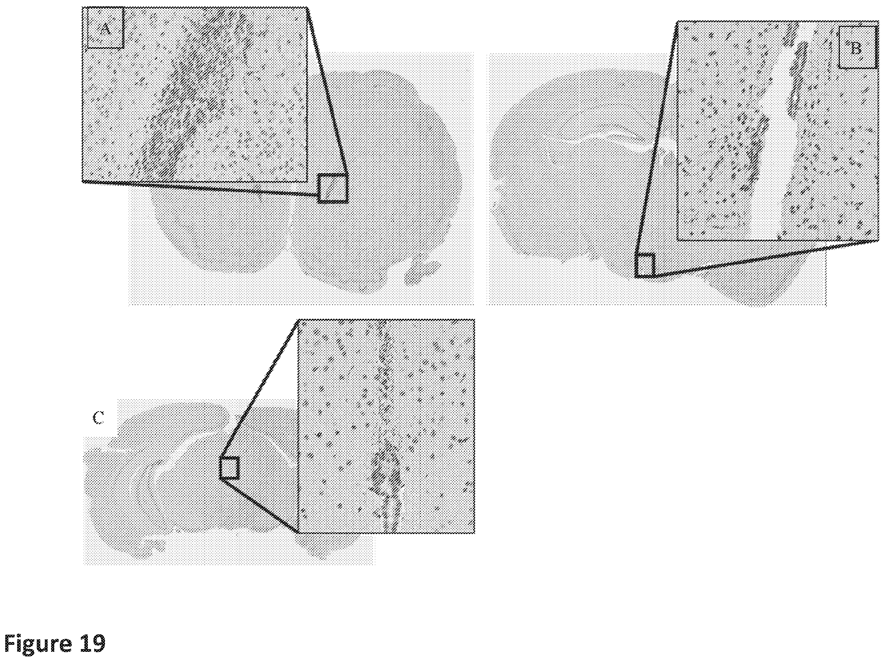

Figures 19A to 19C show nestin expression in the brains of athymic mice. The brain of this athymic mouse was inoculated with vehicle (PBS). At day 4, the mouse was euthanized and brain was harvested. Nestin IHC was performed. Positive nestin IHC is visualized in tanicytes (i.e. cells in ependymal layer of lateral ventricle (upper left panel, Figure 19A) of third ventricle (upper right panel, Figure 19B) and aqueduct (lower left panel, Figure 19C). Each panel shows a low power microphotograph of the entire brain, with the boxed inset showing the high power one.

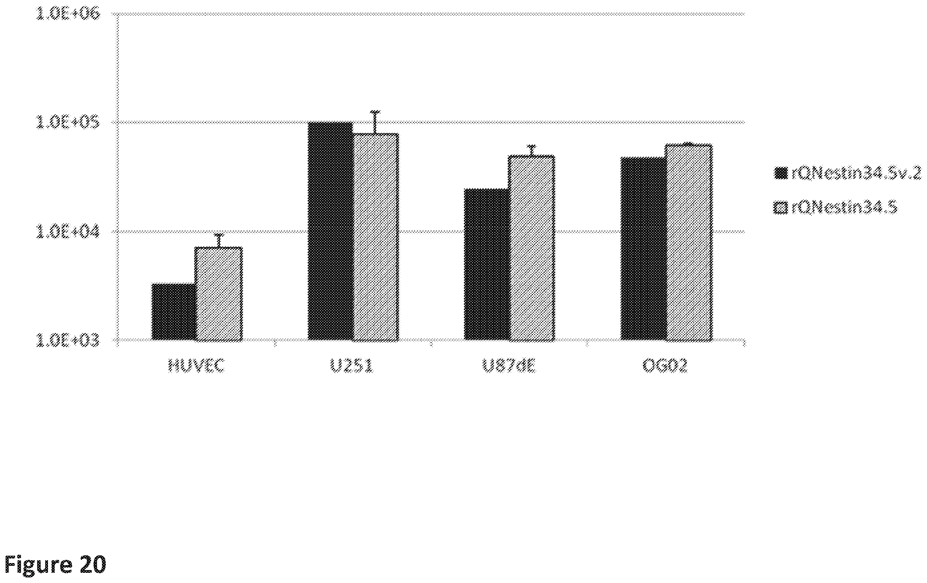

Figure 20 shows bioequivalency of viral yields of rQNestin34.5v.2 and rQNestin in HUVEC cells and established U251, U87dEGFR glioma cells and OG02 glioma "stem-like" cells. The plot shows that the viral yield of rQnestin34.5v.2 was equivalent to that of rQNestin34.5 in 3 glioma cells, including a glioma stem-like cell and superior to that in HUVEC cells.

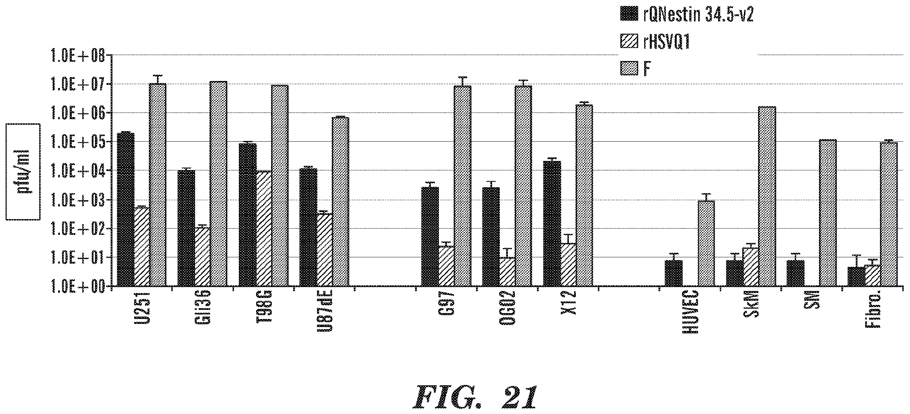

Figure 21 shows plot of replication assay results comparing yields of rQNestin34.5v.2, control rHSVQ1 and wild-type F strain in 4 established glioma cell lines (U251, Gli36, T98G, and U87dE) , 3 glioma stem-like cells (G97, OG02, X12) and 4 normal cells (HUVEC, Skeletal muscle-SKM, Smooth musckle-SM, and Fibroblasts). The plot shows that shows that rQNestin34.5v.2 replication was higher than that of the ICP34.5-negative rHSVQ1 in 4 established glioma cell lines and 3 primary gliomas grown under stem-like condition, but similar to rHSVQ1 in 4 normal cells. F strain replication was higher in all.

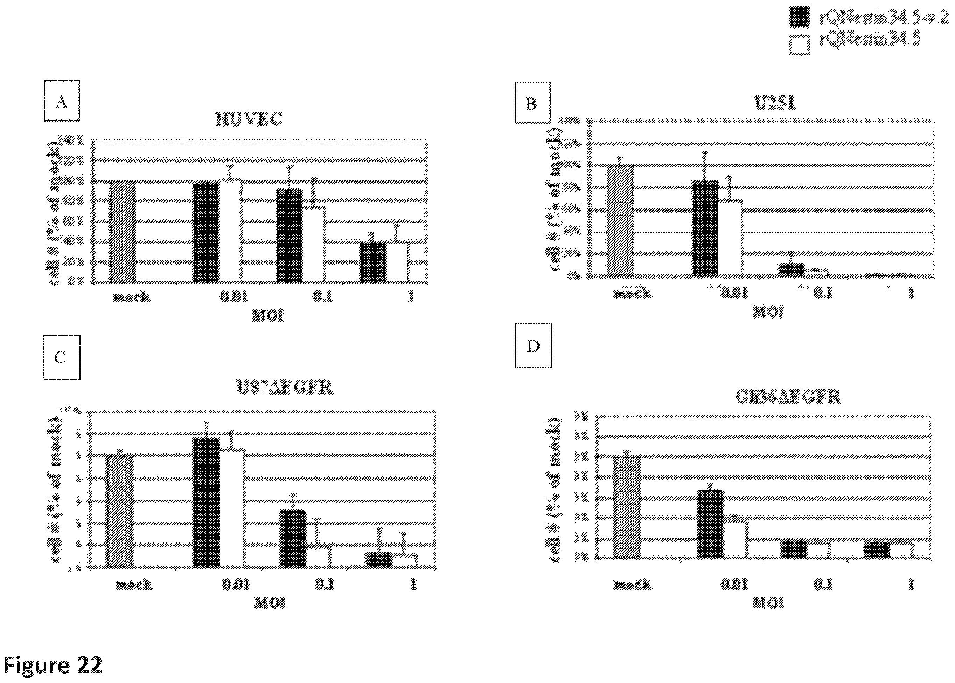

Figures 22A to 22D show cytotoxicity of rQNestin34.5 and QNestin34.5V.2 rQNestin34.5v.2 was equivalent to rQNestin34.5 in cytotoxicity at all tested MOIs against a panel of normal and glioma cells. Figure 22A HUVEC; Figure 22B U251; Figure 22C U87ΔEGFR; Figure 22D Gli36ΔEGFR.

### DESCRIPTION OF THE INVENTION

Unless otherwise defined herein, scientific and technical terms used in connection with the present application shall have the meanings that are commonly understood by those of ordinary skill in the art to which this disclosure belongs. It should be understood that this invention is not limited to the particular methodology, protocols, and reagents, etc., described herein and as such can vary. Definitions of common terms can be found in Singleton et al., Dictionary of Microbiology and Molecular Biology 3rd ed., J. Wiley & Sons New York, NY (2001); March, Advanced Organic Chemistry Reactions, Mechanisms and Structure 5th ed., J. Wiley & Sons New York, NY (2001); Michael Richard Green and Joseph Sambrook, Molecular Cloning: A Laboratory Manual, 4th ed., Cold Spring Harbor Laboratory Press, Cold Spring Harbor, N.Y., USA (2012); Davis et al., Basic Methods in Molecular Biology, Elsevier Science Publishing, Inc., New York, USA (2012); Jon Lorsch (ed.) Laboratory Methods in Enzymology: DNA, Elsevier, (2013); Frederick M. Ausubel (ed.), Current Protocols in Molecular Biology (CPMB), John Wiley and Sons, (2014); John E. Coligan (ed.), Current Protocols in Protein Science (CPPS), John Wiley and Sons, Inc., (2005); and Ethan M Shevach, Warren Strobe, (eds.) Current Protocols in Immunology (CPI) (John E. Coligan, ADA M Kruisbeek, David H Margulies, John Wiley and Sons, Inc., (2003); each of which provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, certain terms are defined below.

In some embodiments, the numbers expressing quantities of ingredients, properties such as molecular weight, reaction conditions, and so forth, used to describe and claim certain embodiments of the application are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

The term "mammal," as used herein, refers to a member of the class Mammalia, including, without limitation, humans, as well as nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like.

The term "vector," as used herein, refers to a carrier nucleic acid molecule into which a nucleic acid sequence can be inserted for introduction into a cell where it can be replicated. A nucleic acid sequence can be "exogenous," which means that it is foreign to the cell into which the vector is being introduced or that the sequence is homologous to a sequence in the cell but in a position within the host cell nucleic acid in which the sequence is ordinarily not found. Vectors include plasmids, cosmids, viruses (bacteriophage, animal viruses, and plant viruses), and artificial chromosomes (e.g., YACs). One of skill in the art would be well equipped to construct a vector through standard recombinant techniques (see, for example, Maniatis et al., 1988 and Ausubel et al., 1994). Additionally, the techniques described herein and demonstrated in the referenced figures are also instructive with regard to effective vector construction.

The term "oncolytic HSV-1 vector" refers to a genetically engineered HSV-1 virus corresponding to at least a portion of the genome of HSV-1 that is capable of transducing a target cell, replicating, and being packaged into HSV-1 virions. The genetically engineered virus comprises deletions and or mutations and or insertions of nucleic acid that render the virus oncolytic such that the engineered virus replicates in- and kills- tumor cells by oncolytic activity. The virus may be attenuated or non-attenuated. The virus may or may not deliver a transgene-that differs from the HSV viral genome. In one embodiment, tbe oncolytic HSV-1 vector does not express a transgene to produce a protein foreign to the virus.

HSV-1 is a human neurotropic virus that is capable of infecting virtually all vertebrate cells. Natural infections follow either a lytic, replicative cycle or establish latency, usually in peripheral ganglia, where the DNA is maintained indefinitely in an episomal state. HSV-1 contains a double-stranded, linear DNA genome, 153 kilobases in length, which has been completely sequenced by McGeoch (McGeoch et al., J. Gen. Virol. 69: 1531 (1988); McGeoch et al., Nucleic Acids Res 14: 1727 (1986); McGeoch et al., J. Mol. Biol. 181: 1 (1985); Perry and McGeoch, J. Gen. Virol. 69:2831 (1988)). DNA replication and virion assembly occurs in the nucleus of infected cells. Late in infection, concatemeric viral DNA is cleaved into genome length molecules which are packaged into virions. In the CNS, herpes simplex virus spreads transneuronally followed by intraaxonal transport to the nucleus, either retrograde or anterograde, where replication occurs.

The term "expression vector" refers to any type of genetic construct comprising a nucleic acid coding for a RNA capable of being transcribed. In some cases, RNA molecules are then translated into a protein, polypeptide, or peptide. In other cases, these sequences are not translated, for example, in the production of antisense molecules or ribozymes. Expression vectors can contain a variety of "control sequences," which refer to nucleic acid sequences necessary for the transcription and possibly translation of an operably linked coding sequence in a particular host cell. In addition to control sequences that govern transcription and translation, vectors and expression vectors may contain nucleic acid sequences that serve other functions as well and are described infra.

The term "promoter," as used herein, refers to a nucleic acid sequence that regulates, either directly or indirectly, the transcription of a corresponding nucleic acid coding sequence to which it is operably linked. The promoter may function alone to regulate transcription, or, in some cases, may act in concert with one or more other regulatory sequences such as an enhancer or silencer to regulate transcription of the gene of interest. The promoter comprises a DNA regulatory sequence, wherein the regulatory sequence is derived from a gene, which is capable of binding RNA polymerase and initiating transcription of a downstream (3'-direction) coding sequence. A promoter generally comprises a sequence that functions to position the start site for RNA synthesis. The best-known example of this is the TATA box, but in some promoters lacking a TATA box, such as, for example, the promoter for the mammalian terminal deoxynucleotidyl transferase gene and the promoter for the SV40 late genes, a discrete element overlying the start site itself helps to fix the place of initiation. Additional promoter elements regulate the frequency of transcriptional initiation. Typically, these are located in the region 30-110 bp upstream of the start site, although a number of promoters have been shown to contain functional elements downstream of the start site as well. To bring a coding sequence "under the control of" a promoter, one can position the 5' end of the transcription initiation site of the transcriptional reading frame "downstream" of (*i.e.,* 3' of) the chosen promoter. The "upstream" promoter stimulates transcription of the DNA and promotes expression of the encoded RNA.

The spacing between promoter elements frequently is flexible, so that promoter function is preserved when elements are inverted or moved relative to one another. Depending on the promoter used, individual elements can function either cooperatively or independently to activate transcription. The promoters described herein may or may not be used in conjunction with an "enhancer," which refers to a cis-acting regulatory sequence involved in the transcriptional activation of a nucleic acid sequence, such as those for the genes, or portions or functional equivalents thereof, listed herein.

A promoter may be one naturally associated with a nucleic acid sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages may be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other virus, or prokaryotic or eukaryotic cell, and promoters or enhancers not "naturally occurring," i.e., containing different elements of different transcriptional regulatory regions, and/or mutations that alter expression. For example, promoters that are most commonly used in recombinant DNA construction include the beta-lactamase (penicillinase), lactose and tryptophan (trp) promoter systems. As demonstrated herein, in some embodiments, a nestin promoter is used to drive expression of the gene of interest. In addition to producing nucleic acid sequences of promoters and enhancers synthetically, sequences may be produced using recombinant cloning and/or nucleic acid amplification technology, in connection with the compositions disclosed herein (see U.S. Pat. Nos. 4,683,202 and 5,928,906). Furthermore, it is contemplated the control sequences that direct transcription and/or expression of sequences within non-nuclear organelles such as mitochondria, chloroplasts, and the like, can be employed as well.

A "gene," or a "sequence which encodes" a particular protein, is a nucleic acid molecule which is transcribed (in the case of DNA) and translated (in the case of mRNA) into a polypeptide *in vitro* or *in vivo* when placed under the control of one or more appropriate regulatory sequences. A gene of interest can include, but is no way limited to, cDNA from eukaryotic mRNA, genomic DNA sequences from eukaryotic DNA, and even synthetic DNA sequences. A transcription termination sequence will usually be located 3' to the gene sequence. Typically, a polyadenylation signal is provided to terminate transcription of genes inserted into a recombinant virus.

The term "polypeptide" or "protein," as used herein, means a polymer of amino acids joined in a specific sequence by peptide bonds. As used herein, the term "amino acid" refers to either the D or L stereoisomer form of the amino acid, unless otherwise specifically designated.

The term "transgene" refers to a particular nucleic acid sequence encoding a polypeptide or a portion of a polypeptide to be expressed in a cell into which the nucleic acid sequence is inserted. The term "transgene" is meant to include (1) a nucleic acid sequence that is not naturally found in the cell (*i.e.,* a heterologous nucleic acid sequence); (2) a nucleic acid sequence that is a mutant form of a nucleic acid sequence naturally found in the cell into which it has been inserted; (3) a nucleic acid sequence that serves to add additional copies of the same (*i.e.,* homologous) or a similar nucleic acid sequence naturally occurring in the cell into which it has been inserted; or (4) a silent naturally occurring or homologous nucleic acid sequence whose expression is induced in the cell into which it has been inserted. A "mutant form" or "modified nucleic acid" or "modified nucleotide" sequence means a sequence that contains one or more nucleotides that are different from the wild-type or naturally occurring sequence, i.e., the mutant nucleic acid sequence contains one or more nucleotide substitutions, deletions, and/or insertions. In some cases, the gene of interest may also include a sequence encoding a leader peptide or signal sequence such that the transgene product may be secreted from the cell.

As used herein, the term "transfection" refers to the uptake of foreign DNA by a mammalian cell. A cell has been "transfected" when exogenous DNA has been introduced inside the cell membrane. A number of transfection techniques are known in the art. See, Michael Richard Green and Joseph Sambrook, Molecular Cloning: A Laboratory Manual, 4th ed., Cold Spring Harbor Laboratory Press, Cold Spring Harbor, N.Y., USA (2012); Davis et al., Basic Methods in Molecular Biology, Elsevier Science Publishing, Inc., New York, USA (2012); Jon Lorsch (ed.) Laboratory Methods in Enzymology: DNA, Elsevier, (2013); Frederick M. Ausubel (ed.), Current Protocols in Molecular Biology (CPMB), John Wiley and Sons, (2014). Such techniques can be used to introduce one or more exogenous DNA moieties, such as a viral vector and other nucleic acid molecules, into suitable host cells. The term refers to both stable and transient uptake of the genetic material.

The term "oncolytic activity," as used herein, refers to cytotoxic effects *in vitro* and/or *in vivo* exerted on tumor cells without any appreciable or significant deleterious effects to normal cells under the same conditions. The cytotoxic effects under *in vitro* conditions are detected by various means as known in prior art, for example, by staining with a selective stain for dead cells, by inhibition of DNA synthesis, or by apoptosis. Detection of the cytotoxic effects under *in vivo* conditions is performed by methods known in the art.

A "biologically active" portion of a molecule, as used herein, refers to a portion of a larger molecule that can perform a similar function as the larger molecule. Merely by way of non-limiting example, a biologically active portion of a promoter is any portion of a promoter that retains the ability to influence gene expression, even if only slightly. Similarly, a biologically active portion of a protein is any portion of a protein which retains the ability to perform one or more biological functions of the full-length protein (e.g. binding with another molecule, phosphorylation, etc.), even if only slightly.

With the aforementioned preliminary descriptions and definitions in mind, additional background is provided herein below to provide context for the genesis and development of the inventive vectors, compositions and methods described herein.

Current mutant HSV-1 vectors that target malignant glioma are based on the two deletion mutant genes, ICP6 (U_{L}39 gene product), the large subunit of HSV-1 ribonucleotide reductase (RR), and ICP34.5 (34.5 gene product), a multifunctional protein that is also related to neurovirulence. While the lack of ICP6 restricts virus replication to non-dividing cells but allows replication to continue in cells with defects in the *p16* tumor suppressor pathway, deletions of both γ₂ 34.5 genes suppresses HSV-1 encephalitis. This may be due to ICP34.5's facilitation of Beclin-1 autophagy function, essential for neurovirulence. Besides this autophagic inhibitory effect, ICP34.5 also counteracts a host defense mechanism triggered by viral infection. This mechanism activates PKR (double-stranded RNA protein kinase) that then phosphorylates the translation factor, eIF2α, leading to translation inhibition. ICP34.5 directly binds and activates PP1 (protein phosphatase 1) that dephosphorylates eIF2α, allowing for viral mRNA translation to continue. Oncolytic HSV-1 with mutated y34.5 genes (e.g. G207, 1716) has proven to be safe for administration in humans with gliomas in multiple clinical trials, but efficacy has been elusive, probably due to their limited viral replication. To overcome this limitation, an HSV1 was previously engineered, wherein the ICP34.5 gene is under the transcriptional control of the glioma stem cell promoter for nestin. rQNestin34.5 has exhibited increased efficacy in glioma models.

Nestin is an intermediate filament predominantly expressed in neural stems cells during embryogenesis, and is considered to be upregulated in glioma. A variety of primary tumors of the central nervous system (CNS) display elevated levels of nestin within tumor and/or endothelial cells. This transcriptionally driven oncolytic virus has shown efficient anti-tumor efficacy against CNS and neuroblastoma tumors in vitro and in vivo. Nestin gene sequence can be found in Genebank Gene ID: 10763, Chromosome 1 - NC_000001.11 (156668763..156677397, complement).

### The Herpes Gamma (γ) 34.5 Gene

Published results have demonstrated that at least one function of the herpes y34.5 gene (which is alternatively known as the γ134.5 gene) is to preclude the host cell's response to viral infection, namely the triggering of host protein synthesis shutoff in an apoptotic-like response (Chou, J., et al., Science 250:1262-1266 (1990); Chou, J. and Roizman, B., Proc. Natl. Acad. Sci. USA 89:3266-3270 (1992); Chou, J., et al., Proc. Natl. Acad. Sci. USA 92:10516-10520 (1995)). A similar function is widespread among pathogenic viruses (Cosentino, G. P., et al., Proc. Natl. Acad. Sci. USA 92:9445-9449 (1995); Gale, M., Jr., et al., Mol. Cell Biol. 18:5208-5218 (1998); Katze, M. G., et al., Trends Microbiol. 3:75-78 (1995); Sharp, T. V., et al., Nuc. Acids Res. 21:4483-4490 (1993)).

While y34.5 is nonessential for viral growth in culture in Vero cells, it enables the virus to spread in the central nervous system (CNS) of mice, and maps to a region of the HSV genome previously implicated in CNS replication (Markovitz, N. S., et al., J. Virol. 71:5560-5569 (1997); Centifanto-Fitzgerald, Y. M., et al., J. Esp. Med 155:475-489 (1982)). This may be due to the fact that the γ34.5-encoded protein inhibits the double-stranded RNA-dependent kinase (PKR). On exposure to double stranded RNA molecules, as seen commonly with viral infection, PKR phosphorylates the alpha subunit of elongation initiation factor eIF-2, resulting in inhibition of protein synthesis (Chou, J., et al., Science 250:1262-1266 (1990); Chou, J. and Roizman, B., Proc. Natl. Acad. Sci USA 89:3266-3270 (1992); Chou, J., et al., J. Virol. 68:8304-8311 (1994)). Infection of cells of neuronal origin with mutants incapable of expressing y34.5 results in shut-off of cellular protein synthesis, with the resultant limitation of viral production.

In summary, in the presence of γ34.5, HSV will prevent apoptosis, thus allowing for production of progeny viruses. In its absence, the cell dies and the infecting HSV cannot generate progeny viruses. Thus, HSV infection/propagation throughout an organ is eliminated.

The nestin promoter/γ34.5 approach of the invention, thus allows for production of virus in cells that can use that promoter, but cells that cannot turn on the promoter will not propagate infection.

The herpes simplex viral mutant of the invention comprises a deletion or inactivating mutation in both copies of the y34.5 gene, wherein at least one copy of the y34.5 gene is reintroduced under the control of a cell-specific or tumor-specific promoter.

As used herein, the term "deletion" is intended to mean the elimination of nucleic acids from a gene, such as the y34.5 gene.

As used herein, the term "inactivating mutation" is intended to broadly mean a mutation or alteration to a gene wherein the expression of that gene is significantly decreased, or wherein the gene product is rendered nonfunctional, or its ability to function is significantly decreased.

The term "gene" encompasses both the regions coding the gene product as well as regulatory regions for that gene, such as a promoter or enhancer, unless otherwise indicated.

Ways to achieve such alterations include: (a) any method to disrupt the expression of the product of the gene or (b) any method to render the expressed gene nonfunctional. Numerous methods to disrupt the expression of a gene are known, including the alterations of the coding region of the gene, or its promoter sequence, by insertions, deletions and/or base changes. (See, Roizman, B. and Jenkins, F. J., Science 229: 1208-1214 (1985)).

We have generated an improved oncolytic rQNestin34.5 HSV virus, a second generation virus referred to as rQNestin34.5.v2. The genetically modified HSV1 rQNestin34.5.v2 has been made glioma-selective by the following maneuvers: 1) one of the viral genes that encodes for a viral protein (ICP6) has been removed. Without this gene, HSV1 has to utilize factors within the infected cells to efficiently grow and replicate and we have shown that such factors are existent in cells that are mitotically active or that have a defect in a cellular gene (p16) that is missing in most gliomas; and 2) the two copies of the viral gene that encode for a protein (ICP34.5) needed for robust viral growth in an infected cell have also been removed and one copy was reinserted under control of a Nestin promoter that is also present selectively in gliomas in the adult human brain. These two maneuvers thus allow this new HSV1 (designated as rQNestin34.5v.2) to be relatively selective in destroying gliomas and not normal brain cells and we have confirmed these statements in cultured cells and in animal models. In addition, there is lack of an ICP6-EGFP fusion protein present in the first generation rQNestin34.5 (Kambara et al. An oncolytic HSV-1 mutant expression ICP34.5 under control of a Nestin promoter increases survival of animals even when symptomatic from a brain tumor, (2005) Cancer Res. 65(7): 2832-2839) , further removing a source of genomic instability and toxicity

Accordingly, provided herein are tumor-selective oncolytic herpes simplex viral vectors comprising: a) a deletion or inactivating mutation in both copies of the gene encoding y35.5; b) an insertion of at least one copy of the HSV y35.5 gene under the transcriptional control of a Nestin promoter; and c) a deletion or inactivating mutation in the gene that encodes for the HSV viral protein ICP6, wherein the tumor-selective oncolytic herpes simplex viral vector does not express green fluorescent protein, e.g. ICP6-EGFP fusion protein present in the first generation HSV-1 vector rQNestin34.5.

In certain embodiments, the vector does not contain UL39 nucleic acid regulatory sequences (promoter and enhancer elements). In certain embodiments, the vector does not contain a fusion protein of ICP6.

In one embodiment, the at least one copy of the y35.5 gene under the transcriptional control of a nestin promoter is inserted into UL39 gene that encodes for the large subunit of ribonucleotide reductase ICP6 (infected cell protein 6). In one embodiment, the nestin promoter comprises SEQ ID NO: 2 or a degenerate variant thereof. In one embodiment, a hybrid promoter comprising a nestin promoter and elements of heat shock protein 68 promoter is used (*See* Kambara et al. An oncolytic HSV-1 mutant expression ICP34.5 under control of a Nestin promoter increases survival of Animals even when symptomatic from a brain tumor, (2005) Cancer Res. 65(7): 2832-2839; and Kawaguchi et al.Nestin EGFP transgenic mice, visualization of the self-renewal and multipotentcy of CNS stem cells Mol. Cell Neurosci. (2001)17:259-273).

In one embodiment, the tumor-selective oncolytic herpes simplex viral vector comprises the sequence of SEQ ID NO: 1, or a degenerate variant thereof.

One of skill in the art would readily appreciate that a modified version of sequences disclosed herein could also be used, so long as it retains similar biological activity. Merely by way of non-limiting example, the nestin 2^{nd} intron sequence (enhancer) represented in SEQ ID NO: 4, and the hsp68 minimum promoter represented in SEQ ID NO: 5, could be used alone or combined when designing various constructs contemplated herein. In some embodiments, the nestin enhancer element may be operably linked to a heat shock protein 68 (hsp68) minimum promoter to drive the expression of γ34.5. In some embodiments, alternative or additional expression control sequences may be incorporated into the oncolytic expression vectors to initiate or influence the expression of any of the aforementioned nucleotide sequences of interest. Merely by way of non-limiting examples, the nestin promoter may incorporate microRNA target sequences. Examples of miR translational control sequences include, but are not limited to: miR128 or miR124 to differentiate glioma cells from normal neural cells.

Also described herein, but not part of the claimed invention, is a method for treating a neoplastic disease in a subject. The method may include administering to the subject a therapeutically effective amount of an expression vector with oncolytic activity. The cancer may be brain cancer. Merely by way of non-limiting examples, the types of brain cancer that can be treated may include glioblastoma, anaplastic astrocytoma, astrocytoma, pilocytic astrocytoma, diffuse intrinsic pontine glioma, oligodendroglioma, anaplastic oligodendroglioma, mixed oligo-astrocytoma, and pendymoma. Cancer stem cells may be treated with the described method. The subject treated may be a mammal. The subject treated may be a human.

Methods of treating any of the neoplastic diseases described herein, including brain cancer, may include administration of the compounds of exemplary embodiments or as described herein as a single active agent, or in combination with additional methods of treatment including, but not limited to, stem cell-based therapy, immunotherapy, radiation therapy, therapy with immunosuppressive agents, chemotherapeutic or anti-proliferative agents, including cytokines. The methods of treatment may be in parallel to, prior to, or following additional methods of treatment.

"Therapeutic benefit," as used herein, includes any decrease in cancer cell number, cancer cell proliferation rate, or metastasis. The promoter used herein may facilitate selective or increased expression of the associated gene of interest in one or more cancer cell type of interest, compared to a normal cell.

The term "operably linked," as used herein, refers to the arrangement of various nucleic acid molecule elements relative to each other such that the elements are functionally connected and are able to interact with each other. Such elements may include, without limitation, a promoter, an enhancer, a polyadenylation sequence, one or more introns and/or exons, and a coding sequence of a gene of interest to be expressed. The nucleic acid sequence elements, when operably linked, can act together to modulate the activity of one another, and ultimately may affect the level of expression of the gene of interest, including any of those encoded by the sequences described above.

One of skill in the art will understand that although specific sequences are provided herein, the nucleic acid molecules used in the inventive vectors, compositions and methods are not limited strictly to molecules including the sequences set forth as SEQ ID NO: 1, SEQ ID NO: 2, SEQ ID NO: 3, SEQ ID NO: 4, SEQ ID NO: 5, SEQ ID NO: 6, and SEQ ID NO: 8. Rather, specific embodiments encompass nucleic acid molecules carrying modifications such as substitutions, small deletions, insertions, or inversions. Included in the invention are nucleic acid molecules, the nucleotide sequence of which is at least 95% identical (e.g., at least 96%, 97%, 98%, or 99% identical) to the nucleotide sequence shown as SEQ ID NOS: 1, 2, 3, 4, 5, 6, 7 and 8 in the Sequence Listing.

Also included in the invention is a nucleic acid molecule that has a nucleotide sequence which is a degenerate variant of a nucleic acid disclosed herein, e.g., SEQ ID NOS: 1, 2, 3, 4, 5, 6, 7, and 8. A sequential grouping of three nucleotides, a "codon," encodes one amino acid. Since there are 64 possible codons, but only 20 natural amino acids, most amino acids are encoded by more than one codon. This natural "degeneracy" or "redundancy" of the genetic code is well known in the art. It will thus be appreciated that the nucleic acid sequences shown in the Sequence Listing provide only an example within a large but definite group of nucleic acid sequences that will encode the polypeptides as described above.

Importantly, the vectors described herein may be useful for the introduction of additional genes in gene therapy. Thus, for example, the HSV vectors may contain one or more additional exogenous gene for the expression of a protein effective in regulating the cell cycle, such as p53, Rb, or mitosin, or a biologically active variant thereof, or in inducing cell death, such as the conditional suicide gene thymidine kinase, the latter must be used in conjunction with a thymidine kinase metabolite in order to be effective, or any other anti-tumor gene, such as for example a toxin.

When used pharmaceutically, oncolytic vector embodiments discussed herein can be combined with various pharmaceutically acceptable carriers. Suitable pharmaceutically acceptable carriers are well known to those of skill in the art. The compositions can then be administered therapeutically or prophylactically, in effective amounts, described in greater detail below.

As used herein, the term "therapeutically effective amount" is intended to mean the amount of vector which exerts oncolytic activity, causing attenuation or inhibition of tumor cell proliferation, leading to tumor regression. An effective amount will vary, depending upon the pathology or condition to be treated, by the patient and his or her status, and other factors well known to those of skill in the art. Effective amounts are easily determined by those of skill in the art. In some embodiments a therapeutic range is from 10³ to 10¹² plaque forming units introduced once. In some embodiments a therapeutic dose in the aforementioned therapeutic range is administered at an interval from every day to every month via the intratumoral, intrathecal, convection-enhanced, intravenous or intra-arterial route.

Although certain routes of administration are provided in the foregoing description, any suitable route of administration of the vectors may be adapted, and therefore the routes of administration described above are not intended to be limiting. Routes of administration may including but are not limited to, intravenous, oral, buccal, intranasal, inhalation, topical application to a mucosal membrane or injection, including intratumoral, intradermal, intrathecal, intracisternal, intralesional or any other type of injection. Administration can be effected continuously or intermittently and will vary with the subject and the condition to be treated. One of skill in the art would readily appreciate that the various routes of administration described herein would allow for the inventive vectors or compositions to be delivered on, in, or near the tumor or targeted cancer cells. One of skill in the art would also readily appreciate that various routes of administration described herein will allow for the vectors and compositions described herein to be delivered to a region in the vicinity of the tumor or individual cells to be treated. "In the vicinity" can include any tissue or bodily fluid in the subject that is in sufficiently close proximity to the tumor or individual cancer cells such that at least a portion of the vectors or compositions administered to the subject reach their intended targets and exert their therapeutic effects.

Pharmaceutically acceptable carriers are well known in the art and include aqueous solutions such as physiologically buffered saline or other solvents or vehicles such as glycols, glycerol, vegetable oils (e.g., olive oil) or injectable organic esters. A pharmaceutically acceptable carrier can be used to administer the compositions of the invention to a cell in vitro or to a subject *in vivo.* A pharmaceutically acceptable carrier can contain a physiologically acceptable compound that acts, for example, to stabilize the composition or to increase the absorption of the agent. A physiologically acceptable compound can include, for example, carbohydrates, such as glucose, sucrose or dextrans, antioxidants, such as ascorbic acid or glutathione, chelating agents, low molecular weight proteins or other stabilizers or excipients. Other physiologically acceptable compounds include wetting agents, emulsifying agents, dispersing agents or preservatives, which are particularly useful for preventing the growth or action of microorganisms. Various preservatives are well known and include, for example, phenol and ascorbic acid. One skilled in the art would know that the choice of a pharmaceutically acceptable carrier, including a physiologically acceptable compound, depends, for example, on the route of administration of the polypeptide.

### EXAMPLES

The following examples are included to demonstrate preferred embodiments of the invention. It should be appreciated by those skilled in the art that the techniques disclosed in the examples which follow represent techniques discovered by the inventors to function well in the practice of the invention, and thus can be considered to constitute preferred modes for its practice. However, it will be apparent that certain agents that are both chemically and physiologically related may be substituted for the agents described herein while the same or similar results would be achieved.

### Example 1 Generation of rQNestin34.5v.2

rQNestin34.5v.2 is a genetically engineered HSV1 (F strain derivative), designed to replicate in glioma cells and not normal cells in the brain or other tissues. HSV1 is present endemically in the human population primarily in a latent form within the sensory neurons of the trigeminal ganglia. The virus is approximately 152-158 kilobases, it is enveloped and measures 150 nm in diameter. The virus will infect and replicate in most, if not all, established human glioma cell lines, as well as freshly established gliomas from patients expanded under conditions that enrich for the "stem-like" glioma subpopulation. The following genetic modification were made to HSV1 F strain ( e.g. commercially available form OriGene Technologies Inc. Rockville, MD 20850 U.S.S) to generate rQNestin34.5v.2:
1- Both endogenous copies of the coding region of the viral gene encoding for ICP34.5 have been mostly removed;
2- A single copy of the ICP34.5 coding region was placed under control of a glioma-selective nestin promoter/enhancer sequence, providing glioma-selective expression of the ICP34.5 viral gene, allowing for more robust replication of the virus in nestin-expressing glioma vs. nestin-non expressing brain cells;
3- The viral ICP6 gene locus is also disrupted, restricting viral replication to glioma cells with defective p16 tumor suppressor pathway signaling (> 90% of gliomas) or mitotic cells.

The schematic of rQNestin34.5v.2 is shown if **Figure 1****.** In infected cells, viral DNA remains extrachromosomal. The virus, upon entry in tumor cells will enter the lytic cycle, usually resulting in tumor cell lysis within 12-18 hours. Latency only occurs in trigeminal sensory neurons.

rQNestin34.5 v. 2 DNA was subjected to next generation sequencing on an Illumina GA IIe machine at the Microarray Core Facility Huntsman Cancer Institute University of Utah, the Sequence is provided in SEQ ID NO: 1.

The nestin-hsp68 minimum enhancer/promoter sequence are derived from the human nestin enhancer/promoter and the human hsp68 promoter fused together to provide specific transcriptional regulation to cells expressing nestin. Specific transcriptional regulation of ICP34.5 in the context of rQNestin was shown in the following set of experiments (Note: ICP34.5 expression in infected cells is leads to dephosphorylation of the translation factor eIF2a. Therefore, we would expect to see dephosphorylated eiF2a in glioma cells that express nestin, while cells that do not express nestin (HUVEC and human astrocytes) should have high levels of phosphor eiF2a, after viral infection).

Several established glioma cell lines (U87dEGFR, U215, U138 and Gli36dEGFR), a freshly explanted glioma cell grown under conditions to enrich for the glioma "stem-like" cell subpopulation (GBM #02), normal human vein endothelial cells (HUVEC) and normal human astrocytes were infected with rQNestin34.5 v.2 or another rQNestin34.5 (v.1- that expressed GFP) **(****Figure 2****)**. In addition, rHSVQ1 (rQ1) (see e.g. US 20020110543 and Hirokazu Kambara et al. An Oncolytic HSV-1 Mutant Expressing ICP34.5 under Control of a Nestin Promoter Increases Survival of Animals even when Symptomatic from a Brain Tumor, Cancer Res April 1, 2005, 65; 2832), an ICP34.5 deleted mutant, wild-type F strain virus, and uninfected cells were also used. Cell lysates were separated and Western blots for eIF2a vs. phosphor-eiF2a were then performed. In normal HUVEC cells and astrocytes, phospo-eiF2a was present, as expected, in rQNestin34.5, rQNestin34.5v.2, and rHSVQ1 virally-infected cells, showing that they functioned as ICP34.5 mutants, while no phosphor-eiF2a was observed in wild-type F strain infected cells. However, in all glioma cells, there was no phosphor-eiF2a detected in in rQNestin34.5 or rQNestin34.5v.2 infected cells, similar to wild-type F strain virus, while phosphor-eiF2a was still visible in tHSVQ1-infected cells. This data thus showed that rQNestin34.5v.2 functioned as an ICP34.5-defective virus in normal cells (HUVEC and astrocytes) unlike wild-type virus. However, in glioma cells, rQNestin34.5v.2 functioned as an ICP345.-positive virus, similar to wild-type virus. Quantitatively, there was clearly a >2-fold decrease in the levels of phosphorylated eiF2α in glioma cells infected with rQNestin34.5v.2 and parental rQNestin34.5 compared to control tHSVQ1 virus. Also, the levels of observed phosphorylated eIF2α in the rQNestin34.5v.2 and parental rQNestin34.5 infected glioma cells were >2-fold less than unphosphorylated eIF2α protein in the same cells.

In the first series of steps, we had to modify fHSVQuik-1, the bacterial artificial chromosome (BAC) that contains the F strain HSV1 sequence with the deleted diploid ICP34.5 genes. The generation of fHSVQ1 was described in Terada et al. (Terada et al. Development of a rapid method to generate multiple oncolytic HSV vectors and their in vivo evaluation using synergenic mouse tumor models. Gene Ther. 13: 705-714, 2006). **Figure 3** shows that the modification of fHSVQuik-1 to remove the entire sequence of EGFP under control of the ICP6 promoter was accomplished by ET-cloning of a GmR selection marker at the site with removal of the ICP6 promoter-EGFP transcriptional cassette, followed by FLP-recombination and removal of the selectable marker to generate fHSVQuik-2. In order to transfer in the Nestin/hsp68 promoter/enhancer- ICP34.5 cassette into fHSVQuik-2, a transfer plasmid with this cassette (marked in X) is then recombined back into the same site using Flp-recombinatiion to give rise to fHsvQ2-X. When X is the Nestin/hsp68 promoter/enhancer- ICP34.5 cassette, it is designated as fHSVQ2-nestin34.5 (see **Figure 4**)

After generating fHSVQ2-nestin34.5 in bacteria, the entire BAC is purified and electroporated into mammalian Vero cells where Cre-Lox recombination is utilized to remove all BAC sequences (figure 4). The viral genome is now "free" to begin its own process of DNA replication, generating viral plaques that can be purified to generate rQNestin34.5v.2. The generated rQnestin34.5v.2 was identified by Southern blotting as possessing the desired genetic identity (data not shown). An isolate of rQNestin34. 5v.2 was then sent to Diamyd, Inc. who proceeded with viral DNA isolation and transfection into their certified Vero derived Master Cell Bank (MCB) followed by expansion to obtain the final seed of rQNestin34.5v.2 employed for subsequent efficacy/toxicity experiments. This Viral Seed Stock (VSS) was subjected to a panel of validated assays in order to allow subsequent cGMP procedures moving toward large scale manufacturing of the vector for the clinical trial. The VSS tests performed under validated GLP conditions included; Sterility (including Bacteristasis and Fungistasis (B&F) immersion), Mycoplasma (Points to Consider), Endotoxin, Polymerase Chain Reaction (PCR) based Reverse Transcription (PBRT) assay, and quantitative PCR (QPCR) testing for the following adventitious agents: Porcine Circovirus 1 and 2; Adenovirus type 5; Adeno-Associated Virus types 1, 2, 3, 4, 6, 7, 8, 9, 10, and 11. The results of this testing panel document the suitability to advance with manufacturing.

### Virus production

Cell Thaw and Expansion. One or more vials of VeroD Master Cell Bank are thawed at 37°C and the cells are transferred to a conical tube and pooled. The cells were vialed at 1.2×10⁷ cell/mL/tube and viable recoveries have been ~ 9.2×10⁶ cell/tube. The cells were gradually diluted with complete medium and a sample is removed to obtain viable cell counts. The cells are plated to flasks at a density of 3.0 - 5.0×10⁴ cells/cm2.

The cells were incubated at 37°C, 7.5% CO₂ and examined periodically by phase microscopy for growth and visual evidence of microbial contamination. When the cells were ~80% confluent, the cells were passaged. Briefly, the complete medium is removed and PBS is added to rinse the cells. The PBS is removed and TryPLE Select is added to the flasks to dissociate the cells. The flasks are incubated at 37°C for approximately 3-5 minutes or until the cells are detached. The cells were re-suspended in complete medium, pooled, counted and seeded into new flasks at a density between 2.0-4.0×10⁴ cells/cm². The cells were expanded to 16×10-layer Nunc Cell Factories and allowed to reach 1-2 days post confluence prior to infection. allowed to reach 1-2 days post confluence prior to infection.

Infection with rQNestin34.5v.2 Vector. When the cells have reached desired confluence, an example flask was treated with TryPLE Select and counted to estimate cell numbers. The rQNestin34.5v.2 Master Virus Bank vector inoculum was prepared by thawing the appropriate volume required to obtain an MOI=0.1. The cell factories were infected by an initial 1.5 hour adsorption period followed by incubation for the first day of infection in complete medium. After ~24 hours, the culture medium was removed and replaced with an equal volume of serum-free medium. The cell factories were placed in the incubator and the temperature is reduced to 33°C/ 7.5%CO2. The cultures were monitored daily and checked visually for percent cytopathic effect. The infection typically lasts for 4 days.

Crude Viral Harvest and Clarification. The infection was stopped by placing the cell factories in the biosafety cabinet and pooling the supernatant and cell debris into a sterile bag. The harvest sodium chloride level was increased to 0.45 M by the addition of a 5 M NaCl stock solution. The harvest was then mixed by hand for 20-30 minutes. The harvest was then aliqotted into 500 mL centrifuge tubes and the cell debris is removed by centrifugation at 1500×g. The supernatant was repooled into a sterile bag. After pre-treatment of a Sartopore clarification filter capsule with sterile water, the virus-containing supernatant was then pumped through the filter capsule into another sterile bag. The virus is followed by pumping sterile water to recover remaining virus in the capsule. The bag was mixed and the filtrate is stored overnight at 4°C.

The next day, the filtrate was warmed and adjusted to ~2mM MgCl₂ by addition of 2 volumes of 3mM MgCl₂ in sterile water. The diluted filtrate is mixed and Benzonase treatment is performed next. This is done by first diluting 100,000-200,000 U of the enzyme into dilution buffer and 7 sequential additions are injected at 5 minute intervals with continuous mixing over a 35 minute period.

Cation Exchange Column Chromatography. A BPG 100 column is packed with SP high performance resin and is sanitized with 0.5N NaOH. After sanitization, the column is equilibrated with wash buffer (PBS pH 7.0), strip buffer (1M NaCl-PBS pH 7.0), and wash buffer before loading benzonase treated virus. Conductivity, UV, and pH are monitored during the run.

The process bag containing the Benzonase treated filtrate was connected to the inlet using a tubing welder and the virus is loaded onto the column. The flow through was collected in a sterile bag. The virus capture step was followed by washing with PBS pH 7.0 until the UV absorbance returns to baseline. The pump was stopped and the process bag containing 0.45 M NaCl-PBS pH 7.0 was connected to the inlet. The outlet tubing was transferred to a sterile container in the biosafety cabinet. The buffer was pumped into the column and when the UV absorbance began to increase sharply, the column outlet was transferred to a new sterile container to collect the eluted virus. The collection was stopped after the UV absorbance returns to near baseline. This is the purified viral elute fraction. The process bag containing strip buffer is next connected to the inlet. The end of the outlet tubing is transferred into a sterile bottle to collect the strip fraction. The buffer is pumped through the column until UV absorbance reaches a peak and returns to near baseline. The elute is stored at 4°C overnight.

Tangential Flow Filtration and Final Filtration. The tangential-flow filtration system is prepared by assembling the tubing and cartridge and sterilizing the system by autoclaving. The system is transferred to the biosafety cabinet and flushed with sterile PBS pH 7.0. An equal volume of sterile PBS pH 7.0 and the 0.45M virus elute fraction are added to the system reservoir and recirculated for 5-10 minutes. After this equilibration, the permeate collection pump is turned on and filtrate is collected at ~5 mL/minute. The system is run until the loaded volume is reduced by half. The retentate in the reservoir is diluted with PBS pH 7.0 to double the volume and continued to reconcentrate. This process is repeated while monitoring the conductivity of the permeate. When the permeate conductivity was within 10% of the diafiltering buffer (PBS pH 7.0) the product was allowed to concentrate to about 40 mL The retentate was recovered and filtered through a 0.45 um Millipack filter unit.

Final Formulation and Packaging. The terminal filtered virus stock was adjusted to 10% final volume with sterile glycerol and mixed well. The product was manually dispensed into cryovials for storage at a volume of 110 uL per vial. The tubes were labeled and stored at ≤-65°C.

### Example2 Preclinical Studies

Three general sets of experiments were performed. In the first, *in vitro* studies utilizing glioma cell lines and normal human cells (particularly human astrocytes) are utilized as models to show the relative selectivity of rQNestin34.5 replication and cytotoxicity against the former and not the latter. In the second, a mouse athymic model of intracranial human glioma xenograft is used to show that a single intratumoral injection of rQNestin34.5v.2 leads to a significant prolongation of animal survival. Due to the lack of replication of the virus in syngeneic murine gliomas grown in C57/B6 mice, a suitable efficacy experiment in an immunocompetent animal model is not possible. Finally, intracranial injections of the agent in the brains HSV-susceptible Balb/c mice were performed to determine the degree of toxicity

We first determined if rQNestin34.5v.2 replicated efficiently in human glioma cells vs. normal human astrocytes. The former express nestin while the latter do not (data not shown). Figure 2 shows that nestin promoter activation of ICP34.5 in rQNestin34.5 infected glioma cells led to dephosphorylation of the translation factor eiF2a thus allowing for efficient translation of viral mRNAs in infected cells, similar to wild-type F strain, while ICP34.4-deleted HSV1 (such as Q1, aka rQ1, rHSVQ1) cannot do this. Therefore, one would expect in human glioma cells robust replication of rQNestin34.5v.2 similar to F strain, while Q1 should replicate poorly. Figure 5 confirms this. Conversely, in normal human astrocytes (the predominant cell population in the brain, whose progenitor is thought to give rise to glioma) that do not express nestin, rQNestin34.5v.2 will not dephosphorylate eiF2a, leading to poor to nil viral replication, similar to other ICP34.5-deleted HSV1 such as Q1 (**Figure 5**). Wild-type F strain HSV1 will still replicate though. In fact, As shown in **Figure 4**, the yield of rQNestin34.5v.2 was at least 4 log units more in U87dEGFR and 5 log units more in U251 compared to that in human astrocytes (where it approached almost no virus obtained). We then tested the cytotoxicity of rQNestin34.5v.2 against a panel of human glioma cells and normal human cells. rQNestin34.5 v.2 was added to panels of gliomas cells and normal human and mouse cells to a MOI of 0.1 (**Figure 6**). Five days later, surviving cells were counted. There were < or = 20% of glioma cells alive and > or = 80% normal cells alive at this time point. The sum of these experiments thus established the relative selectivity in replication and toxicity of rQNestin34.5v.2 against human glioma vs. normal cells.

We then employed an orthotopic model of human glioma growth in athymic mice. For this, we utilized human U87dEGFR glioma cells that usually establish after a few days of intracerebral inoculation and lead to animal death by 3-4 weeks. In the first experiment, rQNestin34.5v.2 was inoculated seven days after tumor cell inhection (**Figure 7A**), while in the second the virus was injected 14 days later, as animals become symptomatic from tumor growth (**Figure 7B**). In both cases, the virus was injected at a dose of 3 x 105 pfus. In both, there was a significant increase in mouse survival, indicating that rQNestin34.5v.2 was an effective antiglioma agent in this mouse model.

Finally, we tested the relative safety of rQNestin34.5v.2 by direct intracerebral inoculation into the brains of Balb/c mice that are relatively sensitive to HSV-induced encephalitis/meningitis
Table 1 in **Figure 8** summarizes the results of all experiments to date with Balb/c mice. Intracerebral injection of 10⁷ pfus in either 8 week old or 6 month old mice was well tolerated (32/33, with one death at day 3 post-injection for unknown reasons). In contrast, F strain caused death in 5/animals at 6, 6, 7, 8, and 12 days after injection. Intrathecal, intrahepatic and intravenous injections of rQNetsin34.5v.2 were also well tolerated without deaths at a dose of 10⁷ pfus, although F strain also did not cause death by these routes.

The sum of these experiments thus indicates that rQNestin34.5v.2 is selectively toxic for human glioma cells vs. normal cells including astrocytes, leads to a prolongation of survival in animal models of gliomas at a dose of 3 x 10⁵ pfus, and 97% of Balb/c mice survive an intracranial injection at a dose of 10⁷ pfus, while less than 30% of mice survive an intracranial injection of wild-type F strain at a dose of 10⁵ pfus.

### Brief description of cloning to remove GFP region

We generated PCR-del-GFP-FRT-Gm-F&R DNA (SEQ ID NO: 8) by PCR amplification. We then performed homologous recombination of this PCR product with fHSVQuik-1 BAC vector using ET-recombination technique, resulting in the replacement of **Figure 15** region schematic (SEQ ID NO: 7) to **Figure 16** schematic, sequence (SEQ ID NO: 8). We then Transformed Flp-T vector in *E.coli* containing above BAC vector to remove the region surrounded by two FRT sites, one of which locates in original fHSVQuik-1 BAC vector. This resultant vector is called fHSVQuik-2.

### In vivo replication

Replication of rQNestin34.5v.2 (v2) was analyzed in 5 glioma stem cells (brain tumor initiating cells - BTIC) freshly generated from humans (G35, G68, G97, OG02, X12). HSVQ1 (Q1) an ICP34.5 deleted virus showed no or minimal replication. F (wild-type HSV) showed the most replication. 34C is an unrelated HSV recombinant HSV. We utilize athymic mice (nu/nu) where implantable human glioma cells (human U87EGFR or Gli36dEGFR) are grown. This model has been widely utilized by us and others to monitor efficacy. These tumors reliably form in animal brains leading to their death within 3-4 weeks. These cells are histologically similar to human glioma cells and the tumors are highly vascular like the clinical tumors. The major difference is that they are not as invasive as the clinical gliomas. We have also shown efficacy in glioma cells freshly excised from humans with tumors and grown to enrich for the glioma stem-like cell population (**Figure 9**).

Injected mice have been kept alive for at least 60 days. In addition, we have conducted biodistribution studies in athymic and Balb/c mice after intracerebral (i.c.) , intravenous (i.v.) or intrathecal (i.t.) inoculation of rQNestin34.5v.2 or F (wild-type) HSv1 strain to detect a viral transcript (LAT) by RT-PCR, expressed during both lytic and latent phases of the viral life cycle. **Figure 10** shows that the transcript was not detectable in the brain or trigeminal ganglia of either Nu/nu or Balb/c mice 60 days after i.c., i.t. or i.c, routes of administration of 10⁷ pfus of rQNestin34.5v.2. In contrast, there were some faint bands in all tested brain and trigeminal ganglia samples at the 30 and 60 day time point after i.c. administration of 10³ pfus of F strain.

In addition, we performed PCR for the HSV DNA polymerase gene. **Figure 11** shows that copies of the gene were not detected in brains or trigeminal ganglia of mice after rQNetsin34.5v.2, indicating lack of active viral replication. In contrast, there was faint detection of DNA pol after injection of F strain, indicating some low level of DNA replication, particularly in brain.

### Example 3 Utilization of a nestin Transcriptional enhancer/promoter

Nestin expression in human brain adjacent to gliomas and in human brain after treatment.

Amongst the several levels of tumor cell selectivity for rQNestin34.5v.2, one of significance, the nestin transcriptional element is composed of the nestin-hsp minimum enhancer/promoter sequence, derived from a the second intron/enhancer of the rat nestin gene enhancer fused to the mouse hsp68 promoter to provide specific transcriptional regulation to cells expressing nestin, including human cells. This construct provides selective expression of the viral ICP34.5 gene to nestin-expressing cells in the brain. The presence or absence of nestin in human brain adjacent to a malignant glioma or in human brain after malignant glioma treatment was confirmed by Nestin IHC in human brain adjacent to gliomas and in human brain after treatment.

An adult male older than 50 years underwent resection of a malignant glioma at the Ohio State University Medical Center. As part of the resection, brain adjacent to tumor that was devoid of gross tumor was also resected up to the lateral ventricle. This brain was stained for expression of GFAP (**Figure 17A**) and Nestin (**Figure 17B-17D**) at the Ohio State University Medical Center Neuropathology Core. A second subject was also over 50 and had a malignant glioma resected and then underwent radiation and chemotherapy. He passed away from causes other than his tumor (**Figure 18**)**.** The primary antibody was Nestin from Millipore at a 1:500 dilution. Deparaffization and dehydration were performed as per routine. Antigen was exposed with standard heat -induced epitope retrieval methods. Slides were immersed in Target Retrieval Solution PH9 (DAKO) and microwaved until the solution came to a boil. Boiling was then carried out for 15min with lower power. After cooling the solution for about 30min, slides were rinsed in PBS. To inhibit internal peroxidase, slides were immerse in methanol with 0.3%H2O2 for 15min and then rinses in PBS-T. Blocking was carried out for 1h at room temperature, before applying the primary antibody to human Nestin (Millipore) 1:500 at 4 °C overnight. The secondary antibody was then applied at 1:500 dilution, followed by DAPI staining and mounting of sections on slides. At autopsy, brain showed little if any nestin immunoreactivity. There was no Nestin immunopositivity in brain or in cells of the ependyma/subependyma within the subventricular zone (SVZ), where neural stem cells usually reside. Therefore the evidence shows that a target population for a clinical trial (human adults with MG) exhibit little if any evidence for nestin expression in brain white matter where the MG usually resides or in the SVZ where neural stem cells are found. Instead, published evidence shows that the MG exhibits high levels of widespread nestin immunoreactivity.

### Athymic mice studies related to nestin expression in brain.

To determine whether there was nestin expression in the brains of athymic mice, since these were the species selected for efficacy and toxicology/biodistribution studies, male and female athymic mice (6-8 week of age) were inoculated intracerebrally with the agent, rQNestin34.5v.2. However, one control group of mice (group 1) was inoculated intracerebrally with vehicle (PBS) alone. At day 4 after this PBS inoculation, mice were euthanized per protocol for brain analyses. It was determined whether there was nestin expression in the brain of one of these mice. **Figure 19** shows nestin positive cells in tanicytes (ependymal cells) lining the lateral and third ventricle as well as the aqueduct. There was also nestin positivity in cells lining the fourth ventricle. The same experiment was performed with an animal from group 2 (preadministration of CPA followed by injection of PBS vehicle), to confirm the same pattern of nestin expression. In addition, nestin expression was seen in astrocytes around the needle tract, indicating up-regulation of nestin in reactive astrocytes in mice.

### Example 4 In vitro characterization of rQNestin34.5v.2.

The viral yield of rQNestin34.5v.2. was determined. Cells (2 x 10⁵) were plated in 6-well plates. The following day, cells were infected with rQNestin34.5v.2 (v2), parental rHSVQ1 (Q1), or wild-type F strain (F) at MOI = 0.1. One hour after infection, cells were washed with glycine saline solution (10 mM glycine, 137 mM NaCl, 24.1 mM KCl, 0.49 mM MgCl2. 0.68 mM CaCl2, pH 3) followed by PBS to remove unattached viruses and fresh medium were added. Cells were incubated for 3 days at 37 °C in an atmosphere containing 5% CO2. The cells and medium were collected, and subjected to three cycles of freeze/thaw with dry ice/Ethanol and 37 °C water bath. After pelleting cell debris by centrifugation (35000 x g, 10 min., 4 °C), supernatant was transferred to new tubes and stored at - 80 °C until titration. The titer of each sample was determined by conventional plaque assay with Vero cells. **Figure 20** shows that the viral yield of rQnestin34.5v.2 was equivalent to that of rQNestin34.5 in 3 glioma cells, including a glioma stem-like cell and superior to that in HUVEC cells. The replication potential of rQNestin34.5v.2. was determined in multiple cell lines. **Figure 21** shows that rQNestin34.5v.2 replication was higher than that of the ICP34.5-negative rHSVQ1 in 4 established glioma cell lines and 3 primary gliomas grown under stem-like condition, but similar to rHSVQ1 in 4 normal cells. F strain replication was higher in all.

The cytotoxicity of rQnestin34.5v.2 was determined. rQNestin34.5v.2 was added to a panel of glioma cells, U87ΔEGFR (U87dE), U87, U251 and OG02 glioma "stem-like" cells and to a panel of normal cells, human astrocytes (HA), human fibroblasts (Fibro.), human smooth muscle (SM), human skeletal muscle cells (SkM) and mouse astrocytes (MA). Cells were seeded on 6-well plates in complete medium prepared by following manufacturer's instructions for normal primary cells, BTSC medium for primary glioma cells or DMEM supplemented with 2% FBS for glioma cell lines and allowed to adhere. The medium for normal cells were changed to basal medium a few hours after cell preparation. Next day, viruses were added at MOI=0.1. rQNestin34.5v.2 inactivated with UV radiation was used as mock control. One hour after infection, cells were washed with glycine saline solution (10 mM glycine, 137 mM NaCl, 24.1 mM KCl, 0.49 mM MgCl2. 0.68 mM CaCl2, pH 3) followed by PBS to remove unattached viruses and fresh medium were added. Cells were incubated at 37 °C in an atmosphere containing 5% CO2. Five days after infection virus cytotoxicity was measured as surviving cells counted with a Coulter counter (Beckman Coulter). **Figure 22** shows the cytotoxity of of rQnestin34.5v.2 and rQnestin34.5.

A clinical trial is planned that will be performed as a dose-escalation study. Clinical outcome will be asssesed (overall survival after injection, progression-free survival after injection), radiologic outcome (regression of tumor visualized on MRI), and tissue analysis of viral biodistribution studied. The trial will be performed as a dose-escalation, starting at 10⁸ pfus (1ml volume, multiple injection sites). This dose was selected because 10⁷ pfus appeared to be safe in a mouse brain. Since a mouse brain weighs about 1 gram, while the human barin weighs 1500 grams, this would translate to a safe dose of 5 x 10¹⁰ in a human. To further ensure safety, we thus will start by almost 3 logs less in humans. Dose-escalations will proceed by half-a -log up to 10¹⁰ pfus.

The maximum tolerated dose (MTD) will be defined as the dose one half-log order less than the dose level at which one-third of the patients have a dose limiting toxicity (DLT) of grade 3 or grade 4 (defined below) related to the administration of rQNestin34.5v.2. Cohorts of three patients will be administered with be escalated by half-log increments at each dose level until a dose limiting toxicity (DLT) is reached. If the MTD is not reached, the phase I dose will be the highest dose reached

A DLT will consist of: ' Any Grade 4 or 5 toxicity on the Common Terminology Criteria for Adverse Events v4.0 (CTCAE) attributed to rQNestin34.5v.2, except for Grade 4 lymphocyte, neutrophil, white blood count decrease on the investigation category of CTCAE v4.0. ' Grade 3 toxicity for encephalitis, encephalomyelitis, meningitis infections/infestations categories on CTCAE v4.0 attributed to rQNestin34.5v.2. ' Grade 3 toxicity for Nervous System disorder category on the CTCAE v4.0 relative to the changes from the pre-treatment neurological status attributed to rQNestin34.5v.2: ataxia, depressed level of consciousness, encephalopathy, extrapyramidal disorder, hydrocephalus, intracranial hemorrhage, leukoencephalopathy, myelitis, pyramidal tract syndrome, somnolence, stroke. ' Grade 3 toxicity for Psychiatric disorder category on CTCAE v4.0 relative to changes from the pre-treatment status attributed to rQNestin34.5v.2: delirium, hallucinations, psychosis.

The first patient in each cohort will be observed for at least 10 days following injection with rQNestin34.5v.2 prior to the next patient being enrolled into the study protocol. If there is no DLT, then the second and third patient will be accrued at the same dose. Patients will only be enrolled into the next dose level if 1/3 DLT is not reached based on CTCAE v.4.

The determination of DLT and MTD will be separate for arm A and arm B since the gliomatous mass will have been largely resected in the former and not the latter and, thus, may lead to toxicities at different doses.

The various methods and techniques described above provide a number of ways to carry out the application. Of course, it is to be understood that not necessarily all objectives or advantages described can be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as taught or suggested herein. A variety of alternatives are mentioned herein. It is to be understood that some preferred embodiments specifically include one, another, or several features, while others specifically exclude one, another, or several features, while still others mitigate a particular feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be employed in various combinations by one of ordinary skill in this art to perform methods in accordance with the principles described herein. Among the various elements, features, and steps some will be specifically included and others specifically excluded in diverse embodiments.

Although the application has been disclosed in the context of certain embodiments and examples, it will be understood by those skilled in the art that the embodiments of the application extend beyond the specifically disclosed embodiments to other alternative embodiments and/or uses and modifications and equivalents thereof.

In some embodiments, the terms "a" and "an" and "the" and similar references used in the context of describing a particular embodiment of the application (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (for example, "such as") provided with respect to certain embodiments herein is intended merely to better illuminate the application and does not pose a limitation on the scope of the application otherwise claimed. No language in the specification should be construed as indicating any non-claimed element essential to the practice of the application.

Other than in the operating examples, or where otherwise indicated, all numbers expressing quantities of ingredients or reaction conditions used herein should be understood as modified in all instances by the term "about." The term "about" when used in connection with percentages can mean ±1%.

As used herein the term "comprising" or "comprises" is used in reference to compositions, methods, and respective component(s) thereof, that are essential to the invention, yet open to the inclusion of unspecified elements, whether essential or not.

As used herein the term "consisting essentially of" or "consists essentially of" refers to those elements required for a given embodiment. The term permits the presence of additional elements that do not materially affect the basic and novel or functional characteristic(s) of that embodiment of the invention.

The term "consisting of" refers to compositions, methods, and respective components thereof as described herein, which are exclusive of any element not recited in that description of the embodiment.

Preferred embodiments of this application are described herein, including the best mode known to the inventors for carrying out the application. Variations on those preferred embodiments will become apparent to those of ordinary skill in the art upon reading the foregoing description. It is contemplated that skilled artisans can employ such variations as appropriate, and the application can be practiced otherwise than specifically described herein. Accordingly, many embodiments of this application include all modifications and equivalents of the subject matter recited in the claims appended hereto as permitted by applicable law. Moreover, any combination of the above-described elements in all possible variations thereof is encompassed by the application unless otherwise indicated herein or otherwise clearly contradicted by context.

### SEQUENCES

**SEQ ID NO: 1** rQNestin34.5v.2Sequence, consensus sequence_BreakPoint_151409 
ATAACTTCGTATAATGTATGCTATACGAAGTTATTAGGTCCCTCGACCTGCAGGAAACTCTAGTCC
GGACCCGGGAGGCCTCCTTTGAGGAGTGGCTGCGATCCAAGGAAGTGGCCCTGGACTTTGGCCTGACGGAAAGGCTTC
GCGAGCACGAAGCCCAGCTGGTGATCCTGGCCCAGGCTCTGGACCATTACGACTGTCTGATCCACAGCACACCGCACA
CGCTGGTCGAGCGGGGGCTGCAATCGGCCCTGAAGTATGAGGAGTTTTACCTAAAGCGCTTTGGCGGGCACTACATGG
AGTCCGTCTTCCAGATGTACACCCGCATCGCCGGCTTTTTGGCCTGCCGGGCCACGCGCGGCATGCGCCACATCGCCC
TGGGGCGAGAGGGGTCGTGGTGGGAAATGTTCAAGTTCTTTTTCCACCGCCTCTACGACCACCAGATCGTACCGTCGA
CCCCCGCCATGCTGAACCTGGGGACCCGCAACTACTACACCTCCAGCTGCTACCTGGTAAACCCCCAGGCCACCACAA
ACAAGGCGACCCTGCGGGCCATCACCAGCAACGTCAGTGCCATCCTCGCCCGCAACGGGGGCATCGGGCTATGCGTGC
AGGCGTTTAACGACTCCGGCCCCGGGACCGCCAGCGTCATGCCCGCCCTCAAGGTCCTCGACTCGCTGGTGGCGGCGC
ACAACAAAGAGAGCGCGCGTCCGACCGGCGCGTGCGTGTACCTGGAGCCGTGGCACACCGACGTGCGGGCCGTGCTCC
GGATGAAGGGGGTCCTCGCCGGCGAAGAGGCCCAGCGCTGCGACAATATCTTCAGCGCCCTCTGGATGCCAGACCTGT
TTTTCAAGCGCCTGATTCGCCACCTGGACGGCGAGAAGAACGTCACATGGACCCTGTTCGACCGGGACACCAGCATGT
CGCTCGCCGACTTTCACGGGGAGGAGTTCGAGAAGCTCTACCAGCACCTCGAGGTCATGGGGTTCGGCGAGCAGATAC
CCATCCAGGAGCTGGCCTATGGCATTGTGCGCAGTGCGGCCACGACCGGGAGCCCCTTCGTCATGTTCAAAGACGCGG
TGAACCGCCACTACATCTACGACACCCAGGGGGCGGCCATCGCCGGCTCCAACCTCTGCACCGAGATCGTCCATCCGG
CCTCCAAGCGATCCAGTGGGGTCTGCAATCTGGGAAGCGTGAATCTGGCCCGATGCGTCTCCAGGCAGACGTTTGACT
TTGGGCGGCTCCGCGACGCCGTGCAGGCGTGCGTGCTGATGGTGAACATCATGATCGACAGCACGCTACAACCCACGC
CCCAGTGCACCCGCGGCAACGACAACCTGCGGTCCATGGGAATCGGCATGCAGGGCCTGCACACGGCCTGCCTGAAGC
TGGGGCTGGATCTGGAGTCTGCCGAATTTCAGGACCTGAACAAACACATCGCCGAGGTGATGCTGCTGTCGGCGATGA
AGACCAGCAACGCGCTGTGCGTTCGCGGGGCCCGTCCCTTCAACCACTTTAAGCGCAGCATGTATCGCGCCGGCCGCT
TTCACTGGGAGCGCTTTCCGGACGCCCGGCCGCGGTACGAGGGCGAGTGGGAGATGCTACGCCAGAGCATGATGAAAC
ACGGCCTGCGCAACAGCCAGTTTGTCGCGCTGATGCCCACCGCCGCCTCGGCGCAGATCTCGGACGTCAGCGAGGGCT
TTGCCCCCCTGTTCACCAACCTGTTTAGCAAGGTGACCCGGGACGGCGAGACGCTGCGCCCCAACACGCTCCTGCTAA
AGGAACTGGAACGCACGTTTAGCGGGAAGCGCCTCCTGGAGGTGATGGACAGTCTCGACGCCAAGCAGTGGTCCGTGG
CGCAGGCGCTCCCGTGCCTGGAGCCCACCCACCCCCTCCGGCGATTCAAGACCGCGTTTGACTACGACCAGAAGTTGC
TGATCGACCTGTGTGCGGACCGCGCCCCCTACGTCGACCATAGCCAATCCATGACCCTGTATGTCACGGAGAAGGCGG
ACGGGACCCTCCCAGCCTCCACCCTGGTCCGCCTTCTGGTCCACGCATATAAGCGCGGACTAAAAACAGGGATGTACT
ACTGCAAGGTTCGCAAGGCGACCAACAGCGGGGTCTTTGGCGGCGACGACAACATTGTCTGCACGAGCTGCGCGCTGT
GACCGACAAACCCCCTCCGCGCCAGGCCCGCCGCCACTGTCGTCGCCGTCCCACGCGCTCCCCCGCTGCCATGGATTC
CGCGGCCCCAGCCCTCTCCCCCGCTCTGACGGCCCATACGGGCCAGAGCGCGCCGGCGGACCTGGCGATCCAGATTCC
 
AAAGTGCCCCGACCCCGAGAGGTACTTCTACACCTCCCAGTGTCCCGACATTAACCACCTGCGCTCCCTCAGCATCCT
TAACCGCTGGCTGGAAACCGAGCTTGTTTTCGTGGGGGACGAGGAGGACGTCTCCAAGCTTTCCGAGGGCGAGCTCAG
CTTTTACCGCTTCCTCTTCGCTTTCCTGTCGGCCGCCGACGACCTGGTTACGGAAAACCTGGGCGGCCTCTCCGGCCT
GTTTGAGCAGAAGGACATTCTCCACTACTACGTGGAGCAGGAATGCATCGAAGTCGTACACTCGCGCGTGTACAACAT
CATCCAGCTGGTGCTTTTTCACAACAACGACCAGGCGCGCCGCGAGTACGTGGCCGGCACCATCAACCACCCGGCCAT
CCGCGCCAAGGTGGACTGGTTGGAAGCGCGGGTGCGGGAATGCGCCTCCGTTCCGGAAAAGTTCATTCTCATGATCCT
CATCGAGGGCATCTTTTTTGCCGCCTCGTTTGCCGCCATCGCCTACCTTCGCACCAACAACCTTCTGCGGGTCACCTG
CCAGTCAAACGACCTCATCAGCCGGGACGAGGCCGTGCACACGACGGCCTCGTGTTACATCTACAACAACTACCTCGG
CGGGCACGCCAAGCCCCCGCCCGACCGCGTGTACGGGCTGTTCCGCCAGGCGGTCGAGATCGAGATCGGATTTATCCG
ATCCCAGGCGCCGACGGACAGCCATATCCTGAGCCCGGCGGCGCTGGCGGCCATCGAAAACTACGTGCGATTCAGCGC
GGATCGCCTGTTGGGCCTTATCCACATGAAGCCACTGTTTTCCGCCCCACCCCCCGACGCCAGCTTTCCGCTGAGCCT
CATGTCCACCGACAAACACACCAATTTTTTCGAGTGTCGCAGCACCTCCTACGCCGGGGCGGTCGTCAACGATCTGTG
AGGGTCGCGGCGCGCTTCTACCCGTGTTTGCCCATAATAAACCTCTGAACCAAACTTTGGGTCTCATTGTGATTCTTG
TCAGGGACGCGGGGGTGGGAGAGGATAAAAGGCGGCGCAAAAAGCAGTAACCAGGTCCGGCCAGATTCTGAGGGCATA
GGATACCATAATTTTATTGGTGGGTCGTTTGTTCGGGGACAAGCGCGCTCGTCTGACGTTTGGGCTACTCGTCCCAGA
ATTTGGCCAGGACGTCCTTGTAGAACGCGGGTGGGGGGCCTGGGTCCGCAGCTGCTCCAGAAACCTGTCGGCGATAT
CAGGGGCCGTGATATGCCGGGTCACGATAGATCGCGCCAGGTTTTCGTCGCGGATGTCCTGGTAGATAGGCAGGCGTT
TCAGAAGAGTCCACGGCCCCCGCTCCTTGGGGCCGATAAGCGATATGACGTACTTAATGTAGCGGTGTTCCACCAGCT
CGGTGATGGTCATGGGATCGGGGAGCCAGTCCAGGGACTCTGGGGCGTCGTGGATGACGTGGCGTCGCCGGCTGGCCA
CATAACTGCGGTGCTCTTCCAGCAGCTGCGCGTTCGGGACCTGGACGAGCTCGGGCGGGGTGAGTATCTCCGAGGAGG
ACGACCTGGGGCCGGGGTGGCCCCCGGTAACGTCCCGGGGATCCAGGGGGAGGTCCTCGTCGTCTTCGTATCCGCCGG
CGATCTGTTGGGTTAGAATTTCGGTCCACGAGACGCGCGTCTCGGTGCCGCCGGTGGCCGGCGGCAGAGGGGGCCTGG
TTTCCGTGGAGCGCGAGCTGGTGTGTTCCCGGCGGATGGCCCGCCGGGTCTGAGAGCGACTCGGGGGGGTCCAGTGAC
ATTCGCGCAGCACATCCTCCACGGAGGCGTAGGTGTTATTGGGATGGAGGTCGGTGTGGCAGCGGACAAAGAGGGCCA
GGAACTGGGGGTAGCTCATCTTAAAGTACTTCAGTATATCGCGACAGTTGATCGTGGGAATGTAGCAGGCGCTAATAT
CCAACACAATATCGCAGCCCATCAACAGGAGGTCAGTGTCCGTGGTGTACACGTACGCGACCGTGTTGGTGTGATAGA
GGTTGGCGCAGGCATCGTCCGCCTCCAGCTGACCCGAGTTAATGTAGGCGTACCCCAGGGCCCGGAGAACGCGAATAC
AGAACAGATGCGCCAGACGCAGGGCCGGCTTCGAGGGCGCGGCGGACGGCAGCGCGGCTCCGGACCCGGCCGTCCCCC
GGGTCCCCGAGGCCAGAGAGGTGCCGCGTCGGCGCATGTTGGAAAAGGCAGAGCTGGGTCTGGAGTCGGTGATGGGGG
AAGGCGGTGGAGAGGCGTCCACGTCACTGGCCTCCTCGTCCGTCCGGCACTGGGCCGTCGTGCGGGCCAGGATGGCCT
TGGCTCCAAACACAACCGGCTCCATACAATTGACCCCGCGATCGGTAACGAAGATGGGGAAAAGGACTTTTGGGTAA
ACACTTTTAATAAGCGACAGAGGCAGTGTAGCGTAATGGCCTCGCGGTCGTAACTGGGGTATCGGCGCTGATATTTGA
CCACCAACGTGTACATGACGTTCCACAGGTCCACGGCAATGGGGGTGAAGTACCCGGCCGGGGCCCCAAGGCCCCGGC
GCTTGACCAGATGGTGTGTGTGGGCAAACTTCATCATCCCGAACAAACCCATGTCAGGTCGATTGTAACTGCGGATCG
GCCTAACTAAGGCGTGGTTGGTGCGACGGTCCGGGACACCCGAGCCTGTCTCTCTGTGTATGGTGACCCAGACAACAA
CACCGACACAAGAGGACAATAATCCGTTAGGGGACGCTCTTTATAATTTCGATGGCCCAACTCCACGCGGATTGGTGC
AGCACCCTGCATGCGCCGGTGCGGGCCAACCTTCCCCCCGCTCATTGCCTCTTCCAAAAGGGTGTGGCCTAACGAGCT
GGGGGCGTATTTAATCAGGCTAGCGCGGCGGGCCTGCCGTAGTTTCTGGCTCGGTGAGCGACGGTCCGGTTGCTTGGG
 
TCCCCTGGCTGCCATCAAAACCCCACCCTCGCAGCGGCATACGCCCCCTCCGCGTCCCGCACCCGAGACCCCGGCCCG
GCTGCCCTCACCACCGAAGCCCACCTCGTCACTGTGGGGTGTTCCCAGCCCGCGTTGGGATGACGGATTCCCCTGGCG
GTGTGGCCCCCGCCTCCCACGTGGAGGACGCGTCGGACGCGTCCCTCGGGCAGCCGGAGGAGGGGCGCCCTGCCAGG
TGGTCCTGCAGGGCGCCGAGCTTAATGGAATCCTACAGGCGTTTGCCCCGCTGCGCACGAGCCTTCTGGACTCGCTTC
TGGTTATGGGAGACCGGGCATCCTTATCCATAACACGATCTTTGGGGAGCAGGTGTTCCTGTCCCTGGAACACTCGC
AATTCAGTCGGTATCGCTGGCGCGGACCCACGGCGGCGTTCCTGTCTCTCGTGGACCAGAAGCGCTCCCTCCTGAGCG
TGTTTCGCGCCAACCAGTACCCGGACCTACGTCGGGTGGAGTTGGCGATCACGGGCCAGGCCCCGTTTCGCACGCTGG
TTCAGCGCATATGGACGACGACGTCCGACGGCGAGGCCGTTGAGCTAGCCAGCGAGACGCTGATGAAGCGCGAACTGA
CGAGCTTTGTGGTGCTGGTTCCCCAGGGAACCCCCGACGTTCAGTTGCGCCTGACGAGGCCGCAGCTCACCAAGGTCC
TTAACGCGACCGGGGCCGATAGTGCCACGCCCACCATGTTCGAGCTCGGGGTTAACGGCAAATTTTCCGTGTTCACCA
CGAGTACCTGCGTCACATTTGCTGCCCGCGAGGAGGGCGTGTCGTCCAGCACCAGCACCCAGGTCCAGATCCTGTCCA
ACGCGCTCACCAAGGCGGGCCAGGCGGCCGCCAACGCCAAGACGGTGTACGGGGAAAATACCCATCGTACCTTCTCTG
TGGTCGTCGACGATTGCAGCATGCGGGCGGTGCTCCGGCGACTGCAGGTCGCCGGGGGCACCCTCAAGTTCTTCCTCA
CGACCCCCGTCCCCAGTCTGTGCGTCACCGCCACCGGTCCCAACGCGGTATCGGCGGTATTTCTCCTGAAACCCCAGA
AGATTTGCCTGGACTGGCTGGGTCATAGCCAGGGGTCTCCTTCCGCCGGGAGCTCGGCCTCCCGGGCCTCTGGGAGCG
AGCCAACAGACAGCCAGGACTCCGCGTCGGACGCGGTCAGCCACGGCGATCCGGAAGACCTCGATGGCGCTGCCCGGG
CGGGAGAGGCGGGGGCCTCGTACGCCTGTCCGATGCCGTCGTCGACCACGCGGGTCACTCCCACGACCAAGCGGGGGC
GCTCGGGGGGCGAGGATGCGCACGCGGACACGGCCCTAAAGAAACCTAAGACGGGGTCGCCCACCGCACCCCCGCCCG
CAGATCCAGTCCCCCTGGACACGGAGGACGACTCCGATGCGGCGGACGGGACAGCGGCCCGTCCCGCCGCTCCAGACG
CCCGAAGCGGAAGCCGTTACGCGTGTTACTTTCGCGACCTCCCGACCGGAGAAGCAAACCCCGGCGCCTTCTCCGCCT
TCCGGGGGGGCCCCCAAACCCCGTCTGGTTTTGGATTCCCCTGACGGGGCGGGGCCTTAGCGGCCGCCCAACCCTCGC
AACATCCCGGGGTTAATGTAAATAAACTTGGTATTGCCCAACACTCTCCCGCGTGTCGCGTGTGGTTCATGTGTGTGC
CTGGCGCCCCCACCCTCGGGTTCGTGTATTTCCTTTCCCTGTCCTTATAAAAGCCGTATGTGGGGCGCTGACGGAACC
ACCCCGCGTGCCATCACGGCCAAGGCGCGGGATGCTCCGCAACGACAGCCACCGGGCCGCGTCCCGGAGGACGGCCA
GGGACGGGTCGACGACGGACGGCCACACCTCGCGTGCGTGGGGGCCCTGGCGCGGGGGTTCATGCATATCTGGCTTCA
GGCCGCCACGCTGGGTTTTGCGGATCGGTCGTTATGTCGCGCGGGCCGTACGCGAATGCCGCGTCTGGGGCGTTCGC
CGTCGGGTGCGCCGTGTTGGGCTTTATGCGCGCGCCCCCTCCCCTCGCGCGGCCCACCGCGCGGATATACGCCTGGCT
CAAACTGGCGGCCGGTGGAGCGGCCCTTGTTCTGTGGAGTCTCGGGGAGCCCGGCACGCAGCCGGGGGCCCTGGCCCC
GGGCCCGGCCACCCAGTGCCTGGCGCTGGGCGCCGCCTATGCGGCGCTCCTGGTGCTCGCCGATGACGTCTATCCGCT
CTTTCTCCTCGCCCCGGGGCCCCTGTTCGTCGGCACCCTGGGGATGGTCGTCGGCGGGCTGACGATCGGAGGCAGCGC
GCGCTACTGGTGGATCGGTGGGCCCGCCGCGGCCGCCCTGGCCGCGGCGGTGTTGGCGGGCCCGGGGGCGACCACCGC
CAGGGACTGCTTCTCCAGGGCGTGCCCCGACCACCGCCGCGTCTGCGTCATCGTCGCAGGCGAGTCTGTTTCCCGCCG
CCCCCCGGAGGACCCAGAGCGACCCGGGGACCCAGGGCCACCGTCCCCCCCGACACCCCAACGATCCCAGGGGCCGCC
GGCCGATGAGGTCGCACCGGCCGGGGTAGCGCGGCCCGAAAACGTCTGGGTGCCCGTGGTCACCTTTCTGGGGGCTGG
CGCGCTCGCCGTCAAGACGGTGCGAGAACATGCCCGGGGAACGCCGGGCCCGGGCCTGCCGCTGTGGCCCCAGGTGTT
TCTCGGAGGCCATGTGGCGGTGGCCCTGACGGAGCTGTGTCAGGCGCTTGCGCCCTGGGACCTTACGGACCCGCTGCT
GTTTGTTCACGCCGGACTGCAGGTCATCAACCTCGGGTTGGTGTTTCGGTTTTCCGAGGTTGTCGTGTATGCGGCGCT
AGGGGGTGCCGTGTGGATTTCGTTGGCGCAGGTGCTGGGGCTCCGGCGTCGCCTGCACAGGAAGGACCCCGGGGACGG
 
GGCCCGGTTGGCGGCGACGCTTCGGGGCCTCTTCTTCTCCGTGTACGCGCTGGGGTTTGGGGTGGGGGCGCTGCTGTG
CCCTCCGGGGTCAACGGCGGGCGGTCGGCGATTGATATATTTTTCAATAAAAGGCATTAGTCCCGAAGACCGCCGG
TGTGTGATGATTTCGCCATAACACCCAAACCCCGGATGGGGCCCGGGTATAAATTCCGGAAGGGGACACGGGCTACCT
TCACTACCGAGGGCGCTTGGTCGGGAGGCCGCATCGAACGCACACCCCCATCCGGTGGTCCGTGTGGAGGTCGTTTTT
CATTGCCCGGTCTCGCTTTGCCGGGAACGCTAGCCGATCCCTCGCGAGGGGGAGGCGTCGGGCATGGCCCCGGGGCGG
GTGGGCCTTGCCGTGGTCCTGTGGAGCCTGGTGTGGCTCGGGGCGGGGGTGTCCGGGGGCTCGGAAACTGCCTCCACC
GGGCCCACGATCACCGCGGGAGCGGTGACGAACGCGAGCGAGGCCCCCACATCGGGGTCCCCCGGGTCAGCCGCCAGC
CCGGAGGTCACCCCCACATCGACCCCAAACCCCAACAATGTCACACAAAACCAAACCACCCCCACCGAGCCGGCCAGC
CCCCCAACAACCCCCAAGCCCACCTCCACACCCAAAAGCCCCCCCACGTCCACCCCCGACCCCAAACCCAAGAACAAC
ACCACCCCCGCCAAGTCGGACCGCCCCACTAAACCCCCCGGGCCCGTGTGGTGCGACCGCCGCGATTTATTGGCCCGG
TACGGCTCGCGGGTGCAGATCCGATGCCGGTTTCGGAATTCCACCCGCATGGAGTTCCGCCTCCAGATATGGCGTTAC
TCCATGGGTCCGTCCCCCCCAATCGCTCCGGCTCCCGACCTAGAGGAGGTCCTGACGAACATCACCGCCCCACCCGGG
GGACTCCTGGTGTACGACAGCGCCCCCAACCTAACGGACCCCCACGTGCTCTGGGCGGAGGGGGCCGGCCCGGGCGCC
GACCCTCCGTTGTATTCTGTCACCGGGCCGCTGCCGACCCAGCGGCTGATTATCGGCGAGGTGACGCCCGCGACCCAG
GGAATGTATTACTTGGCCTGGGGCCGGATGGACAGCCCGCACGAGTACGGGACGTGGGTGCGCGTCCGCATGTTCCGC
CCCCCGTCTCTGACCCTCCAGCCCCACGCGGTGATGGAGGGTCAGCCGTTCAAGGCGACGTGCACGGCCGCCGCCTAC
TACCCGCGTAACCCCGTGGAGTTTGTCTGGTTCGAGGACGACCACCAGGTGTTTAACCCGGGCCAGATCGACACGCAG
ACGCACGAGCACCCCGACGGGTTCACCACAGTCTCTACCGTGACCTCCGAGGCTGTCGGCGGCCAGGTCCCCCCGCGG
ACCTTCACCTGCCAGATGACGTGGCACCGCGACTCCGTGACGTTCTCGCGACGCAATGCCACCGGGCTGGCCCTGGTG
CTGCCGCGGCCAACCATCACCATGGAATTTGGGGTCCGGCATGTGGTCTGCACGGCCGGCTGCGTCCCCGAGGGCGTG
ACGTTTGCCTGGTTCCTGGGGGACGACCCCTCACCGGCGGCTAAGTCGGCCGTTACGGCCCAGGAGTCGTGCGACCGC
CCCGGGCTGGCTACGGTCCGGTCCACCCTGCCCATTTCGTACGACTACAGCGAGTACATCTGTCGGTTGACCGGATAT
CCGGCCGGGATTCCCGTTCTAGAGCACCACGGCAGTCACCAGCCCCCACCCAGGGACCCCACCGAGCGGCAGGTGATC
GAGGCGATCGAGTGGGTGGGGATTGGAATCGGGGTTCTCGCGGCGGGGGTCCTGGTCGTAACGGCAATCGTGTACGTC
GTCCGCACATCACAGTCGCGGCAGCGTCATCGGCGGTAACGCGAGACCCCCCCGTTACCTTTTTAATATCTATATAGT
TTGGTCCCCCTCTATCCCGCCCACCGCTGGGCGCTATAAAGCCGCCACCCTCTCTTCCCTCAGGTCATCCTTGGTCGA
TCCCGAACGACACACGGCGTGGAGCAAAACGCCTCCCCCTGAGCCGCTTTCCTACCAGCGCAACGGCATGCCTCTGCG
GGCATCGGAACACGCCTACCGGCCCCTGGGCCCCGGGACACCCCCCATGCGGGCTCGGCTCCCCGCCGCGGCCTGGGT
TGGCGTCGGGACCATCATCGGGGGAGTTGTGATCATTGCCGCGTTGGTCCTCGTGCCCTCGCGGGCCTCGTGGGCACT
TTCCCCATGCGACAGCGGATGGCACGAGTTCAACCTCGGGTGCATATCCTGGGATCCGACCCCCATGGAGCACGAGCA
GGCGGTCGGCGGCTGTAGCGCCCCGGCGACCCTGATCCCCCGCGCGGCTGCCAAACAGCTGGCCGCCGTCGCACGCGT
CCAGTCGGCAAGATCCTCGGGCTACTGGTGGGTGAGCGGAGACGGCATTCGGGCCTGCCTGCGGCTCGTCGACGGCGT
CGGCGGTATTGACCAGTTTTGCGAGGAGCCCGCCCTTCGCATATGCTACTATCCCCGCAGTCCCGGGGGCTTTGTTCA
GTTTGTAACTTCGACCCGCAACGCGCTGGGGCTGCCGTGAGGCGCGTGTACTGCGGTCTGTCTCGTCTCCTCTTCTCC
CCTTCCCTCCCCCTCCGCATCCCAGGATCACACCGGCCAACGAGGGTTGGGGGGTCCGGCACGGACCCAAAATAATAA
ACACACAATCACGTGCGATAAAAAGAACACGCGGTCCCCTGTGGTGTTTTTGGTTATTTTTATTAAATCTCGTCGTCA
AACAGGGGGAAAGGGGCGTGGTCTAGCGACGGCAGCACGGGTGGAGGCGTTCACCGGCTCCGGCGTCCTTCGCGTTTA
AGCTTGGTCAGGAGGGCGCTCAGGGCGGCGACGTTGGTCGGGCCGTCGTTGGTCAGGGCGTTGGCTCGATGGCGGGCG
 
AGGACGGGCGAGGGGCTCAACGGCGGGGGCGGGGCCCGGTGCGGCCCGGGGGGAAAATAGGGCGGATCCCCCCCAG
TCGTACAGGGGATTTCCGCCTCAATGTACGGGGAGGCCGGCGCTGCATTCGCCGTGTTCGCGCAGACGTTTTCGTAG
ACCCGCATCCATGGTATTTCCTCGTAGACACGCCCCCCGTCCTCGCTCACAGTCTCGTATATTGACTCGTCGTCCTCG
TAGGGGGCGTGCCGTTCGCGGGCCGAGGCGGCGTGGGTGGCTTTGCGGCGGGCGTCGTCGTCGTCGTCGGCCGTC
AGATACGTGGCTTCCATCTGGTCGGGTTCTCCCTCCGGGGCGGGTCCCCACCCCCGTGGCCGATCGAGGCTCCCCAGA
GACGCGCGCCGGACGAGGAGGGGGCACGTCGCCGCCGGCGGTCGCCTGTCGGGTCCCGCGACGTTACGGCCGGGAGG
CGCGGGGGCACCTCCCCCATGTGCGTGTAATACGTGGCCGGCTGTGCGGCCGCAGCGGGGGGCTCGGCGACCGGGTCG
TCCGCATCCGGAAGCGGGGGCGCCGCGCCGTCCGCGCGGCGCCTCCGGAACCGCCGGGTGGCCGCGGGGGTCGAGTGT
AGGCGAGGTCGGGGGAGGGGCGGGGGCTCGTTGTCGCGCCGCGCCCGCTGAATCTTTTCCCGACAGGTCCCACCCCCC
GCGCGATGCCCCCCCGGGCCGCGGGCCATGTCGTCCGGGGGAGGCCCCGCGGACCACGTCGTCCGGCGAGACGCCACG
AGCCGCAGGATGGACTCGTAGTGGAACGACGGCGCCCCGCTGCGGAGCAGATCCGCGGCCAGGGCGGCCCCGAACCAA
GCCTTGATGCTCAACTCCATCCGGGCCCAGCTGGGGGCGGTCATCGTGGGGAACAGGGGGGCGGTGGTCCGACAGAAA
CGCTCCTGGCTGTCCACCGCGGCCCGCAGATACTCGTTGTTCAGGCTGTCGGTGGCCCAGACGCCGTACCCGGTGAGG
GTCGCGTTGATGATATACTGGGCGTGGTGATGGACGATCGACAGAACCTCCACCGTGGATACGACGGTATCCACGGTC
CCGTACGTACCGCCGCTCCGCTTGCCGGTCTGCCACAGGTTGGCTAGGCGCGTCAGGTGGCCCAGGACGTCGCTGACC
GCCGCCCTGAGCGCCATGCACTGCATGGAGCCGGTCGTGCCGCTGGGACCCCGGTCCAGATGGCGCGCGAACGTTTCC
GCGGGCGCCTCCGGGCTGCCGCCGAGCGGGAGGAACCGGCGATTGGAGGGACTCAGCCGGTGGCATACGTGCTTGTCT
GTCGTCCACAGCATCCAGGACGCCCACCGGTACAGCACGGAGACGTAGGCCAGGAGCTCGTTGAGCCGCAGTGCGGTG
TCGGTGCTGGGGCGGCTTGGGTCCGCCGGGCGCATAAAGAACATGTACTGCTGAATCCGATGGAGGGCGTCGCGCAGG
CCGGCCACGGTGGCGGCGTACTTGGCCGCCGCGGCCCCGCTCTTGAACGGGGTGCGCGCCAGCAGCTTTGGCGCCAGG
GTGGGCCGCAGCAGCACGTGAAGGCTGGGGTCGCAGTCGCCCACGGGGTCCTCGGGGACGTCCAGGCCGCTGGGCACC
ACCGTCTGCAGGTACTTCCAGTACTGCGTGAGGATGGCGCGGCTCAACTGGCCGCCGGTGAGCTCCACCTCGCCCAGC
GCCTGGGTGGCGGCCGAAGCGTAGTGCCGGATGTACTCGTAGTGCGGGTCGCTGGCGAGCCCGTCCACGATCAAACTC
TCGGGAACCGTGTTGTGTTGCCGCGCGGCCAACCGGACGCTGCGATCGGTGCAGGTCAGAAACGCCGGCTGCGCGTCG
TCGGAGCGCTGCCGCAAGGCGCCCACGGCCGCGCTAAGGAGCCCCTCCGGGGTGGGGAGCAGACACCCGCCGAAGATG
CGCCGCTCGGGAACGCCCGCGTTGTCGCCGCGGATCAGGTTGGCAGGCGTCAGGCACCGCGCCAGCCGCAGGGAGCTC
GCGCCGCGCGTCCGGCGCTGCATGGTGACGCCCGTTCGGTCGGGACCCGCCGGTCGGAGTTATGCCGCGTCCAGGGCC
ATCGGGGCGCTTTTTATCGGGAGGAGCTTATGGGCGTGGCGGGCCTCCCAGCCCGGTCGCGCGCCTCCCCGACACGTG
CGCCCGCAGGGCGGCGGCCCCCTCGTCTCCCATCAGCAGTTTCCTAAACTGGGACATGATGTCCACCACGCGGACCCG
CGGGCCCAACACGGACCCGCCGCTTACGGGGGCGGGGGGAAGGGCTCCAGGTCCTTGAGAAGAAAGGCGGGGTCTGC
CGTCCCGGACACGGGGGCCCGGGGCGCTGAGGAGGCGGGGCGCAGATCCACGTGCTCCGCGGCCGCGCGGACGTCCGC
CCAGAACTTGGCGGGGGTGGTGCGCGCGTACAGGGGCTGGGTCGCTCGGAGGACGCACGCGTAGCGCAGGGGGGTGTA
TGTGCCCACCTCGGGGGCCGTGAATCCCCCGTCAAACGCGGCCAGTGTCACGCACGCCACCACGGTGTCGGCAAAGCC
CAGCAGCCGCTGCAGGACGAGCCCGGCGGCCAGAATGGCGCGCGTGGCCGCCGCGTCGTCCCGGCGCCGGTGCGCGTC
CCCGCACGCCCGGGCGTACTTTAAGGTCACGGTCGCCAGGGCCGTGTGCAGCGCGTACACCGCAGCGCCCAGCACGGC
GTTGAGCCCGCTGTTGGCGAGCAGCCGGCGCGCTGCGGTGTCGCCCAGCGCCTCGTGCTCGGCCCCCACGACCGCGGG
GCTTCCCAGGGGCAGGGCGCGAAACAGCTCCTCCCGCGCCACGTCCGCAAAGGCGGGGTGGTGCACGTGCGGGTGCAG
GCGCGCCCCCACGACCACCGAGAGCCACTGGACCGTCTGCTCCGCCATCACCGCCAGCACATCCAGCACGCGCCCCAG
 
GAAGGCGGCCTCCCGCGTCAAAACGCACCGGACGGCGTCGGATTGAAGCGGGCGAGCAGGGCCCCGGTGGCCAGGTA
CGTCATGCGGCCGGCATAGCGGGCGGCCACGCGACAGTCGCGGTCCAGCAGCGCGCGCACCCCGGGCCAGTACAGCAG
GGACCCCAGCGAGCTGCGAAACACCGCGGCGTCGGGGCCGGATTGGGGGACACTAACCCCCCCGCGCTCAGTAACGG
CACGGCCGCGGCCCCGACGGGACGCAACGCCGTGAGGCTCGCGAACTGCCGCCTCAGCTCGGCAGCCCTGTCGTCCAG
GTCCGACCCGCGCGCCTCTGCGTGAAGGCGCGTCCCGCACACCCACCCGTTGATGGCCAGCCGCACGACGGCATCCGC
CAAAAAGCTCATCGCCTGGGCGGGGCTGGTTTTGTTCGACGATCCATCAGGTCAAGAATCCCATCGCCCGTGATATA
CCAGGCCAACGCCTCGCCCTGCTGCAGGGTTTGGCGGAAAAACACCGCGGGGTTGTCGGGGGAGGCGAAGTGCATGAC
CCCCACGCGCGATAACCCGAACGCGCTATCCGGACACGGGTAAAACCCGGCCGGATGCCCCAGGGCTAGGGCGGAGCG
CACGGACTCGTCCCACACGGCAACCTGAGGGGCCAGTCGATCCAACGGGAATGCCGCCAGGAGCTCCGGCCCGGCAC
GCGTCCCTCCAGAACCTCCACCTTGGGCGGGGAACGGCCCCGCCGCCGTCCTCCGGCCCGACGTCTTCCGGGTAGTC
GTCCTCCTCGTACTGCAGCTCCTCTAGGAACAGCGGCGACGGCGCCACCCGCGAACCGCCGACCCGCCCCAAAATAGC
CCGCGCGTCGACGGGACCCAGGTATCCCCCCTGCCGGGCCTGCGGAGGACCGCGGGGAACCTCATCATCATCGTCCAG
GCGACCGCGCACCGACTGGCTACGGGCCGCATCGGGCCCGGGGCGCTGCCGGGACGCTCGGCGATGGGATGTGGGCGG
GGCTTCCGACGCGCGCCGTCGTCGGGCTCGCGGGCCTTCCCGTCGACGGCGCACGGGCGGCTCGTCGCCCGCCATCTC
CTCCAGAGCCTCTAGCTCGCTGTCGTCATCCCCGCGGAACACCGCACGCAGGTACCCCATGAACCCCACCCCATCGCC
CGCTGGCTCGTCCGCCACGGGCGAGGCGCGGGGGCGGGTGGATGCGCGCCTCCTACGCCCCGCGGGTTCGCGAGCCGA
CATGGTGGCGATAGACGCGGGTTATCGGATGTCCGCTACCCCCCAAAAAAGAAAAAGACCCCACAGCGCGGATGGAGG
CCGGGGTAGGTGCCGCCGGACCCCCTCGCGATGGGAATGGACGGGAGCGACGGGGCCGGCGCAAAAAACGCAGTATCT
CCCGCGAAGGCTACCCGCCGCCCCAGCCCCCGGCCAAATGCGGAAACGGTCCCGCGCTCTCGCCTTTATACGCGGGCC
GCCCTGCGACACAATCACCCGTCCGTGGTTTCGAATCTACACGACAGGCCCGCAGACGCGGCTAACACACACGCCGGC
AACCCAGACCCCAGTGGGTTGGTTGCGCGGTCCCGTCTCCTGGCTAGTTCTTTCCCCACCACCAAATAATCAGACGA
CAACCGCAGGTTTTTGTAATGTATGTGCTCGTGTTTATTGTGGATACGAACCGGGGACGGAGGGGAAAACCCAGACG
GGGGATGCGGGTCCGGTCGCGCCCCCTACCCACCGTACTCGTCAATTCCAAGGGCATCGGTAAACATCTGCTCAAACT
CGAAGTCGGCCATATCCAGAGCGCCGTAGGGGGCGGAGTCGTGGGGGGTAAATCCCGGACCCGGGGAATCCCCGTCCC
CCAACATGTCCAGATCGAAATCGTCTAGCGCGTCGGCATGCGCCATCGCCACGTCCTCGCCGTCTAAGTGGAGCTCGT
CCCCCAGGCTGACATCGGTCGGGGGGCCGTCGACAGTCTGCGCGTGTGTCCCGCGGGGAGAAAGGACAGGCGCGGAG
CCGCCAGCCCCGCCTCTTCGGGGGCGTCGTCGTCCGGGAGATCGAGCAGGCCCTCGATGGTAGACCCGTAATTGTTTT
TCGTACGCGCGCGGCTGTACGCGTGTTCCCGCATGACCGCCTCGGAGGGCGAGGTCGTGAAGCTGGAATACGAGTCCA
ACTTCGCCCGAATCAACACCATAAAGTACCCAGAGGCGCGGGCCTGGTTGCCATGCAGGGTGGGAGGGGTCGTCAACG
GCGCCCCTGGCTCCTCCGTAGCCGCGCTGCGCACCAGCGGGAGGTTAAGGTGCTCGCGAATGTGGTTTAGCTCCCGCA
GCCGGCGGGCCTCGATTGGCACTCCCCGGACGGTGAGCGCTCCGTTGACGAACATGAAGGGCTGGAACAGACCCGCCA
ACTGACGCCAGCTCTCCAGGTCGCAACAGAGGCAGTCAAACAGGTCGGGCCGCATCATCTGCTCGGCGTACGCGGCCC
ATAGGATCTCGCGGGTCAAAAATAGATACAAATGCAAAAACAGAACACGCGCCAGACGAGCGGTCTCTCGGTAGTACC
TGTCCGCGATCGTGGCGCGCAGCATTTCTCCCAGGTCGCGATCGCGTCCGCGCATGTGCGCCTGGCGGTGCAGCTGCC
GGACGCTGGCGCGCAGGTACCGGTACAGGGCCGAGCAGAAGTTGGCCAACACGGTTCGATAGCTCTCCTCCCGCGCCC
GTAGCTCGGCGTGGAAGAAACGAGAGAGCGCTTCGTAGTAGAGCCCGAGGCCGTCGCGGGTGGCCGGAAGCGTCGGGA
AGGCCACGTCGCCGTGGGCGCGAATGTCGATTTGGGCGCGTTCGGGGACGTACGCGTCCCCCCATTCCACCACATCGC
TGGGCAGCGTTGATAGGAATTTACACTCCCGGTACAGGTCGGCGTTGGTCGGTAACGCCGAAAACAAATCCTCGTTCC
 
AGGTATCGAGCATGGTACATAGCGCGGGCCCGCGCTAAAGCCCAAGTCGTCGAGGAGACGGTTAAAGAGGGCGGCGG
GGGGGACGGGCATGGGCGGGGAGGGCATGAGCTGGGCCTGGCTCAGGCGCCCCGTTGCGTACAGCGGAGGGGCCGCCG
GGGTGTTTTTGGGACCCCCGGCCGGGCGGGGGGGTGGTGGCGAAGCGCCGTCCGCGTCCATGTCGGCAAACAGCTCGT
CGACCAAGAGGTCCATTGGGTGGGGTTGATACGGGAAAGACGATATCGGGCTTTTGATGCGATCGTCCCCGCCCGCCC
AGAGAGTGTGGGACGCCCGACGGCGCGGGAAGAGAAAAACCCCCAAACGCGTTAGAGGACCGGACGGACCTTATGGGG
GGAAGTGGGCAGCGGGAACCCCGTCCGTTCCCGAGGAATGACAGCCCGTGGTCGCCACCCCGCATTTAAGCAACCCGC
ACGGGCCGCCCCGTACCTCGTGACTTCCCCCCACATTGGCTCCTGTCACGTGAAGGCGAACCGAGGGCGGCTGTCCAA
CCCACCCCCCGCCACCCAGTCACGGTCCCCGTCGGATTGGGAAACAAAGGCACGCAACGCCAACACCGAATGAACCCC
TGTTGGTGCTTTATTGTCTGGGTACGGAAGTTTTTCACTCGACGGGCCGTCTGGGGCGAGAAGCGGAGCGGGCTGGGG
CTCGAGGTCGCTCGGTGGGGCGCGACGCCGCAGAACGCCCTCGAGTCGCCGTGGCCGCGTCGACGTCCTGCACCACGT
CTGGATTCACCAACTCGTTGGCGCGCTGAAGCAGGTTTTTGCCCTCGCAGACCGTCACGCGGATGGTGGTGATGCCAA
GGAGTTCGTTGAGGTCTTCGTCTGTGCGCGGACGCGACATGTCCCAGAGCTGGACCGCCGCCATCCGGGCATGCATGG
CCGCCAGGCGCCCGACCGCGGCGCAGAAGACGCGCTTGTTAAAGCCGGCCACCCGGGGGGTCCATGGCGCGTCGGGGT
TTGGGGGGGCGGTGCTAAAGTGCAGCTTTCTGGCCAGCCCCTGCGCGGGTGTCTTGGATCGGGTTGGCGCCGTCGACG
CGGGGGCGTCTGGGAGTGCGGCGGATTCTGGCTGGGCCGATTTCCTGCCGCGGGTGGTCTCCGCCGCCGGGGCCGCGG
GGGCCTTAGTCGCCACCCGCTGGGTTCGGGGGGCCCGGGGGGCGGTGGTGGGTGTGCGTCCGGCCCCTCCGGACCCAG
CGGGCGGCGGAGGCGCCCGCGCAGGCCCCGGGCCGGACAAAACCGCCCCGGAAACGGGACGCCGCGTCCGGGGGACCT
CCGGGTGTTCGTCGTCTTCGGATGACGAGCCCCCGTAGAGGGCATAATCCGACTCGTCGTACTGGACGAAACGGACCT
CGCCCCTCGGGCGCGCGCGTGTCTGTAGGGCGCCACGGCGGGAGGTGGCAGGCGGACTATCGGGACTCGCCATACATG
AAGACGGGGTGTAGTACAGATCCTCGTACTCATCGCGCGGAACCTCCCGCGGACCCGACTTCACGGAGCGGCGAGAGG
TCATGGTTCCACGAACACGCTAGGGTCGGATGCGCGGACAATTAGGCCTGGGTTCGGACGGCGGGGGGTGGTGCAGGT
GTGGAGAGGTCGAGCGATAGGGGCGGCCCGGGAGAGAAGAGAGGGTCCGCAAAACCCACTGGGGATGCGTGAGTGGCC
CTCTGTGGGCGGTGGGGGAGAGTCTTATAGGAAGTGCATATAACCACAACCCATGGGTCTAACCAATCCCCAGGGGCC
AAGAAACAGACACGCCCCAAACGGTCTCGGTTTCCGCGAAGAAGGGGAAGTCCTGGGACACCCTCCACCCCCACCCCT
CACCCCACACAGGGCGGGTTCAGGCGTGCCCGGCAGCCAGTAGCCTCTGGCAGATCTGACAGACGTGTGCGATAATAC
ACACGCCCATCGAGGCCATGCCTACATAAAAGGGCACCAGGGCCCCGGGGGCAGACATTTGGCCAGCGTTTTGGGTCT
CGCACCGCGCGCCCCCGATCCCATCGCGCCCGCCCTCCTCGCCGGGCGGCTCCCCGTGCGGGCCCGCGTCTCCCGCCG
CTAAGGCGACGAGCAAGACAAACAACAGGCCCGCCCGACAGACCCTTCTGGGGGGGCCCATCGTCCCTAACAGGAAGA
TGAGTCAGTGGGGATCCGGGGCGATCCTTGTCCAGCCGGACAGCTTGGGTCGGGGGTACGATGGCGACTGGCACACGG
CCGTCGCTACTCGCGGGGGCGGAGTCGTGCAACTGAACCTGGTCAACAGGCGCGCGGTGGCTTTTATGCCGAAGGTCA
GCGGGGACTCCGGATGGGCCGTCGGGCGCGTCTCTCTGGACCTGCGAATGGCTATGCCGGCTGACTTTTGTGCGATTA
TTCACGCCCCCGCGCTATCCAGCCCAGGGCACCACGTAATACTGGGTCTTATCGACTCGGGGTACCGCGGAACCGTTA
TGGCCGTGGTCGTAGCGCCTAAAAGGACGCGGGAATTTGCCCCCGGGACCCTGCGGGTCGACGTGACGTTCCTGGACA
TCCTGGCGACCCCCCCGGCCCTCACCAAGCCGATTTCCCTGCGGCAGTTCCCGCAACTGGCGCCCCCCCCTCCAACCG
GGGCCGGGATACGCGCAGATCCTTGGTTGGAGGGGGCGCTCGGGGACCCAAGCGTGACTCCGGCCCTACCGGCGCGAC
GCCGAGGGCGGTCCCTCGTCTATGCCGGCGAGCTGACGCCGGTTCAGACGGAACACGGGGACGGCGTACGAGAAGCCA
TCGCCTTCCTTCCAAAACGCGAGGAGGATGCCGGTTTCGACATTGTCGTCCGTCGCCCGGTCACCGTCCCGGCAAACG
GCACCACGGTCGTGCAGCCATCCCTCCGCATGCTCCACGCGGACGCCGGGCCCGCGGCCTGTTATGTGTTGGGGCGGT
 
CGTCGCTCAACGCCCGCGGCCTCCTGGTCGTTCCTACGCGCTGGCTCCCCGGGCACGTATGTGCGTTTGTTGTTTACA
ACCTTACGGGGGTTCCTGTGACCCTCGAGGCCGGCGCCAAGGTCGCCCAGCTCCTGGTTGCGGGGGCGGACGCTCTTC
CTTGGATCCCCCCGGACAACTTTCACGGACCAAAGCGCTTCGAAACTACCCCAGGGGTGTTCCGGACTCAACCGCCG
AACCCAGGAACCCGCCGCTCTTGGTGTTTACGAACGAGTTTGACGCGGAGGCCCCCCCGAGCGAGCGCGGGACCGGGG
GTTTTGGCTCTACCGGTATTTAGCCCATAGCTTGGGGTTCGTTCCGGGCAATAAAAAACGTTTGTATCTCATCTTTCC
TGTGTGTAGTTGTTTCTGTTGGAGGCCTGTGGGTCTATCACACCCGCCCCTCCATCCCACAAACACAGAACACACGGG
TTGGATGAAAACACGCATTTATTGACCCAAAACACACGGAGCTGCTCGAGATGGGCCAGGGCGAGGTGCGGTTGGGGA
GGCTGTAGGTCTGGGAACGGACACGCGGGGACACGATTCCGGTTTGGGGTCCGGGAGGGCGTCGCCGTTTCGGGCGGC
AGGCGCCAGCGTAACCTCCGGGGGCGGCGTGTGGGGGTGCCCCAAGGAGGGCGCCTCGGTCACCCCAAGCCCCCCCAA
GCGGGTTCCCCCGGCAACCCCGAAGGCGGAGAGGCCAAGGGCCCGTTCGGCGATGGCCACATCCTCCATGACCACGTC
GCTCTCGGCCATGCTCCGAATAGCCTGGGAGACGAGCACATCCGCGGACTTGTCAGCCGCCCCCACGGACATGTACAT
CTGCAGGATGGTGGCCATACACGTGTCCGCCAGGCGCCGCATCTTGTCCTGATGGGCCGCCACGGCCCCGTCGATCGT
GGGGGCCTCGAGCCCGGGGTGGTGGCGCGCCAGTCGTTCTAGGTTCACCATGCAGGCGTGGTACGTGCGGGCCAAGGC
GCGGGCCTTCACGAGGCGTCGGGTGTCGTCCAGGGACCCCAGGGTGTCATCGAGCGTGATGGGGGCGGAAGTAGCGC
GTTAACGACCACCAGGGCCTCCTGCAGCCGCGGCTCCGCCTCCGAGGGCGGAACGGCCGCGCGGATCATCTCATATTG
TTCCTCGGGGCGCGCTCCCCAGCCACATATAGCCCCGAGAAGAGAAGCCATCGCGGGCGGGTACTGGCCCTTGGGCGC
GCGGACGCAATGGGGCAGGAAGACGGGAACCGCGGGGAGAGGCGGGCGGCCGGGACTCCCGTGGAGGTGACCGCGCTT
TATGCTACCGACGGGTGCGTTATTACCTCTTCGATCGCCCTCCTCACAAACTCTCTACTGGGGGCCGAGCCGGTTTAT
ATATTCAGCTACGACGCATACACGCACGATGGCCGTGCCGACGGGCCCACGGAGCAAGACAGGTTCGAAGAGAGTCGG
GCGCTCTACCAAGCGTCGGGCGGGCTAAATGGCGACTCCTTCCGAGTAACCTTTTGTTTATTGGGGACGGAAGTGGGT
GGGACCCACCAGGCCCGCGGGCGAACCCGACCCATGTTCGTCTGTCGCTTCGAGCGAGCGGACGACGTCGCCGCGCTA
CAGGACGCCCTGGCGCACGGGACCCCGCTACAACCGGACCACATCGCCGCCACCCTGGACGCGGAGGCCACGTTCGCG
CTGCATGCGAACATGATCCTGGCTCTCACCGTGGCCATCAACAACGCCAGCCCCCGCACCGGACGCGACGCCGCCGCG
GCGCAGTATGATCAGGGCGCGTCCCTACGCTCGCTCGTGGGGCGCACGTCCCTGGGACAACGCGGCCTTACCACGCTA
TACGTCCACCACGAGGCGCGCGTGCTGGCCGCGTACCGCAGGGCGTATTATGGAAGCGCGCAGAGTCCCTTCTGGTTT
CTTAGCAAATTCGGGCCGGACGAAAAAAGCCTGGTGCTCACCACTCGGTACTACCTGCTTCAGGCCCAGCGTCTGGGG
GGCGCGGGGGCCACGTACGACCTGCAGGCCATCAAGGACATCTGCGCCACCTACGCGATTCCCCACGCCCCCCGCCCC
GACACCGTCAGCGCCGCGTCCCTGACCTCGTTTGCCGCCATCACGCGGTTCTGTTGCACGAGCCAGTACGCCCGCGGG
GCCGCGGCGGCCGGGTTTCCGCTTTACGTGGAGCGCCGTATTGCGGCCGACGTCCGCGAGACCAGTGCGCTGGAGAAG
TTCATAACCCACGATCGCAGTTGCCTGCGCGTGTCCGACCGTGAATTCATTACGTACATTTACCTGGCCCATTTTGAG
TGTTTCAGCCCCCCGCGCCTAGCCACGCATCTTCGGGCCGTGACGACCCACGACCCCAACCCCGCGGCCAACACGGAG
CAGCCCTCGCCCCTGGGCAGGGAGGCCGTGGAACAATTTTTTGCCACGTGCGCGCCCAACTGAATATCGGGGAGTAC
GTCAAACACAACGTGACCCCCCGGGAGACCGTCCTGGATGGCGATACGGCCAAGGCCTACCTGCGCGCTCGCACGTAC
GCGCCCGGGGCCCTGACGCCCGCCCCCGCGTATTGCGGGGCCGTGGACTCCGCCACCAAAATGATGGGGCGTTTGGCG
GACGCCGAAAAGCTCCTGGTCCCCCGCGGGTGGCCCGCGTTTGCGCCCGCCAGTCCCGGGGAGGATACGGCGGGCGGC
ACGCCGCCCCCACAGACCTGCGGAATCGTCAAGCGCCTCCTGAGACTGGCCGCCACGGAACAACAGGACACCACGCCC
CCGGCGATCGCGGCGCTTATCCGTAATGCGGCGGTGCAGACTCCCCTGCCCGTCTACCGGATATCCATGGTCCCCACG
GGACAGGCATTTGCCGCGCTGGCCTGGGACGACTGGGCCCGCATAACGCGGGACGCTCGCCTGGCCGAAGCGGTCGTG
 
TCCGCCGAAGCGGCGGCGCACCCCGACCACGGCGCGCTGGGCAGGCGGCTCACGGATCGCATCCGCGCCCAGGGCCCC
GTGATGCCCCCTGGCGGCCTGGATGCCGGGGGCAGATGTACGTGAATCGCAACGAGATATTCAACGGCGCGCTGGCA
ATCACAAACATCATCCTGGATCTCGACATCGCCCTGAAGGAGCCCGTCCCCTTTCGCCGGCTCCACGAGGCCCTGGGC
CACTTTAGGCGCGGGGCTCTGGCTGCGGTTCAGCTCCTGTTTCCCGCGGCCCGCGTGGACCCCGACGCATATCCCTGT
TATTTTTTCAAAAGCGCATGTCGGCCCGGCCCGGCGTCCGTGGGTTCCGGCAGCGGACTCGGCAACGACGACGACGGG
GACTGGTTTCCCTGCTACGACGACGCCGGTGATGAGGAGTGGGCGGAGGACCCGGGCGCCATGGACACATCCCACGAT
CCCCCGGACGACGAGGTTGCCTACTTTGACCTGTGCCACGAAGTCGGCCCCACGGCGGAACCTCGCGAAACGGATTCG
CCCGTGTGTTCCTGCACCGACAAGATCGGACTGCGGGTGTGCATGCCCGTCCCCGCCCCGTACGTCGTCCATGGTTCT
CTAACGATGCGGGGGGTGGCACGGGTCATCCAGCAGGCGGTGCTGTTGGACCGAGATTTTGTGGAGGCCATCGGGAGC
TACGTAAAAAACTTCCTGTTGATCGATACGGGGGTGTACGCCCACGGCCACAGCCTGCGCTTGCCGTATTTTGCCAAA
ATCGCCCCCGACGGGCCTGCGTGCGGAAGGCTGCTGCCAGTGTTTGTGATCCCCCCCGCCTGCAAAGACGTTCCGGCG
TTTGTCGCCGCGCACGCCGACCCGCGGCGCTTCCATTTTCACGCCCCGCCCACCTATCTCGCTTCCCCCCGGGAGATC
CGTGTCCTGCACAGCCTGGGTGGGGACTATGTGAGCTTCTTTGAAAGGAAGGCGTCCCGCAACGCGCTGGAACACTTT
GGGCGACGCGAGACCCTGACGGAGGTCCTGGGTCGGTACAACGTACAGCCGGATGCGGGGGGGACCGTCGAGGGGTTC
GCATCGGAACTGCTGGGGCGGATAGTCGCGTGCATCGAAACCCACTTTCCCGAACACGCCGGCGAATATCAGGCCGTA
TCCGTCCGGCGGGCCGTCAGTAAGGACGACTGGGTCCTCCTACAGCTAGTCCCCGTTCGCGGTACCCTGCAGCAAAGC
CTGTCGTGTCTGCGCTTTAAGCACGGCCGGGCGAGTCGCGCCACGGCGCGGACATTCGTCGCGCTGAGCGTCGGGGCC
AACAACCGCCTGTGCGTGTCCTTGTGTCAGCAGTGCTTTGCCGCCAAATGCGACAGCAACCGCCTGCACACGCTGTTT
ACCATTGACGCCGGTACGCCATGCTCGCCGTCCGTTCCCTGCAGCACCTCTCAACCGTCGTCTTGATAACGGCGTACG
GCCTCGTGCTCGTGTGGTACACCGTCTTCGGTGCCAGTCCGCTGCACCGATGTATTTACGCGGTACGCCCCACCGGCA
CCAACAACGACACCGCCCTCGTGTGGATGAAAATGAACCAGACCCTATTGTTTCTGGGGGCCCCGACGCACCCCCCCA
ACGGGGGCTGGCGCAACCACGCCCATATCTGCTACGCCAATCTTATCGCGGGTAGGGTCGTGCCCTTCCAGGTCCCAC
CCGACGCCATGAATCGTCGGATCATGAACGTCCACGAGGCAGTTAACTGTCTGGAGACCCTATGGTACACACGGGTGC
GTCTGGTGGTCGTAGGGTGGTTCCTGTATCTGGCGTTCGTCGCCCTCCACCAACGCCGATGTATGTTTGGTGTCGTGA
GTCCCGCCCACAAGATGGTGGCCCCGGCCACCTACCTCTTGAACTACGCAGGCCGCATCGTATCGAGCGTGTTCCTGC
AGTACCCCTACACGAAAATTACCCGCCTGCTCTGCGAGCTGTCGGTCCAGCGGCAAAACCTGGTTCAGTTGTTTGAGA
CGGACCCGGTCACCTTCTTGTACCACCGCCCCGCCATCGGGGTCATCGTAGGCTGCGAGTTGATGCTACGCTTTGTGG
CCGTGGGTCTCATCGTCGGCACCGCTTTCATATCCCGGGGGGCATGTGCAATCACATACCCCCTGTTTCTGACCATCA
CCACCTGGTGTTTTGTCTCCACCATCGGCCTGACAGAGCTGTATTGTATTCTGCGGCGGGGCCCGGCCCCCAAGAACG
CAGACAAGGCCGCCGCCCCGGGGCGATCCAAGGGGCTGTCGGGCGTCTGCGGGCGCTGCTGTTCCATCATCCTCTCGG
GCATCGCAGTGCGATTGTGTTATATCGCCGTGGTGGCCGGGGTGGTGCTCGTGGCGCTTCACTACGAGCAGGAGATCC
AGAGGCGCCTGTTTGATGTATGACGTCACATCCAGGCCGGCGGAAACCGGAACGGCATATGCAAATTGGAAACTGTCC
TGTCTTGGGGCCCACCCACCCGACGCGTCATATGCAAATGAAAATCGGTCCCCCGAGGCCACGTGTAGCCTGGATCCC
AACGACCCCGCCCATGGGTCCCAATTGGCCGTCCCGTTACCAAGACCAACCCAGCCAGCATATCCACCCCCGCCCGGG
TCCCCGCGGAAGCGGAACGGTGTATGTGATATGCTAATTAAATACATGCCACGTACTTATGGTGTCTGATTGGTCCTT
GTCTGTGCCGGAGGTGGGGCGGGGGCCCCGCCCGGGGGGCGGAACGAGGAGGGGTTTGGGAGAGCCGGCCCCGGCACC
ACGGGTATAAGGACATCCACCACCCGGCCGGTGGTGGTGTGCAGCCGTGTTCCAACCACGGTCACGCTTCGGTGCCTC
TCCCCGATTCGGGCCCGGTCGCTCGCTACCGGTGCGCCACCACCAGAGGCCATATCCGACACCCCAGCCCCGACGGCA
 
ACCGACAGCCCGGTCATGGCGACTGACATTGATATGCTAATTGACCTCGGCCTGGACCTCTCCGACAGCGATCTGGAC
GAGGACCCACCCGAGCCGGCGGAGAGCCGCCGCGACGACCTGGAATCGGACAGCAGCGGGGAGTGTTCCTCGTCGGAC
GAGGACATGGAAGACCCCCACGGAGAGGACGGACCGGAGCCGATACTCGACGCCGCTCGCCCGGCGGTCCGCCCGTCT
CGTCCAGAAGACCCCGGCGTACCCAGCACCCAGACGCCTCGTCCGACGGAGCGGCAGGGCCCCAACGATCCTCAACCA
GCGCCCCACAGTGTGTGGTCGCGCCTCGGGGCCCGGCGACCGTCTTGCTCCCCCGAGCAGCACGGGGGCAAGGTGGCC
CGCCTCCAACCCCCACCGACCAAAGCCCAGCCTGCCCGCGGCGGACGCCGCGGGCGTCGCAGGGGTCGGGGTCGCGGT
GGTCCCGGGGCCGCCGATGGTTTGTCGGACCCCGCCGGCGTGCCCCCAGAACCAATCGCAACCCGGGGGGACCCCGC
CCCGGGGCGGGGTGGACGGACGGCCCCGGCGCCCCCATGGCGAGGCGTGGCGCGGAAGTGAGCAGCCCGACCCACCC
GGAGGCCCGCGGACACGGGGCGTGCGCCAAGCACCCCCCCGCTAATGACGCTGGCGATTGCCCCCCCGCCCGCGGAC
CCCCGCGCCCCGGCCCCGGAGCGAAAGGCGCCCGCCGCCGACACCATCGACGCCACCACGCGGTTGGTCCTGCGCTCC
ATCTCCGAGCGCGCGGCGGTCGACCGCATCAGCGAGAGCTTTGGCCGCAGCGCACAGGTCATGCACGACCCCTTTGGG
GGGCAGCCGTTTCCCGCCGCGAATAGCCCCTGGGCCCCGGTGTTGGCGGGCCAAGGAGGGCCCTTTGACGCCGAGACC
AGACGGGTCTCCTGGGAAACCTTGGTCGCCCACGGCCCGAGCCTCTATCGCACTTTTGCCGGCAATCCTCGGGCCGCA
TCGACCGCCAAGGCCATGCGCGACTGCGTGCTGCGCCAAGAAAATTTCATCGAGGCGCTGGCCTCCGCCGACGAGACG
CTGGCGTGGTGCAAGATGTGCATCCACCACAACCTGCCGCTGCGCCCCCAGGACCCCATTATCGGGACGGCCGCGGCT
GTGCTGGATAACCTCGCCACGCGCCTGCGGCCCTTTCTCCAGTGCTACCTGAAGGCGCGAGGCCTGTGCGGCCTGGAC
GAACTGTGTTCGCGGCGGCGTCTGGCGGACATTAAGGACATTGCATCCTTCGTGTTTGTCATTCTGGCCAGGCTCGCC
AACCGCGTCGAGCGTGGCGTCGCGGAGATCGACTACGCGACCCTTGGTGTCGGGGTCGGAGAGAAGATGCATTTCTAC
CTCCCCGGGGCCTGCATGGCGGGCCTGATCGAAATCCTAGACACACACCGCCAGGAGTGTTCGAGTCGTGTCTGCGAG
TTGACGGCCAGTCACATCGTCGCCCCCCCGTACGTGCACGGCAAATATTTTTATTGCAACTCCCTGTTTTAGGTACAA
TAAAAACAAAACATTTCAAACAAATCGCCCCACGTGTTGTCCTTCTTTGCTCATGGCCGGCGGGGCGTGGGTCACGGC
AGATGGCGGGGGTGGGCCCGGCGTACGGCCTGGGTGGGCGGAGGGAACTAACCCAACGTATAAATCCGTCCCCGCTCC
AAGGCCGGTGTCATAGTGCCCTTAGGAGCTTCCCGCCCGGGCGCATCCCCCCTTTTGCACTATGACAGCGACCCCCCT
CACCAACCTGTTCTTACGGGCCCCGGACATAACCCACGTGGCCCCCCCTTACTGCCTCAACGCCACCTGGCAGGCCGA
AACGGCCATGCACACCAGCAAAACGGACTCCGCTTGCGTGGCCGTGCGGAGTTACCTGGTCCGCGCCTCCTGTGAGAC
CAGCGGCACAATCCACTGCTTTTTCTTTGCGGTATACAAGGACACCCACCACACCCCTCCGCTGATTACCGAGCTCCG
CAACTTTGCGGACCTGGTTAACCACCCGCCGGTCCTACGCGAACTGGAGGATAAGCGCGGGGTGCGGCTGCGGTGTGC
GCGGCCGTTTAGCGTCGGGACGATTAAGGACGTCTCTGGGTCCGGCGCGTCCTCGGCGGGAGAGTACACGATAAACGG
GATCGTGTACCACTGCCACTGTCGGTATCCGTTCTCAAAAACATGCTGGATGGGGGCCTCCGCGGCCCTACAGCACCT
GCGCTCCATCAGCTCCAGCGGCATGGCCGCCCGCGCGGCAGAGCATCGACGCGTCAAGATTAAAATTAAGGCGTGATC
TCCAACCCCCCCATGAATGTGTGTAACCCCCCAAAAAAATAAACAGCCGTAACCCAATCAAACCAGGCGTGGTGTGAG
TTTGTGGACCCAAAGCCCTCAGAGACAACGCGACAGGCCAGTATGGACCGTGATACTTTTATTTATTAACTCACAGGG
GCGCTTACCGCCACAGGAATACCAGAATAATGACCACCACTATCGCGACCACCCCAAATACAGCATGGCGCCCCACCA
CGCCACAACAGCCCTGTCGCCGGTATGGGGCATGATCAGACGAGCCGCGAGCCGCGCGTTGGGCCCTGTACAGCTCGC
GCGAATTGACCCTAGGAGGCCGCCACGCGCCCGAGTTTTGCGTTCGTCGCTGGTCGTCGGGCGCCAAAGCCCCGGACG
GCTGTTCGGTCGAACGAACGGCCACGACAGTGGCATAGGTTGGGGGGTGGTCCGACATAGCCTCGGCGTACGTCGGGA
GGCCCGACAAGAGGTCCCTTGAGATGTCGGGTGGGGCCACAAGCCTGGTTTCCGGAAGAAACAGGGGGGTTGCCAATA
ACCCGCCAGGGCCAAAACTCCGGCGCTGCGCACGTCGTTCGGCGCGGCGCCGGGCGCGCCGAGCGGCTCGCTGGGCGG
 
CTTGGCGTGAGCGGCCCCGCTCCGACGCCTCGCCCTCTCCGGAGGAGGTTGGCGGAATTGGCACGGACGACAGGGGCC
CAGCAGAGTACGGTGGAGGTGGGTCCGTGGGGGTGTCCAGATCAATAACGACAAACGGCCCCTCGTTCCTACCAGACA
AGCTATCGTAGGGGGGCGGGGGATCAGCAAACGCGTTCCCCGCGCTCCATAGACCCGCGTCGGGTTGCGCCGCCTCCG
AAGCCATGGATGCGCCCCAAAGCCACGACTCCCGCGCGCTAGGTCCTTGGGGTAAGGAAAAGGCCCTACTCCCCATC
CAAGCCAGCCAAGTTAACGGGCTACGCCTTCGGGGATGGACTGGCACCCCGGCGGATTTTGTTGGGCTGGTACGCGT
TGCCCAACCGAGGGCCGCGTCCACGGGACGCGCCTTTTATAACCCCGGGGGTCATTCCCAACGATCACATGCAATCTA
ACTGGCTCCCCTCTCCCCCCCTCTCCCCTCTCCCCCCCTCTCCCCTCTCCCCCCCTCTCCCCTCTCCCCCCCTCTCCC
CTCTCCCCCCCTCTCCCCTCTCCCCCCCTCTCCCCTCTCCCCCCCTCTCCCCTCTCCCCCCCTCTCCCCTCTCCCCCC
CTCTCCCCTCTCCCCCCCTCTCCCCTCTCCCCTCTGCTCTTTCCCCGTGACACCCGACGCTGGGGGGCGTGGCTGCCG
GGAGGGGCCGCGGATGGGCGGGGCCTACTTGGTTTCCCGCCCCCCCCCCCGCCCCCGAACCGCCCCGCCGGCCTTGC
CCCCCTTTGATCCCCTGCTACCCCCAACCCGTGCTGGTGGTGCGGGTTGGGGGGGGAGTGTGGGCGGGGGTGTGCGGG
AGGTGTCGGTGGTGGTGGTGGTGGTAGTAGGAATGGTGGTGAGGGGGGGGGGGCGCTGGTTGGTCAAAAAAGGGA
GGGACGGGGGCCGGCAGACCGACGGCGACAACGCTCCCCGGTGGCCGGGTCGCGGCTCTTACGAGCGGCCCGGCCCGC
GCTCCCACCCCCCGGGCCGTGTCCTTGCTTTCCCCCGTCTCCCCCCCCCCGCCTTCTCCTCCTCCTCCTCGTTTTT
CCAAACCCCGCCCACCCGGCCCGGCCCGGCCCGGCCCGGCCCGGCCACCGCCGCCCACCCACCCACCTCGGGAGACCC
AGCCCCGGTCCCCCGTTCCCCGGGGGCCGTTATCTCCAGCGCCCCGTCCGGCGCGCCGCCCCCCGCCGCTAAACCCCA
TCCCGCCCCCGGGACCCCACATATAAGCCCCCAGCCACACGCAAGAACAGACACGCAGAACGGCTGTGTTTATTTAAA
TAAACCGATGTCGGAATAAACAAACACAAACACCCGCGACGGGGGGACGGAGGGGACGGAGGGAGGGGGGTGACGGGG
GACGGGAACAGACACACCACAAAAAACACCCACCCACCGACACCCCCACCCCAGTCTCCTCGCCTTCTCCCACCCACC
CCACGCCCCCACTGAGCCCGGTCGATCGACGAGCACCCCCGCCCACGCCCCCGCCCCTGCCCCGGCGACCCCCGGCCC
GCACGATCCCGACAACAATAACAACCCCAACGGAAAGCGGCGGGGTGTGGGGGGGGGCGAGGAACAACCGAGGGGAAC
GGGGGATGGAAGGACGGGAAGTGGAAGTCCTGATACCCATCCTACACCCCCCTGCCTTCCACCCTCCGGCCCCCCGCG
AGTCCACCCGCCGGCCGGCTACCGAGACCGAACACGGCGGCCGCCGCAGCCGCCGCAGCCGCCGCCGACACCGCAGAG
CCGGCGCGCGCACACACAAGCGGCAGAGGCAGAAAGGCCCAGAGTCATTGTTTATGTGGCCGCGGGCCAGCAGACGGC
CCGCGACACCCCCCCCCCGCCCGTGTGGGTATCCGGCCCCCCGCCCCGCGCCGGTCCATTAAGGGCGCGCGTGCCCGC
GAGATATCAATCCGTTAAGTGCTCTGCAGACAGGGGCACCGCGCCCGGAAATCCATTAGGCCGCAGACGAGGAAAATA
AAATTACATCACCTACCCACGTGGTGCTGTGGCCTGTTTTTGCTGCGTCATCTGAGCCTTTATAAAAGCGGGGGCGCG
GCCGTGCCGATCGCGGGTGGTGCGAAAGACTTTCCGGGCGCGTCCGGGTGCCGCGGCTCTCCGGGCCCCCCTGCAGCC
GGGGCGGCCAAGGGGCGTCGGCGACATCCTCCCCCTAAGCGCCGGCCGGCCGCTGGTCTGTTTTTTGTTTTCCCCGTT
TCGGGGGTGGGGGGGGTTGCGGTTTCTGTTTCTTTAACCCGTCTGGGGTGTTTTTCGTTCCGTCGCCGGAATGTTTCG
TTCGTCTGTCCCCTCACGGGGCGAAGGCCGCGTACGGCCCGGGACGAGGGGGCCCCCGACCGCGGCGGTCCGGGCCCC
GTCCGGGCCCGCTCGCCGGCACGCGACGCGAAAAAGGCCCCCCGGAGGCTTTTCCGGGTTCCCGGCCCGGGGCCTGAG
ATAAACAATCGGGGTTACCGCCAACGGCCGGCCCCCGTGGCGGCCCGGCCCGGGGCCCCGGCGGACCCAAGGGGCCCC
GGCCCGGGGCCCCACAACGGCCCGGCGCATGCGCTGTGGTTTTTTTTTTTCTCGGTGTTCTGCCGGGCTCCATCGCCT
TTCCTGTTCTCGCTTCTCCCCCCCCCCTTCTTCACCCCCAGTACCCTCCTCCCTCCCTTCCTCCCCCGTTATCCCACT
CGTCAAGGGCGCCCCGGTGTGGTTCAACAAAGACGCCGCGTTTCCAGGTAGGTTAGACACCTGCTTCTCCCCAATAGA
GGGGGGGGACCCAAACGACAGGGGGCGCCCCAGAGGCTAAGGTCGGCCACGCCACTCGCGGGTGGGCTCGTGTTACAG
CACACCAGCCCGTTCTTTTCCCCCCCTCCCACCCTTAGTCAGACTCTGTTACTTACCCGTCCGACCACCAACTGCCCC
 
CTTATCTAAGGGCCGGCTGGAAGACCGCCAGGGGGTCGGCCGGTGTCGCTGTAACCCCCCACGCCAATGACCCACGTA
CTCCAAGAAGGCATGTGTCCCACCCCGCCTGTGTTTTTGTGCCTGGCTCTCTATGCTTGGGTCTTACTGCCTGGGGGG
GGGGAGTGCGGGGGAGGGGGGGTGTGGAAGGAAATGCACGGCGCGTGTGTACCCCCCCTAAAGTTGTTCCTAAAGCGA
GGATATGGAGGAGTGGCGGGTGCCGGGGGACCGGGGTGATCTCTGGCACGCGGGGGTGGGAAGGGTCGGGGGAGGGGG
GATGGGGTACCGGCCCACCTGGCCGACGCGGGTGCGCGTGCCTCTGCACACCAACCCCACGTCCCCCGGCGGTCTCTA
AGAAGCACCGCCCCCCCTCCTTCATACCACCGAGCATGCCTGGGTGTGGGTTGGTAACCAACACGCCCATCCCCTCGT
CTCCTGTGATTCTCTGGCTGCACCGCATTCTTGTTTTCTAACTATGTTCCTGTTTCTGTCTCCCCCCCCCCCACCCCT
CCGCCCCACCCCCCAACACCCACGTCTGTGGTGTGGCCGACCCCCTTTTGGGCGCCCCGTCCCGCCCCGCCACCCCTC
CCGTCCTTTGTTGCCCTATAGTGTAGTTAACCCCCCCCGCCCTTTGTGGCGGCCAGAGGCCAGGTCAGTCCGGGCGGG
CAGGCGCTCGCGGAAACTTAACACCCACACCCAACCCACTGTGGTTCTGGCTCCATGCCAATGGCAGGATGCTTTCGG
GGATCGGTGGTCAGGCAGCCCGGGCCGCGGCTCTGTGGTTAACACCAGAGCCTGCCCAACATGGCACCCCCACTCCCA
CGCACCCCCACTCCCACGCACCCCCACTCCCACGCACCCCCACTCCCACGCACCCCCACTCCCACGCACCCCCACTCC
CACGCACCCCCACTCCCACGCACCCCCACTCCCACGCACCCCCACTCCCACGCACCCCCGAGATCCATCCAACACAGA
CAGGGAAAAGATACAAAAGTAAACCTTTATTTCCCAATAGACAGCAAAAATCCCCTGAGTTTTTATTAGGGCCAACA
CTAAAGACCCGCTGGTGTGTGGTGCCCGTGTCTTTCACTTTTCCCCTCCCCGACACGGATTGGCTGGTGTAGTGGGCG
CGGCCAGAGACCACCCAGCGCCCGACCCCCCCCTCCCCACAAACACGGGGGGCGTCCCTTATTGTTTTCCCTCGTCCC
GGGTCGACGCCCCCTGCTCCCCGGACCACGGGTGCCGAGACCGCAGGCTGCGGAAGTCCAGGGCGCCCACTAGGGTGC
CCTGGTCGAACAGCATGTTCCCCACGGGGGTCATCCAGAGGCTGTTCCACTCCGACGCGGGGGCCGTCGGGTACTCGG
GGGGCATCACGTGGTTACCCGCGGTCTCGGGGAGCAGGGTGCGGCGGCTCCAGCCGGGGACCGCGGCCCGCAGCCGGG
TCGCCATGTTTCCCGTCTGGTCCACCAGGACCACGTACGCCCCGATGTTCCCCGTCTCCATGTCCAGGATGGGCAGGC
AGTCCCCCGTGATCGTCTTGTTCACGTAAGGCGACAGGGCGACCACGCTAGAGACCCCCGAGATGGGCAGGTAGCGCG
TGAGGCCGCCCGCGGGGGCGGCCCCGGAAGTCTCCGCGTGGCGCGTCTTCCGGGCACACTTCCTCGGCCCCCGCGGCC
CAGAAGCAGCGCGGGGGCCGAGGGAGGTTTCCTCTTGTCTCCCTCCCAGGGCACCGACGGCCCCGCCCGAGGAGGCGG
AAGCGGAGGAGGACGCGGCCCCGGCGGCGGAAGAGGCGGCCCCCGCGGGAGTCGGGGCCGAGGAGGAAGAGGCAGAGG
AGGAAGAGGCGGAGGCCGCCGAGGACGTCAGGGGGGTCCCGGGCCACCCTGGCCGCGCCCCCCGGCCCTGAGTCGG
AGGGGGGGTGCGTCGCCGCCCTCTTGGCCCCTGCCGGCGCGAGGGGGGGACGCGTGGACTGGGGGAGGGGTTTTCCT
GGCCCGACCCGCGCCTCTTCCTCGGACGCACCGCCGCCTCCTGCTCGACAGAGGCGGCGGAGGGAGCGGGGGGGCGC
CGGAGGGGGCGGCGCCGCGGGAGGGCCCGTGTCCACCCTCCACGCCCGGCCCCCCCGAGCCGCGCGCCACCGTCGCAC
GCGCCCGGCACAGACTCTGTTCTTGGTTCGCGGCCTGAGCCAGGGACGAGTGCGACTGGGGCACACGGCGCGCGTCCG
CGGGGCGGGCGGCCGGCTCCGCCCCGGGGGCCGGGGCGCGGGGGCCGGGCCCCGGAGGCGGCGCTCGCACGCACGGGG
CCACGGCCGCGCGGGGGCGCGCGGGTCCCGACGCGGCCGAGGACGCGGTGGGCCCGGGGCGGGGGGCGGAGCCTGGCA
TGGGCGCCGCGGGGGGCCTGTGGGGAGAGGCCGGGGGGGAGTCGCTGATCACTATGGGGTCTCTGTTGTTTGCAAGGG
GGGCGGGTCTGTTGACAAGGGGGCCCGTCCGGCCCCTCGGCCGCCCCGCCTCCGCTTCAACAACCCCAACCCCAACCC
CAACCCCCCCGGAGGGGCCAGACGCCCCCCGCGGCGCCGCGGCTCGCGACTGGCGGAGCCGCCGCCGCCGCTGCTGT
TGGTGGTGGTGTTGGTGTTACTGCTGCCGTGTGGCCCGATGGGCGCCGAGGGGGGCGCTGTCCGAGCCGCGGCCGGCT
GGGGGGCTGCGTGAGACGCCCCGCCCGTCACGGGGGGCGCGGCGGCGCCTCTGCGTGGGGGGGCGCGGGGCGTCCGGC
GGGGGGCGGGCGGTACGTAGTCTGCTGCAAGAGACAACGGGGGGCGCGATCAGGTTACGCCCCCTCCCAGGCCCTCCC
TTTCCGCGCCCGCCCGCCTATTCCTCCCTCCCCCCTCCTCCTCCTCCCCCAGGGTCCTCGCCGCCCCCCGCCTCA
 
CCGTCGTCCAGGTCGTCGTCATCCTCGTCCGTGGTGGCTCAGGGTGGGTGGGCGACAGGGCCCTCACCGTGTGCCCC
CCCAGGGTCAGGTACCGCGGGGCGAACCGCTGATTGCCCGTCCAGATAAAGTCCACGGCCGTGCCCGCCCTGACGGCC
TCCTCGGCCTCCATGCGGGTCTGGGGGTCGTTCACGATCGGATGGTGCTGAACGACCCGCTGGGCGTCACGCCCACT
ATCAGGTACACCAGCTTGGCGTTGCACAGCGGGCAGGTGTTGCGCAATTGCATCCAGGTTTTCATGCACGGGATGCAG
AAGCGGTGCATGCACGGGAAGGTGTCGCAGCGCAGGTGGGGCGCGATCTCATCCGTGCACACGGCGCACACGTCGCCC
TCGTCGCTCCCCCCGTCCTCTCGAGGGGGGGCGCCCCCGCAACTGCCGGGGTCTTCCTCGCGGGGGGGGCTCCCCCCC
GAGACCGCCCCCCCATCCACGCCCTGCGGCCCCAGCAGCCCCGTCTCGAACAGTTCCGTGTCCGTGCTGTCCGCCTCG
GAGGCGGAGTCGTCGTCATGGTGGTCGGCGTCCCCCGCCCCCCCACTTCGGTCTCCGCCTCAGAGTCGCTGCTGTCC
GGCAGGTCTCGGTCGCAGGGAAACACCCAGACATCCGGGGCGGGCTAAGGGGAAAAAGGGGGCGGGTAAGAATGGG
GGGATTTCCCGCGTCAATCAGCGCCCACGAGTTCCCCCTCTCCCCCCCCCGCCTCACAAAGTCCTGCCCCCCTGCTGG
CCTCGGAAGAGGGGGGAGAAAGGGGTCTGCAACCAAAGGTGGTCTGGGTCCGTCCTTTGGATCCCGACCCCTCTTCTT
CCCTCTTCTCCCGCCCTCCAGACGCACCGGAGTCGGGGGTCCCACGGCGTCCCCCAAATATGGCGGGCGGCTCCTCCC
CACCCCCCTAGATGCGTGTGAGTAAGGGGGCCCTGCGTATGAGTCAGTGGGGACCACGCCCCCTAACACGGCGACCCC
GGTCCTTGTGTGTTTGTTGTGGGGGCGTGTCTCTGTGTATGAGTCAGGGGGTCCCACGGCGACCCCGGGCCCTGCGTC
TGAGTCAAAGGGGCCATGTGTATGTGTTGGGGGGTCTGTATATATAAAGTCAGGGGGTCACATGGCGACCCCCAACAG
GGCGACCCCGGTCCCTGTATATATAGGGTCAGGGGGTTCCGCGCCCCCTAACATGGCGCCCCCGGTCCCTGTATATAT
AGTGTCACGGGGTTCCACGCCCCCTAACATGGCGCCCGCCCGGCTCCCGTGTATGAGTGGGGGTCCCCCAACATGGCG
GCCGGTTCCAGTGTAAGGGTCGGGGGTCCCCCAACATGGCGCCCCCCAATATGGCGCCCCCCAATATGGCGCCCCAGA
CATGGCGCCCGGCCCCTCACCTCGCGCTGGGGGCGGCCCTCAGGCCGGCGGGTACTCGCTCCGGGGCGGGGCTCCATG
GGGGTCGTATGCGGCTGGAGGGTCGCGGACGGAGGGTCCCTGGGGGTCGCAACGTAGGCGGGGCTTCTGTGGTGATGC
GGAGAGGGGGCGGCCCGAGTCTGCCTGGCTGCTGCGTCTCGCTCCGAGTGCCGAGGTGCAAATGCGACCAGACTGTCG
GGCCAGGGCTAACTTATACCCCACGCCTTTCCCCTCCCCAAAGGGGCGGCAGTGACGATTCCCCCAATGGCCGCGCGT
CCCAGGGGAGGCAGGCCCACCGCGGGGCGGCCCCGTCCCCGGGGACCAACCCGGCGCCCCCAAAGAATATCATTAGCA
TGCACGGCCCGGCCCCCGATTTGGGGGACCAACCCGGTGTCCCCCAAAGAACCCCATTAGCATGCCCCTCCCACCGAC
GCAACAGGGGCTTGGCCTGCGTCGGTGCCCCGGGGCTTCCCGCCTTCCCGAAGAAACTCATTACCATACCCGGAACCC
CAGGGGACCAATGCGGGTTCATTGAGCGACCCGCGGGCCAATGCGCGAGGGGCCGTGTGTTCCGCCAAAAAAGCAATT
AGCATAACCCGGAACCCCAGGGGAGTGGTTACGCGCGGCGCGGGAGGCGGGGAATACCGGGGTTGCCCATTAAGGGCC
GCGGGAATTGCCGGAAGCGGGAAGGGCGGCCGGGGCCGCCCATTAATGAGTTTCTAATTACCATACCGGGAAGCGGAA
CAAGGGTTACCTGGGACTGTGCGGTTGGGACGGCGCCCGTGGGCCCGGGCGGCCGGGGGCGGCGGGGGCCGCGATGGC
GGCGGCGGCGGGCCATGGAGACAGAGAGCGTGCCGGGGTGGTAGAGTTTGACAGGCAAGCATGTGCGTGCAGAGGCGA
GTAGTGCTTGCCTGTCTAACTCGCTCGTCTCGGCCGCGGGGGGCCCGGGCTGCCGCCGCCGCGCTTTAAAGGGCCGCG
CGCGACCCCCGGGGGGTGTGTTTTGGGGGGGGCCCGTTTTCGCTCCTCCCCCCGCTCCTCCCCCCGCTCCTCCCCCCG
CTCCTCCCCCCGCTCCTCCCCCCGCTCCTCCCCCCGCTCCTCCCCCCGCTCCTCCCCCCGCTCCTCCCCCCGCTCCTC
CCCCCGCTCCTCCCCCCGCTCCTCCCCCCGCTCCTCCCCCCGCTCCTCCCCCCGCTCCTCCCCCCGCTCCTCCCCCCG
CTCCTCCCCCCGCTCCTCCCCCCGCTCCCGCGGCCCCGCCCCCAACGCCCGCCGCGCGCGCGCACGCCGCCCGGACCG
CCGCCCGCCTTTTTTGCGCGCGCCCCGCCCGCGGGGGGCCCGGGCTGCCACAGGTGTAACAACACACACGGCTCATCC
ACACGTCACACGTCACGTCATCCACCACACCTGCCCACCAACACAACTCACAGCGACAACTCACCGCGCAACAACTCC
TGTTCCTCATCCACACGTCACCGCGCACCCCCCGCTCCTCCAGACGTCCCCCAGCGCAACACGCCGCTCCTGCTACAC
 
ACCACCGCCCCCTCCCCAGCCCCAGCCCTCCCCAGCCCCAGCCCTCCCCAGCCCCAGCCCTCCCCGGCCCCAGCCCTC
CCCGGCCCCAGCCCTCCCCGGCCCCAGCCCTCCCCGGCCCCAGCCCTCCCCGGCCCCAGCCCTCCCCGCCGCGTCCCG
CGCTCCCTCGGGGGGTTCGGGCATCTCTACCTCAGTGCCGCCAATCTCAGGTCAGAGATCCAAACCCTCCGGGGGCG
CCCGCGCACCACCACCGCCCCTCGCCCCCTCCCGCCCCTCGCCCCCTCCCGCCCCTCGCCCCCTCCCGCCCCTCGCCC
CCTCCCGCCCCTCGCCCCCTCCCGCCCCTCGCCCCCTCCCGCCCCTCGCCCCCTCCCGCCCCTCGCCCCCTCCCGCCC
CTCGCCCCCTCCCGCCCCTCGCCCCCTCCCGCCCCTCGCCCCCTCCCGCCCCTCGCCCCCTCCCGCCCCTCGCCCCCT
CCCGCCCCTCGCCCCCTCCCGCCCCTCGCCCCCTCCCGCCCCTCGCCCCCTCCCGCCCCTCGCCCCCTCCCGCCCCTC
GCCCCCTCCCGCCCCTCGCCCCCTCCCGCCCCTCAAATAAACAACGCTACTGCAAAACTAAATCAGGTCGTTGTCGTT
TATTGTGTCTTCGGGTTTCGCAAGCGCCCCGCCCCGTCCCGGCCCGTTACAGCACCCCGTCCCCCTCGAACGCGCCGC
CGTCGTCGTCGTCCCAGGCGCCTTCCCAGTCCACAACTTCCCGTCGCGGGGGCGTGGCCAAGCCCGCCTCCGCCCCCA
GCACCTCCACGGCCCCCGCCGCCGCCAGCACGGTGCCGCTGCGGCCCGTGGCCGAGGCCCAGCGAATCCCGGGCAACG
CCGGCGGCAGGGCCCCCGGGCCGTCGTCGYCGCCGCGCAGCACCAGCGGGGGGGCGTCGTCGTCGGGCTCCAGCAGGG
CGCGGGCGCAAAAGTCCCTCCGCGGCCCGCGCCACCGGGCCGGGCCGGCGCGCACCGCCTCGCGCCCCAGCGCCACGT
ACACGGGCCGCAGCGGCGCGCCCAGGCCCCAGCGCGCGCAGGCGCGGTGCGAGTGGGCCTCCTCCTCGCAGAAGTCCG
GCGCGCCGGGCGCCATGGCGTCGGTGGTCCCCGAGGCCGCCGCCCGGCCGTCCAGCGCCGGCAGCACGGCCCGGCGGT
ACTCGCGCGGGGACATGGGCACCGGCGTGTCCGGGCCGAAGCGCGTGCGCACGCGGTAGCGCACGTTGCCGCCGCGGC
ACAGGCGCAGCGGCGGCGCGTCGGGGTACAGGCGCGCGTGCGCGGCCTCCACGCGCGCGAAGACCCCCGGGCCGAACA
CGCGGCCCGGGGCCAGCACCGTGCGGCGCAGGTCCCGCGCCGCCGGCCAGCGCACGGCGCACTGCACGGCGGGCAGCA
GGTCGCACGCCAGGTAGGCGTGCTGCCGCGACACCGCGGGCCCGTCGGCGGGCCAGTCGCAGGCGCGCACGGTGTTGA
CCACGATGAGCCGCCGGTCGCCGGCGCTGGCGAGCAGCCCCAGAAACTCCACGGCCCCGGCGAAGGCCAGGTCCCGCG
TGGACAGCAGCAGCACGCCCTGCGCGCCCAGCGCCGACACGTCGGGGGCGCCGGTCCAGTTGCCCGCCCAGGCGGCCG
TGTCCGGCCCGCACAGCCGGTTGGCCAGGGCCGCCAGCAGGCAGGACAGCCCGCCGCGCTCGGCGGACCACTCCGGCG
GCCCCCCCGAGGCCCCGCCGCCGGCCAGGTCCTCGCCCGGCAGCGGCGAGTACAGCACCACCACGCGCACGTCCTCGG
GGTCGGGGATCTGGCGCATCCAGGCCGCCATGCGGCGCAGCGGGCCCGAGGCGCGCAGGGGGCCAAAGAGGCGGCCCC
CGGCGGCCCCGTGGGGGTGGGGGTTATCGTCGTCGTCGCCGCCGCCGCACGCGGCCTGGGCGGCGGGGGCGGGCCCGG
CGCACCGCGCGGCGATCGAGGCCAGGGCCCGCGGGTCAAACATGAGGGCCGGTCGCCAGGGGACGGGGAACAGCGGGT
GGTCCGTGAGCTCGGCCACGGCGCGCGGGGAGCAGTAGGCCTCCAGGGCGGCGGCCGCGGGCGCCGCCGTGTGGCTGG
GCCCCGGGGGCTGCCGCCGCCAGCCGCCCAGGGGGTCGGGGCCCTCGGCGGGCCGGCGCGACACGGCCACGGGGCGCG
GGCGGGCCTGCGCCGCGGCGGCCCGGGGCGCCGCGGGCTGGGCGGGGGCGGGCTCGGGCCCCGGGGGCGTGGAGGGGG
GCGCGGGCGCGGGGAGGGGGGCGCGGGCGTCCGAGCCGGGGGCGTCCGCGCCGCTCTTCTTCGTCTTCGGGGGTCGCG
GGCCGCCGCCTCCGGGCGGCCGGGCCGGGCCGGGACTCTTGCGCTTGCGCCCCTCCCGCGGCGCGGCGGAGGCGGCGG
CGGCCGCCAGCGCGTCGGCGGCGTCCGGTGCGCTGGCGGCCGCCGCCAGCAGGGGGCGCAGGCTCTGGTTCTCAAACA
GCAGGTCCGCGGCGGCGGCGGCCGCGGAGCTCGGCAGGCGCGGGTCCCGCGGCAGCGCGGGGCCCAGGGCCCCGGCGA
CCAGGCTCACGGCGCGCACGGCGGCCACGGCGGCCTCGCTGCCGCCGGCCACGCGCAGGTCCCCGCGCAGGCGCATGA
GCACCAGCGCGTCGCGCACGAACCGCAGCTCGCGCAGCCACGCGCGCAGGCGGGGCGCGTCGGCGTGCGGCGGCGGCG
GGGAAGCGGGGCCCGCGGGTCCCTCCGGCCGCGGGGGGCTGGCGGCCGGGCCCCGGCCAGCCCCGGGACGGCCGCCA
GGTCGCCGTCGAAGCCCTCGGCCAGCGCCTCCAGGATCCCGCGGCAGGCGGCCAGGCACTCCACGGCCACGCGGCCGG
CCTGGGCGCGGCGCCCGGCGTCGTCGTCGGCGTCGGCGTGGCGGGCGGCGTCGGGGTCGTCGCCCCCCGCGGGGGAGG
 
CGGGCGCGGCGGACAGCCGCCCCAGGGCGGCGAGGATCCCCGCGGCGCCGTACCCGGCGGGCACCGCGCGCTCGCCCG
GTGCGGCGACGACGGCGGCGGCGACCCCCTCGTCATCTGCGCCGGCGCCGGGGCTCCCCGCGGCCCCCGTCAGCGCCG
CGTTCTCGCGCGCCAACAGGGGCGCGTAGGCGCGGCGCAGGCTGGTCAGCAGGAAGCCCTTCTGCGCGCGGTCGTATC
GGCGGCTCATGGCCACGGCGGCCGCCGCGTGCGCCAGGCCCCAGCCGAAGCGGCCGGCCGCCATGGCGTAGCCCAGGT
GGGGCACGGCCCGCGCCACGCTGCCGGTGATGAAGGAGCTGCTGTTGCGCGCGGCGCCCGAGATCCGGAAGCAGGCCT
GGTCCAGCGCCACGTCCCCGGGGACCACGCGCGGGTTCTGGAGCCACCCCATGGCCTCCGCGTCCGGGGTGTACAGCA
GCCGCGTGATCAGGGCGTACTGCTGCGCGGCGTCGCCCAGCTCGGGCGCCCACACGGCCGCCGGGGCGCCCGAGGCCT
CGAACCGGCGTCGCGCCTCCTCCGCCTCGGGCGCCCCCCAGAGGCCCGGGCGGCTGTCGCCCAGGCCGCCGTACAGCA
CCCGCCCCGGGGGCGGGGGCCCGGCGCCGGGCCACGGCTCCCCGCTGACGTACCCGTCGCGATAGCGCGCGTAGAAGG
CGCCGGAGGCCGCGTCGGCGTCCAGCTCGACCCGCCGGGGCTGCCCGGCCGTGAAGCGGCCCGTGGCGTCGCGGCCGG
CCACCGCCGCGCGGGCCCGGCGGCGCTCGATGCGGCCCGCGGAGGCCGCGGGGGTCCTCGCCGCCGCCCGGGGCTTGG
GCGCGGCCTCGGAGAGGGGGGGTGGCCCGGGCGGGGGCGGCGTCCGCCCGGGGGCTTCCGGCGCCGCGCTCGACGGAC
CCCGCCCGACGGCCCGCGCCTCGCGTGCGCGGTCGGCCGCGTCGTTGCCGTCGTCGTCCTCGTCCTCGTCGGACGACG
AGGACGAAGAGGATGCGGACGACGAGGACGAGGACCCGGAGTCCGACGAGGTCGATGACGCCGATGGCCGCCGCCGGC
CGTGACGACGTCTCCGCGGCGGCTGGGCCGGCGGGCGCGGCGACAGGCGGTCCGTGGGGTCCGGATACGCGCCGCGTA
GCGGGGCCTCCCGTGCGCGGCCCCGGGCCGGGGCCCGGTCGCCGGCGGCGTCGGCTGCGTCGTCGTACTCGTCCCCGT
CATCGTCGTCGGCTCGAAAGGCGGGGGTCCGGGGCGGCGAGGCCGCGGGGTCGGGCGTCGGGATCGTCCGGACGGCCT
CCTCTACCATGGAGGCCAGCAGGGCCAGCTGTCGCGGCGAGACGGCGTCCCCGGCGTCCTCGCCGGCGTCGGTGCCCG
CCGCGGGGGCCCTCCCGTCCCGCCGGGCGTCGTCGAGGTCGTGGGGGTGGTCGGGGTCGTGGTCGGGGTCGTCCCCGC
CCTCCTCCGTCTCCGCGCCCCACCCGAGGGCCCCCCGCTCGTCGCGGTCTGGGCTCGGGGTGGGCGGCGGCCCGTCGG
TGGGGCCCGGGGAGCCGGGGCGCTGCTTGTTCTCCGACGCCATCGCCGATGCGGGGCGATCCTCCGGGGATACGGCTG
CGACGGCGGACGTAGCACGGTAGGTCACCTACGGACTCTCGATGGGGAGGGGGCGAGACCCACGGACCCCGACGACCC
CCGCCGTCGACGCGGAACTAGCGCGGACCGGTCGATGCTTGGGTGGGAAAAAGGACAGGGACGGCCGATCCCCCTCCC
GCGCTTCGTCCGCGTATCGGCGTCCCGGCGCGGCGAGCGTCTGACGGTCTGTCTCTGGCGGTCCCGCGTCGGGTCGTG
GATCCGTGTCGGCAGCCGCGCTCCGTGTGGACGATCGGGGCGTCCTCGGGCTCATATAGTCCCAGGGGCCGGCGGGAA
GGAGGAGCAGCGGAGGCCGCCGGCCCCCCGCCCCCCAGGCGGGCCCGCCCCGAACGGAATTCCATTATGCACGACCCC
GCCCCGACGCCGGCACGCCGGGGGCCCGTGGCCGCGGCCCGTTGGTCGAACCCCCGGCCCCGCCCATCCGCGCCATCT
GCCATGGGCGGGGCGCGAGGGCGGGTGGGCCCGCGCCCCGCCCCGCATGGCATCTCATTACCGCCCGATCCGGTGGTT
TCCGCTTCCGTTCCGCATGCTAACGAGGAACGGGCCGGGGGCGGGGCCCGGGCCCCGACTTCCCGGTTCGGCGGTAAT
GAGATACGAGCCCCGCGCGCCCGTTGGCCGTCCCCGGCCCCCGGTCCCGCCCGCCGGACGTTGGGACCAACGGGACG
GCGGGCGGCCCAAGGGCCGCCCGCCTTGCCGCCCCCCATTGGCCGGCGGGCGGGACCGCCCCAAGGGGGCGGGGCCG
CCGGGTAAAAGAAGTGAGAACGCGAAGCGTTCGCACTTCGTCCCAATATATATATATTATTAGGGCGAAGTGCGAGCA
CTGGCGCCGTGCCCGACTCCGCGCCGGCCCCGGGGGCGGGCCCGGGCGGCGGGGGGCGGGTCTCTCCGGCGCACATAA
AGGCCCGGCGCGACCGACGCCCGCAGACGGCGCCGGCCACGAACGACGGGAGCGGCTGCGGAGCACGCGGACCGGGAG
CGGGACTCGCAGAGGGCCGTCGGAGCGGACGGCGTCGGCATCGCGACGCCCCGGCTCGGGATCGGGATCGCATCGGAA
AGGGACACGCGGACAAGACCCACCCACCCCACCCACGAAACACAGGGGACGCACCCCGGGGGCCTCCGACGACAGAAA
CCCACCGGTCCGCCTTTGTGCACGGGTAAGCACCTTGGGTGGGCGGAGGAGGGGGGACACGGGGGCGGAGGAGGGGGG
ACACGGGGGCGGAGGAGGGGGGACGCGGGGGCGGAGGAGGGGGGACGCGGGGGCGGAGGAGGGGGGACACGGGGGCGG
 
AGGAGGGGGCTCACCCGCGTTCGTGCCTTCCCGCAGGAGGAACGTCCTCGTCGAGGCGACCGGCGGCGACCGTTGCGT
GGACCGCTTCCTGCTCGTCGGGCGGGGGGAAGCCACTGTGGTCCTCCGGGACGTTTTCTGGATGGCCGACATTTCCCC
AGGCGCTTTTGCGCCTTGTGTAAAAGCGCGGCGTCCCGCTCTCCGATCCCCGCCCCTGGGCACGCGCAAGCGCAAGCG
CCCTTCCCGCCCCCTCTCATCGGAGTCTGAGGTAGAATCCGATACAGCCTTGGAGTCTGAGGTCGAATCCGAGACAGC
ATCGGATTCGACCGAGTCTGGGGACCAGGATGAAGCCCCCCGCATCGGTGGCCGTAGGGCCCCCCGGAGGCTTGGGGG
GCGGTTTTTTCTGGACATGTCGGCGGAATCCACCACGGGGACGGAAACGGATGCGTCGGTGTCGGACGACCCCGACGA
CACATCCGACTGGTCTTATGACGACATTCCCCCACGACCCAAGCGGGCCCGGGTAAACCTGCGGCTCACGAGCTCTCC
CGATCGGCGGGATGGGGTTATTTTTCCTAAGATGGGGCGGGTCCGGTCTACCCGGGAAACGCAGCCCCGGGCCCCCAC
CCCGTCGGCCCCAAGCCCAAATGCAATGCTACGGCGCTCGGTGCGCCAGGCCCAGAGGCGGAGCAGCGCACGATGGAC
CCCCGACCTGGGCTACATGCGCCAGTGTATCAATCAGCTGTTTCGGGTCCTGCGGGTCGCCCGGGACCCCCACGGCAG
TGCCAACCGCCTGCGCCACCTGATACGCGACTGTTACCTGATGGGATACTGCCGAGCCCGTCTGGCCCCGCGCACGTG
GTGCCGTTTGCTGCAGGTGTCCGGCGGAACCTGGGGCATGCACCTGCGCAACACCATACGGGAGGTGGAGGCTCGATT
CGACGCCACCGCGGAACCCGTGTGCAAGCTTCCTTGTTTGGAGACCAGACGGTACGGCCCGGAGTGTGATCTTAGTAA
TCTCGAGATTCATCTCAGCGCGACAAGCGATGATGAAATCTCCGATGCCACCGATCTGGAGGCCGCCGGTTCGGACCA
CACGCTCGCGTCCCAGTCCGACACGGAGGATGCCCCCTCCCCCGTTACGCTGGAAACCCCAGAACCCCGCGGGTCCCT
CGCTGTGCGTCTGGAGGATGAGTTTGGGGAGTTTGACTGGACCCCCCAGGAGGGCTCCCAGCCCTGGCTGTCTGCGGT
CGTGGCCGATACCAGCTCCGTGGAACGCCCGGGCCCATCCGATTCTGGGGCGGGTCGCGCCGCAGAAGACCGCAAGTG
TCTGGACGGCTGCCGGAAAATGCGCTTCTCCACCGCCTGCCCCTATCCGTGCAGCGACACGTTTCTCCGGCCGTGAGT
CCGGTCGCCCCGACCCCCTTGTATGTCCCCAAAATAAAAGACCAAAATCAAAGCGTTTGTCCCAGCGTCTTAATGGCG
GGAAGGGCGGAGAGAAACAGACCACGCGTACATGGGGGGTGTTTGGGGGTTTATTGACATCGGGGCTACAGGGTGGTA
ACCGGATAGCAGATGTGAGGAAGTCTGGGCCGTTCGCCGCGAACGGCGATCAGAGGGTCCGTTTCTTGCGGACCACGG
CCCGGTGATGTGGGTTGTTCGTCTGGGATCTCGGGCATGCCCATACACGCACAACACGGACGCCGCACCGGATGGGAC
GTCGTAAGGGGGCCTGGGGTAGCTGGGTGGGGTTTGTGCAGAGCAATCAGGGACCGCAGCCAGCGCATACAATCGCGC
TCCCGTCCGTTTGTCCCGGGCAGTACCACGCCGTACTGGTATTCGTACCGGCTGAGCAGGGTCTCCAGGGGGTGGTTG
GGGGCCGCGGGGAACGGGGTCCACGCCACGGTCCACTCGGGCAAAAACCGAGTCGGCACGGCCCACGGTTCTCCCACC
CACGCGTCTGGGGTCTTGATGGCGATAAATCTTACCCCGAGCCGGATTTTTGGGCGTATTCGAGAAACGGCACACAC
AGATCCGCCGCGCCTACCACCCACAAGTGGTAGAGGCGAGGGGGGCTGGGTTGGTCTCGGTGCAGCAGTCGGAAGCAC
GCCACGGCGTCCACGACCTCGGTGCTCTCCAAGGGGCTGTCCTCCGCAAACAGGCCCGTGGTGGTGTTTGGGGGGCAG
CGACAGGACCTAGTGCGCACGATCGGGCGGGTGGGTTTGGGTAAGTCCATCAGCGGCTCGGCCAACCGTCGAAGGTTG
GCCGGACGAACGACGACCGGGGTACCCAGGGGTTCTGATGCCAAAATGCGGCACTGCCTAAGCAGGAAGCTCCACAGG
GCCGGGCTTGCGTCGACGGAAGTCCGGGGCAGGGCGTTGTTCTGGTCAAGGAGGGTCATTACGTTGACGACAACAACG
CCCATGTTGGTATATTACAGGCCCGTGTCCGATTTGGGGCACTTGCAGATTTGTAAGGCCACGCACGGCGGGGAGACA
GGCCGACGCGGGGGCTGCTCTAAAAATTTAAGGGCCCTACGGTCCACAGACCCGCCTTCCCGGGGGGGCCCTTGGAGC
GACCGGCAGCGGAGGCGTCCGGGGGAGGGGAGGGTGATTTACGGGGGGGTAGGTCAGGGGGTGGGTCGTCAAACTGCC
GCTCCTTAAAACCCCGGGGCCCGTCGTTCGGGGTGCTCGTTGGTTGGCACTCACGGTGCGGCGAATGGCCTGTCGTAA
GTTTTGTCGCGTTTACGGGGGACAGGGCAGGAGGAAGGAGGAGGCCGTCCCGCCGGAGACAAAGCCGTCCCGGGTGTT
TCCTCATGGCCCCTTTTATACCCCAGCCGAGGACGCGTGCCTGGACTCCCCGCCCCCGGAGACCCCCAAACCTTCCCA
CACCACACCACCCGGCGATGCCGAGCGCCTGTGTCATCTGCAGGAGATCCTGGCCCAGATGTACGGAAACCAGGACTA
 
CCCCATAGAGGACGACCCCAGCGCGGATGCCGCGGACGATGTCGACGAGGACGCCCCGGACGACGTGGCCTATCCGGA
GGAATACGCAGAGGAGCTTTTTCTGCCCGGGGACGCGCCCGGTCCCCTTATCGGGGCCAACGACCACATCCCTCCCCC
GTGTGGCGCATCTCCCCCCGGTATACGACGACGCAGCCGGGATGAGATTGGGGCCACGGATTTACCGCGGAAGAACT
GGACGCCATGGACAGGGAGGCGGCTCGAGCCATCAGCCGCGGCGGCAAGCCCCCCTCGACCATGGCCAAGCTGGTGAC
TGGCATGGGCTTTACGATCCACGGAGCGCTCACCCCAGGATCGGAGGGGTGTGTCTTTGACAGCAGCCACCCAGATTA
CCCCCAACGGGTAATCGTGAAGGCGGGGTGGTACACGAGCACGAGCCACGAGGCGCGACTGCTGAGGCGACTGGACCA
CCCCGCGATCCTGCCCCTCCTGGACCTGCATGTCGTCTCCGGGGTCACGTGTCTGGTCCTCCCCAAGTACCAGGCCGA
CCTGTATACCTATCTGAGTAGGCGCCTGAACCCGCTGGGACGCCCGCAGATCGCAGCGGTCTCCCGGCAGCTCCTAAG
CGCCGTTGACTACATTCACCGCCAGGGCATTATCCACCGCGACATTAAGACCGAAAATATTTTTATTAACACCCCCGA
GGACATTTGCCTGGGGGACTTTGGTGCCGCGTGCTTCGTGCAGGGTTCCCGATCAAGCCCCTTCCCCTACGGAATCGC
CGGAACCATCGACACCAACGCCCCCGAGGTCCTGGCCGGGGATCCGTATACCACCACCGTCGACATTTGGAGCGCCGG
TCTGGTGATCTTCGAGACTGCCGTCCACAACGCGTCCTTGTTCTCGGCCCCCCGCGGCCCCAAAAGGGGCCCGTGCGA
CAGTCAGATCACCCGCATCATCCGACAGGCCCAGGTCCACGTTGACGAGTTTTCCCCGCATCCAGAATCGCGCCTCAC
CTCGCGCTACCGCTCCCGCGCGGCCGGGAACAATCGCCCGCCGTACACCCGACCGGCCTGGACCCGCTACTACAAGAT
GGACATAGACGTCGAATATCTGGTTTGCAAAGCCCTCACCTTCGACGGCGCGCTTCGCCCCAGCGCCGCAGAGCTGCT
TTGTTTGCCGCTGTTTCAACAGAAATGACCGCCCCCAGGGGGCGGTGCTGTTTGCGGGTTGGCACAAAAAGACCCCGA
CCCGCGTCTGTGGTGTTTTTGGCATCATGTCGCCGGGCGCCATGCGTGCCGTTGTTCCCATTATCCCATTCCTTTTGG
TTCTTGTCGGTGTATCGGGGGTTCCCACCAACGTCTCCTCCACCACCCAACCCCAACTCCAGACCACCGGTCGTCCCT
CGCATGAAGCCCCCAACATGACCCAGACCGGCACCACCGACTCTCCCACCGCCATCAGCCTTACCACGCCCGACCACA
CACCCCCCATGCCAAGTATCGGACTGGAGGAGGAGGAAGAGGAGGAGGGGGCCGGGGACGGCGAACATCTTGAGGGGG
GAGATGGGACCCGTGACACCCTACCCCAGTCCCCGGGCCCAGCCTTCCCGTTGGCTGAGGACGTCGAGAAGGACAAAC
CCAACCGTCCCGTAGTCCCATCCCCCGATCCCAACAACTCCCCCGCGCGCCCCGAGACCAGTCGCCCGAAGACACCCC
CCACCATTATCGGGCCGCTGGCAACTCGCCCCACGACCCGACTCACCTCAAAGGACGACCCTTGGTTCCGACGCCTC
AACATACCCCGCTGTTCTCGTTCCTCACTGCCTCCCCCGCCCTGGACACCCTCTTCGTCGTCAGCACCGTCATCCACA
CCTTATCGTTTTTGTGTATTGGTGCGATGGCGACACACCTGTGTGGCGGTTGGTCCAGACGCGGGCGACGCACACACC
CTAGCGTGCGTTACGTGTGCCTGCCGTCCGAACGCGGGTAGGGTATGGGGCGGGGGATGGGGAGAGCCCACACGCGGA
AAGCAAGAACAATAAAGGCGGTGGTATCTAGTTGATATGCATCTCTGGGTGTTTTTGGGGTGTGGCGGACGCGGGGCG
GTCATTGGACGGGGTGCAGTTAAATACATGCCCGGGACCCATGAAGCATGCGCGACTTCCGGGCCTCGGAACCCACCC
GAAACGGCCAACGGACGTCTGAGCCAGGCCTGGCTATCCGGAGAAACAGCACACGACTTGGCGTTCTGTGTGTCGCGA
TGTCTCTGCGCGCAGTCTGGCATCTGGGGCTTTTGGGAAGCCTCGTGGGGGCTGTTCTTGCCGCCACCCATCGGGGAC
CTGCGGCCAACACAACGGACCCCTTAACACACGCCCCAGTGTCCCCTCACCCCAGCCCCCTGGGGGGCTTTGCCGTCC
CCCTCGTAGTCGGTGGGCTGTGCGCCGTAGTCCTGGGGGCGGCGTGTCTGTTTGAGCTCCTGCGTCGTACGTGCCGCG
GGTGGGGGCGTTACCATCCCTACATGGACCCAGTTGTCGTATAATTCCCCCCCCCCCCCCCTTCTCCGCATGGGTGAT
GTCGGGTCCAAACTCCCGACACCACCAGCTGGCATGGTATAAATCACCGGTGCGCCCCCCAAACCATGTCCGGCAGGG
GGATGGGGGGGCGAATGCGGAGGGCACCCAACAACACCGGGCTAACCAGGAAATCCGTGGCCCCGGCCCCCAATAAAG
ATCGCGGTAGCCCGGCCGTGTGACACTATCGTCCATACCGACCACACCGACGAATCCCCTAAGGGGGAGGGGCCATTT
TACGAGGAGGAGGGGTATAACAAAGTCTGTCTTTAAAAAGCAGGGGTTAGGGAGTTGTTCGGTCATAAGCTTCAGCGC
GAACGACCAACTACCCCGATCATCAGTTATCCTTAAGGTCTCTTTTGTGTGGTGCGTTCCGGTATGGGGGGGGCTGCC
 
GCCAGGTTGGGGGCCGTGATTTTGTTTGTCGTCATAGTGGGCCTCCATGGGGTCCGCGGCAAATATGCCTTGGCGGAT
GCCTCTCTCAAGATGGCCGACCCCAATCGCTTTCGCGGCAAAGACCTTCCGGTCCTGGACCAGCTGACCGACCCTCCG
GGGGTCCGGCGCGTGTACCACATCCAGGCGGGCCTACCGGACCCGTTCCAGCCCCCCAGCCTCCCGATCACGGTTTAC
TACGCCGTGTTGGAGCGCGCCTGCCGCAGCGTGCTCCTAAACGCACCGTCGGAGGCCCCCCAGATTGTCCGCGGGGCC
TCCGAAGACGTCCGGAAACAACCCTACAACCTGACCATCGCTTGGTTTCGGATGGGAGGCAACTGTGCTATCCCCATC
ACGGTCATGGAGTACACCGAATGCTCCTACAACAAGTCTCTGGGGGCCTGTCCCATCCGAACGCAGCCCCGCTGGAAC
TACTATGACAGCTTCAGCGCCGTCAGCGAGGATAACCTGGGGTTCCTGATGCACGCCCCCGCGTTTGAGACCGCCGGC
ACGTACCTGCGGCTCGTGAAGATAAACGACTGGACGGAGATTACACAGTTTATCCTGGAGCACCGAGCCAAGGGCTCC
TGTAAGTACGCCCTCCCGCTGCGCATCCCCCCGTCAGCCTGCCTGTCCCCCCAGGCCTACCAGCAGGGGGTGACGGTG
GACAGCATCGGGATGCTGCCCCGCTTCATCCCCGAGAACCAGCGCACCGTCGCCGTATACAGCTTGAAGATCGCCGGG
TGGCACGGGCCCAAGGCCCCATACACGAGCACCCTGCTGCCCCCGGAGCTGTCCGAGACCCCCAACGCCACGCAGCCA
GAACTCGCCCCGGAAGACCCCGAGGATTCGGCCCTCTTGGAGGACCCCGTGGGGACGGTGGCGCCGCAAATCCCACCA
AACTGGCACATACCGTCGATCCAGGACGCCGCGACGCCTTACCATCCCCCGGCCACCCCGAACAACATGGGCCTGATC
GCCGGCGCGGTGGGCGGCAGTCTCCTGGCAGCCCTGGTCATTTGCGGAATTGTGTACTGGATGCGCCGCCGCACTCAA
AAAGCCCCAAAGCGCATACGCCTCCCCCACATCCGGGAAGACGACCAGCCGTCCTCGCACCAGCCCTTGTTTTACTAG
ATACCCCCCCTTAATGGGTGCGGGGGGGTCAGGTCTGCGGGGTTGGGATGGGACCTTAACTCCATATAAAGCGAGTCT
GGAAGGGGGGAAAGGCGGACAGTCGATAAGTCGGTAGCGGGGGACGCGCACCTGTTCCGCCTGTCGCACCCACAGCTT
TTTTTGCGAACCGTCCCGTTCCGGGATGCCGTGCCGCCCGTTGCAGGGCCTGGTGCTCGTGGGCCTCTGGGTCTGTGC
CACCAGCCTGGTTGTCCGTGGCCCCACGGTCAGTCTGGTATCAAACTCATTTGTGGACGCCGGGGCCTTGGGGCCCGA
CGGCGTAGTGGAGGAAGACCTGCTTATTCTCGGGGAGCTTCGCTTTGTGGGGGACCAGGTCCCCCACACCACCTACTA
CGATGGGGTCGTAGAGCTGTGGCACTACCCCATGGGACACAAATGCCCACGGGTCGTGCATGTCGTCACGGTGACCGC
GTGCCCACGTCGCCCCGCCGTGGCTTTCGCCCTGTGTCGCGCGACCGACAGCACTCACAGCCCCGCATATCCCACCCT
GGAGCTGAATCTGGCCCAACAGCCGCTTTTGCGGGTCCGGAGGGCGACGCGTGACTATGCCGGGGTGTACGTGTTACG
CGTATGGGTCGGGGACGCACCAAACGCCAGCCTGTTTGTCCTGGGGATGGCCATAGCCGCCGAAGGGACTCTGGCGTA
CAACGGCTCGGCCCATGGCTCCTGCGACCCGAAACTGCTTCCGTATTCGGCCCCGCGTCTGGCCCCGGCGAGCGTATA
CCAACCCGCCCCTAACCCGGCCTCCACCCCCTCGACCACCACCTCCACCCCCTCGACCACCATCCCCGCTCCCTCGAC
CACCATCCCCGCTCCCCAAGCATCGACCACACCCTTCCCCACGGGAGACCCAAAACCCCAACCTCACGGGGTCAACCA
CGAACCCCCATCGAATGCCACGCGAGCGACCCGCGACTCGCGATACGCGCTAACGGTGACCCAGATAATCCAGATAGC
CATCCCCGCGTCCATTATAGCCCTGGTGTTTCTGGGGAGCTGTATTTGCTTTATACACAGATGTCAACGCCGCTACCG
ACGCTCCCGCCGCCCGATTTACAACCCCCAGATACCCACTGGCATCTCATGCGCGGTGAACGAAGCGGCCATGGCCCG
CCTCGGAGCCGAGCTCAAATCGCATCCGAGCACCCCCCCCAAATCCCGGCGCCGGTCGTCACGCACACCAATGCCCTC
CCTGACGGCCATCGCCGAAGAGTCGGAGCCCGCGGGGGCGGCTGGGCTTCCGACGCCCCCCGTGGACCCCACGACATC
CACCCCCAACGCCTCCCCTGTTGGTATAGGTCCACGGCCACTGGCCGGGGGCACCACATAACCGACCGCAGTCACTGAG
TTGGGAATAAACCGGTATTATTTACCTATATACGTGTATGTCCATTTCTTCCCCCCCCCCCCGGAAACCAAAGAAGGA
AACAAAGAATGGATGGAGGAGTTCAGGAAACCGGGGAGAGGGCCCGCGGCGCATTTAAGGCGTTGTTGTGTTGACTT
TGGCTCTTCTGGCGGGTTGGTGCGGTGCTGTTTGTTGGGCTCCCATTTTACCCGAAGATCGGCTGCTATCCCCGGGAC
ATGGATCGCGGGGCGGTGGTGGGGTTTCTTCTCGGTGTTTGTGTTGTATCGTGCTTGGCGGAACGCCCAAAACGTCC
TGGAGACGGGTGAGTGTCGGCGAGGACGTTTCGTTGCTTCCAGCTCCGGGGCCTACGGGGCGCGGCCCGACCCAGAAA
 
CTACTATGGGCCGTGGAACCCCTGGATGGGTGCGGCCCCTTACACCCGTCGTGGGTCTCGCTGATGCCCCCCAAGCAG
GTGCCCGAGACGGTCGTGGATGCGGCGTGCATGCGCGCTCCGGTCCCGCTGGCGATGGCGTACGCCCCCCCGGCCCCA
TCTGCGACCGGGGGTCTACGGACGGACTTCGTGTGGCAGGAGCGCGCGGCCGTGGTTAACCGGAGTCTGGTTATTTAC
GGGGTCCGAGAGACGGACAGCGGCCTGTATACCCTGTCTGTGGGCGACATAAAGGACCCGGCTCGCCAAGTGGCCTCG
GTGGTCCTGGTGGTGCAACCGGCCCCAGTTCCGACCCCACCCCCGACCCCAGCCGATTACGACGAGGATGACAATGAC
GAGGGCGAGGACGAAAGTCTAGCCGGCACTCCCGCCAGCGGGACCCCCCGGCTCCCGCCTCCCCCCGCCCCCCCGAGG
TCTTGGCCCAGCGCCCCCGAAGTCTCACACGTGCGTGGGGTGACCGTGCGTATGGAGACTCCGGAAGCTATCCTGTTT
TCCCCCGGGGAGGCGTTTAGCACGAACGTCTCCATCCATGCCATCGCCCACGACGACCAGACCTACACCATGGACGTC
GTCTGGTTGAGGTTCGACGTGCCGACCTCGTGTGCCGAGATGCGAATATACGAATCGTGTCTGTATCACCCGCAGCTC
CCAGAGTGTCTGTCCCCGGCCGACGCTCCGTGCGCCGCGAGTACGTGGACGTCTCGCCTGGCCGTCCGCAGCTACGCG
GGGTGTTCCAGAACAAACCCCCCGCCGCGCTGTTCGGCCGAGGCTCACATGGAGCCCTTCCCGGGGCTGGCGTGGCAG
GCGGCCTCCGTCAATCTGGAGTTCCGGGACGCGTCCCCACAACACTCCGGCCTGTATCTGTGCGTGGTGTACGTCAAC
GACCATATTCACGCATGGGGCCACATTACCATCAGCACCGCGGCGCAGTACCGGAACGCGGTGGTGGAACAGCCCCTC
CCACAGCGCGGCGCGGATTTGGCCGAGCCCACCCACCCGCACGTCGGGGCCCCTCCCCACGCGCCCCCAACCCACGGC
GCCCTGCGGTTAGGGGCGGTGATGGGGGCCGCCCTGCTGCTGTCTGCGCTGGGGTTGTCGGTGTGGGCGTGTATGACC
TGTTGGCGCAGGCGTGCCTGGCGGGCGGTTAAAAGCAGGGCCTCGGGTAAGGGGCCCACGTACATTCGCGTGGCCGAC
AGCGAGCTGTACGCGGACTGGAGCTCGGACAGCGAGGGAGAACGCGACCAGGTCCCGTGGCTGGCCCCCCCGGAGAGA
CCCGACTCTCCCTCCACCAATGGATCCGGCTTTGAGATCTTATCACCAACGGCTCCGTCTGTATACCCCCGTAGCGAT
GGGCATCAATCTCGCCGCCAGCTCACAACCTTTGGATCCGGAAGGCCCGATCGCCGTTACTCCCAGGCCTCCGATTCG
TCCGTCTTCTGGTAAGGCGCCCCATCCCGAGGCCCCACGTCGGTCGCCGAACTGGGCGACCGCCGGCGAGGTGGACGT
CGGAGACGAGCTAATCGCGATTTCCGACGAACGCGGACCCCCCCGACATGACCGCCCGCCCCTCGCCACGTCGACCGC
GCCCTCGCCACACCCGCGACCCCCGGGCTACACGGCCGTTGTCTCCCCGATGGCCCTCCAGGCTGTCGACGCCCCCTC
CCTGTTTGTCGCCTGGCTGGCCGCTCGGTGGCTCCGGGGGGCTTCCGGCCTGGGGGCCGTCCTGTGTGGGATTGCGTG
GTATGTGACGTCAATTGCCCGAGGCGCATAAAGGGCCGGTGGTCCGCCTAGCCGCAGCAAATTAAAAATCGTGAGTCA
CTGCGACCGCAACTTCCCACCCGGAGCTTTCTTCCGGCCTCGATGACGTCCCGGCTCTCCGATCCCAACTCCTCAGCG
CGATCCGACATGTCCGTGCCGCTTTATCCCACGGCCTCGCCAGTTTCGGTCGAAGCCTACTACTCGGAAAGCGAAGAC
GAGGCGGCCAACGACTTCCTCGTACGCATGGGCCGCCAACAGTCGGTATTAAGGCGTCGACGCAGACGCACCCGCTGC
GTCGGCATGGTGATCGCCTGTCTCCTCGTGGCCGTTCTGTCGGGCGGATTTGGGGCGCTCCTGATGTGGCTGCTCCGC
TAAAAGACCGCATCGACACGCGCGTCCTTCTTGTCGTCTCTCTTCCCCCCCATCACCCCGCAATTTGCACCCAGCCTT
TAACTACATTAAATTGGGTTCGATTGGCAATGTTGTCTCCCGGTTGATTTTTGGGTGGGTGGGGAGTGGGTGGGTGGG
GAGTGGGTGGGTGGGGAGTGGGTGGGTGGGGAGTGGGTGGGTGGGGAGTGGGTGGGTGGGGAGTGGGTGGGTGGGGAG
TGGGTGGGTGGGGAGTGGGTGGGTGGGGAGTGGGTGGGTGGGGAGTGGCAAGGAAGAAACAAGCCCGACCACCAGACA
GAAAATGTAACCATACCCAAACCGACTCTGGGGGCTGTTTGTGGGGTCGGAACCATAGGATGAACAAACCACCCCGTA
CCTCCCGCACCCTTGGGTGCGGTGGCTCATCGGCATCTGTCCGGTATGGGTTGTTCCCCACCCACTTGCGTTCGGACG
TCTTAGAATCATGGCGGTTTTCTATGCCGACATCGGTTTTCTCCCCCGCAATAAGACACGATGCGATAAAATCTGTTT
GTGAAATTTATTAAGGGTACAAATTGCCCTAGCACAGGGGTGGGGTTAGGGCCGGGTCCCCACACCCAAACGCACCAA
ACAGATGCAGGCAGTGGGTCGAGTACAGCCCCGCGTACGAACACGTCGATGCGTGTGTCAGACAGCACCAGAAAGCAC
AGGCCATCAACAGGTCGTGCATATGTCGGTGGGTTTGGACGCGGGGGGCCATGGTGGTGATAAAGTTAATGGCCGCCG
 
TCCGCCAGGGCCACAGGGGCGACGTCTCTTGGTTGGCCCGGAGCCACTGGGTGTGGACCAGCCGCGCGTGGCGGCCCA
ACATGGCCCCTGTAGCCGGGGGCGGGGATCGCGCACGTTTGCAGCGCACATGCGAGACACCTCGACCACGGTTCGGA
AGAAGGCCCGGTGGTCCGCGGGCAACATCACCAGGTGCGCAAGCGCCCGGGCGTCCAGAGGGTAGAGCCCTGAGTCAT
CCGAGGTTGGCTCATCGCCCGGGTCATGCCGCAAGTGCGTGTGGGTTGGGCTTCCGGTGGGCGGACGCGAACCGCGG
TGTGGAGCCCTACGCGGGCCCGAGCGTACGCTCCATCTTGTGGGGAGAAGGGGTCTGGGCTCGCCAGGGGGGCATACT
TGCCCGGGCTATACAGACCCGCGAGCCGTACGTGGTTCGCGGGGGGTGCGTGGGGTCCGGGGCTCCCGGGGAGGCCGG
GGCTCCCGGGGAGGCCGGGGCTCCCACCGGGGTTGTCGTGGATCCCTGGGGTCACGCGGTACCCTGGGGTCTCTGGGA
GCTCGCGGTACTCTGGGTTCCCTAGGTTCTCGGGGTGGTCGCAGAACCCGGGGCTCCCGGGGAACACGCGGTGTCCTG
GGGATTGTTGGCGGTCGGACGGCTTCAGATGGCTTCGAGATCGTAGTGTCCGCACCGACTCGTAGTAGACCCGAATCT
CCACATTGCCCCGCCGCTTGATCATTATCACCCCGTTGCGGGGGTCCGGAGATCATGCGCGGGTGTCCTCGAGGTGCG
TGAACACCTCTGGGGTGCATGCCGGCGGACGGCACGCCTTTTAAGTAAACATCTGGGTCGCCCGGCCCAACTGGGGCC
GGGGGTTGGGTCTGGCTCATCTCGAGAGCCACGGGGGGAACCACCCTCCGCCCAGAAACTTGGGCGATGGTCGTACCC
GGGACTCAACGGGTTACCGGATTACGGGGACTGTCGGTCACGGTCCCGCCGGTTCTTCGATGTGCCACACCCAAGGAT
GCGTTGGGGGCGATTTTGGGCAGCAGCCCGGGAGAGCGCAGCAGAGGACGCTCCGGGTCGTGCATGGCGGTTTTGGCT
GCCTCCCGGTCCTCACGCCCCCTTTTATTGATCTCATCGCGTACGTCGGCGTACGTCCTGGCCCAACCCGCATGTTG
TCCAGGAAGGTGTCCGCCATTTCCAGGGCCCACGACATGCTCCCCCCCGACGAGCAGGAAGCGGTCCACGCAACGGTC
GCCGCCGGTCGCCTCGACGAGGACGTTCCTCCTGCGGGAAGGCACGAACGCGGGTGAGCCCCCTCCTCCGCCCCCGTG
TCCCCCCTCCTCCGCCCCCGCGTCCCCCCTCCTCCGCCCCCGCGTCCCCCCTCCTCCGCCCCCGCGTCCCCCCTCCTC
CGCCCCCGCGTCCCCCCTCCTCCGCCCCCGCGTCCCCCCTCCTCCGCCCCCGTGTCCCCCCTCCTCCGCCCACCCAAG
GTGCTTACCCGTGCACAAAGGCGGACCGGTGGGTTTCTGTCGTCGGAGGCCCCCGGGGTGCGTCCCCTGTGTTTCGTG
GGTGGGGTGGGTGGGTCTTTCCGCGTGTCCCTTTCCGATGCGATCCCGATCCCGAGCCGGGGCGTCGCGATGCCGACG
CCGTCCGCTCCGACGGCCCTCTGCGAGTCCCGCTCCCGGTCCGCGTGCTCCGCAGCCGCTCCCGTCGTTCGTGGCCGG
CGCCGTCTGCGGGCGTCGGTCGCGCCGGGCCTTTATGTGCGCCGGAGAGACCCGCCCCCCGCCGCCCGGCCCGCCCC
CGGGGCCGGCGCGGAGTCGGGCACGGCGCCAGTGCTCGCACTTCGCCCTAATAATATATATATATTGGGACGAAGTGC
GAACGCTTCGCGTTCTCACTTCTTTACCCGGCGGCCCGCCCCCTTGGGGCGGTCCCGCCCGCCGGCCAATGGGGGG
GCGGCAAGGCGGGCGGCCCTTGGGCCGCCCGCCGTCCCGTTGGTCCCAACGTCCGGCGGGCGGACCGGGGGCCCGGG
GACGGCCAACGGGCGCGCGGGGCTCGTATCTCATTACCGCCGAACCGGGAAGTCGGGGCCCGGGCCCCGCCCCCGGCC
CGTTCCTCGTTAGCATGCGGAACGGAAGCGGAAACCACCGGATCGGGCGGTAATGAGATGCCATGCGGGGCGGGGCGC
GGGCCCACCCGCCCTCGCGCCCCGCCCATGGCAGATGGCGCGGATGGGCGGGGCCGGGGGTTCGACCAACGGGCCGCG
GCCACGGGCCCCCGGCGTGCCGGCGTCGGGGCGGGGTCGTGCATAATGGAATTCCGTTCGGGGCGGGCCCGCCTGGGG
GGCGGGGGGCCGGCGGCCTCCGCTGCTCCTCCTTCCCGCCGGCCCCTGGGACTATATGAGCCCGAGGACGCCCCGATC
GTCCACACGGAGCGCGGCTGCCGACACGGATCCACGACCCGACGCGGGACCGCCAGAGACAGACCGTCAGACGCTCGC
CGCGCCGGGACGCCGATACGCGGACGAAGCGCGGGAGGGGGATCGGCCGTCCCTGTCCTTTTTCCCACCCAAGCATCG
ACCGGTCCGCGCTAGTTCCGCGTCGACGGCGGGGGTCGTCGGGGTCCGTGGGTCTCGCCCCCTCCCCATCGAGAGTCC
GTAGGTGACCTACCGTGCTACGTCCGCCGTCGCAGCCGTATCCCCGGAGGATCGCCCCGCATCGGCGATGGCGTCGGA
GAACAAGCAGCGCCCCGGCTCCCCGGGCCCCACCGACGGGCCGCCGCCCACCCCGAGCCCAGACCGCGACGAGCGGGG
GGCCCTCGGGTGGGGCGCGGAGACGGAGGAGGGCGGGGACGACCCCGACCACGACCCCGACCACCCCCACGACCTCGA
CGACGCCCGGCGGGACGGGAGGGCCCCCGCGGCGGGCACCGACGCCGGCGAGGACGCCGGGGACGCCGTCTCGCCGCG
 
ACAGCTGGCCCTGCTGGCCTCCATGGTAGAGGAGGCCGTCCGGACGATCCCGACGCCCGACCCCGCGGCCTCGCCGCC
CCGGACCCCCGCCTTTCGAGCCGACGACGATGACGGGGACGAGTACGACGACGCAGCCGACGCCGCCGGCGACCGGGC
CCCGGCCCGGGGCCGCGCACGGGAGGCCCCGCTACGCGGCGCGTATCCGGACCCCACGGACCGCCTGTCGCCGCGCCC
GCCGGCCCAGCCGCCGCGGAGACGTCGTCACGGCCGGCGGCGGCCATCGGCGTCATCGACCTCGTCGGACTCCGGGTC
CTCGTCCTCGTCGTCCGCATCCTCTTCGTCCTCGTCGTCCGACGAGGACGAGGACGACGACGGCAACGACGCGGCCGA
CCGCGCACGCGAGGCGCGGGCCGTCGGGCGGGGTCCGTCGAGCGCGGCGCCGGAAGCCCCCGGGCGGACGCCGCCCCC
GCCCGGGCCACCCCCCCTCTCCGAGGCCGCGCCCAAGCCCCGGGCGGCGGCGAGGACCCCCGCGGCCTCCGCGGGCCG
CATCGAGCGCCGCCGGGCCCGCGCGGCGGTGGCCGGCCGCGACGCCACGGGCCGCTTCACGGCCGGGCAGCCCCGGCG
GGTCGAGCTGGACGCCGACGCGGCCTCCGGCGCCTTCTACGCGCGCTATCGCGACGGGTACGTCAGCGGGGAGCCGTG
GCCCGGCGCCGGGCCCCCGCCCCCGGGGCGGGTGCTGTACGGCGGCCTGGGCGACAGCCGCCCGGGCCTCTGGGGGGC
GCCCGAGGCGGAGGAGGCGCGACGCCGGTTCGAGGCCTCGGGCGCCCCGGCGGCCGTGTGGGCGCCCGAGCTGGGCGA
CGCCGCGCAGCAGTACGCCCTGATCACGCGGCTGCTGTACACCCCGGACGCGGAGGCCATGGGGTGGCTCCAGAACCC
GCGCGTGGTCCCCGGGGACGTGGCGCTGGACCAGGCCTGCTTCCGGATCTCGGGCGCCGCGCGCAACAGCAGCTCCTT
CATCACCGGCAGCGTGGCGCGGGCCGTGCCCCACCTGGGCTACGCCATGGCGGCCGGCCGCTTCGGCTGGGGCCTGGC
GCACGCGGCGGCCGCCGTGGCCATGAGCCGCCGATACGACCGCGCGCAGAAGGGCTTCCTGCTGACCAGCCTGCGCCG
CGCCTACGCGCCCCTGTTGGCGCGCGAGAACGCGGCGCTGACGGGGGCCGCGGGGAGCCCCGGCGCCGGCGCAGATGA
CGAGGGGGTCGCCGCCGCCGCCGCCGCCGCACCGGGCGAGCGCGCGGTGCCCGCCGGGTACGGCGCCGCGGGGATCCT
CGCCGCCCTGGGGCGGCTGTCCGCCGCGCCCGCCTCCCCCGCGGGGGGCGACGACCCCGACGCCGCCCGCCACGCCGA
CGCCGACGACGACGCCGGGCGCCGCGCCCAGGCCGGCCGCGTGGCCGTGGAGTGCCTGGCCGCCTGCCGCGGGATCCT
GGAGGCGCTGGCCGAGGGCTTCGACGGCGACCTGGCGGCCGTCCCGGGGCTGGCCGGGGCCCGGCCCGCCAGCCCCCC
GCGGCCGGAGGGACCCGCGGGCCCCGCTTCCCCGCCGCCGCCGCACGCCGACGCGCCCCGCCTGCGCGCGTGGCTGCG
CGAGCTGCGGTTCGTGCGCGACGCGCTGGTGCTCATGCGCCTGCGCGGGGACCTGCGCGTGGCCGGCGGCAGCGAGGC
CGCCGTGGCCGCCGTGCGCGCCGTGAGCCTGGTCGCCGGGGCCCTGGGCCCCGCGCTGCCGCGGACCCGCGCCTGCC
GAGCTCCGCGGCCGCCGCCGCCGCGGACCTGCTGTTTGAGAACCAGAGCCTGCGCCCCCTGCTGGCGGCGGCGGCCAG
CGCACCGGACGCCGCCGACGCGCTGGCGGCCGCCGCCGCCTCCGCCGCGCCGCGGGAGGGGCGCAAGCGCAAGAGTCC
CGGCCCGGCCCGGCCGCCCGGAGGCGGCGGCCCGCGACCCCCGAAGACGAAGAAGAGCGGCGCGGACGCCCCCGGCTC
GGACGCCCGCGCCCCCCTCCCCGCGCCCGCGCCCCCCTCCACGCCCCCGGGGCCCGAGCCCGCCCCCGCCCAGCCCGC
GGCGCCCCGGGCCGCCGCGGCGCAGGCCCGCCCGCGCCCCGTGGCCGTGTCGCGCCGGCCCGCCGAGGGCCCCGACCC
CCTGGGCGGCTGGCGGCGGCAGCCCCCGGGGCCCAGCCACACGGCGGCGCCCGCGGCCGCCGCCCTGGAGGCCTACTG
CTCCCCGCGCGCCGTGGCCGAGCTCACGGACCACCCGCTGTTCCCCGTCCCCTGGCGACCGGCCCTCATGTTTGACCC
GCGGGCCCTGGCCTCGATCGCCGCGCGGTGCGCCGGGCCCGCCCCCGCCGCCCAGGCCGCGTGCGGCGGCGGCGACGA
CGACGATAACCCCCACCCCCACGGGGCCGCCGGGGGCCGCCTCTTTGGCCCCCTGCGCGCCTCGGGCCCGCTGCGCCG
CATGGCGGCCTGGATGCGCCAGATCCCCGACCCCGAGGACGTGCGCGTGGTGGTGCTGTACTCGCCGCTGCCGGGCGA
GGACCTGGCCGGCGGCGGGGCCTCGGGGGGGCCGCCGGAGTGGTCCGCCGAGCGCGGCGGGCTGTCCTGCCTGCTGGC
GGCCCTGGCCAACCGGCTGTGCGGGCCGGACACGGCCGCCTGGGCGGGCAACTGGACCGGCGCCCCCGACGTGTCGGC
GCTGGGCGCGCAGGGCGTGCTGCTGCTGTCCACGCGGGACCTGGCCTTCGCCGGGGCCGTGGAGTTTCTGGGGCTGCT
CGCCAGCGCCGGCGACCGGCGGCTCATCGTGGTCAACACCGTGCGCGCCTGCGACTGGCCCGCCGACGGCCCGCGGT
GTCGCGGCAGCACGCCTACCTGGCGTGCGACCTGCTGCCCGCCGTGCAGTGCGCCGTGCGCTGGCCGGCGGCGCGGGA
 
CCTGCGCCGCACGGTGCTGGCCCCGGGCCGCGTGTTCGGCCCGGGGGTCTTCGCGCGCGTGGAGGCCGCGCACGCGCG
CCTGTACCCCGACGCGCCGCCGCTGCGCCTGTGCCGCGGCGGCAACGTGCGCTACCGCGTGCGCACGCGCTTCGGCCC
GGACACGCCGGTGCCCATGTCCCCGCGCGAGTACCGCCGGGCCGTGCTGCCGGCGCTGGACGGCCGGGCGGCGGCCTC
GGGGACCACCGACGCCATGGCGCCCGGCGCGCCGGACTTCTGCGAGGAGGAGGCCCACTCGCACCGCGCCTGCGCGCG
CTGGGGCCTGGGCGCGCCGCTGCGGCCCGTGTACGTGGCGCTGGGGCGCGAGGCGGTGCGCGCCGGCCCGGCCCGGTG
GCGCGGGCCGCGGAGGGACTTTTGCGCCCGCGCCCTGCTGGAGCCCGACGACGACGCCCCCCCGCTGGTGCTGCGCGG
CGACGACGACGGCCCGGGGGCCCTGCCGCCGGCGTTGCCCGGGATTCGCTGGGCCTCGGCCACGGGCCGCAGCGGCAC
CGTGCTGGCGGCGGCGGGGGCCGTGGAGGTGCTGGGGGCGGAGGCGGGCTTGGCCACGCCCCCGCGACGGGAAGTTGT
GGACTGGGAAGGCGCCTGGGACGACGACGACGGCGGCGCGTTCGAGGGGGACGGGGTGCTGTAACGGGCCGGGACGGG
GCGGGGCGCTTGCGAAACCCGAAGACACAATAAACGGCAACAACCTGATTTAGTTTTGCAGTAGCGTTGTTTATTTGA
GGGGCGGGAGGGGGCGAGGGGCGGGAGGGGGCGAGGGGCGGGAGGGGGCGAGGGGCGGGAGGGGGCGAGGGGCGGGAG
GGGGCGAGGGGCGGGAGGGGGCGAGGGGCGGGAGGGGGCGAGGGGCGGGAGGGGGCGAGGGGCGGGAGGGGGCGAGGG
GCGGGAGGGGGCGAGGGGCGGGAGGGGGCGAGGGGCGGGAGGGGGCGAGGGGCGGGAGGGGGCGAGGGGCGGGAGGGG
GCGAGGGGCGGGAGGGGGCGAGGGGCGGGAGGGGGCGAGGGGCGGGAGGGGGCGAGGGGCGGGAGGGGGCGAGGGGCG
GGAGGGGGCGAGGGGCGGTGGTGGTGCGCGGGCGCCCCCGGAGGGTTTGGATCTCTGACCTGAGATTGGCGGCACTGA
GGTAGAGATGCCCGAACCCCCCCGAGGGAGCGCGGGACGCGCCGGGGAGGGCTGGGGCCGGGGAGGGCTGGGGCCGGG
GAGGGCTGGGGCCGGGGAGGGCTGGGGCCGGGGAGGGCTGGGGCCGGGGAGGGCTGGGGCTGGGGAGGGCTGGGGCTG
GGGAGGGCTGGGGCTGGTGGTGTGTGACAGGAGCGGCGTGTTGCGCTGGGGGACGTCTGGAGGAGCGGGGGGTGCGCG
GTGACGTGTGGATGAGGAACAGGAGTTGTTGCGCGGTGAGTTGTCGCTGTGAGTTGTGTTGGTGGGCAGGTGTGGTGG
ATGACGTGACGTGTGACGTGTGGATGAGGCGTGCTCTGTTGGTTTCACCTGTGGCAGCCCGGGCCCCCCGCGGGCGGG
GCGGCGCGCAAAAAAGGCGGGCGGCGGTCCGGGCGGCGTGCGCGCGCGCGGCGGGCGTTGGGGGCGGGGCCGCGGGAG
CGGGGGGAGGAGCGGGGGGAGGAGCGGGGGGAGGAGCGRRGGGAGGAGCGGGGGGAGGAGCGGGGGGAGGAGCGGGGG
GAGGAGCGGGGGGAGGAGCGGGGGGAGGAGCGGGGGGAGGAGCGGGGGGAGGAGCGGGGGGAGGAGCGGGGGGAGGAG
CGGGGGGAGGAGCGGGGGGAGGAGCGGGGGGAGGAGCGGGGGGAGGAGCGGGGGGAGGAGCGAAAACGGCCCCCCCC
SAAACACACCCCCCGGGGGTCGCGCGCGGCCCTTTAAAGCGGTGGCGGCGCAGCCCGGGCCCCCCGCGGGCGCGCGCG
CGCGCAAAAAAGGCGGGCGGCGGTCCGGGCGGCGTGCGCGCGCGCGGCGGGCGTGGGGGGCGGGGCCGCGGGAGCGGG
GGGAGGAGCGGGGGGAGGAGCGGGGGGAGGAGCGGGGGGAGGAGCGGGGGGAGGAGCGGGGGGAGGAGCGGGGGGAGG
AGCGGGGGGAGGAGCGGGGGGAGGAGCGGGGGGAGGAGCGGGGGGAGGAGCGGGGGGAGGAGCGGGGGGAGGAGCGGG
GGGAGGAGCGGGGGGAGGAGCGGGGGGAGGAGCGGGGGGAGGAGCGGGGGGAGGAGCGAAAACGGCCCCCCCCAAAA
CACACCCCCCGGGGGTCGCGCGCGGCCCTTTAAAGCGGGCGGCGGCAGCCCGGGCCCCCCGCGGCCGAGACGAGCGAG
TTAGACAGGCAAGCACTACTCGCCTCTGCACGCACATGCTTGCCTGTCAAACTCTACCACCCCGGCACGCTCTCTGTC
TCCATGGCCCGCCGCCGCCGCCATCGCGGCCCCCGCCGCCCCCGGCCGCCCGGCCCACGGGCGCCGTCCCAACCGCA
CAGTCCCAGGTAACCCTTGTTCCGCTTCCCGGTATGGTAATTAGAAACTCATTAATGGGCGGCCCCGGCCGCCCTTCC
CGCTTCCGGCAATTCCCGCGGCCCTTAATGGGCAACCCCGGTATTCCCCGCCTCCCGCGCCGCGCGTAACCACTCCCC
TGGGGTTCCGGGTTATGCTAATTGCTTTTTTGGCGGAACACACGGCCCCTCGCGCATTGGCCCGCGGGTCGCTCAATG
AACCCGCATTGGTCCCCTGGGGTTCCGGGTATGGTAATGAGTTTCTTCGGGAAGGCGGGAAGCCCCGGGGCACCGACG
CAGGCCAAGCCCCTGTTGCGTCGGTGGGAGGGGCATGCTAATGGGGTTCTTTGGGGGACACCGGGTTGGTCCCCCAAA
TCGGGGGCCGGGCCGTGCATGCTAATGATATTCTTTGGGGGCGCCGGGTTGGTCCCCGGGGACGGGGCCGCCCCGCGG
 
TGGGCCTGCCTCCCCTGGACGCGCGGCCATTGGGGGAATCGTCACTGCCGCCCCTTTGGGGAGGGGAAAGGCGTGGG
GTATAAGTTAGCCCTGGCCCGACAGTCTGGTCGCATTTGCACCTCGGCACTCGGAGCGAGACGCAGCAGCCAGGCAGA
CTCGGGCCGCCCCCTCTCCGCATCACCACAGAAGCCCCGCCTACGTTGCGACCCCCAGGGACCCTCCGTCCGCGACCC
TCCAGCCGCATACGACCCCCATGGAGCCCCGCCCCGGAGCGAGTACCCGCCGGCCTGAGGGCCGCCCCCAGCGCGAGG
TGAGGGGCCGGGCGCCATGTCTGGGGCGCCATATTGGGGGGCGCCATATTGGGGGGCGCCATGTTGGGGGACCCCCGA
CCCTTACACTGGAACCGGCCGCCATGTTGGGGGACCCCCACTCATACACGGGAGCCGGGCGCCATGTTGGGGCGCCAT
GTTAGGGGGCGTGGAACCCCGTGACACTATATATACAGGGACCGGGGGCGCCATGTTAGGGGGCGCGGAACCCCCTGA
CCCTATATATACAGGGACCGGGGTCGCCCTGTTGGGGGTCGCCATGTGACCCCCTGACTTTATATATACAGACCCCCC
AACACATACACATGGCCCCTTTGACTCAGACGCAGGGCCCGGGGTCGCCGTGGACCCCCTGACTCATACACAGAGAC
ACGCCCCCACAACAAACACACAAGGACCGGGGTCGCCGTGTTAGGGGGCGTGGTCCCACTGACTCATACGCAGGGCC
CCCTTACTCACACGCATCTAGGGGGGTGGGGAGGAGCCGCCCGCCATATTTGGGGGACGCCGTGGGACCCCCGACTCC
GGTGCGTCTGGAGGGCGGGAGAAGAGGGAAGAAGAGGGGTCGGGATCCAAAGGACGGACCCAGACCACCTTTGGTTGC
AGACCCCTTTCTCCCCCCTCTTCCGAGGCCAGCAGGGGGGCAGGACTTTGTGAGGCGGGGGGGGGAGAGGGGGAACTC
GTGGGCGCTGATTGACGCGGGAAATCCCCCCATTCTTACCCGCCCCCCTTTTTTCCCCTTAGCCCGCCCCGGATGTCT
GGGTGTTTCCCTGCGACCGAGACCTGCCGGACAGCAGCGACTCTGAGGCGGAGACCGAAGTGGGGGGGCGGGGGGACG
CCGACCACCATGACGACGACTCCGCCTCCGAGGCGGACAGCACGGACACGGAACTGTTCGAGACGGGGCTGCTGGGGC
CGCAGGGCGTGGATGGGGGGGCGGTCTCGGGGGGGAGCCCCCCCGCGAGGAAGACCCCGGCAGTTGCGGGGGCGCCC
CCCCTCGAGAGGACGGGGGGAGCGACGAGGGCGACGTGTGCGCCGTGTGCACGGATGAGATCGCGCCCCACCTGCGCT
GCGACACCTTCCCGTGCATGCACCGCTTCTGCATCCCGTGCATGAAAACCTGGATGCAATTGCGCAACACCTGCCCGC
TGTGCAACGCCAAGCTGGTGTACCTGATAGTGGGCGTGACGCCCAGCGGGTCGTTCAGCACCATCCCGATCGTGAACG
ACCCCCAGACCCGCATGGAGGCCGAGGAGGCCGTCAGGGCGGGCACGGCCGTGGACTTTATCTGGACGGGCAATCAGC
GGTTCGCCCCGCGGTACCTGACCCTGGGGGGGCACACGGTGAGGGCCCTGTCGCCCACCCACCCTGAGCCCACCACGG
ACGAGGATGACGACGACCTGGACGACGGTGAGGCGGGGGGCGGCGAGGACCCTGGGGGAGGAGGAGGAGGAGGGGGG
AGGGAGGAATAGGCGGGCGGGGGAGGAAAGGAGGGCCTGGGAGGGGGCGTAACCTGATCGCGCCCCCCGTTGTCTCT
TGCAGCAGACTACGTACCGCCCGCCCCCCGCCGGACGCCCCGCGCCCCCCCACGCAGAGGCGCCGCCGCGCCCCCCGT
GACGGGCGGGGCGTCTCACGCAGCCCCCCAGCCGGCCGCGGCTCGGACAGCGCCCCCCTCGGCGCCCATCGGGCCACA
CGGCAGCAGTAACACCAACACCACCACCAACAGCAGCGGCGGCGGCGGCTCCCGCCAGTCGCGAGCCGCGGCGCCGCG
GGGGGCGTCTGGCCCCTCCGGGGGGGTTGGGGTTGGGGTTGGGGTTGTTGAAGCGGAGGCGGGGCGGCCGAGGGGCCG
GACGGGCCCCCTTGTCAACAGACCCGCCCCCCTTGCAAACAACAGAGACCCCATAGTGATCAGCGACTCCCCCCCGGC
CTCTCCCCACAGGCCCCCCGCGGCGCCCATGCCAGGCTCCGCCCCCCGCCCCGGGCCCACCGCGTCCTCGGCCGCGTC
GGGACCCGCGCGCCCCCGCGCGGCCGTGGCCCCGTGCGTGCGAGCGCCGCCTCCGGGGCCCGGCCCCCGCGCCCCGGC
CCCCGGGGCGGAGCCGGCCGCCCGCCCCGCGGACGCGCGCCGTGTGCCCCAGTCGCACTCGTCCCTGGCTCAGGCCGC
GAACCAAGAACAGAGTCTGTGCCGGGCGCGTGCGACGGTGGCGCGCGGCTCGGGGGGGCCGGGCGTGGAGGGTGGACA
CGGGCCCTCCCGCGGCGCCGCCCCCTCCGGCGCCCCCCCGCTCCCCTCCGCCGCCTCTGTCGAGCAGGAGGCGGCGGT
GCGTCCGAGGAAGAGGCGCGGGTCGGGCCAGGAAAACCCCTCCCCCCAGTCCACGCGTCCCCCCCTCGCGCCGGCAGG
GGCCAAGAGGGCGGCGACGCACCCCCCCTCCGACTCAGGGCCGGGGGGGCGCGGCCAGGGTGGGCCCGGGACCCCCCT
GACGTCCTCGGCGGCCTCCGCCTCTTCCTCCTCTGCCTCTTCCTCCTCGGCCCCGACTCCCGCGGGGGCCGCCTCTTC
CGCCGCCGGGGCCGCGTCCTCCTCCGCTTCCGCCTCCTCGGCGGGGCCGTCGGTGCCCTGGGAGGGAGACAAGAGGA
 
AACCTCCCTCGGCCCCCGCGCTGCTTCTGGGCCGCGGGGGCCGAGGAAGTGTGCCCGGAAGACGCGCCACGCGGAGAC
TTCCGGGGCCGCCCCCGCGGGCGGCCTCACGCGCTACCTGCCCATCTCGGGGGTCTCTAGCGTGGTCGCCCTGTCGCC
TTACGTGAACAAGACGATCACGGGGGACTGCCTGCCCATCCTGGACATGGAGACGGGGAACATCGGGGCGTACGTGGT
CCTGGTGGACCAGACGGGAAACATGGCGACCCGGCTGCGGGCCGCGGTCCCCGGCTGGAGCCGCCGCACCCTGCTCCC
CGAGACCGCGGGTAACCACGTGATGCCCCCCGAGTACCCGACGGCCCCCGCGTCGGAGTGGAACAGCCTCTGGATGAC
CCCCGTGGGGAACATGCTGTTCGACCAGGGCACCCTAGTGGGCGCCCTGGACTTCCGCAGCCTGCGGTCTCGGCACCC
GTGGTCCGGGGAGCAGGGGGCGTCGACCCGGGACGAGGGAAAACAATAAGGGACGCCCCCGTGTTTGTGGGGAGGGG
GGGGTCGGGCGCTGGGTGGTCTCTGGCCGCGCCCACTACACCAGCCAATCCGTGTCGGGGAGGGGAAAGTGAAAGAC
ACGGGCACCACACACCAGCGGGTCTTTAGTGTTGGCCCTAATAAAAAACTCAGGGGATTTTTGCTGTCTATTGGGAAA
TAAAGGTTTACTTTTGTATCTTTTCCCTGTCTGTGTTGGATGGATCTCGGGGGTGCGTGGGAGTGGGGGTGCGTGGGA
GTGGGGGTGCGTGGGAGTGGGGGTGCGTGGGAGTGGGGGTGCGTGGGAGTGGGGGTGCGTGGGAGTGGGGGTGCGTGG
GAGTGGGGGTGCGTGGGAGTGGGGGTGCGTGGGAGTGGGGGTGCCATGTTGGGCAGGCTCTGGTGTTAACCACAGAGC
CGCGGCCCGGGCTGCCTGACCACCGATCCCCGAAAGCATCCTGCCATTGGCATGGAGCCAGAACCACAGTGGGTTGGG
TGTGGGTGTTAAGTTTCCGCGAGCGCCTGCCCGCCCGGACTGACCTGGCCTCTGGCCGCCACAAAGGGCGGGGGGGGT
TAACTACACTATAGGGCAACAAAGGACGGGAGGGGTGGCGGGGCGGGACGGGGCGCCCAAAAGGGGGTCGGCCACACC
ACAGACGTGGGTGTTGGGGGGTGGGGCGGAGGGGTGGGGGGGGGGGAGACAGAAACAGGAACATAGTTAGAAAACAAG
AATGCGGTGCAGCCAGAGAATCACAGGAGACGAGGGGATGGGCGTGTTGGTTACCAACCCACACCCAGGCATGCTCGG
TGGTATGAAGGAGGGGGGGCGGTGCTTCTTAGAGACCGCCGGGGGACGTGGGGTTGGTGTGCAGAGGCACGCGCACCC
GCGCGGCCAGGTGGGCCGGTACCCCATCCCCCCTCCCCCGACCCTTCCCACCCCCGCGTGCCAGAGATCACCCCGGTC
CCCCGGCACCCGCCACTCCTCCATATCCTCGCTTTAGGAACAACTTTAGGGGGGGTACACACGCGCCGTGCATTTCCT
TCCACACCCCCCCTCCCCCGCACTCCCCCCCCCCCGGCAGTAAGACCCAAGCATAGAGAGCCAGGCACAAAAACACAG
GCGGGGTGGGACACATGCCTTCTTGGAGTACGTGGGTCATTGGCGTGGGGGGTTACAGCGACACCGGCCGACCCCCTG
GCGGTCTTCCAGCCGGCCCTTAGATAAGGGGGCAGTTGGTGGTCGGACGGGTAAGTAACAGAGTCTGACTAAGGGTGG
GAGGGGGGGAAAAGAACGGGCTGGTGTGCTGTAACACGAGCCCACCCGCGAGTGGCGTGGCCGACCTTAGCCTCTGGG
GCGCCCCCTGTCGTTTGGGTCCCCCCCCTCTATTGGGGAGAAGCAGGTGTCTAACCTACCTGGAAACGCGGCGTCTTT
GTTGAACCACACCGGGGCGCCCTTGACGAGTGGGATAACGGGGGAGGAAGGGAGGGAGGAGGGTACTGGGGGTGAAGA
AGGGGGGGGGGAGAAGCGAGAACAGGAAAGGCGACGGAGCCCGACAAAACACCGAGAAAAAAAAACCACAGCGCATG
CGCCGGGCCGTTGTGGGGCCCCGGGCCGGGGCCCCTTGGGTCCGCCGGGGCCCCGGGCCGGGCCGCCACGGGGGCCGG
CCGTTGGCGGTAACCCCGATTGTTTATCTCAGGCCCCGGGCCGGGAACCCGGAAAAGCCTCCGGGGGGCCTTTTTCGC
GTCGCGTGCCGGCGAGCGGGCCCGGACGGGGCCCGGACCGCCGCGGTCGGGGGCCCCCTCGTCCCGGGCCGTACGCGG
CCTTCGCCCCGTGAGGGGACAGACGAACGAAACATTCCGGCGACGGAACGAAAAACACCCCAGACGGGTTAAAGAAAC
AGAAACCGCAACCCCCCCACCCCCGAAACGGGGAAAACAAAAAACAGACCAGCGGCCGGCCGGCGCTTAGGGGGAGG
ATGTCGCCGACGCCCCTTGGCCGCCCCGGCTGCAGGGGGGCCCGGAGAGCCGCGGCACCCGGACGCGCCCGGAAAGTC
TTTCGCACCACCCGCGATCGGCACGGCCGCGCCCCCGCTTTTATAAAGGCTCAGATGACGCAGCAAAAACAGGCCACA
GCACCACGTGGGTAGGTGATGTAATTTATTTTCCTCGTCTGCGGCCTAATGGATTTCCGGGCGCGGTGCCCCTGTCT
GCAGAGCACTTAACGGATTGATATCTCGCGGGCACGCGCGCCCTTAATGGACCGGCGCGGGGCGGGGGGCCGGATACC
CACACGGGCGGGGGGGGGGTGTCGCGGGCCGTCTGCTGGCCCGCGGCCACATAAACAATGACTCTGGGCCTTTCTGCC
TCTGCCGCTTGTGTGTGCGCGCGCCGGCTCTGCGGTGTCGGCGGCGGCTGCGGCGGCTGCGGCGGCCGCCGTGTTCGG
 
TCTCGGTAGCCGGCCGGCGGGTGGACTCGCGGGGGGCCGGAGGTGGAAGGCAGGGGGGTGTAGGATGGTATCAGGA
CTTCCACTTCCCGTCCTTCCATCCCCCGTTCCCCTCGGTTGTTCCTCGCCCCCCCCAACACCCCGCCGCTTTCCGTTG
GGGTTGTTATTGTTGTCGGGATCGTGCGGGCCGGGGGTCGCCGGGGCAGGGGCGGGGGCGTGGGCGGGGGTGCTCGTC
GATCGACCGGGCTCAGTGGGGGCGTGGGGTGGGTGGGAGAAGGCGAGGAGACTGGGGTGGGGGTGTCGGTGGGTGGTT
GTTTTTTGTGGTTGTTTTTGTGGCTGTTCCCGTCCCCCGTCACCCCCCTCCCTCCGTCCCCTCCGTCCCCCCGTCGCG
GGTGTTTGTGTTTGTTTATTCCGACATCGGTTTATTTAAATAAACACAGCCGTTCTGCGTGTCTGTTCTTGCGTGTGG
CTGGGGGCTTATATGTGGGGTCCCGGGGGCGGGATGGGGTTTAGCGGCGGGGGGCGGCGCGCCGGACGGGGCGCTGGA
GATAACGGCCCCCGGGGAACGGGGGACCGGGGCTGGGTCTCCCGAGGTGGGTGGGTGGGCGGCGGTGGCCGGGCCGGG
CCGGGCCGGGCCGGGCCGGGTGGGCGGGGTTTGGAAAAACGAGGAGGAGGAGGAGAAGGAGGGGGGGGGGGAGACGGG
GGGAAAGCAAGGACACGGCCCGGGGGGTGGAGCGCGGGCCGGGCCGCTCGTAAGAGCCGCGACCCGGCCACCGGGGA
GCGTTGTCGCCGTCGGTCTGCCGGCCCCCGTCCCTCCCTTTTTTGACCAACCAGCGCCCCCCCCCCCCCTCACCACCA
TTCCTACTACCACCACCACCACCACCACCGACACCTCCCGCGCACCCCCGCCCACATCCCCCCCCAACCCGCACCACG
AGCACGGGTTGGGGGTAGCAGGGGATCAAAGGGGGGCAAGGCCGGCGGGGCGGTTCGGGGGGGGGGGGGGGGGCGGG
AGACCGAGTAGGCCCCGCCCATCCGCGGCCCCTCCCGGCAGCCACGCCCCCCAGCGTCGGGTGTCACGGGAAAGAGC
AGAGGGGAGAGGGGAGAGGGGGGGAGAGGGGAGAGGGGGGGAGAGGGGAGAGGGGGGGAGAGGGGAGAGGGGGGGAGA
GGGGAGAGGGGGGGAGAGGGGAGAGGGGGGGAGAGGGGAGAGGGGGGGAGAGGGGAGAGGGGGGGAGAGGGGAGAGGG
GGGGAGGGGGAGAGAGGGGAGAGGGGGTATATAAACCAACGAAAAGCGCGGGAACGGGGATACGGGGCTTGTGTGGCA
CGACGTCGTGGTTGTGTTACTGGGCAAACACTTGGGGACTGTAGGTTTCTGTGGGTGCCGACCCTAGGCGCTATGGGG
ATTTTGGGTTGGGTCGGGCTTATTGCCGTTGGGGTTTTGTGTGTGCGGGGGGGCTTGTCTTCAACCGAATATGTTATT
CGGAGTCGGGTGGCTCGAGAGGTGGGGGATATATTAAAGGTGCCTTGTGTGCCGCTCCCGTCTGACGATCTTGATTGG
CGTTACGAGACCCCCTCGGCTATAAACTATGCTTTGATAGACGGTATATTTTTGCGTTATCACTGTCCCGGATTGGAC
ACGGTCTTGTGGGATAGGCATGCCCAGAAGGCATATTGGGTTAACCCCTTTTTATTTGTGGCGGGTTTTTTGGAGGAC
TTGAGTCACCCCGCGTTTCCTGCCAACACCCAGGAAACAGAAACGCGCTTGGCCCTTTATAAAGAGATACGCCAGGCG
CTGGACAGTCGCAAGCAGGCCGCCAGCCACACACCTGTGAAGGCTGGGTGTGTGAACTTTGACTATTCGCGCACCCGC
CGCTGTGTAGGGCGACAGGATTTGGACCTACCAACGGAACGTCTGGACGGACCCCGGTTCTGCCGCCGGACGATGAA
GCGGGCCTGCAGCCGAAGCCCCTCACCACGCCGCCGCCCATCATCGCCACGTTGGACCCCACCCCGCGACGGGACGCC
GCCGCAAAAAGCAGACGCCGACGACCCCACTCCCGGCGCATCTAATGATGCCGCGACGGAAACCCGTCCGGGTTCGGG
GGGCGAACCGGCCGCCTGTCGCTCGTCAGGGCCGGCGGGCGCTCCTCGCCGCCCTAGAGGCTGTCCCGCTGGTGTGAC
GTTTTCCTCGTCCGCGCCCCCCGACCCTCCCATGGATTTAACAAACGGGGGGGTGTCGCCTGTGGCGACCTCGGCGCC
TCTGGACTGGACCACGTTTCGGCGTGTGTTTCTGATCGACGACGCGTGGCGGCCCCTGTTGGAGCCTGAGCTGGCGAA
CCCCTTAACCGCCCACCTCCTGACCGAATATAATCGTCGGTGCCAGACCGAAGAGGTGCTGCCGCCGCGGGAGGATGT
GTTTTCATGGACTCGTTATTGCACCCCCGACGAGGTGCGCGTGGTTATCATCGGCCAGGACCCATATCACCACCCCGG
CCAGGCGCACGGACTTGCGTTTAGCGTGCGCGCGAACGTGCCGCCTCCCCTGAGTCTTCGGAATGTCTTGGCGGCCGT
CAAGAACTGTTATCCCGAGGCACGGATGAGCGGCCACGGTTGCCTGGAAAAGTGGGCGCGGGACGGCGTCCTGTTACT
AAACACGACCCTGACCGTCAAGCGCGGGGCGGCGGCGTCCCACTCTAGAATCGGTTGGGACCGCTTCGTGGGCGGAGT
TATCCGCCGGTTGGCCGCGCGCCGCCCCGGCCTGGTGTTTATGCTCTGGGGCGCACATGCCCAGAATGCCATCAGGCC
GGACCCTCGGGTCCATTGCGTCCTCAAGTTTTCGCACCCGTCGCCCCTCTCCAAGGTTCCGTTCGGAACATGCCAGCA
TTTCCTCGTGGCGAATCGATATCTCGAGACCCGGTCGATTTCACCCATCGACTGGTCGGTTTGAAAGGCATCGACGTC
 
CGGGGTTTTCGTCTGTGGGGGCTTTTGGGTATTTCCGATGAATAAAGACGGTTAATGGTTAAACCTCTGGTCTCATAC
GGGTCGGTGATGTCGGGCGTCGGGGGAGAGGGAGTTCCCTCTGTGCTTGCGATTCTAGCCTCGTGGGGCTGGACGTTC
GACACGCCAAACCACGAGTCAGGGATATCGCCAGATACGACTCCCGCAGATTCCATTCGGGGGGCCGCTGTGGCCTCA
CCTGACCAACCTTTACACGGGGGCCCGGAACGGGAGGCCACAGCGCCGTCTTTCTCCCCAACGCGCGCGGATGACGGC
CCGCCCTGTACCGACGGGCCCTACGTGACGTTTGATACCCTGTTTATGGTGTCGTCGATCGACGAATTAGGGCGTCGC
CAGCTCACGGACACCATCCGCAAGGACCTGCGGTTGTCGCTGGCCAAGTTTAGCATTGCGTGCACCAAGACCTCCTCG
TTTTCGGGAAACGCCCCGCGCCACCACAGACGCGGGGCGTTCCAGCGCGGCACGCGGGCGCCGCGCAGCAACAAAAGC
CTTCAGATGTTTGTGTTGTGCAAACGCACCCACGCCGCTCGAGTGCGAGAGCAGCTTCGGGTCGTTATTCAGTCCCGC
AAGCCGCGCAAGTATTACACGTGATCTTCGGACGGGCGGCTCTGCCCCGCCGTCCCCGTGTTCGTCCACGAGTTCGTC
TCGTCCGAGCCAATGCGCCTCCACCGAGATAACGTCATGCTGGCCTCGGGGGCCGAGTAACCGCCCCCCCGCGCCACC
CTCACTGCCCGTCGCGCGTGTTTGATGTTAATAAATAACGCATAAATTTGGCTGGTTGTTTGTTGTCTTTAATGGACC
GCCCGCAAGGGGGGGGKGGCATTTCAGTGTCGGGTGACGAGCGCGATCCGGCCGGGATCCTAGGACCCCAAAAGTTTG
TCTGCGTATTCCAGGGCGGGGCTCAGTTGAATCTCCCGCAGCACCTCTACCAGCAGGTCCGCGGTGGGCTGGAGAAAC
TCGGCCGTCCCGGGGCAGGCGGTCGTCGGGGGTGGAGGCGCGGCGCCCACCCCGTGTGCCGCGCCTGGCGTCTCCTCT
GGGGGCGACCCGTAAATGGTTGCAGTGATGTAAATGGTGTCCGCGGTCCAGACCACGGTCAAAATGCCGGCCGTGGCG
CTCCGGGCGCTTTCGCCGCGCGAGGAGCTGACCCAGGAGTCGAACGGATACGCGTACATATGGGCGTCCCACCCGCGT
TCGAGCTTCTGGTTGCTGTCCCGGCCTATAAAGCGGTAGGCACAAAATTCGGCGCGACAGTCGATAATCACCAACAGC
CCAATGGGGGTGTGTTGGATAACAACGCCTCCGCGCGGCAGGCGGTCCTGGCGCTCCCGGCCCCGTACCATGATCGCG
CGGGTGCCGTACTCAAAAACATGCACCACCTGCGCGGCGTCGGGCAGTGCGCTGGTCAGCGAGGCCCTGGCGTGGCAT
AGGCTATACGCGATGGTCGTCTGTGGATTGGACATCTCGCGGTGGGTAGTGAGTCCCCCGGGCCGGGTTCGGTGGAAC
TGTAAGGGGACGGCGGGTTAATATACAATGACCACGTTCGGATCGCGCAGAGCCGATAGTATGTGCTTACTAATGACG
TCATCGCGCTCGTGGCGCTCCCGGAGCGGATTTAAGTTCATGCGAAGGAATTCGGAGGAGGTGGTGCGGGACATGGCC
ACGTACGCGCTGTTGAGGCGCAGGTTGCCGGGCGTAAAGCAGATGGCGACCTTGTCCAGGCTAAGGCCCTGGGAGCGC
GTGATGGTCATGGCAAGCTTGGAGCTGATGCCGTAGTCGGCGTTTATGGCCATGGCCAGCTCCGTAGAGTCAATGGAC
TCGACAAACTCGCTGATGTTGGTGTTGACGACGGACATGAAGCCGTGTTGGTCCCGCAAGACCACGTAAGGCAGGGGG
GCCTCTTCCAGTAACTCGGCCACGTTGGCCGTCGCGTGCCGCCTCCGCAGCTCGTCCGCAAAGGCAAACACCCGTGCG
TACGTGTATCCCATGAGCGTATAATTGTCCGTCTGCAGGGCGACGGACATCAGCCCCCGCGCGGCGAGCCGGTCAGC
ATCTCGCAGCCCCGGAAGATAACGTTGTCCACGTACGTGCTAAAGGGGGCGCCTTCAAATGCCTCCCCAAAGAGCTCT
TGGAGGATTCGGAATCTCCCGAGGAAGGCCCGCTTCAGCAGCGCAAACTGGGTGTGAACGGCGGCGGTGGTCTCCGGT
TCCCCGGGGGTGTAGTGGCAGTAAAACACGTCGAGCTGTTGTTCGTCCAGCCCCGCGAAAATAACGTCGAGGTCGTCG
TCGGGAAAATCGTCCGGGCCCCCGTCCCGCGGCCCCAGTTGCTTAAAATCAAACGCACGCTCGCCGGGGGCGCCTGCG
TCGGCCATTACCGACGCCTGCGTCGGCACCCCCGAAGATTTGGGGCGCAGAGACAGAATCTCCGCCGTTAGTTCTCCC
ATGCGGGCGTAGGCGAGGGTCCTCTGGGTCGCATCCAGGCCCGGGCGCTGCAGAAAGTTGTAAAAGGAGATAAGCCCG
CTAAATATGAGCCGCGACAGGAACCTGTAGGCAAACTCCACCGAAGTCTCCCCCTGAGTCTTTACAAAGCTGTCGTCA
CGCAACACTGCCTCGAAGGCCCGGAACGTCCCACTAAACCCAAAAACCAGTTTTCGCAGGCGCGCGGTTACCGCGATC
TGGCTGTTGAGGACGTAAGTGACGTCGTTGCGGGCCACGACCAGCTGCTGTTTGCTGTGCACCTCGCAGCGCATGTGC
CCCGCGTCCTGGTCCTGGCTCTGCGAGTAGTTGGTGATGCGGCTGGCGTTGGCCGTGAGCCACTTTTCAATAGTCAGG
CCGGGCTGGTGTGTCAGCCGTCGGTATTCGTCAAACTCCTTGACCGACACGAACGTAAGCACGGGGAGGGTGAACACG
 
ACAAACTCCCCCTCACGGGTCACCTTCAGGTAGGCGTGGAGCTTGGCCATGTACGCGCTCACCTCTTTGTGGGAGGAG
AACAACCGCGTCCAGCCGGGGAGGTTGGCGGGGTTGGTGATGTAGTTTTCCGGGACGACGAAGCGATCCACGAACTGC
ATGTGCTCCTCGGTGATGGGTAGGCCGTACTCCAGCACCTTCATGAGGTTACCGAACTCGTGCTCGATGCACCGTTTG
TTGTTAATAAAAATGGCCCAGCTATACGAGAGGCGGGCGTACTCCCGCAGCGTGCGGTTGCAGATGAGGTACGTGAGC
ACGTTCTCGCTCTGGCGGACGGAACACCGCAGTTTCTGGTGCTCGAAGGTCGACTCCAGGGACGCCGTCTGTGTCGGC
GAGCCCACACACACCAACACGGGCCGCAGGCGGCCGCGTACTGGGGGGTGTGGTACAGGGCGTTAATCATCCACCAG
CAATACACCACGGCCGTGAGGAGGTGACGCCCAAGGAGCCCGGCCTCGTCGATGACGATCACGTTGCTGCGGGTAAAG
GCCGGCAGCGCCCCGTGGGTGGCCGGGGCCAACCGCGTCAGGGCGCCCTCGGCCAACCCCAGGGTCCGTTCCAGGGCG
GCCAGGGCGCGAAACTCGTTCCGCGACTCCTCGCCCCCGGAGGCGGCCAGGGTGCGCTTCGTGAGGTCCAAAATCACC
TCCCAGTAGTACGTCAGATCTCGTCGCTGCAGGTCCTCCAGCGAGGCGGGGTTGCTGGTCAGGGTGTACGGGTACTGC
CCCAGTTGGGCCTGGACGTGATTCCCGCGAAACCCAAATTCATGAAAGATGGTGTTGATGGGTCGGCTGAGAAAGGCG
CCCGAGAGTTTGGCGTACATGTTTTGGGCCGCAATGCGCGTGGCGCCCGTCACCACACAGTCCAAGACCTCGTTGATT
GTCTGCACGCACGTGCTCTTTCCGGAGCCAGCGTTGCCGGTGATAAGATACACCGCGAACGGAAACTCCCTGAGGGGC
AGGCCTGCGGGGGACTCTAAGGCCGCCACGTCCCGGAACCACTGCAGACGGGGCACTTGCGCTCCGTCGAGCTGTTGT
TGCGAGAGCTCTCGGATGCGCTTAAGGATTGGCTGCACCCCGTGCATAGACGTAAAATTTAAAAGGCCTCGGCCCTC
CCTGGAACGGCTGGTCGGTCCCCGGGTTGCTGAAGGTGCGGCGGGCCGGGTCTCTGTCCGTCTAGCTGGCGCTCCCCG
CCGGCCGCCGCCATGACCGCACCACGCTCGCGGGCCCCCACTACGCATGCGCGGGGGGACACGGAAGCGCTGTGCTCC
CCCGAGGACGGCTGGGTAAAGGTTCACCCCACCCCCGGTACGATGCTGTTCCGCGAGATTCTCCACGGGCAGCTGGGG
TATACCGAGGGCCAGGGGGTGTACAACGTCGTCCGGTCCAGCGAGGCGACCACCCGGCAGCTGCAGGCGGCGATCTTT
CACGCGCTCCTCAACGCCACCACTTACCGGGACCTCGAGGCGGACTGGCTCGGCCACGTGGCGGCCCGCGGTCTGCAG
CCCCAACGGCTGGTTCGCCGGTACAGGAACGCCCGGGAGGCGGATATCGCCGGGGTGGCCGAGCGGGTGTTCGACACG
TGGCGGAACACGCTTAGGACGACGCTGCTGGACTTTGCCCACGGGTTGGTCGCCTGCTTTGCGCCGGGCGGCCCGAGC
GGCCCGTCAAGCTTCCCCAAATATATCGACTGGCTGACGTGCCTGGGGCTGGTCCCCATATTACGCAAGCGACAAGAA
GGGGGTGTGACGCAGGGTCTGAGGGCGTTTCTCAAGCAGCACCCGCTGACCCGCCAGCTGGCCACGGTCGCGGAGGCC
GCGGAGCGCGCCGGCCCCGGGTTTTTGAGCTGGCGCTGGCCTTCGACTCCACGCGCGTGGCGGACTACGACCGCGTG
TATATTTACTACAACCACCGCCGGGGCGACTGGCTCGTGCGAGACCCCATCAGCGGGCAGCGCGGAGAATGTCTGGTG
CTGTGGCCTCCCTTGTGGACCGGGGACCGTCTGGTCTTCGATTCGCCCGTACAGCGGCTGTTTCCCGAGATCGTCGCG
TGTCACTCCCTCCGGGAACACGCGCACGTCTGCCGGCTGCGCAATACCGCGTCCGTCAAGGTGCTGCTGGGGCGCAAG
AGCGACAGCGAGCGCGGGGTGGCCGGCGCCGCGCGGGTCGTTAACAAGGTGTTGGGGGAGGACGACGAGACCAAGGCC
GGGTCGGCCGCCTCGCGCCTCGTGCGGCTTATCATCAACATGAAGGGCATGCGCCACGTAGGCGACATTAACGACACT
GTGCGTGCCTACCTCGACGAGGCCGGGGGGCACCTGATAGACGCCCCGGCCGTCGACGGTACCCTCCCGGGATTCGGC
AAGGGCGGAAACAGCCGCGGGTCTGCGGGCCAGGACCAGGGGGGGCGGGCGCCGCAGCTTCGCCAGGCCTTCCGCACG
GCCGTGGTTAACAACATCAACGGCGTGTTGGAGGGCTATATAAATAACCTGTTTGGAACCATCGAGCGCCTGCGCGAG
ACCAACGCGGGCCTGGCGACCCAGTTGCAGGAGCGCGACCGCGAGCTCCGGCGCGCAACATCGGGGGCCCTGGAGCGC
CAGCAGCGCGCGGCCGACCTGGCGGCCGAGTCCGTGACCGGGGGATGCGGCAGCCGCCCTGCGGGGGCGGACCTGCTC
CGGGCCGACTATGACATTATCGACGTCAGCAAGTCCATGGACGACGACACGTACGTCGCCAACAGTTTTCAGTACCCG
TACATCCCTTCGTACGCCCAGGACCTGGAGCGCCTGTCGCGCCTCTGGGAGCACGAGCTGGTGCGCTGTTTCAAAATT
CTGTGTCACCGCAACAACCAGGGCCAAGAGACGTCGATCTCGTACTCCAGCGGGGCGATCGCCGCATTCGTCGCCCCC
 
TACTTTGAGTCAGTGCTTCGGCCCCCCGGGTAGGCGCGCCATCACGGGCTCCGATGTCATCCTGGGGGAGGAGGAGT
TATGGGATGCGGTGTTTAAGAAAACCCGCCTGCAAACGTACCTGACAGACATCGCGGCCCTGTTCGTCGCGGACGTCC
AGCACGCAGCGCTGCCCCCGCCCCCCTCCCCGGTCGGCGCCGATTTCCGGCCCGGCGCGTCCCCGCGGGGCCGGTCCA
GATCGCGGTCGCCCGGAAGAACTGCGCGAGGCGCGCCGGACCAGGGCGGGGGCATCGGGCACCGGGATGGCCGCCGCG
ACGGCCGACGATGAGGGGTCGGCCGCCACCATCCTCAAACAGGCCATCGCCGGGGACCGCAGCCTGGTCGAGGCGGCC
GAGGCGATTAGCCAGCAGACGCTGCTCCGCCTGGCCTGCGAGGTGCGCCAGGTCGGCGACCGCCAGCCGCGGTTTACC
GCCACCAGCATCGCGCGCGTCGACGTCGCGCCTGGGTGCCGGTTGCGGTTCGTTCTGGACGGGAGTCCCGAGGACGCC
TATGTGACGTCGGAGGATTACTTTAAGCGCTGCTGCGGCCAGTCCAGTTATCGCGGCTTCGCGGTGGCGGTCCTGACG
GCCAACGAGGACCACGTGCACAGCCTGGCCGTGCCCCCCCTCGTTCTGCTGCACCGGTTCTCCCTGTTCAACCCCAGG
GACCTCCTGGACTTTGAGCTTGCCTGTCTGCTGATGTACCTGGAGAACTGCCCCCGAAGCCACGCCACCCCGTCGACC
TTTGCCAAGGTTCTGGCGTGGCTCGGGGTCGCGGGTCGCCGCACGTCCCCATTCGAACGCGTTCGCTGCCTTTTCCTC
CGCAGTTGCCACTGGGTCCTAAACACACTCATGTTCATGGTGCACGTAAAACCGTTCGACGACGAGTTCGTCCTGCCC
CACTGGTACATGGCCCGGTACCTGCTGGCCAACAACCCGCCCCCCGTTCTCTCGGCCCTGTTCTGTGCCACCCCGACA
AGCTCCTCATTCCGGCTGCCGGGGCCGCCCCCCGCTCCGACTGCGTGGCCTATAACCCCGCCGGGATCATGGGGAGC
TGCTGGGCGTCGGAGGAGGTGCGCGCGCCTCTGGTCTATTGGTGGCTTTCGGAGACCCCAAAACGACAGACGTCGTCG
CTGTTTTATCAGTTTTGTTGAATTTTAGGAAATAAACCCGGTTTTGTTTCTGTGGCCTCCCGACGGATGCGCGTGTCC
TTACTCCGTCTTGGTGGGTGGGTGGCTGTGTATGGCGTCCCATCTGTGCGGGGAGGGGGGCAAGTCGGCACGTATTCG
GACAGACTCAAGCACACACGGGGGAGCGCTCTTGTCTCAGGGCAATGTTTTTATTGGTCAAACTCAGGCAAACAGAAA
CGACATCTTGTCGTCAAAGGATACACAAACTTCCCCCCCTCGCCCCATACTCCCGCCAGCACCCCGGTAAACACCAA
CTCAATCTCGCGCAGGATTTCGCGCAGGTGATGAGCGCAGTCCACGGGGGGGAGCACAAGGGGCCGCGGGTATAGATC
GACGGGGACGCCGACCGACTCCCCGCCTCCGGGACAGACACGCACGACGCGCCGCCAGTAGTGCTCTGCGTCCAGCAA
GGCGCCGCCGCGGAAGGCAGTGGGGGGCAAGGGGTCGCTGGCCTCAAAGGGGGACACCCGAACGCTCCAGTACTCCGC
GTCCAACCGTTTATTAAACGCGTCCAAGATAAGGCGGTCGCAGGCGTCCTCCATAAGGCCCCGGGCCGTGAGTGCGTC
CTCCTCCGGCACGCCTGCCGTTGTCAGGCCCAGGACCCGTCGCAGCGTGTCGCGTACGACCCCGGCCGCCGTGGTGTA
CGCGGGCCCGCGGAGAGGAAATCCCCCAAGATGGTCAGTGTTGTCGCGGGAGTTCCAGAACCACACTCCCGCCTGGCT
CCAGGCGACTGCGTGGGTGTAGACGCCCTCGAGGGCCAGGCACAGTGGGTGCCGCAGCCGGAGGCCGTTGGCCCTAAG
CACGGCTCCCACGGCCGTCTCGATGGCCCGCCGGGCGTCCTCGATCACCCCGGAAGCCGCATCCGCGTCTTGGGGGTC
CACGTTAAAGACACCCCAGAACGCACCCCCATCGCCCCCGCAGACCGCGAACTTCACCGAGCTGGCCGTCTCCTCGAT
CTGCAGGCAGACGGCGGCCATTACCCCACCCAGGAGCTGCCGCAGCGCAGGGCAGGTGTTGCACGTGTCCGGGACCAG
GCGCTCCAAGACGGCCCCGGCCCAGGGCTCTGAGGGAGCGGCCACCACCAGCGCGTCCAGTCTTGCTAGGCCCGTCCG
GCCGTGGGGGTCCGCCAGCCCGCTCCCCCCGAGGTCGGCCAGGGCCGCCAGGAGCTGGGCGCGAAGTCCGGGGAAGCA
AAACCGCGCCGTCCAGACGGGCCCGACGGCCGCGGGCGGGTCTAACAGTTGGATGATTTAGTGGCGGGATGCCACCG
CGCCACCGCCTCCCGCACTGCGGGCAGGAGGCATCCGGCTGCCGCCGAGGCCACGCCGGGCCAGGCTCGCGGGGGGAG
GACGACCCTGACCCCCACCGCGGGCCAGGCCCCCAGGAGCGCGGCGTAAGCGGCCGCGGCCCCGCGCACCAGGTCCCG
TGCCGACTCGGCCGTGGCCGGCACGGTGAACGTGGGCCAACCCGGAAACCCCAGGACGGCAAAGTACGGACGGGTCC
CCCCCGGACCTCAAACTCGGGCCCCAGAAAGGCAAAGACGGGGGCCAGGGCCCCGGGGGCGGCGTGGACCGTGGTATG
CCACTGCCGGAAAAGGGCGACGAGCGCCGGCGCGGAGAACTTCTCGCCGGCGCTTACAAAGTAGTCGTAATCGCGGGG
CAGCAGCACCCGTGCCGTGACTCGTTGTGGGTGCCCGCGTGGCCGCAGGCCCACCTCGCACACCTCGACCAGGTCCCC
 
GAACGCGCCCTCCTTCTTGATCGGCGGAAACGCAAGAGTCTGGTATTCGCGCGCAAATAGCGCGGTTCCGGTGGTGAT
GTTAACGGTCAGCGAAGCGGTGGACGCGCACTGGGGGGTGTCGCGAATGGCCGCCAGGCGCGCCCACGCCAGCCGCGC
GTCGGGATGCTCGGCAACGCGCGCCGCCAGGGCCATAGGGTCGATGTCAATGTTGGCCTCCGCGACCAGGAGAGCGGC
GCGAGGGGCGGCGGGCGGGCCCCACGACGCTCTCTCAACTTTCACCACCAGTCCCGTGCGTGGGTCCGAGCCGATACG
CAGCGGGGCGAACAGGGCCACCGGCCCGGTCTGGTGCTCCAGGGCCGCCAGGACGCACGCGTACAGCGCCCGCCACAG
AGTCGGGTTCTCCAGGGGCTCCAGCGGGGAGGCGGCCGGCGTCGTCGCGGCGCGGGCGGCCGCCACGACGGCCTGGAC
GGAGACGTCCGCGGAGCCGTAGAAATCCCGCAGCTCCGTCGCGGTGACGGAGACCTCCGCAAAGCGCGCGCGACCCTC
CCCTGCGGCGTTGCGACATACAAAATACACCAGGGCGTGGAAGTACTCGCGAGCGCGGGGGGGCAGCCATACCGCGTA
AAGGGTAATGGCGCTGACGCTCTCCTCCACCCACACGATATCTGCGGTGTCCATCGCACGGCCCCTAAGGATCACGGG
CGGTCTGTGGGTCCCATGCTGCCGTGCCTGGCCGGGCCCGGTGGGTTGCGGAAACCGGTGACGGGGGGGGGCGGTTT
TTGGGGTTGGGGTGGGAAACGGCCCGGGTCCGGGGGCCAACTTGGCCCCTCGGTGCGTTCCGGCAACAGCGCCGCCGG
TCCGCGGACGACCACGTACCGAACGAGTGCGGTCCCGAGACTTATAGGGTGCTAAAGTTCACCGCCCCCTGCATCATG
GGCCAGGCCTCGGTGGGGAGCTCCGACAGCGCCGCCTCCAGGATGATGTCAGCGTTGGGGTTGGCGCTGGATGAGTGC
GTGCGCAAACAGCGCCCCCACGCGGGCACGCGTAGCTTGAAGCGCGCGCCCGCAAACTCCCGCTTGTGGGCCATAAGC
AGGGCGTACAGCTGCCTGTGGGTCCGGCAGGCGCTGTGGTCGATGTGGTGGGCGTCCAACAACCCCACGATTGTCTGT
TTGGTGAGGTTTTTAACGCGCCCCGCCCCGGGAAACGTCTGCGTGCTTTTGGCCATCTGCACGCCAAACAGTTCGCCC
CAGATTATCTTGAACAGCGCCACCGCGTGGTCCGTCTCACTAACGGACCCGCGCGGGGGACAGCCGCTTAGGGCGTCG
GCGACGCGCTTGACGGCTTCCTCCGAGAGCAGAAGTCCGTCGGTTACGTTACAGTGGCCCAGTTCGAACACCAGCTGC
ATGTAGCGGTCGTAGTGGGGGGTCAGCAGGTCCAGCACGTCATCGGGGCCGAAGGTCCTCCCAGATCCCCCGGCCGCC
GAGTCCCAATGCAGGCGCGCGGCCATGGTGCTGCACAGGCACAACAGCTCCCAGACAGGGGTTACGTTCAGGGTGGGG
GGCAGGGCCACGAGCTCCAGCTCTCCGGTGACGTTGATCGTGGGGATGACGCCCGTGGCGTAGTGGTCATAGATCCGC
CGAAATATGGCGCTGCTGCGGGTGGCCATGGGAACGCGGAGACAGGCCTCCAGCAACGCCAGGTAAATAAACCGCGTG
CGTCCCATCAGGCTGTTGAGGTTGCGCATGAGCGCGACAATTTCCGCCGGCGCGACATCGGACCGGAGGTATTTTTCG
ACGAAAAGACCCACCTCCTCCGTCTCGGCGGCCTGGGCCGGCAGCGACGCCTCGGGATCCCGGCACCGCAGCTCCCGT
AGATCGCGCTGGGCCCTGAGGGCGTCGAAATGTACGCCCCGCAAAAACAGACAGAAGTCCTTTGGGGTCAGGGTATCG
TCGTGTCCCCAGAAGCGCACGCGTATGCAGTTTAGGGTCAGCAGCATGTGAAGGATGTTAAGGCTGTCCGAGAGACAC
GCCAGCGTGCATCTCTCAAAGTAGTGTTTGTAACGGAATTTGTTGTAGATGCGCGACCCCCGCCCCAGCGACGTGTCG
CATGCCGACGCGTCACAGCGCCCCTTGAACCGGCGACACAGCAGGTTTGTGACCTGGGAGAACTGCGCGGGCCACTGG
CCGCAGGAACTGACCACGTGGTTCAGGAGCATGGGCGTAAAGACGGGCTCCGAGCGCGCCCCGGAGCCGTCCATGTAA
ATCAGTAGCTCCCCCTTGCGGAGGGTGCGCACCCGTCCCAGGGACTGGTACACGGACACCATGTCCGGTCCGTAGTTC
ATGGGTTTCACGTAGGCGAACATGCCATCAAAGTGCAGGGGATCGAAGCTGAGGCCCACGGTTACGACCGTCGTGTAT
ATAACCACGCGGTATTGGCCCCACGTGGTCACGTCCCCGAGGGGGGTGAGCGAGTGAAGCAACAGCACGCGGTCCGTA
AACTGACGGCAGAACCGGGCCACGATCTCCGCGAAGGAGACCGTCGACGAAAAAATGCAGATGTTATCGCCCCCGCCA
AGGCGCGCTTCCAGCTCCCCAAAGAACGTGGCCCCCCGGGCGTCCGGAGAGGCGTCCGGAGACGGGCCGCTCGGCGGC
CCGGGCGGGCGCAGGGCAGCCTGCAGGAGCTCGGTCCCCAGACGCGGGAGAAACAGGCACCGGCGCGCCGAAAACCCG
GGCATGGCGTACTCGCCGACCACCACATGCACGTTTTTTCGCCCCGGAGACCGCACAGGAAGTCCACCAACTGCGCG
TTGGCGGTTGCGTCCATGGCGATGATCCGAGGACATGTGCGCAGCAGGCGTAGCATTAACGCATCCACGCGGCCCAGT
TGCTGCATCGTTGGCGAATAGAGCTGGCCCAGCGTCGACATAACCTCGTCCAGAACGAGGACGTCGTAGTTGTTCAGA
 
AGGTTGGGGCCCACGCGATGAAGGCTTTCCACCTGGACGATAAGTCGGTGGAAGGGGCGGTCGTTCATAATGTAATTG
GTGGATGAGAAGTAGGTGACAAAGTCGACCAGGCCTGACTCAGCGAACCGCGTCGCCAGGGTCTGGGTAAAACTCCGA
CGACAGGAGACGACGAGCACACTCGTGTCCGGAGAGTGGATCGCTTCCCGCAGCCAGCGGATCAGCGCGGTAGTTTTT
CCCGACCCCATTGGCGCGCGGACCACAGTCACGCACCTGGCCGTCGGGGCGCTCGCGTTGGGGAAGGTGACGGGTCCG
TGCTGCTGCCGCTCGATCGTTGTTTTCGGGTGAACCCGGGGCACCCATTCGGCCAAATCCCCCCCGTATAACATCCGC
GCTAGCGATACGCTCGACGTGTACTGTTCGCACTCGTCGTCCCCAATGGGACGCCCGGCCCCCAGAGGATCCCCCGAC
TCCGCGCCCCCCACGAAAGGCATGACCGGGGCGCGGACGGCGTGGTGGGTCTGGTGTGTGCAGGTGGCGACGTTTGTG
GTCTCTGCGGTCTGCGTCACGGGGCTCCTCGTCCTGGCCTCTGTGTTCCGGGCACGGTTTCCCTGCTTTTACGCCACG
GCGAGCTCTTATGCCGGGGTTAACTCCACGGCCGAGGTGCGCGGGGGTGTAGCCGTGCCCCTCAGGTTGGACACGCAG
AGCCTTGTGGGCACTTATGTAATCACGGCCGTATTGTTGTTGGCCGCGGCCGTGTATGCCGTGGTCGGCGCCGTGACC
TCCCGCTACGACCGCGCCCTGGACGCGGGCCGCCGTCTGGCTGCGGCCCGCATGGCCATGCCGCACGCCACGCTGATC
GCCGGAAACGTCTGCTCTTGGTTGCTGCAGATCACCGTCCTGCTGCTGGCCCATCGCATCAGCCAGCTGGCCCACCTG
GTTTACGTCCTGCACTTTGCGTGTCTGGTGTATTTTGCGGCCCATTTTTGCACCAGGGGGGTCCTGAGCGGGACGTAT
CTGCGTCAGGTGCACGGCCTGATGGAGCTGGCCCCGACCCATCATCGCGTCGTCGGCCCGGCTCGCGCCGTGCTGACA
AACGCCTTGCTGTTGGGCGTCTTCCTGTGCACGGCCGACGCCGCGGTATCCCTGAATACCATCGCCGCGTTCAACTTT
AATTTTTCGGCCCCGGGCATGCTCATCTGCCTGACCGTGCTGTTCGCCATTCTCGTCGTATCGCTGTTGTTGGTGGTC
GAGGGGGTGTTGTGTCACTACGTGCGCGTGTTGGTGGGCCCCCACCTGGGGGCCGTTGCCGCCACGGGCATCGTCGGC
CTGGCCTGCGAGCACTATTACACCAACGGCTACTACGTGGTGGAGACGCAGTGGCCGGGGGCACAGACGGGAGTGCGC
GTCGCCCTCGCCCTGGTCGCCGCCTTTGCCCTCGGCATGGCCGTGCTCCGCTGCACCCGCGCCTATCTGTATCACAGG
CGGCACCACACCAAATTTTTATGCGCATGCGCGACACGCGACACCGCGCACATTCCGCCCTCAAGCGCGTACGCAGT
TCCATGCGCGGATCGCGAGACGGCCGCCACAGGCCCGCGCCCGGCAGCCCGCCCGGGATTCCCGAATATGCGGAAGAC
CCCTACGCGATCTCATACGGCGGCCAGCTCGACCGGTACGGAGATTCCGACGGGGAGCCGATTTACGACGAGGTGGCG
GACGACCAAACCGACGTATTGTACGCCAAGATACAACACCCGCGGCACCTGCCCGACGACGAGCCCATCTATGACACC
GTTGGGGGGTACGACCCCGAGCCCGCCGAGGACCCCGTGTACAGCACCGTCCGCCGTTGGTAGCTGTTTGGTTCCGTT
TTAATAAACCGTTTGTGTTTAACCCGACCGTGGTGTATGTCTGGTGTGTGGCGTCCGATCCCGTTACTATCACCGTCC
CCCCCCCCCCTCAACCCCGGCGATTGTGGGTTTTTAAAAACGACACGCGTGCGACCGTATACAGAACATTATTTTGG
TTTTTATTCGCTATCGGACATGGGGGGTGGAAACTGGGTGGCGGGGCAGGCGCCTCCGGGGGTCCGCCGGTGAGTGTG
GCGCGAGGGGGGGTCCGACGAACGCAGGCGCGGTCTCCCCGGGGCCCGCGTAACCACGCGCATATCCGGGGGCACGTA
GAAATTACCTTCCTCTTCGGACTCGATATCCACGACGTCAAAGTCGTGGGCGGTCAGCGAGACGACCTCCCCGTCGTC
GGTGATGAGGACGTTGTTTCGGCAGCAGCAGGGCCGGGCCCCGGAGAACGAGAGGCCCATAGCTCGGCGAGCGTGTCG
TCGAACGCCAGGCGGCTGCTTCGCTGGATGGCCTTATAGATCTCCGGATCGATGCGGACGGGGGTAATGATCAGGGCG
ATCGGAACGGCCTGGTTCGGGAGAATGGACGCCTTGCTGGGTCCTGCGGCCCCGAGAGCCCCGGCGCCGTCCTCCAGG
CGGAACGTTACGCCCTCCTCCGCGCTGGTGCGGTGCCTGCCGATAAACGTCACCAGATGCGGGTGGGGGGGGCAGTCG
GGGAAGTGGCTGTCGAGCACGTAGCCCTGCACCAAGATCTGCTTAAAGTTCGGGTGGCGGGGGTTCGCGAAGACGGGC
TCGCGGCGGACCAGATCCCCGGAGCTCCAGGACACGGGGGAGATGGTGTGGCGTCCGAGGTCGGGGGCGCCAAACAGA
AGCACCTCCGAGACAACGCCGCTATTTAACTCCACCAAGGCCCGATCCGCGGCGGAGCACCGCCTTTTTTCGCCCGAG
GCGTGGGCCTCTGACCAGGCCTGGTCTTGCGTGACGAGAGCCTCCTCCGGGCCGGGGACGCGCCCGGGCGCGAAGTAT
CGCACGCTGGGCTTCGGGATCGACCGGATAAATGCCCGGAACGCCTCCGGGGACCGGTGTGCCATCAAGTCCTCGTAC
 
GCGGAGGCCGTGGGGTCGCTGGGGTCCATGGGGTCGAAAGCGTACTTGGCCCGGCATTTGACCTCGTAAAAGGCCAGG
GGGGTCTTGGGGACTGGGGCCAGGTAGCCGTGAATGTCCCGAGGACAGACGAGAATATCCAGGGACGCCCCGACCATC
CCCGTGTGACCGTCCATGAGGACCCCACACGTATGCACGTTCTCTTCGGCGAGGTCGCTGGGTTCGTGGAAGATAAAG
CGCCGCGTGTCGGCGCCGGCCTCGCCGCCGTCGTCCGCGCGGCCCACGCAGTAGCGAAACAGCAGGCTTCGGGCCGTC
GGCTCGTTCACCCGCCCGAACATCACCGCCGAAGACTGTACATCCGGTCGCAGGCTGGCGTTGTGCTTCAGCCACTGG
GGCGAGAAACACGGACCCTGGGGGCCCCAGCGGAGGGTGGATGCGGTCGTGAGGCCCCGCCGGAGCAGGGCCCATAGC
TGGCAGTCGGCCTGGTTTTGCGTGGCCGCCTCGTAAAACCCCATGAGGGGCCGGGGCGCCACGGCGTCCGCGGCGGCC
GGGGGGGCGCGGCGCGTCAGGCGCCATAGGTGCCGGCCGAGTCCGCGGTCCACCATACCCGCCTCCTCGAGGACCACG
GCCAGGGAACACAGATAATCCAGGCGGGCCCAGAGGGGACCGATGGCCAGAGGGGCGCGGACGCCGCGCAGCAACCCG
CGCAGGTGGCGCTCGAACGTCTCGGCTAGTATATGGAGGGCAGCGCGTTGGGGATCACCGACGCCGACCACATAGAG
TCAAGGTCCGGGGAGTCGGGATCGGCGTCCGGGTCGCGGGCGTGGGTGCCCCCAGGAGATAGCGGAATGTCCGGGGTC
GGAGGCCCGGAGGCGTCAGAAAGTGCCGGCGACGCGGCCCGGGGCTTTTCGTCTGCGGTGTCGGTGGCGTGCTGATCA
CGTGGGGGGTTATCGGGCGAATGGGAGCTCGGGTCCACAGCTGACGTCGTCTGGGGTGGGGGGGCAGGGGACGGAAG
GTGGTTGTCAGCGGAAGACTGTTAGGGCGGGGGCGCTTGGGGGGGCTGTCGGGGCCACGAGGGGTGTCCTCGGCCAGG
GCCCAGGGACGCTTAGTCACGGTGCGTCCCGGCGGACATGCTGGGCCTACCGTGGACTCCATTTCCGAGACGACGTGG
GGGGAGCGGTGGTTGAGCGCGCCGCCGGGTGAACGCTGATTCTCACGACAGCGCGTGCCGCGCGCACGGGTTGGTGTG
ATACAGGCGGGACACCAGCACCAGGAGAGGCTTAAGCTCGGGAGGCAGCGCCACCGACGACAGTATCGCCTTGTGTGT
GTGCTGGTAATTTATACACCGATCCGTAAACGCGCGCCGAATCTTGGGATTGCGGAGGTGGCGCCGGATGCCCTCTGG
GACGTCATACGCCAGGCCGTGGGTGTTGGTCTCGGCCGAGTTGACAAACAGGGCTGGGTGCAGCACGCAGCGATAGGC
GAGCAGGGCCAGGGCGAAGTCCGGCGACAGCTGGTTGTTGAAATACTGGTAACCGGGAAACCGGGTCACGGGTACGCC
CAGGCTCGGGGCGACGTACACGCTAACCACCAACTCCAGCAGCGTCTGGCCCAGGGCGTACAGGTCAACCGCTAGCCC
GACGTCGTGCTTCAGGCGGTGGTTGGTAAATTCGGCCCGTTCGTTGTTAAGGTATTTCACCAACAGCTCCGGGGGCTG
GTTATACCCGTGACCCACCAGGGTGTGAAAGTTGGCTGTGGTTAGGGCGGTGGCATGCCAAACATCCGGGGGGACTT
GAGGTCCGGCTCCTGGAGGCAAAACTGCCCCCGGGCGATCGTGGAGTTGGAGTTGAGGGTGACGAGGCTAAAGTCGGC
GAGGACGGCCCGCCGGAGCGAGACGGCGTCCGACCGCAGCATGACGAGGATGTTGGCGCACTTGATATCCAGGTGGCT
GATCCCGCAGGTGGTGTTTAAAAACACAACGGCACGGGCCAGCTCCGTGAAGCACTGGTGGAGGGCCGTCGAGACCGA
GGGGTTTGTTGTGCGCAGGGACGCCAGTTGGCCGATATACTTACCGAGGTCCATGTCGTACGCGGGAACACTATCTG
TCGTTGTTGCAGCGAGAACCCGAGGGGCGCGATGAAGCCGCGGATGTTGTGGGTGCGGCCGGCGCGTAGAGCGCACTC
CCCGACCAACAGGGTCGCGATGAGCTCAACGGCAAACCACTCCTTTTCCTTTATGGTCTTAACGGCAAGCTTATGTTC
GCGAATCAGTTGGACTTCGCCGTATCCCCCAGACCCCCCGAAGCTTCGGGCCCCGGGGATCTCGAGGGTCGTGTAGTG
TAGGGCGGGGTTGATGGCGAACACGGGGCTGCATAGCTTGCGGATGCGCGTGAGGGTGAGGATGTGCGAGGGGGACGA
GGGGGGTGCGGTTAACGCCGCCTGGGATCTGCGCAGGGGCGGGCGGTTCAGTTTGGCCGCCGTACCGGGCGCCTCGGG
GGACGCGCGGCGATGAGACGAGCGGCTCATTCGCCATCGGGATAGTCCCGCGCGAAGCCGCTCGCGGAGGCCGGATCG
GTGGCGGCACCCGTGGGAGGAGCGGGAGACGGCGGCGTTCTGGAGAGAGGGGCCGCTGGGGCGCCCGGAGGCCCCATG
GGGGTTGGAGTGTATGTAGGATGCGAGCCAATCCTTGAAGGACCGTTGGCGTGCACCTTGGGGGCTGAGGTTAGCTGC
CACATGACCAGCAGGTCGCTGTCTGCGGGACTCATCCATCCTTCGGCCAGGTCGCCGTCTCCCCACAGAGAAGCGTTG
GTCGCTGCCTCCTCGAGTTGCTCCTCCTGGTCCGCAAGACGATCGTCCACGGCGTCCAGGCGCTCACCAAGCGCCGGA
TCGAGGTACCGTCGGTGTGCGGTTAGAAAGTCACGACGCGCCGCTTGCTCCTCCACGCGAATTTTAACACAGGTCGCG
 
CGCTGTCGCATCATCTCTAAGCGCGCGGGACTTTAGCCGCGCCTCCAATTCCAAGTGGGCCGCCTTTGCAGCCATA
AAGGCGCCAACAAACCGAGGATCTTGGGTGCTGACGCCCTCCCGGTGCAGCTGCAGGGTCTGGTCCTTGTAAATCTCG
GCTCGGAGGTGCGTCTCGGCCAGGCGTCGGCGCAGGGCCGCGTGGGCGGCATCTCGGTCCATTCCGCCACCCTGCGGG
CGACCCGGGGGTGCTCTGATAGTCTCGCGTGCCCAAGGCCCGTGATCGGGGTACTTCGCCGCCGCGACCCGCCACCCG
GTGTGCGCGATGTTTGGTCAGCAGCTGGCGTCCGACGTCCAGCAGTACCTGGAGCGCCTCGAGAAACAGAGGCAACTT
AAGGTGGGCGCGGACGAGGCGTCGGCGGGCCTCACAATGGGCGGCGATGCCCTACGAGTGCCCTTTTTAGATTTCGCG
ACCGCGACCCCCAAGCGCCACCAGACCGTGGTCCCGGGCGTCGGGACGCTCCACGACTGCTGCGAGCACTCGCCGCTC
TTCTCGGCCGTGGCGCGGCGGCTGCTGTTTAATAGCCTGGTGCCGGCGCAACTAAAGGGGCGTGATTTCGGGGGCGAC
CACACGGCCAAGCTGGAATTCCTGGCCCCCGAGTTGGTACGGGCGGTGGCGCGACTGCGGTTTAAGGAGTGCGCGCCG
GCGGACGTGGTGCCTCAGCGTAACGCCTACTATAGCGTTCTGAACACGTTTCAGGCCCTCCACCGCTCCGAAGCCTTT
CGCCAGCTGGTGCACTTTGTGCGGGACTTTGCCCAGCTGCTTAAACCTCCTTCCGGCCTCCAGCCTCACGGAGACCA
CGGGCCCCCCAAAAAACGGGCCAAGGTGGACGTGGCCACCCACGGCCGGACGTACGGCACGCTGGAGCTGTTCCAAA
AAATGATCCTTATGCACGCCACCTACTTTCTGGCCGCCGTGCTCCTCGGGGACCACGCGGAGCAGGTCAACACGTTCC
TGCGTCTCGTGTTTGAGATCCCCCTGTTTAGCGACGCGGCCGTGCGCCACTTCCGCCAGCGCGCCACCGTGTTTCTCG
TCCCCCGGCGCCACGGCAAGACCTGGTTTCTGGTGCCCCTCATCGCGCTGTCGCTGGCCTCCTTTCGGGGGATCAAGA
TCGGCTACACGGCGCACATCCGCAAGGCGACCGAGCCGGTGTTTGAGGAGATCGACGCCTGCCTGCGGGGCTGGTTCG
GTTCGGCCCGAGTGGACCACGTTAAAGGGGAAACCATCTCCTTCTCGTTTCCGGACGGGTCGCGCAGTACCATCGTGT
TTGCCTCCAGCCACAACACAAACGTAAGTCCTCTTTTCTTTCGCATGGCTCTCCCAAGGGGCCCCGGGTCGACCCGAC
CCACACCCACCCACCCACATACACACACAACCAGACGCGGGAGGAAAGTCTGCCCCGTGGGCACTGATTTTTATTCGG
GATCGCTTGAGGAGGCCCGGGCAACGGCCCGGGCAACGGTGGGGCAACTCGTAGCAAATAGGCGACTGATGTACGAAG
AGAAGACACACAGGCGCCACCCGGCGCTGGTCGGGGGATGTTGTCCGCGCCGCACCGTCCCCCGACGACCTCTTGCA
GACGGTCCGTGATGCAAGGACGGCGGGGGGCCTGCAGCAGGGTGACCGTATCCACGGGATGGCCAAAGAGAAGCGGAC
ACAGGCTAGCATCCCCCTGGACCGCCAGGGTACACTGGGCCATCTTGGCCCACAGACACGGGGCGACGCAGGGACAGG
ACTCCGTTACGACGGAGGAGAGCCACAGTGCGTTGGCGGAATCGATGTGGGGCGGCGGGGCGCAGGACTCGCAGCCCC
CCGGGTGGTTAGTGATCCTGGCCAGGAGCCATCCCAGATGGCGGGCCCTGCTTCCCGGTGGACAGAGCGACCCCAGGT
CGCTGTCCATGGCCCAGCAGTAGATCTGGCCGCTGGGGAGGTGCCACCAGGCCCCCGGGCCCAAGGCGCAGCACGCGC
CCGGCTCCGGGGGGGTCTTCGCGGGACCAGATACGCGCCATCCAGCTCGCCGACCACTGGCTCCTCCGCGAGCTGTT
CGGTGGTTGGGTCGGGGGTTTCCTCCGGGGGGGTGGCCGCCCGTATGCGGGCGAACGTGAGGGTGCACAGGAGCGGGG
TCAGGGGGTGCGTCACGCTCCGGAGGTGGACGATCGCGCAGTAGCGGCGCTCGCGGTTAAAGAAAAAGAGGGCAAAGA
AGGTGTTCGGGGGCAACCGCAGCGCCTTGGGGCGCGTCAGATACAGAAAAATCTCGCAGAAGAGGGCGCGCCCGGGGT
CTGGGTTAGGAAGGGCCACCTGACACAGAGGCTCGGTGAGGACCGTTAGACACCGAAAGATCTTGAGCCGCTCGTCCG
CCCGAACGACGCGCCACACAAAGACGGAGTTGACAATGCGCGCGATAGAGTCGACGTCCGTCCCCAGGTCGTCGACTC
TGTCGCGCGTGCCGCGAGCTCCGGCCCGGGAATCCGGCCGGGGCAAGGTCCCCGGGGGACCAGGCGGCGCCAGGGGCC
GCCGGGGTCCCAGCTGCGCCATGCCGGGGGCGGGGGAGGGCAAACCCCAGAGGCGGGGGCCAACGGCGCGGGGAGGA
GTGGATGGGCGAGGTGGCCGGGGGAAGGCGCCCGCTAGCGAGAACGGCCGTTCCCGGACGACACCTTGCGACAAAACC
TAAGGACAGCGGCCCGCGCGACGGGGTCCGAGAGGCTAAGGTAGGCCGCGATGTTAATGGTGAACGCAAAGCCGCCGG
GAAAGACAACTATGCCACAGAGGCGGCGATTAAACCCCAGGCAGAGGTAGGCGTAGCTTTCCCCGGGCAGGTATTGCT
CGCAGACCCTGCGTGGGGCTGTGGAGGGGACGGCCTCCATGAAGCGACATTTACTCTGCTCGCGTTTACTGACGTCAC
 
CATCCATCGCCACGGCGATTGGACGATTGTTAAGCCGCAGCGTGTCTCCGCTTGTGCTGTAGTAGTCAAAAACGTAAT
GGCCGTCGGAGTCGGCAAAGCGGGCCGGGAGGTCGTCGCCGAGCGGGACGACCCGCCGCCCCCGACCGCCCCGTCCCC
CCAGGTGTGCCAGGACGGCCAGGGCATACGCGGTGTGAAAAAAGGCGTCGGGGGCGGTCCCCTCGACGGCGCGCATCA
GGTTCTCGAGGAGAATGGGGAAGCGCCTGGTCACCTCCCCCAGCCACGCGCGTTGGTCGGGGCCAAAGTCATAGCGCA
GGCGCTGTGAGATTCGCGGGCCGCCCTGAAGCGCGGCCCGGATGGCCTGGCCCAGGGCCCGGAGGCACGCCAGATGTA
TGCGCGCGGTAAAGGCGACCTCGGCGGCGATGTCAAAGGGCGGCAGGACGGGGCGCGGGTGGCGCAGGGGCACCTCGA
GCGCGGGAAAGCGGAGCAGCAGCTCCGCCTGCCCAGCGGGAGACAGCTGGTGGGGGCGCACGACGCGTTCTGCGGCGC
AGGCCTCGGTCAGGGCCGTGGCCAGCGCCGAGGACAGCAGCGGAGGGCGGGCGCGTCGCCCGCCCCACGCCACTGAGT
TCTCGTAGGAGACGACGACGAAGCGCTGCTTGGTTCCGTAGTGGTGGCGCAGGACCACGGAGATAGAACGACGGCTCC
ACAGCCAGTCCGGCCGGTCGCCGCCGGCCAGGGCTTCCCATCCGCGATCCAACCACTCGACCAGCGACCGCGGCTTTG
TGGTACCAGGGGTAAGGGTTAGAACGTCGTTCAGGATGTCCTCGCCCCCGGGCCCGTGGGGCGCTGGGGCCACAAAGC
GGCCCCCGCCGGGGGGCTCCAGACCCGCCAGCACCGCATCTGCGTCAGCCGCCCCCATGGCGCCCCCGCTGACGGCCT
GGTGAACCAGGGCGCCCTGGCGGAGCCCCGATGCAACGCCACAGGCCGCACGCCCGGTCCGAGCGCGGACCGGGTGGC
GGCGGGTGACGTCCTGCACTGCCCGCTGAACCAACGCGAGGATCTCCTCGTTCTCCTGTGCGATGGACACGTCCTGGG
CCGCGGTCGTGTCGCCGCCGGGGGCCGTCAGCTGCTCCTCCGGGGAGATGGGGGGGTCGGACGCCCCGACGATGGGCG
GGTCTGCGGGCGCCCCCGCGTGGGGCCGGGCCAAGGGCTGCGGACGCGGGGACGCGCTTTCCCCCAGACCCATGGACA
GGTGGGCCGCGGCCTCCTTCGCGGCCGGCGGGGCGGCGGCGCCAAGCAGAGCGACGTAGCGGCACAAATGCCGACAGA
CGCGCATGATGCGCGTGCTGTCGGCCGCGTAGCGCGTGTTGGGGGGGACGAGCTCGTCGTAACTAAACAGAATCACGC
GGGCACAGCTCGCCCCCGAGCCCCACGCGAGGCGCAGCGCCGCCACGGCGTACGGGTCATAGACGCCCTGCGCGTCAC
ACACCACGGGCAGGGAGACGAACAACCCCCCGGCGCTGGACGCACGCGGAAGGAGGCCAGGGTGTGCCGGCACGACGG
GGGCCAGAAGCTCCCCCACCGCATCCGCGGGCACGTAGGCGGCAAACGCCGTGCACCACGGGGTACAGTCGCCGGTGG
CATGAGCCCGAGTCTGGATTTCGACCTGGAAGTTTGCGGCCGTCCCGAGTCCGGGGCGGCCGCGCATCAGGGCGGCCA
GAGGGATTCCCGCGGCCGCCAGGCACTCGCTGGATATGATGACGTGAACCAAAGACGAGGGCCGACCCGGGCCGTGGC
CGAGATCGTACTGGACCTCGTTGGCCAAGTGCGCGTTCATGGTTCGGGGTGGGTGTGGGTGTGTAGGCGATGCGGGTC
CCCCGAGTCCGCGGGAAGGGCGTGGGTTTGGCGCGCGTATGCGTATTCGCCAACGGAGGCGTGCGTGCTTATGCGCGG
CGCGTTTCTTCTGTCTCCAGGGAATCCGAGGCCAGGACTTTAACCTGCTCTTTGTCGACGAGGCCAACTTTATTCGCC
CGGATGCGGTCCAGACGATTATGGGCTTTCTCAACCAGGCCAACTGCAAGATTATCTTCGTGTCGTCCACCAACACCG
GGAAGGCCAGTACGAGCTTTTTGTACAACCTCCGCGGGGCCGCCGACGAGCTTCTCAACGTGGTGACCTATATATGCG
ATGATCACATGCCGAGGGTGGTGACGCACACAAACGCCACGGCCTGTTCTTGTTATATCCTCAACAAGCCCGTTTTCA
TCACGATGGACGGGGCGGTTCGCCGGACCGCCGATTTGTTTCTGGCCGATTCCTTCATGCAGGAGATCATCGGGGGCC
AGGCCAGGGAGACCGGCGACGACCGGCCCGTTCTGACCAAGTCTGCGGGGGAGCGGTTTCTGTTGTACCGCCCCTCGA
CCACCACCAACAGCGGCCTCATGGCCCCCGATTTGTACGTGTACGTGGATCCCGCGTTCACGGCCAACACCCGAGCCT
CCGGGACCGGCGTCGCTGTCGTCGGGCGGTACCGCGACGATTATATCATCTTTGCCCTGGAGCACTTTTTTCTCCGCG
CGCTCACGGGCTCGGCCCCCGCCGACATCGCCCGCTGCGTCGTCCACAGTCTGACGCAGGTCCTGGCCCTGCATCCCG
GGGCGTTTCGCGGCGTCCGGGTGGCGGTCGAGGGAAATAGCAGCCAGGACTCGGCCGTCGCCATCGCCACGCACGTGC
ACACAGAGATGCACCGCCTACTGGCCTCGGAGGGGGCCGACGCGGGCTCGGGCCCCGAGCTTCTCTTCTACCACTGCG
AGCCTCCCGGGAGCGCGGTGCTGTACCCCTTTTTCCTGCTCAACAAACAGAAGACGCCCGCCTTTGAACACTTTATTA
AAAAGTTTAACTCCGGGGGCGTCATGGCCTCCCAGGAGATCGTTTCCGCGACGGTGCGCCTGCAGACCGACCCGGTCG
 
AGTATCTGCTCGAGCAGCTGAATAACCTCACCGAAACCGTCTCCCCAACACTGACGTCCGTACGTATTCCGGAAAAC
GGAACGGCGCCTCGGATGACCTTATGGTCGCCGTCATTATGGCCATCTACCTTGCGGCCCAGGCCGGACCTCCGCACA
CATTCGCTCCCATCACACGCGTTTCGTGAGCGCCCAATAAACACACCCAGGTATGCTACGCACGACCACGGTGTCGCC
TGTTAAGGGGGGGGAAGGGGGTGTTGGCGGGAAGCGTGGGAACACGGGGGATTCTCTCACGACCGGCACCAGTACCAC
CCCCCTGTGAACACAGAAACCCAACCCAAATCCCATAAACATACGACACACAGGCATATTTTGGAATTTCTTGGGTTT
TTATTTATTTAGGTATGCTGGGGTTTCTCCCTGGATGCCCACCCCCCACCCCCCCGTGGGTCTAGCCGGGCCTTAGGG
ATAGCGTATAACGGGGGCCATGTCTCCGGACCGCACAACGGCCGCGCCGTCAAAGGTGCACACCCGAACCACGGGAGC
CAGGGCCAAGGTGTCTCCTAGTTGGCCCGCGTGGGTCAGCCAGGCGACGAGCGCCTCGTAAAGCGGCAGCCTTCGCTC
TCCATCCTGCACCAGGGCCGGGGCTTCGGGGTGAATGAGCTGGGCGGCCTCCCGCGTGACACTCTGCATCTGCAGGAG
AGCGTTCACGTACCCGTCCTGGGCACTTAGCGCAAAGAGCCGGGGGATTAGCGTAAGGATGATGGTGGTTCCCTCCGT
GATCGAGTAAACCATGTTAAGGACCAGCGATCGCAGCTCGGCGTTTACGGGACCGAGTTGTTGGACGTCCGCCAGCAG
CGAGAGGCGACTCCCGTTGTAGTACAGCACGTTGAGGTCTGGCAGCCCTCCGGGGTTTCTGGGGCTGGGGTTCAGGTC
CCGGATGCCCCTGGCCACGAGCCGCGCCACGATTTCGCGCGCCAGGGGCGATGGAAGCGGAACGGAAACCGCAACGT
GAGGTCCAGCGAATCCAGGCGCACGTCCGTCGCTTGGCCCTCGAACACGGGCGGGACGAGGCTGATGGGGTCCCCGTT
ACAGAGATCTACGGGGGAGGTGTTGCGAAGGTTAACGGTGCCGGCGTGGGTGAGGCCCACGTCCAGGGGGCAGGCGAC
GATTCGCGTGGGAAGCACCCGGGTGATGACCGCGGGGAAGCGCCTTCGGTACGCCAGCAACAACCCCAACGTGTCGGG
ACTGACGCCTCCGGAGACGAAGGATTCGTGCGCCACGTCGGCCAGCGTCAGTTGCCGGCGGATGGTCGGCAGGAATAC
CACCCGCCCTTCGCAGCGCTGCAGCGCCGCCGCATCGGGGCGCGAGATGCCCGAGGGTATCGCGATGTCAGTTTCAAA
GCCGTCCGCCAGCATGGCGCCGATCCACGCGGCAGGGAGTGCAGTGGTGGTTCGGGTGGCGGGAGGAGCGCGGTGGGG
GTCAGCGGCGTAGCAGAGACGGGCGACCAACCTCGCATAGGACGGGGGGTGGGTCTTAGGGGGTTGGGAGGCGACAGG
GACCCCAGAGCATGCGCGGGGAGGTCTGTCGGGCCCAGACGCACCGAGAGCGAATCCGTCCACGGAGTCCCGGTCTGG
GTTTTATGGGGCCCGGCCCTCGGAATCGCGGCTTGTCGGCGGGGACAAAGGGGGCGGGGCTAGGGGGCTTGCGGAAAC
AGAAGACGCGTGGGATAAAAGAATCGCACTACCCCAAGGAAGGGCGGGGCGGTTTATTACAGAGCCAGTCCCTTGAGC
GGGGATGCGTCATAGACGAGATACTGCGCGAAGTGGGTCTCCCGCGCGTGGGCTTCCCCGTTGCGGGCGCTGCGGAGG
AGGGCGGGGTCGCTGGCGCAGGTGAGCGGGTAGGCCTCCTGAAACAGGCCACACGGGTCCTCCACGAGTTCGCGGCAC
CCCGGGGGGCGCTTAAACTGTACGTCGCTGGCGGCGGTGGCCGTGGACACCGCCGAACCCGTCTCCACGATCAGGCGC
TCCAGGCAGCGATGTTTGGCGGCGATGTCGGCCGACGTAAAGAACTTAAAGCAGGGGCTGAGCACCGGCGAGGCCCCG
TTGAGGTGGTAGGCCCCGTTATAGAGCAGGTCCCCGTACGAAAATCGCTGCGACGCCCACGGGTTGGCCGTGGCCGCA
AAGGCCCGGGACGGGTCGCTCTGGCCGTGGTCGTACATGAGGGCGGTGACATCCCCCTCCTTGTCCCCCGCGTAAACG
CCCCCGGCGGCGCGTCCCCGGGGGTTGCAGGGCCGGCGGAAGTAGTTGACGTCGGTCGACACGGGGGTGGCGATAAAC
TCACACACGGCGTCCTGGCCGTGGTCCATCCCTGCGCGCCGCGGCACCTGGGCGCACCCGAACACGGGGACGGGCTGG
GCCGGCCCCAGGCGGTTTCCCGCCACGACCGCGTTCCGCAGGTACACGGCTGCCGCGTTGTCCAGGAGAGGGGGAGCC
CCGCGGCCCAGGTAAAAGTTTTGGGGAAGGTTGCCCATGTCGGTGACGGGGTTGCGGACGGTTGCCGTGGCCACGACG
GCGGTGTAGCCCACGCCCAGGTCCACGTTCCCGCGCGGCTGGGTGAGCGTGAAGTTTACCCCCCCGCCAGTTTCGTGC
CGGGCCACCTGGAGCTGGCCCAGGAAGTACGCCTCCGACGCGCGCTCCGAGAACAGCATGTTCTCAGTCACAAAGCGG
TCCTGTCGGACGACGGTGAACCCAAACCCGGGATGGAGGCCCGTCTTGAGCTGATGATGCAAGGCCACGGGACTGATC
TTGAAGTACCCCGCCATGAGCGCGTAGGTCAGCGCGTTCTCCCCGGCCGCGCTCTCGCGGACGTGCTGCACGACGGGC
TGTCGGATCGACGAAAAGTAGTTGGCCCCCAGAGCCGGGGGACCAGGGGGACCTGCCGCGACAGGTCGCGCAGGGCC
 
GGGGGGAAATTGGGCGCGTTCGCCACGTGGTCGGCCCCGGCGAACAGCGCGTTGACGGGAAGGGGGTAAAAATAGTCG
CCATTTTGGATGGTATGGTCCAGATGCTGGGGGGCCATCAGCAGGATTCCGGCGTGCAACGCCCCGTCGAATATGCGC
ATGTTGGTGGTGGACGCGGTGTTGGCGCCCGCGTCGGGCGCCGCCGAGCAGAGCAGCGCCGTTGTGCGTTCGGCCATG
TTGTGGGCCAGCACCTGCAGCGTGAGCATGGCGGGCCCGTCCACTACCACGCGCCCGTTGTGAAACATGGCGTTGACC
GTGTTGGCCACCAGATTGGCCGGGTGCAGGGGGTGCGCGGGGTCCGTCACGGGGTCGCTGGGGCACTCCTCGCCGGGG
GCGATCTCCGGGACCACCATGTTCTGCAGGGTGGCGTATACGCGGTCGAAGCGAACCCCCGCGGTGCAGCAGCGGCCC
CGCGAGAAGGCGGGCACCATCACGTAGTAGTAAATCTTGTGGTGCACGGTCCAGTCCGCCCCCCGGTGCGGCCGGTCA
TCCGCGGCGTCCGCGGCTCGGGCCTGGGTGTTGTGCAGCAGCTGGCCGTCGTTGCGGTTGAAGTCCGCGGTCGCCACG
TTACATGCCGCCGCGTACACGGGGTCGTGGCCCCCCGCGCTAACCCGGCAGTCGCGATGGCGGTCCAGGGCCGCGCGC
CGCATCAGGGCGTCACAGTCCCACACGAGGGGTGGCAGCAGCGCCGGGTCTCGCATTAGGTGATTCAGCTCGGCTTGC
GCCTGCCCGCCCAGCTCCGGGCCGGTCAGGGTAAAGTCATCAACCAGCTGGGCCAGGGCCTCGACGTGCGCCACCAGG
TCCCGGTACACGGCCATGCACTCCTCGGGAAGGTCTCCCCCGAGGTAGGTCACGACGTACGAGACCAGCGAGTAGTCG
TTCACGAACGCCGCGCACCGCGTGTTGTTCCAGTAGCTGGTGATGCACTGGACAACGAGCCGGGCCAGGGCGCAGAAG
ACGTGCTCGCTGCCGTGTATGGCGGCCTGCAGCAGGTAAAACACCGCCGGGTAGTTGCGGTCGTCGAACGCCCCGCGA
ACGGCGGCGATGGTGGCGGGGGCCATGGCGTGGCGTCCCACCCCCAGCTCCAGGCCCCGGGCGTCCCGGAACGCCGCC
GGACATAGCGCCAGGGGCAAGTTGCCGTTCACCACGCGCCAGGTGGCCTGGATCTCCCCCGGCCGGCCGGGGGAACG
TCCCCCCCCGGCAGCTCCACGTCGGCCACCCCCACGAAGAAGTCGAACGCGGGGTGCAGCTCAAGAGCCAGGTTGGCG
TTGTCGGGCTGCATAAACTGCTCCGGGGTCATCTGGCCTTCCGCGACCCATCGGACCCGCCCGTGGGCCAGGCGCTGC
CCCCAGGCGTTCAAAAACAGCTGCTGCATGTCTGCGGCGGGGCCGGCCGGGGCCGCCACGTACGCCCCGTACGGATTG
GCGGCTTCGACGGGGTCGCGGTTAAGGCCCCCGACCGCCGCGTCAACGTTCATCAGCGAAGGGTGGCACACGGTCCCG
ATCGCGTGTTCCAGAGACAGGCGCAGCACCTGGCGGTCCTTCCCCCAAAAAAACAGCTGGCGGGGCGGGAAGGCGCGG
GGATCCGGGTGGCCGGGGGCGGGGACTAGGTCCCCGGCGTGCGCGGCAAACCGTTCCATGACCGGATTGAACAGGCCC
AGGGGCAGGACGAACGTCAGGTCCATGGCGCCCACCAGGGGGTAGGGAACGTTGGTGGCGGCGTAGATGCGCTTCTCC
AGGGCCTCCAGAAAGACCAGCTTCTCGCCGATGGACACCAGATCCGCGCGCACGCGCGTCGTCTGGGGGGCGCTCTCG
AGCTCGTCCAGCGTCTGCCGGTTCAGGTCGAGCTGCTCCTCCTGCATCTCCAGCAGGTGGCGGCCCACGTCGTCCAGA
CTTCGCACGGCCTTGCCCATCACGAGCGCCGTGACCAGGTTGGCCCCGTTCAGGACCATCTCGCCGTACGTCACCGGC
ACGTCGGCTTCGGTGTCCTCCACTTTCAGGAAGGACTGCAGGAGGCGCTGTTTGATCGGGGCTGTGGTGACTAGCACC
CCGTCGACCGGCCGCCCGCGCGTGTCGGCATGCGTCAGACGGGGCACGGCCACGGAGGGCTGCGTGGCCGTGGTGAGG
TCCACGAGCCAGGCCTCGACGGCCTCCCGGCGGTGGCCCGCCTTGCCCAGGAAAAAGCTCGTCTCGCAGAAGCTTCGC
TTTAGCTCGGCGACCAGGGTCGCCCGGGCCACCCTGGTGGCCAGGCGGCCGTTGTCCAGGTATCGTTGCATCGGCAAC
AACAAAGCCAGGGGCGGCGCCTTTTCCAGCAGCACGTGCAGCATCTGGTCGGCCGTGCCGCGCTCAAACGCCCCGAGG
ACGGCCTGGACGTTGCGAGCGAGCTGTTGGATGGCGCGCAACTGGCGATGCGCGCTGATACCCGTCCCGTCCAGGGCC
TCCCCCGTGAGCAGGGCGATGGCCTCGGTGGCCAGGCTGAAGGCGGCGTTCAGGGCCCGGCGGTCGATAATCTTGGTC
ATGTAATTGTGTGTGGGTTGCTCGATGGGGTGCGGGCCGTCGCGGGCAATCAGCGGCTGGTGGACCTCGAACTGTACG
CGCCCCTCGTTCATGTAGGCCAGCTCCGGAAACTTGGTACACACGCACGCCACCGACAACCCGAGCTCCAGAAAGCGC
ACGAGCGACAGGGTGTTGCAATACGACCCCAGCAGGGCGTCGAACTCGACGTCGTACAGGCTGTTTGCATCGGAGCGC
ACGCGGGAAAAAAAATCGAACAGGCGTCGATGCGACGCCACCTCGATCGTGCTAAGGAGGGACCCGGTCGGCACCATG
GCCGCGGCATACCGGTATCCCGGAGGGTCGCGGTTGGGAGCGGCCATAGGGTCGCGTGGAGATCGGCTGTCTCTAGCG
 
ATATTGGCCCGGGGAGGCTAAGATCCACCCCAACGCCCGGCCACCCGTGTACGTGCCCGACGGCCCAAGGTCCACCGA
AAGACACGACGGACCCGGACCCAAAGAGGCGGGGGATGCTGTGTGAGAGGCCGGGTGTCGGTCGGGGGGGAAAGGCAC
CGGGAGAAGGCTGCGGCCTCGTTCCAGGAGAACCCAGTGTCCCCAACAGACCCGGGGACGTGGGATCCCCGGCCTTAT
ATACCCCCCCCCCCGCCCCACCCCCGTTAGAACGCGACGGGTGCATTCAAGATGGCCCTGGTCCAAAAGCGTGCCAGG
AAGAAATTGGCAGAGGCGGCAAAGCTGTCCGCCGCCGCCACCCACATCGAGGCCCCGGCCACACAGGCTATCCCCAGG
GCCCGTGTGCGCAGGGGATCGGTGGGTGGCAGCATTTGGTTGGTGGCGATAAAGTGGAAAAGCCCGTCCGGACTGAAG
GTCTCGTGGGCGGCGGCGAACAAGGCACACAGGGCCGTGCCTCCCAAAAACATGGACATCCCCCAAAACACGGGCGCC
GACAACGGCAGACGATCCCTCTTGATGTTAACGTACAGGAGGAGCGCCCGCACCGCCCACGTAACGTAGTAGCCGACG
ATGGCGGCCAGGATACAGGCCGGCGCCACCACCCTTCCGGTCAGCCCGTAATACATGCCCGCTGCCACCATCTCCAAC
GGCTTCAGGACCAAAAACGACCAAAGGAACAGAATCACGCGCTTTGAAAAGACCGGCTGGGTATGGGGCGGAAGACGC
GAGTATGCCGAACTGACAAAAAAATCAGAGGTGCCGTACGAGGACAATGAAAACTGTTCCTCCAGCGGCAGTTCTCCC
TCCTCCCCCCCGAAGGCGGCCTCGTCGACCAGATCTCGATCCACCAGAGGAAGGTCATCCCGCATGGTCATGGGGTGT
GCGGTGGAGGTGGGGAGACCGAAACCGCAAAGGGTCGCTTACGTCAGCAGGATCCCGAGATCAAAGACACCCGGGTTC
TTGCACAAACACCACCCGGGTTGCATCCGCGGAGGCGAGTGTTTTGATAAGGCCGTTCCGCGCCTTGATATAACCTTT
GATGTTGACCACAAAACCCGGAATTTACGCCTACGCCCCAATGCCCACGCAAGATGAGGTAGGTAACCCCCCCGTGGG
TGTGACGTTGCGTTTAGTTCATTGGAGGCCAAGGGGAAAAATGGGGTGGGGAGGAAACGGAAAACCCAGTAGGCCGTG
TCGGGAACACGCCCGGGGTTGTCCTCAAAAGGCAGGGTCCATACTACGGAAGCCGTCGTTGTATTCGAGACCTGCCTG
TGCGACGCACGTCGGGGTTGCCTGTGTCCGGTTCGGCCCCCACCGCGTGCGGCACGCACGAGGACGAGTCCGCGTGCT
TTATTGGCGTTCCAAGCGTTGCCCTCCAGTTTCTGTTGTCGGTGTTCCCCCATACCCACGCCCACATCCACCGTAGGG
GGCCTCTGGGCCGTGTTACGTCGCCGCCCGCGATGGAGCTTAGCTACGCCACCACCATGCACTACCGGACGTTGTGT
TTTACGTCACAACGGACCGAAACCGGGCCTACTTTGTGTGCGGGGGGTGTGTTTATTCCGTGGGGCGGCCGTGTGCCT
CGCAGCCCGGGGAGATTGCCAAGTTTGGTCTGGTCGTTCGAGGGACAGGCCCAGACGACCGCGTGGTCGCCAACTATG
TACGAAGCGAACTCCGACAACGCGGCCTGCAGGACGTGCGTCCCATTGGGGAGGACGAGGTGTTTCTGGACAGCGTGT
GTCTTCTAAACCCGAACGTGAGCTCCGAGCTGGATGTGATTAACACGAACGACGTGGAAGTGCTGGACGAATGTCTGG
CCGAGTACTGCACCTCGCTGCGAACCAGCCCGGGTGTGCTAATATCCGGGCTGCGCGTGCGGGCGCAAGACAGAATCA
TCGAGTTGTTTGAACACCCAACGATAGTCAACGTTTCCTCGCACTTTGTGTATACCCCGTCCCCATACGTGTTCGCCC
TGGCCCAGGCGCACCTCCCCCGGCTCCCGAGCTCGCTGGAGGCCCTGGTGAGCGGCCTGTTTGACGGCATCCCCGCCC
CACGCCAGCCACTTGACGCCCACAACCCGCGCACGGATGTGGTTATCACGGGCCGCCGCGCCCCACGACCCATCGCCG
GGTCGGGGGCGGGGTCGGGGGGCGCGGGCGCCAAGCGGGCCACCGTCAGCGAGTTCGTGCAAGTCAAACACATTGACC
GCGTGGGCCCCGCTGGCGTTTCGCCGGCGCCTCCGCCAAACAACACCGACTCGAGTTCCCTGGTGCCCGGGGCCCAGG
ATTCCGCCCCGCCCGGCCCCACGCTAAGGGAGCTGTGGTGGGTGTTTTATGCCGCAGACCGGGCGCTGGAGGAGCCCC
GCGCCGACTCTGGCCTCACCCGCGAGGAGGTACGTGCCGTACGTGGGTTCCGGGAGCAGGCGTGGAAACTGTTTGGCT
CCGCGGGGGCCCCGCGGGCGTTTATCGGGGCCGCGTTGGGCCTGAGCCCCCTCCAAAAGCTGGCCGTTTACTACTATA
TCATCCACCGAGAGAGGCGCCTGTCCCCCTTCCCCGCGCTAGTCCGGCTCGTAGGCCGGTACACACAGCGCCACGGCC
TGTACGTCCCTCGGCCCGACGACCCAGTCTTGGCCGATGCCATCAACGGGCTGGTTCGCGACGCGCTGGCGGCCGGAA
CCACAGCCGAGCAGCTCCTCATGTTCGACCTTCTCCCCCCAAAGGACGTGCCGGTGGGAAGCGACGTGCAGGCCGACA
GCACCGCTCTGCTGCGCTTTATAGAATCGCAACGTCTCGCCGTCCCCGGGGGGGTGATCTCCCCCGAGCACGTCGCGT
ACCTTGGTGCGTTCCTGAGCGTGCTGTACGCTGGCCGCGGGCGCATGTCCGCAGCAACGCACACCGCGCGGCTGACAG
 
GGGTGACCTCCCTGGTGCTAGCGGTGGGTGACGTGGACCGTCTTTCCGCGTTTGACCGCGGAGCGGCGGGCGCGGCCA
GCCGCACGCGGGCCGCCGGGTACCTGGATGTGCTTCTTACCGTTCGTCTCGCTCGCTCCAAACACGGACAGTCTGTGT
AACAGACCCCAATAAACGTATGTCGCTACCACACCCTTGTGTGTCAATGGACGCCTCTCCGGGGGGGAGAGGGAAAAC
AAAGAGGGGCTGGGGGAGCGGCACCACTGGGGCCTGAACAAACAAACCACAGACACGGTTACAGTTTATTCGGTCGGG
CGGATAAACGGCCGAAGCCACGCCCCCTTTATTCGCGTCTCCAAAAAAACGGGACACTTGTCCGGAGAACCTTTAGGA
TGCCAGCCAGGGCGGCGGTAATCATAACCACGCCCAGCGCAGAGGCGGCCAGAAACCCGGGCGCAATTGCGGCCACGG
GCTGCGTGTCAAAGGCTAGCAAATGAATGACGGTTCCGTTTGGAAATAGCAACAAGGCCGTGGACGGCACGTCGCTCG
AAAACACGCTCGGGGCGCCCTCCGTCGGCCCGGCGGCGATTTGCTGCTGTGTGTTGTCCGTATCCACCAGCAACACAG
ACATGACCTCCCCGGCTGGGGTGTAGCGCATAAACACGGCCCCCACGAGCCCCAGGTCGCGCTGGTTTTGGGTGCGCA
CCAGCCGCTTGGACTCGATATCCCGGGTGGAGCCTTCGCATGTCGCGGTGAGGTAGGTTAGGAACAGTGGGCGTCGGA
CGTCGACGCCGGTGAGCTTGTAGCCGATCCCCCGGGGCAGAGGGGAGTGGGTGACGACGTAGCTGGCGTTGTGGGTGA
TGGGTACCAGGATCCGTGGCTCGACGTTGGCAGACTGCCCCCCGCACCGATGTGAGGCCTCAGGGACGAAGGCGCGGA
TCAGGGCGTTGTAGTGTGCCCAGCGCGTCAGGGTCGAGGCGAGGCCGTGGGTCTGCTGGGCCAGGACTTCGACCGGGG
TCTCGGATCGGGTGGCTTGAGCCAGCGCGTCCAGGATAAACACGCTCTCGTCTAGATCAAAGCGCAGGGAGGCCGCGC
ATGGCGAAAAGTGGTCCGGAAGCCAAAAGAGGGTTTTCTGGTGGTCGGCCCGGGCCAGCGCGGTCCGGAGGTCGGCGT
TGGTCGCTGCGGCGACGTCGGACGTACACAGGGCCGATGCTATCAGAAGGCTCCGGCGGGCGCGTTCCCGCTGCACCG
CCGAGGGGACGCCCGCCAAGAACGGCTGCCGGAGGACAGCCGAGGCGTAAAATAGCGCCCGGTGGACGACCGGGGTGG
TCAGCACGCGGCCCCCTAGAAACTCGGCATACAGGGCGTCGATGAGATGGGCTGCGCTGGGCGCCACTGCGTCGTACG
CCGAGGGGCTATCCAGCACGAAGGCCAGCTGATAGCCCAGCGCGTGTAATGCCAAGCTCTGTTCGCGCTCCAGAATCT
CGGCCACCAGGTGCTGGAGCCGAGCCTCTAGCTGCAGGCGGGCCGTGGGATCCAAGACTGACACATTAAAAAACACAG
AATCCGCGGCACAGCCCGCGGCCCCGCGGGCGGCCAACCCGGCAAGCGCGCGCGAGTGGGCCAAAAAGCCTAGCAGGT
CGGAGAGGCAGACCGCGCCGTTTGCGTGGGCGGCGTTCACGAAAGCAAAACCCGACGTCGCGAGCAGCCCCGTTAGGC
GCCAGAAGAGAGGGGGGCGCGGGCCCTGCTCGGCGCCCGCGTCCCCCGAGAAAAACTCCGCGTATGCCCGCGACAGGA
ACTGGGCGTAGTTCGTGCCCTCCTCCGGGTAGCCGCCCACGCGGCGGAGGGCGTCCAGCGCGGAGCCGTTGTCGGCCC
GCGTCAGGGACCCTAGGACAAAGACCCGATACCGGGGGCCGCCCGGGGGCCCGGGAAGAGCCCCCGGGGGGTTTTCGT
CCGCGGGGTCCCCGACCCGATCTAGCGTCTGGCCCGCGGGGACCACCATCACTTCCACCGGAGGGCTGTCGTGCATGG
ATATCACGAGCCCCATGAATTCCCGCCCGTAGCGCGCGCGCACCAGCGCGGCATCGCACCCGAGCACCAGCTCCCCCG
TCGTCCAGATGCCCACGGGCCACGTCGAGGCCGACGGGGAGAAATACACGTACCTACCTGGGGATCTCAACAGGCCCC
GGGTGGCCAACCAGGTCGTGGACGCGTTGTGCAGGTGCGTGATGTCCAGCTCCGTCGTCGGGTGCCGCCGGGCCCCAA
CCGGCGGTCGGGGGGGCGGTGTATCACGCGGCCCGCTTGGGTGGCTCGCCGTCGCCACGTTGTCTCCCCGCGGGAACG
TCAGGGCCTCGGGGTCAGGGACGGCCGAAAACGTTACCCAGGCCCGGGAACGCAGCAACACGGAGGCGACTGGATTGT
ACAAGAGACCCTTAAGGGGGGCGACCGAGGGGGGAGGCTGGGCGGTCGGCTCGACCGTGGTGGGGGCGGCAGGCTCG
CGTTCGGGGGCCGGCCGAGCAGGTAGGTCTTCGGGATGTAAAGCAGCTGGCCGGGGTCCCGCGGAAACTCGGCCGTGG
TGACCAATACAAAACAAAAGCGCTCCTCGTACCAGCGAAGAAGGGGCAGAGATGCCGTAGTCAGGTTTAGTTCGTCCG
GCGGCGCCAGAAATCCGCGCGGTGGTTTTTGGGGGTCGGGGGTGTTTGGCAGCCACAGACGCCCGGTGTTTGTGTCGC
GCCAGTACATGCGGTCCATGCCCAGGCCATCCAAAAACCATGGGTCTGTCTGCTCAGTCCAGTCGTGGACCTGACCCC
ACGCAACGCCCAAAATAATAACCCCCACGAACCATAAACCATTCCCCATGGGGGACCCCGTCCCTAACCCACGGGGCC
CGTGGCTATGGCAGGGCTTGCCGCCCCGACGTTGGCTGCGAGCCCTGGGCCTTCACCCGAACTTGGGGGGTGGGGTGG
 
GGAAAAGGAAGAAACGCGGGCGTATTGGCCCCAATGGGGTCTCGGTGGGGTATCGACAGAGTGCCAGCCCTGGGACCG
AACCCCGCGTTTATGAACAAACGACCCAACACCCGTGCGTTTTATTCTGTCTTTTTATTGCCGTCATAGCGCGGGTTC
CTTCCGGTATTGTCTCCTTCCGTGTTTCAGTTAGCCTCCCCCATCTCCCGGGCAAACGTGCGCGCCAGGTCGCAGATC
GTCGGTATGGAGCCGGGGGTGGTGACGTGGGTCTGGACCATCCCGGAGGTAAGTTGCAGCAGGGCGTCCCGGCAGCCG
GCGGGCGATTGGTCGTAATCCAGGATAAAGACGTGCATGGGACGGAGGCGTTTGGCCAAGACGTCCAAGGCCCAGGCA
AACACGTTGTACAGGTCGCCGTTGGGGGCCAGCAACTCGGGGGCCCGAAACAGGGTAAATAACGTGTCCCCGATATGG
GGTCGTGGGCCCGCGTTGCTCTGGGGCTCGGCACCCTGGGGCGGCACGGCCGTCCCCGAAAGCTGTCCCCAATCCTCC
CGCCACGACCCGCCGCCCTGCAGATACCGCACCGTATTGGCAAGCAGCCCGTAAACGCGGCGAATCGCGGCCAGCATA
GCCAGGTCAAGCCGCTCGCCGGGGCGCTGGCGTTTGGCCAGGCGGTCGATGTGTCTGTCCTCCGGAAGGGCCCCCAAC
ACGATGTTTGTGCCGGGCAAGGTCGGCGGGATGAGGGCCACGAACGCCAGCACGGCCTGGGGGGTCATGCTGCCCATA
AGGTATCGCGCGGCCGGGTAGCACAGGAGGGCGGCGATGGGATGGCGGTCGAAGATGAGGGTGAGGGCCGGGGGCGGG
GCATGTGAGCTCCCAGCCTCCCCCCCGATATGAGGAGCCAGAACGGCGTCGGTCACGGCATAAGGCATGCCCATTGTT
ATCTGGGCGCTTGTCATTACCACCGCCGCGTCCCCGGCCGATATCTCACCCTGGTCGAGGCGGTGTTGTGTGGTGTAG
ATGTTCGCGATTGTCTCGGAAGCCCCCAGCACCTGCCAGTAAGTCATCGGCTCGGGTACGTAGACGATATCGTCGCGC
GAACCCAGGGCCACCAGCAGTTGCGTGGTGGTGGTTTCCCCATCCCGTGAGGACCGTCTATATAAACCCGCAGTAGC
GTGGGCATTTTCTGCTCCAGGCGGACTTCCGTGGCTTCTTGCTGCCGGCGAGGGCGCAACGCCGTACGTCGGTTGCTA
TGGCCGCGAGAACGCGCAGCCTGGTCGAACGCAGACGCGTGTTGATGGCAGGGGTACGAAGCCATACGCGCTTCTACA
AGGCGCTTGCCGAAGAGGTGCGGGAGTTTCACGCCACCAAGATCTGCGGCACGCTGTTGACGCTGTTAAGCGGGTCGC
TGCAGGGTCGCTCGGTGTTCGAGGCCACACGCGTCACCTTAATATGCGAAGTGGACCTGGGACCGCGCCGCCCCGACT
GCATCTGCGTGTTCGAATTCGCCAATGACAAGACGCTGGGCGGGGTTTGTGTCATCATAGAACTAAAGACATGCAAAT
ATATTTCTTCCGGGGACACCGCCAGCAAACGCGAGCAACGGGCCACGGGGATGAAGCAGCTGCGCCACTCCCTGAAGC
TCCTGCAGTCCCTCGCGCCTCCGGGTGACAAGATAGTGTACCTGTGCCCCGTCCTGGTGTTTGTCGCCCAACGGACGC
TCCGCGTCAGCCGCGTGACCCGGCTCGTCCCGCAGAAGGTCTCCGGTAATATCACCGCAGTCGTGCGGATGCTCCAGA
GCCTGTCCACGTATACGGTCCCCATGGAGCCTAGGACCCAGCGAGCCCGTCGCCGCCGCGGCGGCGCCGCCCGGGGGT
CTGCGAGCAGACCGAAAAGGTCACACTCTGGGGCGCGCGACCCGCCCGAGTCAGCGGCCCGCCAATTACCACCCGCCG
ACCAAACCCCCGCCTCCACGGAGGGCGGGGGGTGCTTAAGAGGATCGCGGCGCTCTTCTGCGTGCCCGTGGCCACCA
AGACCAAACCCCGAGCCGCCTCCGAATGAGAGTGTTTCGTTCCTTCCCCCTCCCCCGCGTCAGACAAACCCTAACCA
CCGCTTAAGCGGCCCCCGCGAGGTCCGAAGACTCATTTGGATCCGGCGGGAGCCACCCGACAACAGCCCCCGGGTTTT
CCCACGCCAGACGCCGGTCCGCTGTGCCATCGCGCCCCCTCATCCCACCCCCCATCTTGTCCCCAAATAAAACAAGGT
CTGGTAATTAGGACAACGACCGCAGTTCTCGTGTGTTATTTTCGCTCTCCGCCTCTCGCAGATGGACCCGTACTGCCC
ATTTGACGCTCTGGACGTCTGGAACACAGGCGCTTCATAGTCGCCGATTCCCGAAACTTCATCACCCCCGAGTTCCC
CCGGGACTTTTGGATGTCGCCCGTCTTTAACCTCCCCCGGGAGACGGCGGCGGAGCAGGTGGTCGTCCTACAGGCCCA
GCGCACAGCGGCTGCCGCTGCCCTGGAGAACGCCGCCATGCAGGCGGCCGAGCTCCCCGTCGATATCGAGCGCCGGTT
ACGCCCGATCGAACGGAACGTGCACAAGATCGCAGGCGCCCTGGAGGCGCTGGAGACGGCGGCGGCCGCCGCCGAAGA
GGCGGATGCCGCGCGCGGGGATGAGCCGGCGGGTGGGGGCGACGGGGGGGCGCCCCCGAGTCTGGCCGTCGCGGAGAT
GGAGGTCCAGATCGTGCGCAACGACCCGCCGCTACGATACGACACCAACCTCCCCGTGGATCTGCTACACATGGTGTA
CGCGGGCCGCGGGGCGACCGGATCGTCGGGGGTGGTGTTCGGGACCTGGTACCGCACTATCCAGGACCGCACCATCAC
GGACTTTCCCCTGACCACCCGCAGTGCCGACTTTCGGGACGGCCGTATGTCCAAGACCTTCATGACGGCGCTGGTACT
 
GTCCCTGCAGTCGTGCGGCCGGCTGTATGTGGGCCAGCGCCACTATTCCGCCTTCGAGTGCGCCGTGTTGTGTCTCTA
CCTGCTGTACCGAAACACGCACGGGGCCGCCGACGATAGCGACCGCGCTCCGGTCACGTTCGGGGATCTGCTGGGCCG
GCTGCCCCGCTACCTGGCGTGCCTGGCCGCGGTGATCGGGACCGAGGGCGGCCGGCCACAGTACCGCTACCGCGACGA
CAAGCTCCCCAAGACGCAGTTCGCGGCCGGCGGGGGCCGCTACGAACACGGAGCGCTGGCGTCGCACATCGTGATCGC
CACGCTGATGCACCACGGGGTGCTCCCGGCGGCCCCGGGGGACGTCCCCCGGGACGCGAGCACCCACGTTAACCCCGA
CGGCGTGGCGCACCACGACGACATAAACCGCGCCGCCGCCGCGTTCCTCAGCCGGGGCCACAACCTATTCCTGTGGGA
GGACCAGACTCTGCTGCGGGCAACCGCGAACACCATAACGGCCCTGGGCGTTATCCAGCGGCTCCTCGCGAACGGCAA
CGTGTACGCGGACCGCCTCAACAACCGCCTGCAGCTGGGCATGCTGATCCCCGGAGCCGTCCCTTCGGAGGCCATCAC
CCGTGGGGCCTCCGGGTCCGACTCGGGGGCCATCAAGAGCGGAGACAACAATCTGGAGGCGCTATGTGCCAATTACGT
GCTTCCGCTGTACCGGGCCGACCCGGCGGTCGAGCTGACCCAGCTGTTTCCCGGCCTGGCCGCCCTGTGTCTTGACGC
CCAGGCGGGGCGGCCGGTCGGGTCGACGCGGCGGGTGGTGGATATGTCATCGGGGGCCCGCCAGGCGGCGCTGGTGCG
CCTCACCGCCCTGGAACTCATCAACCGCACCCGCACAAACCCCACCCCCGTGGGGGAGGTTATCCACGCCCACGACGC
CCTGGCGATCCAATACGAACAGGGGCTTGGCCTGCTGGCGCAGCAGGCACGCATTGGCTTGGGCTCCAACACCAAGCG
TTTCTCCGCGTTCAACGTTAGCAGCGACTACGACATGTTGTACTTTTTATGTCTGGGGTTCATTCCACAGTACCTGTC
GGCGGTTTAGTGGGTGGTGGGCGAGGGGGGAGGGGGCATTAGGGAGAAAGAACAAGAGCCTCCGTTGGGTTTTCTTTG
TGCCTGTACTCAAAAGGTCATACCCCGTAAACGGCGGGCTCCAGTCCCGGCCCGGCGGTTGGCGTGAACGCAACGGCG
GGAGCTGGGTTAGCGTTTAGTTTAGCATTCGCTCTCGCCTTTCCGCCCGCCCCCCGACCGTTGCGCCTTTTTTTTTTT
CGTCCACCAAAGTCTCTGTGGGTGCGCGCATGACAGCCGATGCCCCGGGAGACCGGATGGAGGAGCCCCTGCCAGACA
GGGCCGTGCCCATTTACGTGGCTGGGTTTTTGGCCCTGTATGACAGCGGGGACTCGGGCGAGTTGGCATTGGATCCGG
ATACGGTGCGTGCGGCCCTGCCTCCGGATAACCCACTCCCGATTAACGTGGACCACCGCGCTGGCTGCGAGGTGGGGC
GGGTGCTGGCCGTGGTCGACGACCCCCGCGGGCCGTTTTTTGTGGGACTGATCGCCTGCGTGCAACTGGAGCGCGTCC
TCGAGACGGCCGCCAGCGCTGCGATTTTCGAGCGCCGCGGGCCGCCGCTCTCCCGGGAGGAGCGCCTGTTGTACCTGA
TCACCAACTACCTGCCCTCGGTCTCCCTGGCCACAAAACGCCTGGGGGGCGAGGCGCACCCCGATCGCACGCTGTTCG
CGCACGTCGCGCTGTGCGCGATCGGGCGGCGCCTCGGCACTATCGTCACCTACGACACCGGTCTCGACGCCGCCATCG
CGCCCTTTCGCCACCTGTCGCCGGCGTCTCGCGAGGGGGCGCGGCGACTGGCCGCCGAGGCCGAGCTCGCGCTGTCCG
GACGCACCTGGGCGCCCGGCGTGGAGGCGCTGACCCACACGCTGCTTTCCACCGCCGTTAACAACATGATGCTGCGGG
ACCGCTGGAGCCTGGTGGCCGAGCGGCGGCGGCAGGCCGGGATCGCCGGACACACCTACCTCCAGGCGAGCGAAAAAT
TCAAAATGTGGGGGGCGGAGCCTGTTTCCGCGCCGGCGCGCGGGTATAAGAACGGGGCCCCGGAGTCCACGGACATAC
CGCCCGGCTCGATCGCTGCCGCGCCGCAGGGTGACCGGTGCCCAATCGTCCGTCAGTGCGGGGTCGCCTCGCCCCCGG
TACTGCCCCCCATGAACCCCGTTCCGGCATCGGGCACCCCGGCCCCCGCGCCGCCCGGCGACGGAGCTACCTGTGGA
TCCCGGCCTCCCATTACAACCAGCTCGTCGCCGGCCACGCCGCGCCCCAACCCCAGCCGCATTCCGCGTTTGGTTTCC
CGGCTGCGGCGGGGGCCGTGGCCTATGGGCCTCACGGCGCGGGTCTTTCCCAGCATTACCCTCCCCACGTCGCCCATC
AGTATCCCGGGGTGCTGTTCTCGGGACCCAGCCCACTCGAGGCGCAGATAGCCGCGTTGGTGGGGGCCATAGCCGCGG
ACCGCCAGGCGGGCGGTCAGACGGCCGCGGGAGACCCTGGGGTCCGGGGGTCGGGAAAGCGTCGCCGGTACGAGGCGG
GGCCGTCGGAGTCCTACTGCGACCAGGACGAACCGGACGCGGACTACCCGTACTACCCCGGGGAGGCTCGAGGCGGGC
CGCGCGGGGTCGACTCTCGGCGCGCGGCCCGCCAGTCTCCCGGGACCAACGAGACCATCACGGCGCTGATGGGGGCGG
TGACGTCTCTGCAGCAGGAACTGGCGCACATGCGGGCTCGGACCAGCGCCCCCTATGGAATGTACACGCCGGTGGCGC
ACTATCGCCCTCAGGTGGGGGAGCCGGAACCAACAACGACCCACCCGGCCCTTTGTCCCCCGGAGGCCGTGTATCGCC
 
CCCCACCACACAGCGCCCCCTACGGTCCTCCCAGGGTCCGGCGTCCCATGCCCCCACTCCCCCGTATGCCCCAGCTG
CCTGCCCGCCAGGCCCGCCACCGCCCCCATGTCCTTCCACCCAGACGCGCGCCCCTCTACCGACGGAGCCCGCGTTCC
CCCCCGCCGCCACCGGATCCCAACCGGAGGCATCCAACGCGGAGGCCGGGGCCCTTGTCAACGCCAGCAGCGCAGCAC
ACGTGGACGTTGACACGGCCCGCGCCGCCGATTTGTTCGTCTCTCAGATGATGGGGGCCCGCTGATTCGCCCCGGTCT
TTGGTACCATGGGATGTCTTACTGTATATCTTTTTAAATAAACCAGGTAATACCAAATAAGACCCATTGGTGTATGTT
CTTTTTTTATTGGGAGGCGCGGGTAGGCGGGTAGCTTTACAATGCAAAAACCTTCGACGTGGAGGAAGGCGTGGGGGG
GGGGAATCGGCACTGACCAAGGGGGTCCGTTTTGTCACGGGAAAGGAAAGAGGAAACAGGCCGCGGACACCCGGGGGA
GTTTATGTGTTCCCTTTTCTTTCTTCCCACACACACAAAAGGCGTACCAAACAAACAAACCAAAAGATGCACATGCGG
TTTAACACCCGTGGTTTTTATTTACAACAAACCCCCCGTCACAGGTCGTCCTCGTCGGCGTCACCGTCTTTGTTGGGA
ACTTGGGTGTAGTTGGTGTTGCGGCGCTTGCGCATGACCATGTCGGTGACCTTGGCGCTGAGCAGCGCGCTCGTGCCC
TTCTTCTTGGCCTTGTGTTCCGTGCGCTCCATGGCAGACACCAGGGCCATGTACCGTATCATCTCCCGGGCCTCGGCT
AGCTTGGCCTCGTCAAAGTCGCCGCCCTCCTCGCCCTCCCCGGACGCGTCCGGGTTGGTGGGGTTCTTGAGCTCCTTG
GTGGTTAGCGGGTACAGGGCCTTCATGGGGTTGCTCTGCAGCCGCATGACGTAGCGAAAGGCGAAGAAAGCCGCCGCC
AGGCCGGCCAGGACCAACAGACCCACGGCCAGCGCCCCAAAGGGGTTGGACATGAAGGAGGACACGCCCGACACGGCC
GATACCACGCCGCCCACGATGCCCATCACCACCTTGCCGACCGCGCGCCCCAGGTCGCCCATCCCCTCGAAGAACGCG
CCCAGGCCCGCGAACATGGCGGCGTTGGCGTCGGCGTGGATGACCGTGTCGATGTCGGCGAAGCGCAGGTCGTGCAGC
TGGTTGCGGCGCTGGACCTCCGTGTAGTCCAGCAGGCCGCTGTCCTTGATCTCGTGGCGGGTGTACACCTCCAGGGGG
ACAAACTCGTGATCCTCCAGCATGGTGATGTTGAGGTCGATGAAGGTGCTGACGGTGGTGATGTCGGCGCGGCTCAGC
TGGTGGGAGTACGCGTACTCCTCGAAGTACACGTAGCCCCCGCCGAAGGTGAAGTAGCGCCGGTGTCCCACGGTGCAC
GGCTCGATCGCATCGCGCGTCAGCCGCAGCTCGTTGTTCTCCCCCAGCTGCCCCTCGACCAACGGGCCCTGGTCTTCG
TACCGAAAGCTGACCAGGGGGCGGCTGTAGCAGGCCCCGGGCCGCGAGCTGATGCGCATCGAGTTTTGGACGATCACG
TTGTCCGCGGCGACCGGCACGCACGTGGAGACGGCCATCACGTCGCCGAGCATCCGCGCGCTCACCCGCCGGCCCACG
GTGGCCGAGGCGATGGCGTTGGGGTTCAGCTTGCGGGCCTCGTTCCACAGGGTCAGCTCGTGATTCTGCAGCTCGCAC
CACGCGATGGCAACGCGGCCCAACATATCGTTGACATGGCGCTGTATGTGGTTGTACGTAAACTGCAGCCGGGCGAAC
TCGATGGAGGAGGTGGTCTTGATGCGCTCCACGGACGCGTTGGCGCTGGCCCCGGGCGGCGGGGGCGTGGGGTTTGGG
GGCTTGCGGCTCTGCTCTCGGAGGTGTTCCCGCACGTACAGCTCCGCGAGCGTGTTGCTGAGAAGGGGCTGGTACGCG
ATCAGAAAACCCCCATTGGCCAGGTAGTACTGCGGCTGGCCCACCTTGATGTGCGTCGCGTTGTACCTGCGGGCGAAG
ATGCGGTCCATGGCGTCGCGGGCGTCCTTGCCGATGCAGTCCCCCAGGTCCACGCGCGAGAGCGGGTACTCGGTCAGG
TTGGTGGTGAAGGTGGTGGATATGGCGTCGGAGGAGAATCGGAAGGAGCCGCCGTACTCGGAGCGCAGCATCTCGTCC
ACCTCCTGCCACTTGGTCATGGTGCAGACCGACGGGCGCTTTGGCACCCAGTCCCAGGCCACGGTGAACTTGGGGGTC
GTGAGCAGGTTCCGGGTGGTCGGCGCCGTGGCCCGGGCCTTGGTGGTGAGGTCGCGCGCGTAGAAGCCGTCAACCTGC
TTGAAGCGGTCGGCGGCGTAGCTGGTGTGTTCGGTGTGCGACCCCTCCCGGTAGCCGTAAAACGGGGACATGTACACA
AAGTCGCCAGTCGCCAGCACAAACTCGTCGTACGGGTACACCGAGCGCGCGTCCACCTCCTCGACGATGCAGTTTACC
GTCGTCCCGTACCGGTGGAACGCCTCCACCCGCGAGGGGTTGTACTTGAGGTCGGTGGTGTGCCAGCCCCGGCTCGTG
CGGGTCGCGGCGTTGGCCGGTTTCAGCTCCATGTCGGTCTCGTGGTCGTCCCGGTGAAACGCGGTGGTCTCCAGGTTG
TTGCGCACGTACTTGGCCGTGGACCGACAGACCCCCTTGGCGTTGATCTTGTCGATCACCTCCTCGAAGGGGACGGGG
GCGCGGTCCTCAAAGATCCCCATAAACTGGGAGTAGCGGTGGCCGAACCACACCTGCGAAACGGTGACGTCTTTGTAG
TACATGGTGGCCTTGAACTTGTACGGGGCGATGTTCTCCTTGAAGACCACCGCGATGCCCTCCGTGTAGTTCTGACCC
 
TCGGGCCGGGTCGGCAGCGGCGCGGCTGCTCGAACTGCACCACCGTGGCGCCCGTGGGGGTGGCACACGTAAAAG
TTTGCATCGGTGTTCTCCGCCTTGATGTCCCGCAGGTGCTCGCGCAGGGTGGCGTGGCCCGCGGCGACGGTCGCGTTG
TCGCCGGCGGGGCGCGGCGGCGGTGGGGGTTTCGGTTTTTGTTCTTCTTCGGTTTCGTGTCCCCCGTTGGGCGGGG
CCAGGGGCGGGCGGCGCCGGAGTGGCAGGTCCCCCGTTCGCCGCCTGGGTCGCGGCCGCGACCCCAGGCGTGCCGGGG
GAACTCGGAGCCGCCGACGCCACCAGGACCCCCAGCGTCAACCCCAAGAGCGCCCATACGACGAACCACCGGCACCCC
CGCGCGGGGGCGCCCTGGCGCATGGCGGACTACGGGGGCCCGTCGTGCCCCCCGTCAGGTAGCCTGGGGGCGAGGTG
CTGGAGGACCGAGTAGAGGATCGAGAAAACGTCTCGGTCGTAGACCACGACCGACCGGGGGCCGATACAGCCGTCGGG
GGCGCTCTCGACGATGGCCACCAGCGGACAGTCGGAGTCGTACGTGAGATATACGCCGGGCGGGTAACGGTAACGACC
TTCGGAGGTCGGGCGGCTGCAGTCCGGGCGGCGCAACTCGAGCTCCCCGCACCGGTAGACCGAGGCAAAGAGTGTGGT
GGCGATAATCAGCTCGCGAATATATCGCCAGGCGGCGCGCTGAGTGGGCGTTATTCCGGAAATGCCGTCAAAACAGTA
AAACCTCTGAAATTCGCTGACGGCCCAATCAGCACCCGAGCCCCCGCCCCCATGATGAACCGGGCGAGCTCCTCCTT
CAGGTGCGGCAGGAGCCCCACGTTCTCGACGCTGTAATACAGCGCGGTGTTGGGGGGCTGGGCGAAGCTGTGGGTGGA
GTGATCAAAGAGGGGCCCGTTGACGAGCTCGAAGAAGCGATGGGTGATGCTGGGAGCAGGGCCGGGTCCACCTGGTG
TCGCAGGAGAGACGCTCGCATGAACCGGTGCGCGTCGAACACGCCCGGCGCCGAGCGGTTGTCGATGACCGTGCCCGC
GCCCGCCGTCAGGGCGCAGAAGCGCGCGCGCCGCAAAGCCGTTGGCGACCGCGGCGAACGTCGCGGGCAGCACCTC
GCCGTGGACGCTGACCCGCAGCATCTTCTCGAGCTCCCCGCGCTGCTCGCGGACGCAGCGCCCCAGGCTGGCCAACGA
CCGCTTCGTCAGGCGGTCCGCGTACAGCCGCCGTCGCTCCCGCACGTCCGCGGCCGCTTGCGTGGCGATGTCCCCCCA
CGTCTCGGGCCCCTGCCCCCCGGGCCCGCGGCGACGGTCTTCGTCCTCGCCCCCGCCCCCGGGAGCTCCCAACCCCCG
TGCCCCTTCCTCTACGGCGACACGGTCCCCGTCGTCGTCGGGGCCCGCGCCGCCCTTGGGCGCGTCCGCCGCGCCCCC
CGCCCCCATGCGCGCCAGCACGCGACGCAGCGCCTCCTCGTCGCACTGTTCGGGGCTGACGAGGCGCCGCAAGAGCGG
CGTCGTCAGGTGGTGGTCGTAGCACGCGCGGATGAGCGCCTCGATCTGATCGTCGGGTGACGTGGCCTGACCGCCGAT
TATTAGGGCGTCCACCATATCCAGCGCCGCCAGGTGGCTCCCGAACGCGCGATCGAAATGCTCCGCCCGCCGCCCGAA
CAGCGCCAGTTCCACGGCCACCGCGGCGGTCTCCTGCTGCAACTCGCGCCGCGCCAGCGCGGTCAGGTTGCTGGCAAA
CGCGTCCATGGTGGTCTGGCCGGCGCGGTCGCCGGACGCGAGCCAGAATCGCAATTCGCTGATGGCGTACAGGCCGGG
CGTGGTGGCCTGAAACACGTCGTGCGCCTCCAGCAGGGCGTCGGCCTCCTTGCGGACCGAGTCGTTCTCGGGCGACGG
GTGGGGCTGCCCGTCGCCCCCCGCGGTCCGGGCCAGCGCATGGTCCAACACGGAGAGCGCCCGCGCGGTCGGCGTC
CGACAGCCCGGCGGCGTGGGGCAGGTACCGCCGCAGCTCGTTGGCGTCCAGCCGCACCTGCGCCTGCTGGGTGACGTG
GTTACAGATACGGTCCGCCAGGCGGCGGGCGATCGTCGCCCCCTGGTTCGCCGTCACACACAGTTCCTCGAAACAGAC
CGCGCAGGGGTGGGACGGGTCGCTAAGCTCCGGGGGGACGATAAGGCCCGACCCCACCGCCCCCACCATAAACTCCCG
AACGCGCTCCAGCGCGGCGGTGGCGCCGCGCGAGGGGGTGATGAGGTGGCAGTAGTTTAGCTGCTTTAGAAAGTTCTC
GACGTCGTGCAGGAAACACAGCTCCATATGGACGGTCCCGCCATACGTATCCAGCCTGACCCGTTGGTGATACGGACA
GGGTCGGGCCAGGCCCATGGTCTCCGTGAAAAACACCGCGACGTCTCCCGCGGTCGCGAACGTCTCCAGGCTGCCCAG
GAGCCGCTCGCCCTCGCGCCACGCGTACTCTAGCAGCAACTCCAGGGTGACCGACAGCGGGGTGAGAAAGGCCCCGGC
CTGGGCCTCCAGGCCCGGCCTCAGACGACGCCGCAGCGCCCGCACCTGAAGCGCGTTCAGCTTCAGTTGGGGGAGCTT
CCCCCGTCCGATGTGGGGGTCGCACCGCCGGAGCAGCTCTATCTGAAACACATAGGTCTGCACCTGTCCGAGCAGGGC
TAACAACTTTTGACGGGCCACGGTGGGCTCGGACACCGGGGCGGCCATCTCGCGGCGCCGATCTGTACCGCGGCCGGA
GTATGCGGTGGACCGAGGCGGTCCGTACGCTACCCGGCGTCTGGCTGAGCCCCGGGGTCCCCCTATTCGGGGCGGCCT
CCCGCGGGCCCGCCGACCGGCAAGCCGGGAGTCGGCGGCGCGTGCGTTTCTGTTCTATTCCCAGACACCGCGGAGAGG
 
AATCACGGCCCGCCCAGAGATATAGACACGGAACACAAACAAGCACGGATGTCGTAGCAATAATTTATTTTACACACA
TTCCCCGCCCCGCCCTAGGTTCCCCCACCCCCCAACCCCTCACAGCATATCCAACGTCAGGTCTCCCTTTTTGTCGGG
GGGCCCCTCCCCAAACGGGTCATCCCCGTGGAACGCCCGTTTGCGGCCGGCAAATGCCGGTCCCGGGGCCCCCGGGCC
GCCGAACGGCGTCGCGTTGTCGTCCTCGCAGCCAAAATCCCCAAAGTTAAACACCTCCCCGACGTTGCCGAGTTGGCT
GACTAGGGCCTCGGCCTCGTGCGCCACCTCCAGGGCCGCGTCCGTCGACCACTCGCCGTTGCCGCGCTCCAGGGCACG
TGCGGTCAGCTCCATCATCTCCTCGCTTAGGTACTCGTCCTCCAGGAGCGCCAGCCAGTCCTCGATCTGCAGCTGTTG
GGTGCGGGGCCCCAGGCTTTTCACGGTCGCCACGAACACGCTACTGGCGACGGCCGCCCCGCCCTCGGAGATAATGCC
CCGGAGCTGCTCGCACAGCGAGCTTTCGTGCGCTCCGCCGCCGAGGCTCGAGGCCGCGCACACAAACCCGGCCCGGGG
ACAGGCCAGGACGAACTTGCGGGTGCGGTCAAAAATAAGGAGCGGGCACGCGTTTTTGCCGCCCATCAGGCTGGCCCA
GTTCCCGGCCTGAAACACACGGTCGTTGCCGGCCATGCCGTAGTATTTGCTGATGCTCAACCCCAACACGACCATGGG
GCGTGCCGCCATGACGGGCCGCAGCAGGTTGCAGCTGGCGAACATGGAGGTCCACGCGCCCGGATGCGCGTCCACGGC
GTCCATCAGCGCGCGGGCCCCGGCCTCCAGGCCCGCCCCGCCCTGCGCGGACCACGCGGCCGCCGCCTGCACGCTGGG
GGGACGGCGGACCCCGCGATGATGGCCGTGAGGGTGTTGATGAAGTACGTCGAGTGATCGCAGTACCGCAGAATCTG
GTTTGCCATGTAGTACATCGCCAGCTCGCTCACGTTGTTGGGGGCCAGGTTAATAAAGTTGATCGCGCCGTAGTCCAG
GGAAAACTTTTTAATGAACGCGATGGTCTCGATGTCCTCGCGCGACAGGAGCCGGGCGGGAAGCTGGTTGCGTTGGAG
GGCCGTCCAGAACCACTGCGGGTTCGGCTGGTTGGACCCCGGGGGCTTGCCGTTGGGGAAGATGGCCGCGTGGAACTG
CTTCAGCAGAAAGCCCAGCGGTCCGAGGAGGATGTCCACGCGCTTGTCGGGCTTCTGGTAGGCGCTCTGGAGGCTGGC
GACCCGCGCCTTGGCGGCCTCGGACGCGTTGGCGCTCGCGCCCGCGAACAACACGCGGCTCTTGACGCGCAGCTCCTT
GGGAAACCCCAGGGTCACGCGGGCAACGTCGCCCTCGAAGCTGCTCTCGGCGGGGGCCGTCTGGCCGGCCGTCAGGCT
GGGGGCGCAGATAGCCGCACCCTCCGAGAGCGCGACCGTCAGCGTTTTGGCCGACAGAAACCCGTTGTTAAACATGTC
CATCACGCGCCGCCGCAGCACCGGTTGGAATTGATTGCGAAAGTTGCGCCCCTCGACCGACTGCCCGGCGAACACCCC
GTGGCACTGGCTCAGGGCCAGGTCCTGGTACACGGCGAGGTTGGATCGCCGCCCGAGAAGCTGAAGCAGGGGGCACGG
CCCGCACGCGTACGGGTCCAGCGTCAGGGACATGGCGTGGTTGGCCTCGCCCAGACCGTCGCGAAACTTGAAGTTCCT
CCCCTCCACCAGGTTGCGCATCAGCTGCTCCACCTCGCGGTCCACGACCTGCCTGACGTTGTTCACCACCGTATGCAG
GGCCTCGCGGTTGGTGATGATGGTCTCCAGCCGCCCCATGGCCGTGGGGACCGCCTGGTCCACGTACTGCAGGGTCTC
GAGTTCGGCCATGACGCGCTCGGTCGCCGCGCGGTACGTCTCCTGCATGATGGTCCGGGCGGTCTCGGATCCGTCCGC
GCGCTTCAGGGCCGAGAAGGCGGCGTAGTTTCCCAGCACGTCGCAGTCGCTGTACATGCTGTTCATGGTCCCGAAGAC
GCCGATGGCTCCGCGGGCGGCGCTGGCGAACTTGGGATGGCGCGCCCGGAGGCGCATGAGCGTCGTGTGTACGCAGGC
GTGGCGCGTGTCGAAGGTGCACAGGTTACAGGGCACGTCGGTCTGGTTGGAGTCCGCGACGTATCGAAACACGTCCAT
CTCCTGGCGCCCGACGATCACGCCGCCGTCGCAGCGCTCCAGGTAAAACAGCATCTTGGCCAGCAGCGCCGGGGAAAA
CCCACACAACATGGCCAGGTGCTCGCCGGCAAATTCCTGGGTTCCGCCGACGAGGGGCGCGGTGGCCGACCCTCGAA
CCCGGGCACCACGTGTCCCTCGCGGTCCACCTGTGGGTTGGCCGCCACGTGGGTCCCGGGCACGAGGAAGAAGCGGTA
AAAGGAGGGTTTGCTGTGGTCCTTTGGGTCCGCCGGGCCGGCGTCGTCCACCTCGGTGAGATGGAGGGCCGAGTTGGT
GCTAAATACCATGGCCCCCACGAGTCCCGCGGCGCGCGCCAGGTACGCCCCGACGGCGTTGGCGCGGGCCGCGGCCGT
GTCCTGGCCCTCGAACAGCGGCCACGCGGAGATGTCGGTGGGCGGCTCGTCAAAGACGGCCATCGACACGATAGACTC
GAGGGCCAGGGCGGCATCTCCGGCCATGACGGAGGCCAGGCGCTGTTCGAACCCGCCCGCAGGGCCCTTGCCGCCGCC
GTCACGCCCGCCCCGCGGGGTCTTACCCTGGCTGGCTTCGAAGGCCGTGAACGTAATGTCGGCGGGGAGGGCGGCGCC
CTCGTGGTTTTCGTCAAACGCCAGGTGGGCGGCCGCGCGGGCCACGGCGTCCACGTTTCGGCATCGCAGTGCCACGGC
 
GGCGGGTCCCACGACCGCCTCGAACAGGAGGCGGTGGAGGGGGCGGTTAAAAAACGGAAGCGGGTAGGTAAAATTCTC
CCCGATAGATCGGTGGTTGGCGTTGAACGGCTCTGCGATGACACGGCTAAAATCCGGCATGAACAGCTGCAACGGGTA
CACGGGTATGCGGTGCACCTCCGCCCCGCCTATGGTTACCTTGTCCGAGCCTCCCAGGTGCAGAAAGGTGTTGTTGAT
GCACACGGCCTCCTTGAAGCCCTCGGTAACGACCAGATACAGGAGGGCGCGGTCCGGGTCCAGGCCGAGGCGCTCACA
CAGCGCCTCCCCCGTCGTCTCGTGTTTGAGGTCGCCGGGCCGGGGGGTGTAGTCCGAAAAGCCAAAATGGCGGCGTGC
CCGCTCGCAGAGTCGCGTCAGGTTCGGGGCCTGGGTGCTGGGGTCCAGGTGCCGGCCGCCGTGAAAGACGTACACGGA
CGAGCTGTAGTGCGAGGGCGTCAGTTTCAGGGACACCGCGGTACCCCCGAGCCCCGTCGTGCGAGAACCCACGACCAC
GGCCACGTTGGCCTCAAAGCCGCTCTCCACGGTCAGGCCCACGACCAGGGGCGCCACGGCGACGTCGGCATCGCCGCT
GCGCGCCGACAGTAACGCCAGAAGCTCGATGCCTTCGGACGGACACGCGCGAGCGTACACGTATCCCAGGGGCCCGGG
GGGGACCTTGATGGTGGTTGCCGTCTTGGGCTTTGTCTCCATGTCCTTCTGTCAATCGGTCCGCGAACGGAGGTAATC
CCGGCACGACGACGGACGCCCGACAAGGTATGTCTCCCGAGCGTCAAAATCCGGGGGGGGGCGGCGACGGTCAAGGGG
AGGGTTGGAGACCGGGGTTGGGGAATGAATCCCTACCCTTCACCGACAACCCCCCGGGTAATCACGGGGTGCCGATGA
ACCCCGGCGGCCGGCAACGCGGGGTCCCTGCGAGAGGCACAGATGCTTACGGTCAGGTGCTCCGGGTCGGGTGCGTCT
GGTATGCGGTTGGTATATGTACACTTTACCTGGGGGCGTGCCTGGCCGCCCCAGCCCCTCCCACGCCCCGCGCGTCAT
CAGCCGGTGGGCGTGGCCGCTATTATAAAAAAAGTGAGAACGCGAAGCGTTCGCACTTTGTCCTAATAATATATATAT
TATTAGGACAAAGTGCGAACGCTTCGCGTTCTCACTTTTTTTATAATAGCGCCCACGCCCACCGGCTACGTCACGCTC
CTGTCGGCCGCCGGCGGTCCATAAGCCCGGCCGGCCGGGCCGACGCGAATAAACCGGGCCGCCGGCCGGGGCGCCGCG
CAGCAGCTCGCCGCCCGGATCCGCCAGACAAACAAGGCCCTTGCACATGCCGGCCCGGGCGAGCCTGGGGGTCCGGTA
ATTTTGCCATCCCACCCAAGCGGCTTTTTGGGTTTTTCTCTTCCCCCCTCCCCACATCCCCCCTCTTTAGGGGTTCGG
GTGGTAACAACCGCGATGTTTTCCGGTGGCGGCGGCCCGCTGTCCCCCGGAGGAAAGTCGGCGGCCAGGGCGGCGTCC
GGGTTTTTTGCGCCCGCCGGCCCTCGCGGAGCCGGCCGGGGACCCCCGCCTTGCTTGAGGCAAAACTTTTACAACCCC
TACCTCGCCCCAGTCGGGACGCAACAGAAGCCGACCGGGCCAACCCAGCGCCATACGTACTATAGCGAATGCGATGAA
TTTCGATTCATCGCCCCGCGGGTGCTGGACGAGGATGCCCCCCCGGAGAAGCGCGCCGGGGTGCACGACGGTCACCTC
AAGCGCGCCCCCAAGGTGTACTGCGGGGGGGACGAGCGCGACGTCCTCCGCGTCGGGTCGGGCGGCTTCTGGCCGCGG
CGCTCGCGCCTGTGGGGCGGCGTGGACCACGCCCCGGCGGGGTTCAACCCCACCGTCACCGTCTTTCACGTGTACGAC
ATCCTGGAGAACGTGGAGCACGCGTACGCATGCGCGCGGCCCAGTTCCACGCGCGGTTTATGGACGCCATCACACCGA
CGGGGACCGTCATCACGCTCCTGGGCCTGACTCCGGAAGGCCACCGGGTGGCCGTTCACGTTTACGGCACGCGGCAGT
ACTTTTACATGAACAAGGAGGAGGTCGACAGGCACCTACAATGCCGCGCCCCACGAGATCTCTGCGAGCGCATGGCCG
CGGCCCTGCGCGAGTCCCCGGGCGCGTCGTTCCGCGGCATTTCCGCGGACCACTTCGAGGCGGAGGTGGTGGAGCGCA
CCGACGTGTACTACTACGAGACGCGCCCCGCTCTGTTTTACCGCGTCTACGTCCGAAGCGGGCGCGTGCTGTCGTACC
TGTGCGACAACTTCTGCCCGGCCATCAAGAAGTACGAGGGTGGGGTCGACGCCACCACCCGGTTCATCCTGGACAACC
CCGGGTTCGTCACCTTCGGCTGGTACCGTCTCAAACCGGGCCGGAACAACACGCTAGCCCAGCCGGCGGCCCCGATGG
CCTTCGGGACATCCAGCGACGTCGAGTTTAACTGTACGGCGGACAACCTGGCCATCGAGGGGGGCATGAGCGACCTAC
CGGCATACAAGCTCATGTGCTTCGATATCGAATGCAAGGCGGGGGGGGAGGACGAGCTGGCCTTTCCGGTGGCCGGGC
ACCCGGAGGACCTGGTCATCCAGATATCCTGTCTGCTCTACGACCTGTCCACCACCGCCCTGGAGCACGTCCTCCTGT
TTTCGCTCGGTTCCTGCGACCTCCCCGAATCCCACCTGAACGAGCTGGCGGCCAGGGGCCTGCCCACGCCCGTGGTTC
TGGAATTCGACAGCGAATTCGAGATGCTGTTGGCCTTCATGACCCTTGTGAAACAGTACGGCCCCGAGTTCGTGACCG
GGTACAACATCATCAACTTCGACTGGCCCTTCTTGCTGGCCAAGCTGACGGACATTTACAAGGTCCCCCTGGACGGGT
 
ACGGCCGCATGAACGGCCGGGGCGTGTTTCGCGTGTGGACATAGGCCAGAGCCACTTCCAGAAGCGCAGCAAGATAA
AGGTGAACGGCATGGTGAACATCGACATGTACGGATTATAACCGACAAGATCAAGCTCTCGAGCTACAAGCTCAACG
CCGTGGCCGAAGCCGTCCTGAAGGACAAGAAGAAGGACCTGAGCTATCGCGACATCCCCGCCTACTACGCCGCCGGGC
CCACGCAACGCGGGGTGATCGGCGAGTACTGCATACAGGATTCCCTGCTGGTGGGCCAGCTGTTTTTTAAGTTTTTGC
CCCATCTGGAGCTCTCGGCCGTCGCGCGCTTGGCGGGTATTAACATCACCCGCACCATCTACGACGGCCAGCAGATCC
GCGTCTTTACGTGCCTGCTGCGCCTGGCCGACCAGAAGGGCTTTATTCTGCCGGACACCCAGGGGCGATTTAGGGGCG
CCGGGGGGGAGGCGCCCAAGCGTCCGGCCGCAGCCCGGGAGGACGAGGAGCGGCCAGAGGAGGAGGGGGAGGACGAGG
ACGAACGCGAGGAGGGCGGGGGCGAGCGGGAGCCGGAGGGCGCGCGGGAGACCGCCGGCCGGCACGTGGGGTACCAGG
GGGCCAGGGTCCTTGACCCCACTTCCGGGTTTCATGTGAACCCCGTGGTGGTGTTCGACTTTGCCAGCCTGTACCCCA
GCATCATCCAGGCCCACAACCTGTGCTTCAGCACGCTCTCCCTGAGGGCCGACGCAGTGGCGCACCTGGAGGCGGGCA
AGGACTACCTGGAGATCGAGGTGGGGGGGCGACGGCTGTTCTTCGTCAAGGCTCACGTGCGAGAGAGCCTCCTCAGCA
TCCTCCTGCGGGACTGGCTCGCCATGCGAAAGCAGATCCGCTCGCGGATTCCCCAGAGCAGCCCCGAGGAGGCCGTGC
TCCTGGACAAGCAACAGGCCGCCATCAAGGTCGTGTGTAACTCGGTTTACGGGTTCACGGGAGTGCAGCACGGACTCC
TGCCGTGCCTGCACGTTGCCGCGACGGTGACGACCATCGGCCGCGAGATGCTGCTCGCGACCCGCGAGTACGTCCACG
CGCGCTGGGCGGCCTTCGAACAGCTCCTGGCCGATTTCCCGGAGGCGGCCGACATGCGCGCCCCCGGGCCCTATTCCA
TGCGCATCATCTACGGGGACACGGACTCCATCTTTGTGCTGTGCCGCGGCCTCACGGCCGCCGGGCTGACGGCCGTGG
GCGACAAGATGGCGAGCCACATCTCGCGCGCGCTGTTTCTGTCCCCCATCAAACTCGAGTGCGAAAAGACGTTCACCA
AGCTGCTGCTGATCGCCAAGAAAAAGTACATCGGCGTCATCTACGGGGGTAAGATGCTCATCAAGGGCGTGGATCTGG
TGCGCAAAAACAACTGCGCGTTTATCAACCGCACCTCCAGGGCCCTGGTCGACCTGCTGTTTTACGACGATACCGTAT
CCGGAGCGGCCGCCGCGTTAGCCGAGCGCCCCGCAGAGGAGTGGCTGGCGCGACCCCTGCCCGAGGGACTGCAGGCGT
TCGGGGCCGTCCTCGTAGACGCCCATCGGCGCATCACCGACCCGGAGAGGGACATCCAGGACTTTGTCCTCACCGCCG
AACTGAGCAGACACCCGCGCGCGTACACCAACAAGCGCCTGGCCCACCTGACGGTGTATTACAAGCTCATGGCCCGCC
GCGCGCAGGTCCCGTCCATCAAGGACCGGATCCCGTACGTGATCGTGGCCCAGACCCGCGAGGTAGAGGAGACGGTCG
CGCGGCTGGCCGCCCTCCGCGAGCTCGACGCCGCCGCCCCAGGGGACGAGCCCGCCCCCCCCGCGGCCCTGCCCTCCC
CGGCCAAGCGCCCCCGGGAGACGCCGTTGCATGCCGACCCCCCGGGAGGCGCGTCCAAGCCCCGCAAGCTGCTGGTGT
CCGAGCTGGCCGAGGATCCCGCATACGCCATTGCCCACGGCGTCGCCCTGAACACGGACTATTACTTCTCCCACCTGT
TGGGGGCGGCGTGCGTGACATTCAAGGCCCTGTTTGGGAATAACGCCAAGATCACCGAGAGTCTGTTAAAAAGGTTTA
TTCCCGAAGTGTGGCACCCCCCGGACGACGTGGCCGCGCGGCTCCGGGCCGCAGGGTTCGGGGCGGTGGGTGCCGGCG
CTACGGCGGAGGAAACTCGTCGAATGTTGCATAGAGCCTTTGATACTCTAGCATGAGCCCCCCGTCGAAGCTGATGTC
CCTCATTTTACAATAAATGTCTGCGGCCGACACGGTCGGAATCTCCGCGTCCGTGGGTTTCTCTGCGTTGCGCCGGAC
CACGAGCACAAACGTGCTCTGCCACACGTGGGCGACGAACCGGTACCCCGGGCACGCGGTGAGCATCCGGTCTATGAG
CCGGTAGTGCAGGTGGGCGGACGTGCCGGGAAAGATGACGTACAGCATGTGGCCCCCGTAAGTGGGGTCCGGGTAAAA
CAACAGCCGCGGGTCGCACGCCCCGCCTCCGCGCAGGATCGTGTGGACGAAAAAAAGCTCGGGTTGGCCAAGAATCCC
GGCCAAGAGGTCCTGGAGGGGGGCGTTGTGGCGGTCGGCCAACACGACCAAGGAGGCCAGGAAGGCGCGATGCTCGAA
TATCGTGTTGATCTGCTGCACGAAGGCCAGGATTAGGGCCTCGCGGCTGGTGGCGGCGAACCGCCCGTCTCCCGCGTT
GCACGCGGGACAGCAACCCCCGATGCCTAGGTAGTAGCCCATCCCGGAGAGGGTCAGGCAGTTGTCGGCCACGGTCTG
GTCCAGACAGAAGGGCAGCGAGACGGGAGTGGTCTTCACCAGGGGCACCGAGAGCGAGCGCACGATGGCGATCTCCTC
GGAGGGCGTCTGGGCGAGGGCGGCGAAAGGCCCCGATAGCGCTGGCGCTCGTGTAAACACAGCTCCTGTTTGCGGGC
 
GTGAGGCGGCAGGCTCTTCCGGGAGGCCCGACGCACCACGCCCAGAGTCCCGCCGGCCGCAGAGGAGCGCGACCGCCG
GCGCTCCTTGCCGTGATAGGGCCCGGGCCGGGAGCCGCGGCGATGGGGGTCGGTGTCATACATAGGTACACAGGGTGT
GCTCCAGGGACAGGAGCGAGATCGAGTGGCGTCTAAGCAGCGCGCCCGCCTCACGGACAAATGTGGCGAGCGCGGTGG
GCTTTGGTACAAATACCTGATACGTCTTGAAGGTGTAGATGAGGGCACGCAACGCTATGCAGACACGCCCCTCGAACT
CGTTCCCGCAGGCCAGTTTGGCCTTGTGGAGCAGCAGCTCGTCGGGATGGGTGGCGGGGGGATGGCCGAACAGAACCC
AGGGGTCAACCTCCATCTCCGTAATGGCGCACATGGGGTCACAGAACATGTGCTTAAAGATGGCCTCGGCCCCCGCGG
CCCGAAGCAGGCTCACAAACCGGCCCCCGTCCCCGGGCTGCGTCTCGGGGTCAGCCTCGAGCTGGTCGACGACGGGTA
CGATACAGTCGAAGAGGCTCGTGTTGTTTTCCGAGTAGCGGACCACGGAGGCCCGGAGTCTGCGCAGGGCCAGCCAGT
AAGCACGCACCAGTAACAGGTTACACAGCAGGCATTCTCCGCCGGTGCGCCCGCGCCCCCGGCCGTGTTTCAGCACGG
TGGCCATCAGAGGGCCCAGGTCGAGGTCGGGCTGGGCATCGGGTTCGGTAAACTGCGCAAAGCGGAGCCACGTCGC
GCGTGCGTGCCCCGCGATGCGCTTCCCAGGACTGGCGGACCGTGGCGCGACGGGCCTCCGCGGCAGCGCGCAGCTGGG
GCCCCGACTCCCAGACGGCGGGGTGCCGGCGAGGAGCAGCAGGACCAGATCCGCGTACGCCCACGTATCCGGCGACT
CCTCCGGCTCGCGGTCCCCGGCGACCGTCTCGAATTCCCCGTTGCGAGCGGCGGCGCGCGTACAGCAGCTGTCCCCGC
CCCCGCGCCGACCCTCCGTGCAGTCCAGGAGACGGGCGCAATCCTTCCAGTTCATCAGCGCGGTGGTGAGCGACGGCT
GCGTGCCGGATCCCGCCGACCCCGCCCCCTCCTCGCCCCCGGAGGCCAAGGTTCCGATGAGGGCCCGGGTGGCAGACT
GCGCCAGGAACGAGTAGTTGGAGTACTGCACCTTGGCGGCTCCCGGGGAGGGCGAGGGCTTGGGTTGCTTCTGGGCAT
GCCGCCCGGGCACCCCGCCGTCGGTACGGAAGCAGCAGTGGAGAAAAAGTGCCGGTGGATGTCGTTTATGGTGAGGG
CAAAGCGTGCGAAGGAGCCGACCAGGGTCGCCTTCTTGGTGCGCAGAAAGTGGCGGTCCATGACGTACACAAACTCGA
ACGCGGCCACGAAGATGCTAGCGGCGCAGTGGGGCGCCCCCAGGCATTTGGCACAGAGAAACGCGTAATCGGCCACCC
ACTGAGGCGAGAGGCGGTAGGTTTGCTTGTACAGCTCGATGGTGCGGCAGACCAGACAGGGCCGGTCCAGCGCGAAGG
TGTCGATGGCCGCCGCGGAAAAGGGCCCGGTGTCCAAAAGCCCCTCCCCACAGGGATCCGGGGGCGGGTTGCGGGGTC
CTCCGCGCCCGCCCGAACCCCCTCCGTCGCCCGCCCCCCCGCGGGCCCTTGAGGGGGCGGTGACCACGTCGGCGGCGA
CGTCCTCGTCGAGCGTACCGACGGGCGGCACACCTATCACGTGACTGGCCGTCAGGAGCTCGGCGCAGAGAGCCTCGT
TAAGAGCCAGGAGGCTGGGATCGAAGGCCACATACGCGCGCTCGAACGCCCCCGCCTTCCAGCTGCTGCCGGGGGACT
CTTCGCACACCGCGACGCTCGCCAGGACCCCGGGGGGCGAAGTTGCCATGGCTGGGCGGGAGGGGCGCACGCGCCAGC
GAACTTTACGGGACACAATCCCCGACTGCGCGCTGCGGTCCCAGACCCTGGAGAGTCTAGACGCGCGCTACGTCTCGC
GAGACGGCGCGCATGACGCGGCCGTCTGGTTCGAGGATATGACCCCCGCCGAGCTGGAGGTTGTCTTCCCGACTACGG
ACGCCAAGCTGAACTACCTGTCGCGGACGCAGCGGCTGGCCTCCCTCCTGACGTACGCCGGGCCTATAAAAGCGCCCG
ACGACGCCGCCGCCCCGCAGACCCCGGACACCGCGTGTGTGCACGGCGAGCTGCTCGCCCGCAAGCGGGAAAGATTCG
CGGCGGTCATTAACCGGTTCCTGGACCTGCACCAGATTCTGCGGGGCTGACGCGCGTGCTGTTGGGCGGACGGTTCG
CGAACCCTTTGGTGGGTTTACGCGGGCACGCACGCTCCCATCGCGGGCGCCATGGCGGGACTGGGCAAGCCCTACACC
GGCCACCCAGGTGACGCCTTCGAGGGTCTCGTTCAGCGAATTCGGCTTATCGTCCCATCTACGTTGCGGGGCGGGGAC
GGGGAGGCGGGCCCCTACTCTCCCTCCAGCCTCCCCTCCAGGTGCGCCTTTCAGTTTCATGGCCATGACGGGTCCGAC
GAGTCGTTTCCCATCGAGTATGTACTGCGGCTTATGAACGACTGGGCCGAGGTCCCGTGCAACCCTTACCTGCGCATA
CAGAACACCGGCGTGTCGGTGCTGTTTCAGGGGTTTTTTCATCGCCCACACAACGCCCCCGGGGGCGCGATTACGCCA
GAGCGGACCAATGTGATCCTGGGGTCCACCGAGACGACGGGGTTGTCCCTCGGCGACCTGGACACCATCAAGGGGCGG
CTCGGCCTGGATGCCCGGCCGATGATGGCCAGCATGTGGATCAGCTGCTTTGTGCGCATGCCCCGCGTGCAGCTCGCG
TTTCGGTTCATGGGCCCCGAAGATGCCGGACGGACGAGACGGATCCTGTGCCGCGCCGCCGAGCAGGCTATTACCCGT
 
CGCCGCCGAACCCGGCGGTCCCGGAGGCGTACGGGCCGAGGCCGGGCTGGGGGTGGCCGGAACGGGTTTCCGGGCC
AGGGGGGACGGTTTTGGCCCGCTCCCCTTGTTAACCCAAGGGCCCTCCCGCCCGTGGCACCAGGCCCTGCGGGGTCTT
AAGCACCTACGGATTGGCCCCCCCGCGCTCGTTTTGGCGGCGGACTCGTCCTGGGGGCCGCTATTTGGTGGGTGGTT
GGTGCTGGCGCGCGCCTATAAAAAAGGACGCACCGCCGCCCTAATCGCCAGTGCGTTCCGGACGCCTTCGCCCCACAC
AGCCCTCCCGACCGACACCCCCATATCGCTTCCCGACCTCCGGTCCCGATGGCGTCCCGCAATTTCACCGCCCCAACA
CCGTTACCACCGATAGCGTCCGGGCGCTTGGCATGCGCGGCTCGTCTTGGCCACCAATAACTCTCAGTTTATCATGG
ATAACAACCACCCACACCCCCAGGGCACCCAAGGGGCCGTGCGGGAGTTTCTCCGCGGTCAGGCGGCGGCACTGACGG
ACCTTGGTCTGGCCCACGCAAACAACACGTTTACCCCGCAGCCTATGTTCGCGGGCGACGCACCGGCCGCCTGGTTGC
GGCCCGCGTTTGGCCTGCGGCGCACCTATTCACCTTTTGTCGTTCGAGAACCTTCGACGCCCGGGACCCCGTGAGGCC
CAGGGAGTTCCTTCTGGGGTGTTTTAATCAATAAAAGACCACACCAACGCACGAGCCTTGCGTTTAATGTCGTGTTTA
TTCAAGGGAGTGGGATAGGGTTCGACGGTTCGAAACTTAACACACCAAATAATCGAGCGCGTCTAGCCCAGTAACATG
CGCACGTGATGTAGGCTGGTCAGCACGGCGTCGCTGTGATGAAGCAGCGCCCGGCGGGTCCGCTGTAACTGCTGTTGT
AGGCGGTAACAGGCGCGGATCAGCACCGCCAGGGCGCTACGACCGGTGCGTTGCACGTAGCGTCGCGACAGAACTGCG
TTTGCCGATACGGGCGGGGGGCCGAATTGTAAGCGCGTCACCTCTTGGGAGTCATCGGCGGATAACGCACTGAATGGT
TCGTTGGTTATGGGGGAGTGTGGTTCCCCAGGGAGTGGGTCGAACGCCTCGGCCTCGGAATCCGAGAGGAACAACGAG
GTGGCGTCGGAGTCTTCGTCGTCAGAGACATACAGGGTCTGAAGCAGCGACACGGGCGGGGGGGTAGCGTCGATGTGT
AGCGCGAGGGAGGATGCCCACGAAGACACCCCAGACAAGGAGCTGCCCGTGCGTGGATTTGTGGAAGACGCGGAAGCC
GGGACGGATGGGCGGTTTTGCGGTGCCCGGAACCGAACCGCCGGATACTCCCCGGGTGCTACATGCCCGTTTTGGGGC
TGGGGTTGGGGCTGGGGTTGGGGCTGGGGTTGGGGCTGGGGTTGGGGCTGGGGTTGGGGCTGGGGTTGGGGTTGGGGT
TGGGGCTGGGGTTGGGGTTGGGGCTGGGGCTGGGGCTGGGGCTGGGGCTGGGGCTGGGGCTGGGGCTGGGGCTGGGGC
TGGGGCTGGGGCTGGGGCTGGGGCTGGGGCTGGGGTTGGGGCTGGGGCGCGGACAGGCGGCTGACGGTCAAATGCCCC
CGGGGGCGCGCAGATGTGGTGGGCGTGGCCACCGGCTGCCGTGTAGTGGGGCGGCGGGAAACCGGGCCTCCGGGCGTA
ACACCGCCCTCCAGCGTCAAGTATGTGGGGGGCGGGCCTGACGTCGGGGGCGGGGTGACGGTTGGACCGCGGGAGGC
GGGGGAGAGGGACCTGCGGGAGAGGATGAGGTCGGCTCGGCCGGGTTGCGGCCTAAAACAGGGGCCGTGGGGTCGGCG
GGGTCCCAGGGTGAAGGGAGGGATTCCCGCGATTCGGACAGCGACGCGACAGCGGGGCGCGTAAGGCGCCGCTGCGGC
CCGCCTACGGGAACCCTGGGGGGGGTTGGCGCGGGACCCGAGGTTAGCGGGGGGCGGCGGTTTTCGCCCCCGGGCAAA
ACCGTGCCGGTTGCGACCGGGGGCGGAACGGGATCGATAGGGAGAGCGGGAGAAGCCTGGCCGGCGGACTGGGGACCG
AGCGGGAGGGGCACACCAGACACCAAAGCGTGGGGCGCTGGCTCTGGGGGTTTGGAGGGGCCGGGGGGCGCGCGAAA
TCGGTAACCGGGGCGACCGTGTCGGGGAGGGCAGGCGGCCGCCAACCCTGGGTGGTCGCGGAAGCCTGGGTGGCGCGC
GCCAGGGAGCGTGCCCGGCGGTGTCGGCGCGCGCGCGACCCGGACGAAGAAGCGGTAGAAGCGCGGGAGGAGGCGGGG
GGGCGGGGGGCGGTGGCATCGGGGGGCGCCGGGGAACTTTGGGGGGACGGCAAGCGCCGGAAGTCGTCGCGGGGGCCC
ACGGGCGCCGGCCGCGTGCTTTCGGCCGGGACGCCCGGTCGTGCTTCGCGAGCCGTGACTGCCGGCCCAGGGGGCCGC
GGTGCACACTGGGACGTGGGGACGGACTGATCGGCGGTGGGCGAAAGGGGGTCCGGGGCAAGGAGGGGCGCGGGGCCG
CCGGAGTCGTCAGACGCGAGCTCCTCCAGGCCGTGAATCCATGCCCACATGCGAGGGGGGACGGCTCGCCGGGGGTG
GCGTCGGTGAATAGCGTGGGGGCCAGGCTTCCGGGCCCCAACGAGCCCTCCGCCCCAACAAGGTCCGCCGGGCCGGGG
GTCGGGTTCGGGACCGAGGGGCTCTGGTCGTCGGGGGCGCGCTGGTACACCGGATGCCCCGGGAATAGCTCCCCCGAC
AGGAGGGAGGCGTCGAACGGCCGCCCGAGGATAGCTCGCGCGAGGAAGGGGTCCTCGTCGGTGGCGCTCGCGGCGAGG
ACGTCCTCGCCGCCCACCACAAACGGGAGCTCCTCGGTGGCCTCGCTGCCAACAAACCGCACGTCGGGGGGGCCGGGG
 
GGGTCCGGGTTTTCCCACAACACCGCGACCGGGGTCATGGAGATGTCCACGAGCACCAGGCACGGCGGGCCCCGGGCG
AGGGGCCGCTCGGCGATGAGCGCGGACAGGCGCGGGAGCTGTGCCGCCAGACACGCGTTTTCGATCGGGTTAAGGTCG
GCGTGCAGGAGGCGGACGGCCCACGTCTCGATGTCGGACGACACGGCATCGCGCAAGGCGGCGTCCGGCCCGCGAGCG
CGTGAGTCAAACAGCGTGAGGCACAGCTCCAGTTCCGACTCGCGGGAAAAGGCCGTGGTGTTGCGGAGCGCCACGACG
ACGGGCGCGCCCAGGAGCACTGCCGCCAGCACCAGGTCCATGGCCGTAACGCGCGCCGCGGGGGTGCGGTGGGTGGCG
GCGGCCGGCACGGCGACGTGCTGGCCCGTGGGCCGGTAGAGGGCGTTGGGGGGAGCGGGGGGTGACGCCTCGCGCCCC
CCCGAGGGGCTCAGCGTCTGCCCAGATTCCAGACGCGCGGTCAGAAGGGCGTCGAAACTGTCATACTCTGTGTAGTCG
TCCGGAAACATGCAGGTCCAAAGAGCGGCCAGCGCGGTGCTTGGGAGACACATGCGCCCGAGGACGCTCACCGCCGCC
AGCGCCTGGGCGGGACTCAGCTTTCCCAGCGCGGCGCCGCGCTCGGTTCCCAGCTCGGGGACCGAGCGCCAGGGCGCC
AGGGGGTCGGTTTCGGACAACTTGCCGCGGCGCCAGTCTGCCAGCCGCGTGCCGAACATGAGGCCCCGGGTCGGAGGG
CCTCCGGCCGAAAACGCTGGCAGCACGCGGATGCGGGCGTCTGGATGCGGGGTCAGGCGCTGCACGAATAGCATGGAA
TCTGCTGCGTTCTGAAACGCACGGGGGAGGGTGAGATGCATGTACTCGTGTTGGCGGACCAGATCCAGGCGCCAAAAG
GTGTAAATGTGTTCCGGGGAGCTGGCCACCAGCGCCACCAGCACGTCGTTCTCGTTAAAGGAAACGCGGTGCCTAGTG
GAGCTCTGGGGTCCGAGCGGCGGCCCCGGGGCCGCCGCGTCACCCCCCATTCCAGCTGGGCCCAGCGACACCCAAAC
TCGCGCGTGAGAGTGGTCGCGACGAGGGCGACGTAGAGCTCGGCCGCCGCATCCATCGAGGCCCCCCATCTCGCCTGG
CGGTGGCGCACAAAGCGTCCGAAGAGCTGAAAGTTGGCGGCCTGGGCGTCGCTGAGGGCCAGCTGAAACCGGTTGATG
ACGGTGAGGACGTACATGGCCGTGACGGTCGAGGCCGACTCCAGGGTGTCCGTCGGAAGCGGGGGGCGAATGCATGCC
GCCTCGGGACACATCAGCAGCGCGCCGAGCTTGTCGGTCACGGCCGGGAAGCAGAGCGCGTACTGCAGTGGCGTTCCA
TCCGGGACCAAAAAGCTGGGGGCGAACGGCCTATCCAGCGTACTGGTGGCCTCGCGCAGCACCAGGGGCCCCGGGCCT
CCGCTCACTCGCAGGTACGCCTCGCCCCGGCGGCGCAGCATCTGCGGGTCGGCCTCTTGGCCGGGTGGGGCGGACGCC
CGGGCGCGGGCGTCTAGGGCGCGAAGATCCACGAGCAGGGGCGCGGGCGCGGCCGCCGCGCCCGCGCCCGTCTGGCCT
GTGGCCTTGGCGTACGCGCTATATAAGCCCATGCGGCGTTGGATGAGCTCCCGCGCGCCCCGGAACTCCTCCACCGCC
CATGGGGCCAGGTCCCCGGCCACCGCGTCCAATTCCGCCAACAGGCCCCCCAGGGTGTCAAAGTTCATCTCCCAGGCC
ACCCTTGGCACCACCTCGTCCCGCAGCCGGGCGCTCAGGTCGGCGTGTTGGGCCACGCGCCCCCCGAGCTCCTCCACG
GCCCCGGCCCGCTCGGCGCTCTTGGCGCCCAGGACGCCCTGGTACTTGGCGGGAAGGCGCTCGTAGTCCCGCTGGGCT
CGCAGCCCCGACACAGTGTTGGTGGTGTCCTGCAGGGCGCGAAGCTGCTCGCATGCCGCGCGAAATCCCTCGGGCGAT
TTCCAGGCCCCCCCGCGAACGCGGCCGAAGCGACCCCATACCTCGTCCCACTCCGCCTCGGCCTCCTCGAAAGACCTC
CGCAGGGCCTCGACGCGGCGACGGGTGTCGAAGAGCGACTGCAGGCGCGCGCCCTGTCGCGTCAGGAGGCCCGGGCCG
TCGCCGCTGGCCGCGCTTAGCGGGTGCGTCTCAAAGGTGCGCTGGGCATGTTCCAACCAGGCGACCGCCTGCACGTCG
AGCTCGCGCGCCTTCTCCGTCTGGTCCAACAGAATCTCGACCTGATCCGCGATCTCCTCCGCCGAGCGCGCCTGGTCC
AGCGTCTTGGCCACGGTCGCCGGGACGGCAACCACCTTCAGCAGGGTCTTCAGATTGGCCAGACCCTCGGCCTCGAGC
TGGGCCCGGCGCTCGCGCGCGGCCAGCACCTCCCGCAACCCCGCCGTGACCCGCTCGGTGGCTTCGGCGCGCTGCTGT
TTGGCGCGCACCACGGCGTCCTTGGTATCGGCCAGGTCCTGTCGGGTCACGAATGCGACGTAGTCGGCGTACGCCGTG
TCCTTCACGGGGCTCTGGTCCACGCGCTCCAGCGCCGCCACACACGCCACCAGCGCGTCCTCGCTCGGGCAGGGCAGG
GTGACCCCTGCCCGGACAAGCTCGGCGGCCGCCGCCGGGTCGTTGCGCACCGCGGATATCTCCTCCGCGGCGGCGGCC
AGGTCCAGCGCCACGCTTCCGATCGCGCGCCGCGCGTCGGCCCGGAGGGCGTCCAGGCGATCGCGGATATCCACGTAC
TCGGCGTAGCCCTTTTGAAAAAACGGCACGTACTGGCGCAGGGCCGGCACGCCCCCCAAGTCTTCCGACAGGTGTAGG
ACGGCCTCGTGGTAGTCGATAAACCCGTCGTTCACCTGGGCCCGCTCCAGCAGCCCCCCCGCGAGCCGCAGAAGCCGC
 
GCCAGGGGCTCGGTGTCCACCCGAAACATGTCGGCGTACGTGTCGGCCGCGGCCCCGAAGGCCGCGCTCCAGTCGATG
CGGTGAATGGCTGCGAGCGGGGGAGCATGGGGTGGCGCTGGTTCTCGGGGGTGTATGGGTTAAACGCAAGGCCGTC
TCCAGGGCAAGGGTCACCGCCTTGGCGTTGGTTCCCAGCGCCTGCTCGGCCCGCTTTCGGAAGTCCCGGGGGTTGTAG
CCGTGCGTGCCCGCCAGCGCCTGCAGGCGACGGAGCTCGACCACGTCAAACTCGGCACCGCTTTCCACGCGGTCCAGC
ACGGCCTCCACGTCGGCGGCCCAGCGCTCGTGGCTACTGCGGGCGCGCTGGGCCGCCATCTTCTCTCTGAGGTCGGCG
GTGGCGGCCTCAAGTTCGTCGGCGCGGCGTCGCGTGGCGCCGATGACCTTTCCCAGCTCCTGCAGGGCGCGCCCGCTG
GGGGAGTGGTCCCCGGCCGTCCCTTCGGCGTGCAACAGGCCCCCGAACCTGCCCTCGTGGCCCGCGAGGCTTTCCCGC
GCGCCGGTGGTCGCGCGCGTCGCGGCCTGGATCAGGGAGGCATGCTCTCCCTCCGGTTGGTTGGCGGCCCGGCGCACC
TGGACGACAAGGTCGGCGGCAGCCGACCCTAAGGTCGTGAGCTGGGCGATGGCCCCCCGCGCGTCCAGGGCCAACCGA
GTCGCCTTGACGTATCCCGCGGCGCTGTCGGCCATGGCCGCTAGGAAGGCCAGGGGGGAGGCCGGGTCGCTGGCGGCC
GCGCCCAGGGCCGTCACTGCGTCGACCAGGACGCGGTGCGCCCGCACGGCCGCATCCACCGTCGACGCGGGGTCTGCC
GTCGCGACGGCGGCGCTGCCGGCGTTGATGGCGTTCGAGACGGCGTGGGCTATGATCGGGGCGTGATCGGCGAAGAAC
TGCAAGAGAAACGGAGTCTCGGGGGCGTTGGCGAACAGGTTCTTCAGCACCACCACGAAGCTGGGATGCAAGCCGGAC
AGAGCCGTCGCCGTGTCCGGAGTCGGGTGCTCCAGGGCATCTCGGTACTGCCCCAGCAGCCCCCACATGTCCGCCCGC
AGCGCCGCCGTAACCTCCGGGGGCGCCCCCCGAACGGCCTCGGGGAGGTCCGACCAGCCCGCCGGCAGGGAGGCCCGC
AGGGTCGTCAGGACGGCCGGACAGGCCTTTAGCCCACAAAGTCAGGGAGGGGCCGCAGGACCCCCTGGAGTTTGTGC
AAGAACTTCTCCCGGGCGTCGCGGGCCACCTTCGCCCGCTCCCGCGCTCCCTCGAGCATTGCCTCCAGGGAGCGCGCG
CGCTCCCGCAAACGGGCACGCGCATCGGGGGCGAGCTCTGCCGTCAGCTTGGCGGCATCCATGGCCCGCGCCTGCCGC
AGCGCTTCCTCGGCCATGCGCGTGGCCTCTGGCGACAGCCCGCCGTCGTCGGGGTAGGGCGACGCGCCGGGCGCAGGA
ACAAAGGCCGCGTCGCTGTCCAGCTGCTGGCCCAGGGCCGCATCTAGGGCGTCGAAGCGCCGCAGCTCGGCCAGACCC
GAGCTGCGGCGCGCCTGCTGGTCGTTAATGTCGCGGATGCTGCGCGCCAGCTCGTCCAGCGGCTTGCGTTCTATCAGC
CCTTGGTTGGCGGCGTCCGTCAGGACGGAGAGCCAGGCCGCCAGGTCCTCGGGGGCGTCCAGCGTCTGGCCCCGCTGT
ATCAGATCCCGCAACAGGATGGCCGTGGGGCTGGTCGCGATCGGGGGCGGGGCGGGAATGGCGGCGCTCTGCGCGATG
TCCCGCGTGTGCTGGTCGAAGACAGGCAGGGACTCTAGCAGCTGGACCACGGGCACGACGGCGGCCGAAGCCACGTGA
AACCGGCGGTCGTTGTTGTCGCTGGCCTGCAGAGCCTTGGCGCTGTATACGGCCCCCCGGTAAAGTACTCCTTAACC
GCGCCCTCGATCGCCCGACGGGCCTGGGTCCGCACCTCCTCCAGCCGAACCTGAACGGCCTCGGGGCCCAGGGGGGGT
GGGCGCGGAGCCCCCTGCGGGGCCGCCCCGGCCGGGGCGGGCATTACGCCGAGGGGCCCGGCGTGCTGTGAGACCGCG
TCGACCCCGCGAGCGAGGGCGTCGAGGGCCTCGCGCATCTGGCGATCCTCCGCCTCCACCCTAATCTCTTCGCCACGG
GCAAATTTGGCCAGAGCCTGGACTCTATACAGAAGCGGTTCTGGGTGCGTCGGGGTGGCGGGGGCAAAAAGGGTGTCC
GGGTGGGCCTGCGAGCGCTCCAGAAGCCACTCGCCGAGGCGTGTATACAGATTGGCCGGCGGGGCCGCGCGAAGCTGC
AGCTCCAGGTCCGCGAGTTCCCCGTAAAGGCGTCCGTCTCCCGAATGACATCCCTAGCCACAAGGATCAGCTTCGCC
AGCGCCAGGCGACCGATCAGAGAGTTTTCGTCCAGCACGTGCTGGACGAGGGGCAGATGGGCGGCCACGTCGGCCAGG
CTCAGGCGCGTGGAGGCCAGAAAGTCCCCCACGGCCGTTTTCCGGGGCAGCATGCTCAGGGTAAACTCCAGCAGGGCG
GCGGCCGGGCCGGCCACCCCGGCCTGGGTGTGCGTCCGGGCCCCGTTCTCGATGAGAAAGGCGAGGACGCGTTCAAAG
AAAAAAATAACACAGAGCTCCAGCAGCCCCGGAGAAGCCGGATACGGCGACCGTAAGGCGCTGATGGTGAGCCGCGAA
CACGCGGCGACCTCGCGGGCCAGGGCGGCGGAGCACGCGGTGAACTTAACCGCCGTGGCGGCCACGTTTGGGTGGGCC
TCGAACAGCTGGGCAAGGTCTGCGCCCGGGGGCTCGGGTGAGCGGCGAGTCTTCAGCGCCTCGAGGGCCTGCGAGGAC
GCCGGAACCGTGGGCCCGTCGTCCTCGCCCGCCTCGGCGACCGGCGGCCCGGCCGGGTCGGGGGGTGCCGAGGCGAGG
 
ACAGGCTCCGGAACGGAGGCGGGACCGCGGCCCCGACGGGGTTTTGCCTTTGGGGTGGATTTCTTCTTGGTTTTG
GCAGGGGGGGCCGAGCGTTTCGTTTTCTCCCCCGAAGTCAGGTCTTCGACGCTGGAAGGCGGAGTCCAGGTGGGTCGG
CGGCGCTTGGGAAGGCCGGCCGAGTAGCGTGCCCGGTGCCGACCAACCGGGACGACGCCCATCTCCAGGACCCGCATG
TCGTCGTCATCTTCTTCGGCCGCCTCTGCGGCGGGGGGCTTGGGGGCGGAGGGAGGCGGTGGTGGGATCGCGGAGGGT
GGGTCGGCGGAGGGTGGGTCGGCGGAGGGGGGATCCGTGGGTGGGGTACCCTTCAGGGCCACCGCCCATACATCGTCG
GGCGCCCGATTCGGGCGCTTGGCCTCTGGTTTTGCCGACGGACCGGCCGTCCCCCGGGATGTCTCGGAGGCCCTGTCG
TCGCGACGGGCCCGGGTCGGTGGCGGCGACTGGGCGGCTGTGGGCGGGTGGGGCCCCGTGCCCCCTACCCCCTCCCGG
GGGCCCACGCCGACGCAGGGCTCCCCCAGGCCCGCGATCTCGCCCCGCAGGGGGTGCGTGATGGCCACGCGCCGTTCG
CTGAACGCTTCGTCCTGCAGGTAAGTCTCGCTGGCCCCGTAAAGATGCAGAGCCGCGGCCGTCAAGTCCGCAGGAGCC
GCGGGTTCCGGGCCCGACGGCACGAAAAACACCATGGCTCCCGCCCACCGTACGTCCGGGCGATCGCGGGTGTAATAC
GTCAGGTATGGATACATGTCCCCCGCCCGCACTTTGGCGATGAACGCGGGGGTGCCCTCCGGAAGGCCGTGCGGGTCA
AAAAGGTATGCGGTGTCGCCGTCCCTGAACAACCCCATCCCTAGGGGGCCAATGGTTAGGAGCGTGTACGACAGGGGG
CGCAGGGCCCACGGGCCGGCGAAGAACGTGTGTGCGGGGCATTGTGTCTCCAGCAGGCCCGCCGCGGGCTCCCCGAAG
AAGCCCACCTCGCCGTATACGCGCGAGAAGACACAGCGCAGTCCGCCGCGCGCCCCTGGGTACTCGAGGAAGTTGGGG
AGCTCGACGATCGAACACATGCGCGGCGGCCCAGGGCCCGCGGTCGCGCGCGTCCACTCGCCCCCCTCGACCAAACAA
CCCTCGATGGCCTCCGCGGACAGAACGTCGCGAGGGCCCACATCAAATATGAGGCTGAGAAAGGACAGCGACGAGCGC
ATGCACGATACCGACCCCCCCGGCTCCAGGTCGGGCGCGAACTGGTTCCGAGCACCGGTGACCACGATGTCGCGATCC
CCCCCGCGTTCCATCGTGGAGTGCGGTGGGGTGCCCGCGATCATATGTGCCCTACTGGCCAGAGACCCGGCCTGTTTA
TGGACCGGACCCCCGGGGTTAGTGTTGTTTCCGCCACCCATGCCCCCGTACCATGGCCCCGGTTCCCCTGATTAGGCT
ACGAGTCGCGGTGATCGCTTCCCAAAAACCGAGCTGCGTTTGTCTGTCTTGATCTTTCCCCCCCCCCCGCCCGCCCG
CACACCATAACACCGAGAACAACACACGGGGGTGGGCGTAACATAATAAAGCTTTATTGGTAACTAGTTAACGGCAAG
TCCGTGGGTGGCGCGACGGTGTCCTCCGGGCTCATCTCGTCGTCCTCGACGGGGGTGTTGGAATGAGGCGCCCCCTCG
CGGTCCGCCTGGCGTGGGCCGTGCCCATAGGCCTCCGGCTTCTGTGCGTCCATGGGCATAGGCGCGGGGAGACTGTTT
CCGGCGTCGCGGACCTCCAGGTCCCTGGGAGACTCCGGTCCGGCTAACGGACGAAACGCGGAAGCGCGAAACACGCCG
TCGGTGACCCGCAGGAGCTCGTTCATCAGTAACCAATCCATACTCAGCGTAACGGCCAGCCCCTGGCGAGACAGATCC
ACGGAATCCGGAACCGCGGTCGTCTGGCCCAGGGGGCCGAGGCTGTAGTCCCCCCAGGCCCCTAGGTCGCGACGGCTC
GTAAGCACGACGCGGTCGGCCGCGGGGCTTTGCGGGGGGGCGTCCTCGGGCGCATGCGCCATTACCTCTCGGATGGCC
GCGGCGCGCTGGTCGGCCGAGCTGACCAAGGGCGCCACGACCACGGCGCGCTCCGTCTGCAGGCCCTTCCACGTGTCG
TGGAGTTCCTGGACAAACTCGGCCACGGGCTCGGGTCCCGCGGCCGCGCGCGCGGCTTGATAGCAGGCCGAGAGACGC
CGCCAGCGCGCTAGAAACTGACCCATGAAGCAAAACCCGGGGACCTGGTCTCCCGACAGCAGCTTCGACGCCCGGGCG
TGAATGCCGGACACGACGGACAGAAACCCGTGAATTTCGCGCCGGACCACGGCCAGCACGTTGTCCTCGTGCGACACC
TGGGCCGCCAGCTCGTCGCACACCCCCAGGTGCGCCGTGGTTTCGGTGATGACGGAACGCAGGCTCGCGAGGGACGCG
ACCAGCGCGCGCTTGGCGTCGTGATACATGCTGCAGTACTGACTCACCGCGTCCCCCATGGCCTCGGGGGGCCAGGGC
CCCAGGCGGTCGGGCGTGTCCCCGACCACCGCATACAGGCGGCGCCCGTCGCTCTCGAACCGACACTCGAAAAAGGCG
GAGAGCGTGCGCATGTGCAGCCGCAGCAGCACGATGGCGTCCTCCAGTTGGCGAATCAGGGGGTCTGCGCGCTCGGCG
AGGTCCTGCAGCACCCCCCGGGCGGCCAGGGCGTACATGCTAATCAACAGGAGGCTGGTGCCCACCTCGGGGGGCGGG
GGGGGCTGCAGCTGGACCAGGGGCCGCAGCTGCTCGACGGCACCCCTGGAGATCACGTACAGCTCCCGGAGCAGCTGC
TCTATGTTGTCGGCCATCTGCATAGTGGGGCCGAGGCCGCCCCGGGCGGCCGGTTCGAGGAGGGTAATCAGCGCGCCC
 
AGTTTGGTGCGATGGCCCTCGACCGTGGGAGATAGCCCAGCCCAAAGTCCCGGGCCCAGGCCAACACACGCAGGGCG
AACTCGACCGGGCGTGGAAGGTAGGCCGCGCTACACGTGGCCCTCAACGCGTCCCCGACCACCAGGGCCAGAACGTAG
GGGACGAAGCCCGGGTCGGCGAGGACGTTGGGGTGAATGCCCTCGAGGGCGGGGAAGCGGATCTGGGTCGCCGCGGCC
AGGTGGACAGAGGGGGCGTGGCTGGGCTGCCCGACGGGGAGAAGCGCGGACAGCGGCGTGGCCGGGGTGGTGGGGGTG
ATGTCCCAGTGGGTCTGACCATACACGTCGATCCAGATGAGCGCCGTCTCGCGGAGAAGGCTGGGTTGACCGGAACTA
AAGCGGCGCTCGGCCGTCTCAAACTCCCCCACGAGCGCCCGCCGCAGGCTCGCCAGATGTTCCGTCGGCACGGCCGGA
CCCATGATACGCGCCAGCGTCTGGCTCAGAACGCCCCCCGACAGGCCGACCGCCTCGCAGAGCCGCCCGTGCGTGTGC
TCGCTGGCGCCCTGGACCCGCCTGAAAGTTTTACGTAGTTGGCATAGTACCCGTATTCCCGCGCCAGACCAAACACG
TTCGACCCCGCGAGGGCAATGCACCCAAAGAGCTGCTGGACTTCGCCGAGTCCGTGGCCGGCGGGCGTCCGCGCGGGG
ACGCCCGCCGCCAGAAACCCCTCCAGGGCCGAAAGGTAGTGCGTGCAGTGCGAGGGCGTGAACCCAGCGTCGATCAGG
GTGTTGATCACCACGGAGGGCGAATTGGTATTCTGGATCAACGTCCACGTCTGCTGCAGCAGAGCCAACAGCCGCTGC
TGGGCGCCGGCGGAGGGCTGCTCCCCGAGCTGCAGCAGGCTGGAGACGGCAGGCTGGAAGACTGCCAGTGCCGACGAA
CTCAGGAACGGCACGTCGGGATCAAACACGGCCACGTCCGTCCGCACGCGCGCCATTAGCGTCCCCGGGGGCGCACAG
GCCGAGCGCGGGCTGACGCGGCTGAGGGCCGTCGACACGCGCACCTCCTCGCGGCTGCGAACCATCTTGTTGGCCTCC
AGTGGCGGAATCATTATGGCCGGGTCGATCTCCCGCACGGTGTGCTGAAACTGCGCCAACAGGGGCGGCGGGACCACA
GCCCCCCGCTCGGGGGTCGTCAGGTACTCGTCCACCAGGGCCAACGTAAAGAGGGCCCGTGTGAGGGGAGTGAGGGTC
GCGTCGTCTATGCGCTGGAGGTGCGCCGAGAACAGCGTCACCCGATTACTCACCAGGGCCAAGAACCGGAGGCCCTCT
TGCACGAACGGGGCGGGGAAGAGCAGGCTGTACGCCGGGGTGGTAAGGTTCGCGCTGGGCTGCCCCAACGGGACCGGC
GCCATCTTGAGCGACGTCTCCCCAAGGGCCTCGATGGAGGTCCGCGGGCTCATGGCCAAGCAGCTCTTGGTGACGGTT
TGCCAGCGGTCTATCCACTCCACGGCGCACTGGCGGACGCGGACCGGCCCCAGGGCCGCCGCGGTCGCAGGCCGGCGG
AATCCAGCGCATGGGACGTGTCGGAGCCGGTGACCGCGAGGATGGTGTCCTTGATGACCTCCATCTCCCGGAAGGCCT
GGTCGGGGGCCTCGGGGAGAGCCACCACCAAGCGGTGTACGAGCAACCCGGGGAGGTTCTCGGCCAAGAGCGCCGTCT
CCGGAAGCCCGTGGGCCCGGTGGAACGCGCACAGGTGTTCCAGCAGCGGCCGCCAGCATGCCCGCGCGTCTGCCGGGG
CGATGGCCGTTCCCGACAACAGAAACGCCGCCATGGCGGCGCGCAGCTTGGCCGTGGCCAGAAACGCCGGGTCGTCCG
CCCCGTTTGCCGTCTCGGCCGTGGGGGTTGGCGGTTGGCGAAGGCCGGCTAGGCTCGCCAATAGGCGCTGCATAGGTC
CGTCCGAGGGCGGACCGGCGGGTGAGGTCGTGACGACGGGGGCCTCGGACGGAGACCGCGGTCTGCCATGACGCCCG
GCTCGCGTGGGTGGGGGACAGCGTAGACCAACGACGAGACCGGGCGGGAATGACTGTCGTGCGCTGTAGGGAGCGGCG
AATTATCGATCCCCTGCGGCCCTCCAGGAACCCCGCAGGCGTTGCGAGTACCCCGCGTCTTCGCGGGGTGTTATACGG
CCACTTAAGTCCCGGCATCCCGTTCGCGGACCCAGGCCCGGGGGATTGTCCGGATGTGCGGGCAGCCCGGACGGCGTG
GGTTGCGGACTTTCTGCGGGGCGGCCCAAATGGCCCTTTAAACGTGTGTATACGGACGCGCCGGGCCAGTCGGCCAAC
ACAACCCACCGGAGGCGGTAGCCGCGTTTGGCTGTGGGGTGGGTGGTTCCGCCTTGTGTGAGTGTCCTTTCGACCCCC
CCCCCCCCCCTCCCCGGGTCTTGCTAGGTCGCGATCTGTGGTCGCAATGAAGACCAATCCGCTACCCGCAACCCCTT
CCGTGTGGGGCGGGAGTACCGTGGAACTCCCCCCCACCACACGCGATACCGCGGGGCAGGGCCTGCTTCGGCGCGTCC
TGCGCCCCCCGATCTCTCGCCGCGACGGCCCAGTGCTCCCCAGGGGGTCGGGACCCCGGAGGCGGCCAGCACGCTGT
GGTTGCTTGGCCTGGACGGCACAGACGCGCCCCCTGGGGCGCTGACCCCCAACGACGATACCGAACAGGCCCTGGACA
AGATCCTGCGGGGCACCATGCGCGGGGGGGCGGCCCTGATCGGCTCCCCGCGCCATCATCTAACCCGCCAAGTGATCC
TGACGGATCTGTGCCAACCCAACGCGGATCGTGCCGGGACGCTGCTTCTGGCGCTGCGGCACCCCGCCGACCTGCCTC
ACCTGGCCCACCAGCGCGCCCCGCCAGGCCGGCAGACCGAGCGGCTGGGCGAGGCCTGGGGCCAGCTGATGGAGGCGA
 
CCGCCCTGGGGTCGGGGCGAGCCGAGAGCGGGTGCACGCGCGGGCCTCGTGTCGTTTAACTTCCTGGTGGCGGCGT
GTGCCGCCTCGTACGACGCGCGCGACGCCGCCGATGCGGTACGGGCCCACGTCACGGCCAACTACCGCGGACGCGGG
TGGGGGCGCGCCTGGATCGTTTTTCCGAGTGTCTGCGCGCCATGGTTCACACGCACGTCTTCCCCCACGAGGTCATGC
GGTTTTTCGGGGGGCTGGTGTCGTGGGTCACCCAGGACGAGCTAGCGAGCGTCACCGCCGTGTGCGCCGGGCCCCAGG
AGGCGGCGCACACCGGCCACCCGGGCCGGCCCCGCTCGGCCGTGATCCTCCCGGCGTGTGCGTTCGTGGACCTGGACG
CCGAGCTGGGGCTGGGGGGCCCGGGCGCGGCGTTTCTGTACCTGGTATTCACTTACCGCCAGCGCCGGGACCAGGAGC
TGTGTTGTGTGTACGTGATCAAGAGCCAGCTCCCCCGCGCGGGTTGGAGCCGGCCCTGGAGCGGCTGTTTGGGCGCC
TCCGGATCACCAACACGATTCACGGCACCGAGGACATGACGCCCCCGGCCCCAAACCGAAACCCCGACTTCCCCCTCG
CGGGCCTGGCCGCCAATCCCAAACCCCGCGTTGCTCGGCTGGCCAGGTCACGAACCCCCAGTTCGCCGACAGGCTGT
ACCGCTGGCAGCCGGACCTTCGGGGGCGCCCCACCGCACGCACCTGTACGTACGCCGCCTTTGCAGAGCTCGGCATGA
TGCCCGAGGATAGTCCCCGCTGCCTGCACCGCACCGAGCGCTTTGGGGCGGTCAGCGTCCCCGTTGTTATTCTGGAAG
GCGTGGTGTGGCGCCCCGGCGAGTGGCGGGCATGCGCGTGAGCGTAGCAAACGCCCCGCCCACACAACGCTCCGCCCC
CAACCCCTTCCCCGCTGTCACTCGTGGTTCGTTGACCCGGACGTCCGCCAAATAAAGCCACTGAAACCCGAAACGCGA
GTGTTGTAACGTCCTTTGGGCGGGAGGAAGCCCGTATAGCATACATTATACGAAGTTATAGCGAAGTTCCTATTCT
CTAGAAAGTATAGGAACTTCGAATTGGTCGACGGATCCAACCGCGGAAGACCCAGGCCGCCTCGGGTGTAACGTTAGA
CCGAGTTCGCCGGGCCGGCTCCGCGGGCCAGGGCCCGGGCACGGGCCTCGGGCCCCAGGCACGGCCCGATGACCGCCT
CGGCCTCCGCCACCCGGCGCCGGAACCGAGCCCGGTCGGCCCGCTCGCGGGCCCACGAGCCGCGGCGCGCCAGGCGGG
CGGCCGAGGCCCAGACCACCAGGTGGCGCACCCGGACGTGGGGCGAGAAGCGCACCCGCGTGGGGGTCGCGGGGGTCG
CGGGGGTCGCGGGGGGCTTCGGCGCCCCCTCCCCGCCCGCGCGTCGCAGGCGCAGGCGCGCCAGGTGCTCTGCGGTGA
CGCGCAGGCGGAGGGCGAGGCGCGGCGGAAGGCGGAAGGGGCGTGAGGGGGGGTGGAGGGGTTAGCCCCGCCCCCCG
GGCCCGCGCCGGGCGGTGGGGGCCGGGGCCGGGGGGCGGCGGCGGTGGGCCGGGCCTCTGGCGCCGGCTCGGGCGGGG
GGCTGTCCGGCCAGTCGTCGTCATCGTCGTCGTCGGACGCGGACTCGGGAACGTGGAGCCACTGGCGCAGCAGCAGCG
AACAAGAAGGCGGGGGCCCACTGGCGGGGGGCGGCGGCGGGGCGGCCGCGGGCGCGCTCCTGACCACGGGTTCCGAGT
TGGGCGTGGAGGTTACCTGGGACTGTGCGGTTGGGACGGCGCCCGTGGGCCCGGGCGGCCGGGGGCGGCGGGGGCCGC
GATGGCGGCGGCGGCGGGCCATGATCAAGCTCATGGCGCCGCGCTCTGCTTCTGGAAGGCTGCGCTCCGCGGCGTGGA
TGCTCCGGGGAAAGTTGCGCTCCGCGGCAGGGATGCTCCTGGGAAGGTTGCGCTCCGCGGCAGGGATGCTCTGGGGAA
GGCTGGTCCTGGCCGAGGATCGGGAACGCGCCGCTCGCTCTGCTTCTCTTGTCTTCGCTTGTCTCTGGATGGAACCAG
ATTTGGTTCTGAGTAGCTGTCAGCGTCTGGTGACCTGCTCGCCGCCCTGCGCCTTTAAGGAGTCTTCACCGGCCCCGC
CCACTCTCCGCTGGGCCAATCAGCGAGCCGGAGGAGGCCTTGGGGCCAGGAATCTTCCAGCAGTTTCGCGTCTGGTGG
AGCTTCCCCGCCTCCCTTGAGTAATCGGAGTTGTGGGTTCCGCCCTTGTCCAGAACTCTCCAGAGGTTTCTGGGGTTC
ACTGGAGAGTACGGATTCCTGAGGGGGAGGGTGTGGGGAAGTGCTGGTGCTACTAGTGACACTGTTGCTATGGCGACG
CATTACTAAGGCCTGTGTGGAATGGACAAGAAAGATCACCTCTAGCTCGGTGTTGTGTACAGTTTGTTGTGATTTGTG
GGGTTTCGCCAACTCGCACAGTTCTGAATATGGGGGTTAAAGGCTAAAACTTAAGGGCTAAAACTTCTCCCCGCCAAG
TTTAGGAGACCCAGGGAGATGCCTGGGGGCGTGTCCGGTGACGTGATCCTCTCCAATCGCGTTACAATGGCAGTGCTG
CCTCTGACCTCATGGACTAATTTAGGAACTAGAGGCTCTGTCCCAGCACAGGCTCAAAGTTGCCGGGAGGGGCGGGGT
GGGGGGTGGGGGGGACCCCGGCTGCTCAGTTTGGATGTTCCTGGAGCTCGGTACCCGCGATCGCCCCTAGAGGATCTA
CTAGTCATATGGATAAGCCTGGGAACCTCGTCCAGGTGTCTGCAACCGAGAGTTCTCAGCCTCCAGCAGAGTCCTGGT
GGGGAGTGGGGAGATAGGGTCAGCTCCAGCTGAGGTAGCATGTCCTGCCACTGCAGGATCAATCTCTATTGTGACCAT
 
TGTCATATAAAAGCCACACAGTCATATACCCACAGATATATACTTAGCCAACCCATATTTGAGACACAGGGAGACCCC
ACATGCAGATTCCCACAGTCGGAGGCAGGGCCAAATGAATTGCTAACACTTATATCAGACTCCTCAGATCAGTCTCCG
CCTCCCCACCCAAGGCCAAGGCCGATGACCTCATCCTCTGGGAGGGAGGCCGATTCTCATGCTAATTATTGCCTTTTG
TCCACACTACCATCTGGAGGGCCTAAGAAGGGAGGGCTCCTCAGGGGAAGTGGAATTCTCAGGCTGTTCCCAGGGGA
TGGCTCTCTCTCTGCCCCCAGAGCTGGTAACAGACAAAAGCAAATGAATTCAGCTCCCCTTCTCCAAATCCTTTTCAG
ACCTCAAACGCCAGTGGTTACATTCCTCAGAGCTGCCTGGACCCTTCCCCTCAGAGGACTGACTGGGGCTAAAGCCCT
CATCTCAGGATCACAAACTCTTCAGGGATCGGATCTCGGCCCGGGCTAGCACGCGTAAGAGCTCGGTACCTATCGATA
GAGAAATGTTCTGGCACCTGCACTTGCACTGGGGACAGCCTATTTTGCTAGTTTGTTTTGTTTCGTTTTGTTTTGATG
GAGAGCGTATGTTAGTACTATCGATTCACACAAAAAACCAACACACAGATGTAATGAAAATAAAGATATTTTATTGCG
GCGATCCGGAACCCTTAAT (SEQ ID NO: 1)
**SEQ ID NO: 2 Nestin Promoter**

aaccctgaag agtttgtgat cctgagatga gggctttagc cccagtcagt cctctgaggg 60
gaagggtcca ggcagctctg aggaatgtaa ccactggcgt ttgaggtctg aaaaggattt 120
ggagaagggg agctgaattc atttgctttt gtctgttacc agctctgggg gcagagagag 180
agccatcccc tgggaacagc ctgagaattc ccacttcccc tgaggagccc tcccttctta 240
ggccctccag atggtagtgt ggacaaaagg caataattag catgagaatc ggcctccctc 300
ccagaggatg aggtcatcgg ccttggcctt gggtggggag gcggagactg atctgaggag 360
tctgatataa gtgttagcaa ttcatttggc cctgcctccg actgtgggaa tctgcatgtg 420
gggtctccct gtgtctcaaa tatgggttgg ctaagtatat atctgtgggt atatgactgt 480
gtggctttta tatgacaatg gtcacaatag agattgatcc tgcagtggca ggacatgcta 540
cctcagctgg agctgaccct atctccccac tccccaccag gactctgctg gaggctgaga 600
actctcggtt gcagacacct ggacgaggtt caggcttatc atatgactag tagatcctct 660
aggggcgatc gcgggtaccg agctccagga acatccaaac tgagcagccg ggtccccccc 720
caccccccac cccgcccctc ccggcaactt tgagcctgtg ctgggacaga gcctctagtt 780
cctaaattag tccatgaggt cagaggcagc actgccattg taacgcgatt ggagaggatc 840
acgtcaccgg acacgccccc aggcatctcc ctgggtctcc taaacttggc ggggagaagt 900
tttagccctt aagttttagc ctttaacccc catattcaga actgtgcgag ttggcgaaac 960
cccacaaatc acaacaaact gtacacaaca ccgagctaga ggtgatcttt cttgtccatt 1020
ccacacaggc cttagtaatg cgtcgccata gcaacagtgt cactagtagc accagcactt 1080
ccccacaccc tccccctcag gaatccgtac tctccagtga accccagaaa cctctggaga 1140
gttctggaca agggcggaac ccacaactcc gattactcaa gggaggcggg gaagctccac 1200
cagacgcgaa actgctggaa gattcctggc cccaaggcct cctccggctc gctgattggc 1260
ccagcggaga gtgggcgggg ccggtgaaga ctccttaaag gcgcagggcg gcgagcaggt 1320
caccagacgc tgacagctac tcagaaccaa atctggttcc atccagagac aagcgaagac 1380
aagagaagca gagcgagcgg cgcgttcccg atcctcggcc aggaccagcc ttccccagag 1440
catccctgcc gcggagcgca accttcccag gagcatccct gccgcggagc gcaactttcc 1500
ccggagcatc cacgccgcgg agcgcagcct tccagaagca 1540 (SEQ ID NO: 2)

### UL39 Sequences ribonucleotide reductase subunit 1 [Human herpesvirus 1]

LOCUS **NP_044641** 1137 aa linear VRL 24-AUG-2010
DEFINITION ribonucleotide reductase subunit 1 [Human herpesvirus 1].
ACCESSION NP_044641
VERSION NP_044641.1 GI:9629420
DBLINK BioProject: PRJNA15217
DBSOURCE REFSEQ: accession NC_001806.1
KEYWORDS RefSeq.
SOURCE Human herpesvirus 1 (Herpes simplex virus 1)
   ORGANISM Human herpesvirus 1
   Viruses; dsDNA viruses, no RNA stage; Herpesvirales; Herpesviridae;
   Alphaherpesvirinae; Simplexvirus.
REFERENCE 1 (residues 1 to 1137)
   AUTHORS McGeoch,D.J., Dalrymple,M.A., Davison,A.J., Dolan,A., Frame,M.C., McNab,D., Perry,L.J., Scott,J.E. and Taylor,P.
   TITLE The complete DNA sequence of the long unique region in the genome of herpes simplex virus type 1
   JOURNAL J. Gen. Virol. 69 (PT 7), 1531-1574 (1988)
   PUBMED 2839594
REFERENCE 2 (residues 1 to 1137)
   CONSRTM NCBI Genome Project
   TITLE Direct Submission
   JOURNAL Submitted (01-AUG-2000) National Center for Biotechnology Information, NIH, Bethesda, MD 20894, USA
REFERENCE 3 (residues 1 to 1137)
   AUTHORS McGeoch,D.J.
   TITLE Direct Submission
   JOURNAL Submitted (17-JAN-1989) McGeoch D.J., MRC Virology Institute, Institute of Virology, Church Street, Glasgow G11 5JR, GB
COMMENT PROVISIONAL REFSEQ: This record has not yet been subject to final NCBI review. The reference sequence was derived from CAA32314.
   CURATION: The original gene nomenclature has been retained. Genes presumably inherited from the common ancestor of alpha-, beta- and gammaherpesviruses (core genes) and non-core genes presumably inherited from the ancestor of alphaherpesviruses (alpha genes) are indicated. Initiation codons are assigned with as much confidence as is possible for each protein-coding region. A standard protein nomenclature has been applied so that orthologs have the same name in all herpesviruses. Protein information may have been propagated from other herpesvirus species.
   Method: conceptual translation.
FEATURES Location/Qualifiers
   source 1..1137
      /organism="Human herpesvirus 1"
      /strain="17"
      /host="Homo sapiens"
      /db_xref="taxon:10298"
      /acronym="HHV-1"
      /acronym="HSV-1"
   Protein 1..1137
      /product="ribonucleotide reductase subunit 1"
      /EC_number="1.17.4.1"
      /function="nucleotide metabolism"
      /note="translation initiation factor-associated protein
      (N-terminalregion)"
      /calculated_mol_wt=123920
   Region 491..576
      /region_name="Ribonuc_red_lgN"
      /note="Ribonucleotide reductase, all-alpha domain;
      pfam00317"
      /db_xref="CDD:249765"
   Region 537..1113
      /region_name="RNR_PFL"
      /note="Ribonucleotide reductase and Pyruvate formate
      lyase; cl09939"
      /db_xref="CDD:245211"
   Region 580..1113
      /region_name="Ribonuc_red_lgC"
      /note="Ribonucleotide reductase, barrel domain; pfam02867"
      /db_xref="CDD:251578"
   CDS 1..1137
      /gene="UL39"
      /locus_tag="HHV1gp057"
      /coded_by="NC_0018061 :8644489857"
      /db_xref="GOA:P08543"
      /db_xref="InterPro:IPR000788"
      /db_xref="InterPro:IPR003010"
      /db_xref="UniProtKB/Swiss-Prot:P08543"
      /db_xref="GeneID:2703361"
ORIGIN

1 masrpaassp vearapvggq eaggpsaatq geaagaplah ghhvycqrvn gvmvlsdktp
61 gsasyrisdn nfvqcgsnct miidgdvvrg rpqdpgaaas papfvavtni gagsdggtav
121 vafggtprrs agtstgtqta dvptealggp pppprftlgg gccscrdtrr rsavfggegd
181 pvgpaefvsd drssdsdsdd sedtdsetls hassdvsgga tyddaldsds ssddslqidg
241 pvcrpwsndt apldvcpgtp gpgadaggps avdphaptpe agaglaadpa varddaegls
301 dprprlgtgt aypvpleltp enaeavarfl gdavnrepal mleyfcrcar eetkrvpprt
361 fgspprlted dfgllnyalv emqrlcldvp pvppnaympy ylreyvtrlv ngfkplvsrs
421 arlyrilgvl vhlrirtrea sfeewlrske valdfglter lreheaqlvi laqaldhydc
481 lihstphtlv erglqsalky eefylkrfgg hymesvfqmy triagflacr atrgmrhial
541 gregswwemf kfffhrlydh qivpstpaml nlgtrnyyts scylvnpqat tnkatlrait
601 snvsailarn ggiglcvqaf ndsgpgtasv mpalkvldsl vaahnkesar ptgacvylep
661 whtdvravlr mkgvlageea qrcdnifsal wmpdlffkrl irhldgeknv twtlfdrdts
721 msladfhgee feklyqhlev mgfgeqipiq elaygivrsa attgspfvmf kdavnrhyiy
781 dtqgaaiags nlcteivhpa skrssgvcnl gsvnlarcvs rqtfdfgrlr davqacvlmv
841 nimidstlqp tpqctrgndn lrsmgigmqg lhtaclklgl dlesaefqdl nkhiaevmll
901 samktsnalc vrgarpfnhf krsmyragrf hwcrfpdarp rycgcwcmlr qsmmkhglrn
961 sqfvalmpta asaqisdvse gfaplftnlf skvtrdgetl rpntlllkel ertfsgkrll
1021 evmdsldakq wsvaqalpcl epthplrrfk tafdydqkll idlcadrapy vdhsqsmtly
1081 vtekadgtlp astlvrllvh aykrglktgm yyckvrkatn sgvfggddni vcmscal (SEQ ID NO: 3)

**Nestin 2^{nd} intron sequence SEQ ID NO: 4** 
aaccctgaag agtttgtgat cctgagatga gggctttagc cccagtcagt cctctgaggg 60

gaagggtcca ggcagctctg aggaatgtaa ccactggcgt ttgaggtctg aaaaggattt 120

ggagaagggg agctgaattc atttgctttt gtctgttacc agctctgggg gcagagagag 180

agccatcccc tgggaacagc ctgagaattc ccacttcccc tgaggagccc tcccttctta 240

ggccctccag atggtagtgt ggacaaaagg caataattag catgagaatc ggcctccctc 300

ccagaggatg aggtcatcgg ccttggcctt gggtggggag gcggagactg atctgaggag 360

tctgatataa gtgttagcaa ttcatttggc cctgcctccg actgtgggaa tctgcatgtg 420

gggtctccct gtgtctcaaa tatgggttgg ctaagtatat atctgtgggt atatgactgt 480

gtggctttta tatgacaatg gtcacaatag agattgatcc tgcagtggca ggacatgcta 540

cctcagctgg agctgaccct atctccccac tccccaccag gactctgctg gaggctgaga 600

actctcggtt gcagacacct ggacgaggtt 630 (SEQ ID NO: 4)
**hsp68: heat shock protein 68 SEQ ID NO: 5** 
caggcttatc atatgactag tagatcctct aggggcgatc gcgggtaccg agctccagga 60

acatccaaac tgagcagccg gggtcccccc caccccccac cccgcccctc ccggcaactt 120

tgagcctgtg ctgggacaga gcctctagtt cctaaattag tccatgaggt cagaggcagc 180

actgccattg taacgcgatt ggagaggatc acgtcaccgg acacgccccc aggcatctcc 240

ctgggtctcc taaacttggc ggggagaagt tttagccctt aagttttagc ctttaacccc 300

catattcaga actgtgcgag ttggcgaaac cccacaaatc acaacaaact gtacacaaca 360

ccgagctaga ggtgatcttt cttgtccatt ccacacaggc cttagtaatg cgtcgccata 420

gcaacagtgt cactagtagc accagcactt ccccacaccc tccccctcag gaatccgtac 480

tctccagtga accccagaaa cctctggaga gttctggaca agggcggaac ccacaactcc 540

gattactcaa gggaggcggg gaagctccac cagacgcgaa actgctggaa gattcctggc 600

cccaaggcct cctccggctc gctgattggc ccagcggaga gtgggcgggg ccggtgaaga 660

ctccttaaag gcgcagggcg gcgagcaggt caccagacgc tgacagctac tcagaaccaa 720
 
atctggttcc atccagagac aagcgaagac aagagaagca gagcgagcgg cgcgttcccg 780
atcctcggcc aggaccagcc ttccccagag catccctgcc gcggagcgca accttcccag 840
gagcatccct gccgcggagc gcaactttcc ccggagcatc cacgccgcgg agcgcagcct 900
tccagaagca 910 (SEQ ID NO: 5)
**Genome sequence of UL39 is** (nts. 86217..90988) of NC_001806.1 (SEQ ID NO: 6) 
86217 acaa caggtgggtg ctttggaaac ttgccggtcg ccgtgctcct gtgagcttgc gtccctcccc
86281 ggtttccttt gcgctcccgc cttccggacc tgctctcgcc tatcttcttt ggctctcggt
86341 gcgattcgtc aggcagcggc cttgtcgaat ctcgacccca ccactcgccg gacccgccga
86401 cgtcccctct cgagcccgcc gaaacccgcc gcgtctgttg aaatggccag ccgcccagcc
86461 gcatcctctc ccgtcgaagc gcgggccccg gttgggggac aggaggccgg cggccccagc
86521 gcagccaccc agggggaggc cgccggggcc cctctcgccc acggccacca cgtgtactgc
86581 cagcgagtca atggcgtgat ggtgctttcc gacaagacgc ccgggtccgc gtcctaccgc
86641 atcagcgata gcaactttgt ccaatgtggt tccaactgca ccatgatcat cgacggagac
86701 gtggtgcgcg ggcgccccca ggacccgggg gccgcggcat cccccgctcc cttcgttgcg
86761 gtgacaaaca tcggagccgg cagcgacggc gggaccgccg tcgtggcatt cgggggaacc
86821 ccacgtcgct cggcggggac gtctaccggt acccagacgg ccgacgtccc caccgaggcc
86881 cttgggggcc cccctcctcc tccccgcttc accctgggtg gcggctgttg ttcctgtcgc
86941 gacacacggc gccgctctgc ggtattcggg ggggaggggg atccagtcgg ccccgcggag
87001 ttcgtctcgg acgaccggtc gtccgattcc gactcggatg actcggagga cacggactcg
87061 gagacgctgt cacacgcctc ctcggacgtg tccggcgggg ccacgtacga cgacgccctt
87121 gactccgatt cgtcatcgga tgactccctg cagatagatg gccccgtgtg tcgcccgtgg
87181 agcaatgaca ccgcgcccct ggatgtttgc cccgggaccc ccggcccggg cgccgacgcc
87241 ggtggtccct cagcggtaga cccacacgcg ccgacgccag aggccggcgc tggtcttgcg
87301 gccgatcccg ccgtggcccg ggacgacgcg gaggggcttt cggacccccg gccacgtctg
87361 ggaacgggca cggcctaccc cgtccccctg gaactcacgc ccgagaacgc ggaggccgtg
87421 gcgcgctttc tgggagatgc cgtgaaccgc gaacccgcgc tcatgctgga gtacttttgc
87481 cggtgcgccc gcgaggaaac caagcgtgtc ccccccagga cattcggcag cccccctcgc
87541 ctcacggagg acgactttgg gcttctcaac tacgcgctcg tggagatgca gcgcctgtgt
87601 ctggacgttc ctccggtccc gccgaacgca tacatgccct attatctcag ggagtatgtg
87661 acgcggctgg tcaacgggtt caagccgctg gtgagccggt ccgctcgcct ttaccgcatc
87721 ctgggggttc tggtgcacct gcggatccgg acccgggagg cctcctttga ggagtggctg
87781 cgatccaagg aagtggccct ggattttggc ctgacggaaa ggcttcgcga gcacgaagcc
87841 cagctggtga tcctggccca ggctctggac cattacgact gtctgatcca cagcacaccg
87901 cacacgctgg tcgagcgggg gctgcaatcg gccctgaagt atgaggagtt ttacctaaag
 
87961 cgttttggcg ggcactacat ggagtccgtc ttccagatgt acacccgcat cgccggcttt
88021 ttggcctgcc gggccacgcg cggcatgcgc cacatcgccc tggggcgaga ggggtcgtgg
88081 tgggaaatgt tcaagttctt tttccaccgc ctctacgacc accagatcgt accgtcgacc
88141 cccgccatgc tgaacctggg gacccgcaac tactacacct ccagctgcta cctggtaaac
88201 ccccaggcca ccacaaacaa ggcgaccctg cgggccatca ccagcaacgt cagtgccatc
88261 ctcgcccgca acgggggcat cgggctatgc gtgcaggcgt ttaacgactc cggccccggg
88321 accgccagcg tcatgcccgc cctcaaggtc cttgactcgc tggtggcggc gcacaacaaa
88381 gagagcgcgc gtccgaccgg cgcgtgcgtg tacctggagc cgtggcacac cgacgtgcgg
88441 gccgtgctcc ggatgaaggg ggtcctcgcc ggcgaagagg cccagcgctg cgacaatatc
88501 ttcagcgccc tctggatgcc agacctgttt ttcaagcgcc tgattcgcca cctggacggc
88561 gagaagaacg tcacatggac cctgttcgac cgggacacca gcatgtcgct cgccgacttt
88621 cacggggagg agttcgagaa gctctaccag cacctcgagg tcatggggtt cggcgagcag
88681 atacccatcc aggagctggc ctatggcatt gtgcgcagtg cggccacgac cgggagcccc
88741 ttcgtcatgt tcaaagacgc ggtgaaccgc cactacatct acgacaccca gggggcggcc
88801 atcgccggct ccaacctctg caccgagatc gtccatccgg cctccaagcg atccagtggg
88861 gtctgcaacc tgggaagcgt gaatctggcc cgatgcgtct ccaggcagac gtttgacttt
88921 gggcggctcc gcgacgccgt gcaggcgtgc gtgctgatgg tgaacatcat gatcgacagc
88981 acgctacaac ccacgcccca gtgcacccgc ggcaacgaca acctgcggtc catgggaatc
89041 ggcatgcagg gcctgcacac ggcctgcctg aagctggggc tggatctgga gtctgccgaa
89101 tttcaggacc tgaacaaaca catcgccgag gtgatgctgc tgtcggcgat gaagaccagc
89161 aacgcgctgt gcgttcgcgg ggcccgtccc ttcaaccact ttaagcgcag catgtatcgc
89221 gccggccgct ttcactggga gcgctttccg gacgcccggc cgcggtacga gggcgagtgg
89281 gagatgctac gccagagcat gatgaaacac ggcctgcgca acagccagtt tgtcgcgctg
89341 atgcccaccg ccgcctcggc gcagatctcg gacgtcagcg agggctttgc ccccctgttc
89401 accaacctgt tcagcaaggt gacccgggac ggcgagacgc tgcgccccaa cacgctcctg
89461 ctaaaggaac tggaacgcac gtttagcggg aagcgcctcc tggaggtgat ggacagtctc
89521 gacgccaagc agtggtccgt ggcgcaggcg ctcccgtgcc tggagcccac ccaccccctc
89581 cggcgattca agaccgcgtt tgactacgac cagaagttgc tgatcgacct gtgtgcggac
89641 cgcgccccct acgtcgacca tagccaatcc atgaccctgt atgtcacgga gaaggcggac
89701 gggaccctcc cagcctccac cctggtccgc cttctggtcc acgcatataa gcgcggacta
89761 aaaacaggga tgtactactg caaggttcgc aaggcgacca acagcggggt ctttggcggc
89821 gacgacaaca ttgtctgcat gagctgcgcg ctgtgaccga caaaccccct ccgcgccagg
89881 cccgccgcca ctgtcgtcgc cgtcccacgc tctcccctgc tgccatggat tccgcggccc
89941 cagccctctc ccccgctctg acggccctta cgggccagag cgcgacggcg gacctggcga
90001 tccagattcc aaagtgcccc gaccccgaga ggtacttcta cacctcccag tgtcccgaca
90061 ttaaccacct gcgctccctc agcatcctta accgctggct ggaaaccgag cttgttttcg
90121 tgggggacga ggaggacgtc tccaagcttt ccgagggcga gctcagcttt taccgcttcc
 
90181 tcttcgcttt cctgtcggcc gccgacgacc tggttacgga aaacctgggc ggcctctccg
90241 gcctgtttga gcagaaggac attctccact actacgtgga gcaggaatgc atcgaagtcg
90301 tacactcgcg cgtgtacaac atcatccagc tggtgctttt ccacaacaac gaccaggcgc
90361 gccgcgagta cgtggccggt accatcaacc acccggccat ccgcgccaag gtggactggt
90421 tggaagcgcg ggtgcgggaa tgcgcctccg ttccggaaaa gttcattctc atgatcctca
90481 tcgagggcat ctttttgccc gcctcgtttg ccgccatcgc ctaccttcgc accaacaacc
90541 ttctgcgggt cacctgccag tcaaacgacc tcatcagccg ggacgaggcc gtgcacacga
90601 cggcctcgtg ttacatctac aacaactacc tcggcgggca cgccaagccc ccgcccgacc
90661 gcgtgtacgg gctgttccgc caggcggtcg agatcgagat cggatttatc cgatcccagg
90721 cgccgacgga cagccatatc ctgagcccgg cggcgctggc ggccatcgaa aactacgtgc
90781 gattcagcgc ggatcgcctg ttgggcctta tccacatgaa gccactgttt tccgccccac
90841 cccccgacgc cagctttccg ctgagcctca tgtccaccga caaacacacc aattttttcg
90901 agtgtcgcag cacctcctac gccggggcgg tcgtcaacga tctgtgagtg tcgcggcgcg
90988 cttctacccg tgtttgccca taataaac
>del-seq of fHSV Quik-1 (SEQ ID NO: 7) 
aataaagccactgaaacccgaaacgcgagtgttgtaacgtcctttgggcgggaggaagccacaaaatgca
aatgggatacatggaaggaacacacccccgtgactcaggacatcggtgtgtccttttgggtttcactgaa
actggcccgcgccccacccctgcgcgatgtggataaaaagccagcgcgggtggtttagggtaccacaggt
gggtgctttggaaacttgccggtcgccgtgctcctgtgagcttgcgtccctccccggtttcctttgcgct
cccgccttccggacctgctctcgcctactcttctttggctctcggtgcgattcgtcaggcagcggccttg
tcgaatctcgaccccaccactcgccggacccgccgacgtcccctcagcttgcatgcctgcaggtcgagcc
cgccgaaacccgccgcgtctgttgaaatggccagccgccccgccgcatcctctcccgtcgaagcgcgggc
cccggttgggggacaggaggccggcggccccagcgcagccacccaggggggaggccgccggggcccctctc
gcccgcggccaccacgtgtactgccagcgagtcaatggcgtgatggtgctttccgacaagacgcccgggt
ccgcgtcctaccgcatcagcgatagcaactttgtccaatgtggttccaactgcaccatgatcatagacgg
agacgtggtgcgcgggcgcccccaggacccgggggccgcggcatcccccgctcccttcgttgcggtgaca
aacatcggagccggcagcgacggcgggaccgccgtcgtggcattcgggggaaccccacgtcgctcggcgg
ggacgtctaccggtacccagacgaccgacgtccccaccgaggcccttgggggccccccctcctccccg
cttcaccctgggtggcggctgttgttcctgtcgcgacacacggcgccgctctgcggtattcgggggggag
ggggatcgatccatcgccaccatggtgagcaagggcgaggagctgttcaccggggtggtgcccatcctgg
tcgagctggacggcgacgtaaacggccacaagttcagcgtgtccggcgagggcgagggcgatgccaccta
cggcaagctgaccctgaagttcatctgcaccaccggcaagctgcccgtgccctggcccaccctcgtgacc
accctgacctacggcgtgcagtgcttcagccgctaccccgaccacatgaagcagcacgacttcttcaagt
ccgccatgcccgaaggctacgtccaggagcgcaccatcttcttcaaggacgacggcaactacaagacccg
cgccgaggtgaagttcgagggcgacaccctggtgaaccgcatcgagctgaagggcatcgacttcaaggag
gacggcaacatcctggggcacaagctggagtacaactacaacagccacaacgtctatatcatggccgaca
agcagaagaacggcatcaaggtgaacttcaagatccgccacaacatcgaggacggcagcgtgcagctcgc
 
cgaccactaccagcagaacacccccatcggcgacggccccgtgctgctgcccgacaaccactacctgagc
acccagtccgccctgagcaaagaccccaacgagaagcgcgatcacatggtcctgctggagttcgtgaccg
ccgccgggatcactctcggcatggacgagctgtacaagtaaagcggccgatcc (SEQ ID NO: 7)
>PCR-del-GFP-FRT-Gm-F&R **(SEQ ID NO: 08)**

aataaagccactgaaacccgaaacgcgagtgttgtaacgtcctttgggcgggaggaagcccgtatagcat
acattatacgaagttatagcgcgaagttcctattctctagaaagtataggaacttcaagctttaaacttt
tgccattctcaccggattcagtcgtcactcatggtgatttctcacttgataaccttatttttgacgaggg
gaaattaggccgggaagccgatctcggcttgaacgaattgttaggtggcggtacttgggtcgatatcaaa
gtgcatcacttcttcccgtatgcccaactttgtatagagagccactgcgggatcgtcaccgtaatctgct
tgcacgtagatcacataagcaccaagcgcgttggcctcatgcttgaggagattgatgagcgcggtggcaa
tgccctgcctccggtgctcgccggagactgcgagatcatagatatagatctcactacgcggctgctcaaa
cttgggcagaacgtaagccgcgagagcgccaacaaccgcttcttggtcgaaggcagcaagcgcgatgaat
gtcttactacggagcaagttcccgaggtaatcggagtccggctgatgttgggagtaggtggctacgtctc
cgaactcacgaccgaaaagatcaagagcagcccgcatggatttgacttggtcagggccgagcctacatgt
gcgaatgatgcccatacttgagccacctaactttgttttagggcgactgccctgctgcgtaacatcgttg
ctgctgcgtaacatcgttgctgctccataacatcaaacatcgacccacggcgtaacgcgcttgctgcttg
gatgcccgaggcatagactgtacaaaaaaacagtcataacaagccatgaaaaccgccactgcgccgttac
caccgctgcgttcggtcaaggttctggaccagttgcgtgagcgcatacgctacttgcattacagtttacg
aaccgaacaggcttatgtcaactgggttcgtgccttcatccgtttccacggtgtgcgtcacccggcaacc
ttgggcagcagcgaagtcgaggcatttctgtcctggctggcgaacgagcgcaaggtttcggtctccacgc
atcgtcaggcattggcggccttgctgttcttctacggcaaggtgctgtgaccgccgccgggatcactctc
ggcatggacgagctgtacaagtaaagcggccgatcc (SEQ ID NO:8)

## Claims

1. A tumor-selective oncolytic herpes simplex viral vector, comprising:
(a) a deletion or inactivating mutation in both copies of the gene encoding y34.5, and
(b) an insertion of at least one copy of the HSV y34.5 gene under the transcriptional control of a Nestin promoter;
(c) a deletion or inactivating mutation in the gene that encodes for the HSV viral protein ICP6, wherein the tumor-selective oncolytic herpes simplex viral vector does not express green fluorescent protein.

2. The tumor-selective oncolytic herpes simplex viral vector of claim 1, wherein the vector does not contain UL39 nucleic acid regulatory sequences.

3. The tumor-selective oncolytic herpes viral simplex vector of any of claims 1-2, wherein the vector does not contain a fusion protein of ICP6.

4. The tumor-selective oncolytic herpes simplex viral vector of any of claims 1-3, wherein the at least one copy of the y34.5 gene under the transcriptional control of the Nestin promoter is inserted into UL39 gene that encodes for the large subunit of ribonucleotide reductase ICP6.

5. The tumor-selective oncolytic herpes simplex viral vector of any of claims 1-4, wherein the Nestin promoter comprises SEQ ID NO: 2.

6. The tumor-selective oncolytic herpes simplex viral vector of claim 1, wherein the vector comprises the sequence of SEQ ID NO: 1.

7. The tumor-selective oncolytic herpes simplex viral vector of any of claims 1-6, for use in selectively killing intracranial tumor cells in a subject.

8. The tumor-selective oncolytic herpes simplex viral vector for the use of claim 7, further comprising the administration of cyclophosphamide (CPA).

9. The tumor-selective oncolytic herpes simplex viral vector for the use of any of claims 7-8, wherein the CPA is administered two days before the oncolytic herpes simplex viral vector.

10. The tumor-selective oncolytic herpes simplex viral vector for the use of any of claims 7-9, wherein the tumor cells comprise a glioblastoma cell.

11. The tumor-selective oncolytic herpes simplex viral vector for the use of any of claims 7-10, wherein the tumor cells comprise a cancer stem cell.

12. The tumor-selective oncolytic herpes simplex viral vector for the use of any of claims 7-11, wherein the subject is a mammal.

13. The tumor-selective oncolytic herpes simplex viral vector for the use of any of claims 7-12, wherein the subject is a human.

## Patentansprüche

1. Tumorselektiver onkolytischer Herpes-simplex-Virusvektor, umfassend:
(a) eine Deletion oder inaktivierende Mutation in beiden Kopien des Gens, das für y34.5 codiert, und
(b) eine Insertion von mindestens einer Kopie des HSV-y34.5-Gens unter der transkriptionellen Kontrolle eines Nestin-Promotors;
(c) eine Deletion oder inaktivierende Mutation in dem Gen, das für das HSV-Virusprotein ICP6 codiert, wobei der tumorselektive onkolytische Herpes-simplex-Virusvektor kein grün fluoreszierendes Protein exprimiert.

2. Tumorselektiver onkolytischer Herpes-simplex-Virusvektor nach Anspruch 1, wobei der Vektor keine UL39-Nukleinsäure-Regulationssequenzen enthält.

3. Tumorselektiver onkolytischer Herpes-simplex-Virusvektor nach einem der Ansprüche 1 bis 2, wobei der Vektor kein Fusionsprotein von ICP6 enthält.

4. Tumorselektiver onkolytischer Herpes-simplex-Virusvektor nach einem der Ansprüche 1 bis 3, wobei die mindestens eine Kopie des y34.5-Gens unter der transkriptionellen Kontrolle des Nestin-Promotors in das UL39-Gen eingesetzt ist, das für die große Untereinheit der Ribonukleotidreduktase ICP6 codiert.

5. Tumorselektiver onkolytischer Herpes-simplex-Virusvektor nach einem der Ansprüche 1 bis 4, wobei der Nestin-Promotor SEQ ID NO: 2 umfasst.

6. Tumorselektiver onkolytischer Herpes-simplex-Virusvektor nach Anspruch 1, wobei der Vektor die Sequenz von SEQ ID NO: 1 umfasst.

7. Tumorselektiver onkolytischer Herpes-simplex-Virusvektor nach einem der Ansprüche 1 bis 6 zur Verwendung beim selektiven Abtöten von intrakraniellen Tumorzellen bei einem Subjekt.

8. Tumorselektiver onkolytischer Herpes-simplex-Virusvektor zur Verwendung nach Anspruch 7, ferner umfassend die Verabreichung von Cyclophosphamid (CPA).

9. Tumorselektiver onkolytischer Herpes-simplex-Virusvektor zur Verwendung nach einem der Ansprüche 7 bis 8, wobei das CPA zwei Tage vor dem onkolytischen Herpes-simplex-Virusvektor verabreicht wird.

10. Tumorselektiver onkolytischer Herpes-simplex-Virusvektor zur Verwendung nach einem der Ansprüche 7 bis 9, wobei die Tumorzellen eine Glioblastomzelle umfassen.

11. Tumorselektiver onkolytischer Herpes-simplex-Virusvektor zur Verwendung nach einem der Ansprüche 7 bis 10, wobei die Tumorzellen eine Krebsstammzelle umfassen.

12. Tumorselektiver onkolytischer Herpes-simplex-Virusvektor zur Verwendung nach einem der Ansprüche 7 bis 11, wobei das Subjekt ein Säugetier ist.

13. Tumorselektiver onkolytischer Herpes-simplex-Virusvektor zur Verwendung nach einem der Ansprüche 7 bis 12, wobei das Subjekt ein Mensch ist.

## Revendications

1. Vecteur viral herpès simplex oncolytique à sélectivité tumorale, comprenant :
(a) une délétion ou une mutation inactivante dans les deux copies du gène codant pour y34.5, et
(b) une insertion d'au moins une copie du gène y34.5 du HSV sous le contrôle transcriptionnel d'un promoteur de la nestine ;
(c) une délétion ou une mutation inactivante dans le gène qui code pour la protéine virale ICP6 du HSV, dans lequel le vecteur viral herpès simplex oncolytique à sélectivité tumorale n'exprime pas la protéine fluorescente verte.

2. Vecteur viral herpès simplex oncolytique à sélectivité tumorale selon la revendication 1, dans lequel le vecteur ne contient pas de séquences régulatrices d'acide nucléique UL39.

3. Vecteur viral herpès simplex oncolytique à sélectivité tumorale selon l'une quelconque des revendications 1 à 2, dans lequel le vecteur ne contient pas de protéine de fusion ICP6.

4. Vecteur viral herpès simplex oncolytique à sélectivité tumorale selon l'une quelconque des revendications 1 à 3, dans lequel l'au moins une copie du gène y34.5 sous le contrôle transcriptionnel du promoteur de la nestine est insérée dans le gène UL39 qui code pour la grande sous-unité de la ribonucléotide réductase ICP6.

5. Vecteur viral herpès simplex oncolytique à sélectivité tumorale selon l'une quelconque des revendications 1 à 4, dans lequel le promoteur de la nestine comprend SEQ ID NO: 2.

6. Vecteur viral herpès simplex oncolytique à sélectivité tumorale selon la revendication 1, dans lequel le vecteur comprend la séquence de SEQ ID NO: 1.

7. Vecteur viral herpès simplex oncolytique à sélectivité tumorale selon l'une quelconque des revendications 1 à 6, destiné à être utilisé pour éliminer de manière sélective des cellules tumorales intracrâniennes chez un sujet.

8. Vecteur viral herpès simplex oncolytique à sélectivité tumorale destiné à l'utilisation selon la revendication 7, comprenant en outre l'administration de cyclophosphamide (CPA).

9. Vecteur viral herpès simplex oncolytique à sélectivité tumorale destiné à l'utilisation selon l'une quelconque des revendications 7 à 8, dans lequel le CPA est administré deux jours avant le vecteur viral herpès simplex oncolytique.

10. Vecteur viral herpès simplex oncolytique à sélectivité tumorale destiné à l'utilisation selon l'une quelconque des revendications 7 à 9, dans lequel les cellules tumorales comprennent une cellule de glioblastome.

11. Vecteur viral herpès simplex oncolytique à sélectivité tumorale destiné à l'utilisation selon l'une quelconque des revendications 7 à 10, dans lequel les cellules tumorales comprennent une cellule souche cancéreuse.

12. Vecteur viral herpès simplex oncolytique à sélectivité tumorale destiné à l'utilisation selon l'une quelconque des revendications 7 à 11, dans lequel le sujet est un mammifère.

13. Vecteur viral herpès simplex oncolytique à sélectivité tumorale destiné à l'utilisation selon l'une quelconque des revendications 7 à 12, dans lequel le sujet est un humain.
